(19) **Europäisches Patentamt European Patent Office Office européen des brevets**

(11) **EP 4 347 876 B1**

(12) **EUROPEAN PATENT SPECIFICATION**

(45) Date of publication and mention of the grant of the patent:
**23.04.2025 Bulletin 2025/17**

(21) Application number: **23724152.6**

(22) Date of filing: **22.04.2023**

(51) International Patent Classification (IPC):
**C12Q 1/6827** *(2018.01)*

(52) Cooperative Patent Classification (CPC):
(C-Sets available)
**C12Q 1/6827** (Cont.)

(86) International application number:
**PCT/US2023/066097**

(87) International publication number:
**WO 2023/205803 (26.10.2023 Gazette 2023/43)**

(54) **METHODS, COMPOSITIONS, AND KITS FOR DETERMINING CHROMOSOME STABILITY, GENOTOXICITY, AND INSERT NUMBER**

VERFAHREN, ZUSAMMENSETZUNGEN UND KITS ZUR BESTIMMUNG DER CHROMOSOMENSTABILITÄT, GENOTOXIZITÄT UND EINSATZNUMMER

MÉTHODES, COMPOSITIONS ET KITS POUR DÉTERMINER LA STABILITÉ CHROMOSOMIQUE, LA GÉNOTOXICITÉ ET LE NUMÉRO D'INSERTION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **22.04.2022 US 202263363476 P**
**09.06.2022 US 202263366141 P**

(43) Date of publication of application:
**10.04.2024 Bulletin 2024/15**

(73) Proprietor: **Kromatid, Inc.**
**Longmont, CO 80501 (US)**

(72) Inventors:
- **CROSS, Erin Marie**
  **Longmont, Colorado 80501 (US)**
- **SEBESTA, Henry Cecil**
  **Longmont, Colorado 80501 (US)**
- **TOMPKINS, Christopher John**
  **Longmont, Colorado 80501 (US)**

(74) Representative: **Paulraj, Leonita Theresa**
**Venner Shipley LLP**
**200 Aldersgate**
**London EC1A 4HD (GB)**

(56) References cited:
WO-A1-2014/008425    WO-A1-2023/102501
WO-A2-2021/119002    US-A- 5 871 932

- **SO GUN HONG ET AL: "Assessing the Risks of Genotoxicity in the Therapeutic Development of Induced Pluripotent Stem Cells", MOLECULAR THERAPY, vol. 21, no. 2, 4 December 2012 (2012-12-04), US, pages 272 - 281, XP055327237, ISSN: 1525-0016, DOI: 10.1038/mt.2012.255**
- **LUXTON JARED J. ET AL: "Telomere Length Dynamics and Chromosomal Instability for Predicting Individual Radiosensitivity and Risk via Machine Learning", JOURNAL OF PERSONALIZED MEDICINE, vol. 11, no. 3, 8 March 2021 (2021-03-08), pages 188, XP093067351, ISSN: 2075-4426, DOI: 10.3390/jpm11030188**
- **RAY F. ANDREW ET AL: "Directional genomic hybridization for chromosomal inversion discovery and detection", CHROMOSOME RESEARCH, vol. 21, no. 2, 10 April 2013 (2013-04-10), NL, pages 165 - 174, XP093066897, ISSN: 0967-3849, Retrieved from the Internet <URL:https://link.springer.com/content/pdf/10.1007/s10577-013-9345-0.pdf?pdf=button> DOI: 10.1007/s10577-013-9345-0**

**(Cont. next page)**

EP 4 347 876 B1

(52) Cooperative Patent Classification (CPC): (Cont.)

C-Sets
**C12Q 1/6827, C12Q 2537/143, C12Q 2543/00,
C12Q 2545/101, C12Q 2563/107, C12Q 2565/102,
C12Q 2565/601**

**Description**

**CROSS-REFERENCE TO RELATED APPLICATIONS**

**FIELD OF THE INVENTION**

The present disclosure relates generally to analysis of cells using microscopy and more specifically to detection of chromosome structural variants, repair events and the instability of genomes.

**BACKGROUND OF THE DISCLOSURE**

Chromosome variants, however, they are formed, can be harmless and show no genotoxicity, can negatively affect cellular function, can cause genomic instability, can kill the cell, or can form genotoxic products. Non-harmless chromosome variants negatively affect cells and contribute to disease through the formation of oncogenes, gene inactivation or knock out, regulatory element disruption, loss of heterozygosity, duplication of genes or promoters, and other mechanisms that disrupt necessary metabolic pathways, activate inert metabolic pathways or cause fatal damage to the cells (collectively genotoxic effects or genotoxicity). If chromosome variation is congenital, even if it does not result in any obvious pathology, mistakes in meiotic crossover caused by misalignment can produce genetic abnormalities in the offspring of the affected individual. In a typical mendelian fashion, recessive chromosome variants inherited from both parents can cause disease in children not active in either parent. X-linked structural variations selectively impact male offspring because the Y chromosome of the XY pair does not have a compensating normal gene.

The detection and identification of both non-recurrent chromosome variants in individual cells resulting from DNA double-stranded break (DSB) mis-repair, as well as the chromosome variants present in an individual genome and their representation in individual cells (heterogeneity/ mosaicism) is clinically relevant and important across a wide spectrum of human disease and conditions. Because of the potential for both cell death and risk to patient's DNA, mis-repairs and the resulting chromosome variants, there is a need for methods for measuring chromosomal structural variants. An ideal method would be to measure structural variation in single cells at sufficient resolution to detect kilobase scale variants and allow for the direct determination of target sequence orientation and the detection of inverted sequences. Widely adopted techniques, such as G-Banding can provide partial information by staining the secondary structure of chromosomes, however at a relatively crude resolution of 10s of megabases and without any direct measure of orientation. Next-generation sequencing (NGS) and Sanger sequencing methods can also provide partial information, through short and long read whole genome sequencing and analysis. However, these methods' reliance (typically) on pooled DNA means that they cannot provide accurate distributions of variation across heterogenous populations, and since the breakpoints that define structural variation can occur in repetitive sequences, de novo analysis of NGS and Sanger sequencing often contains significant uncertainty in determining the true identity of structural variations.

To detect chromosomal variants, improved methods are needed. Two types of approaches that can be employed, for example, are array-based detection/ comparative genome hybridization (array cGH), and sequence based computational analysis. Each can measure some products of mis-repair through chromosomal variant detection algorithms and can be more effective when used in concert to cross-validate findings. However, since these techniques measure the sequence of DNA bases, from isolated pools of DNA from a population of edited cells, and not the relationship or structure of the genes, promotors or large segments of DNA in single cells, they can be used only to hypothesize genomic structure through bioinformatic reconstruction results that are often compromised by numerous false positive and false negative variant calls. Additionally, sequencing approaches rely on PCR amplification of a specific genomic region of interest. This type of amplification subjects results to PCR biases and makes *de novo* analysis and analysis of regions high in repeats challenging. Thus, the cellular context in which genetic aberrations occur is entirely lost. The dGH process, however, gathers data from actively dividing cells, resulting in a cellular "snapshot" of the genetic structural variation that has taken place within that cell up until the point of arrest and fixation.

Additional methods for analyzing chromosomal variants include fluorescence based microscopic methods such as, for example, fluorescence in situ hybridization (FISH) and directional genomic hybridization (dGH). These methods can detect and characterize chromosome variants, and dGH in particular provides high resolution detection of structural variation with a direct measure of sequence orientation. There remains a need for methods that can utilize this information to characterize the genotoxic risk of a cell population. Such improved methods would have utility for cancer research and diagnostics, cell therapy safety and quality control, cellular engineering, genome editing, radiation biodosimetry, and genetic disease research.

LUXTON JARED J. ET AL: "Telomere Length Dynamics and Chromosomal Instability for Predicting Individual Radiosensitivity and Risk via Machine Learning", JOURNAL OF PERSONALIZED MEDICINE, vol. 11, no. 3, 8 March 2021 (2021-03-08), page 188, presents the first implementation of individual telomere length data in a machine learning model, XGBoost, trained on pre-radiotherapy (baseline) and in vitro exposed (4 Gy $\gamma$-rays) telomere length measurements, to predict post radiotherapy telomeric outcomes, which together with chromosomal instability provide insight into individual radiosensitivity and risk for radiation-induced late effects.

RAY F. ANDREW ET AL: "Directional genomic hybridization for chromosomal inversion discovery and detection", CHROMOSOME RESEARCH, vol. 21, no. 2 10 April 2013 (2013-04-10), pages 165-174, presents the directional genomic hybridization methodology of chromatid painting that can be employed with high resolution on a cell-by-cell basis, and demonstrate its basic capabilities for genome-wide discovery and targeted detection of inversions.

## SUMMARY

Provided herein are methods and compositions related to analyzing genotoxicity and genotoxicity potential of a sample or population of cells.

In one aspect, provided herein is a method of determining a genotoxicity risk for a test population of cancer cells, the method comprising the steps of:

a) performing a directional genomic hybridization (dGH) reaction by contacting one or more pairs of single-stranded sister chromatids on at least four cells from the test population, with a plurality of sets of dGH probes, wherein the dGH probes of each set of dGH probes binds to a different target DNA sequence on a same single-stranded sister chromatid, wherein each set of dGH probes binds to a corresponding set of target DNA sequences on a different single-stranded sister chromatid, and wherein each single-stranded sister chromatid can be assigned to a chromosome number based on a color of the set of dGH probes that binds thereto and other visual features of the single-stranded sister chromatid;

b) assessing a presence of two or more types of chromosome variants and/or repair events in each of the at least four cells of the test population of cells individually based on results of the dGH reaction;

c) measuring the number and variability of each of the two or more chromosomal variant and/or repair event types detected in each of the at least four cells of the test population of cells; and

d) comparing the number and/or variability of each of the two or more chromosomal variant and/or repair event types detected in the test population of cells to a number and/or variability for a corresponding type of chromosomal variant and/or repair event of a control cell or a population of control cells, wherein a weight is assigned to the number and/or variability for each type of chromosomal variant and/or repair event to provide a series of weighted factors, wherein the weight assigned to obtain each weighted factor of the series of weighted factors depends on a characteristic of and/or a type of cells of the test population of cells to determine a weighted chromosomal stability score, wherein the weighted chromosomal stability score is used to determine the genotoxicity risk of the test population of cells, and wherein an increased weighted chromosomal stability score in the test population of cells compared to the control cell or the population of control cells is indicative of an increased genotoxicity risk for the test population of cells. The preferred features of the method of determining a genotoxicity risk for a test population of cells are defined in claims 2 to 15.

Further details regarding aspects and embodiments of the present disclosure are provided throughout this patent application. Sections and section headers are for ease of reading and are not intended to limit combinations of disclosure, such as methods, compositions, and kits or functional elements therein across sections.

## BRIEF DESCRIPTION OF THE DRAWINGS

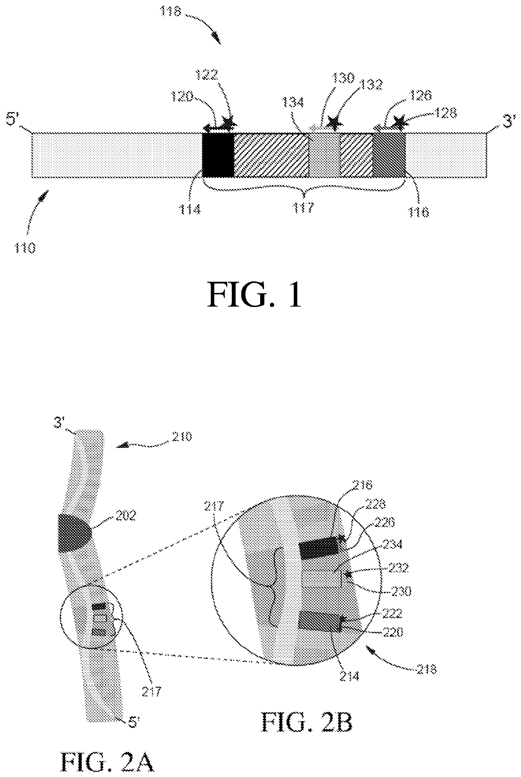

FIG. 1 is a diagram illustrating an example of hybridization between a dGH probe (118) that is made up of a pool of single-stranded oligonucleotides, including a first single-stranded oligonucleotide (120) having a first fluorescent label (122), a second single-stranded oligonucleotide (126) having the first fluorescent label (128), a third single-stranded oligonucleotide (130) having the first fluorescent label (132), from a dGH probe (118) made up of these three labeled single-stranded oligonucleotides, namely, 120, 126 and 130, which bind to their respective complementary DNA sequences (114, 116 and 134), which are located within a target DNA sequence (117) of a portion of a single-stranded sister chromatid (110).

FIG. 2A is a diagram illustrating an example of a single-stranded sister chromatid (210) with a centromere (202) and a target DNA sequence (217).

FIG. 2B is a diagram illustrating an example of a probe (218) made up of a pool of three fluorescently labeled single-stranded oligonucleotides (220, 226, 230) hybridizing with their respective complementary DNA sequences (214, 216, 234), which are located within the target DNA sequence (217) of the single-stranded sister chromatid (210) of FIG. 2A.

FIG. 3A is a cartoon of an example of a metaphase spread (300) wherein the single-stranded sister chromatids (310a, 310b) of a cell, of a sample of cells, have been hybridized with five sets of probes (i.e., 301, 302, 303, 304, and 305), wherein each probe is made up of a pool of fluorescently labeled single-stranded oligonucleotides, wherein each of the five probes is labeled with a different colored fluorescent label.

FIG. 3B is a cartoon illustrating an example of a karyogram (320), of the metaphase spread (300) of FIG. 3A, wherein the chromosomes have been rearranged into orderly rows according to the color of the fluorescent labelled probes used, the size of the chromosomes, and centromeric position. The color of the fluorescent labelled probes is indicated with the letter Y, B, R, G or P, where Y = yellow, B = blue, R= red, G= green, and P= purple.

FIG. 4 illustrates a dGH SCREEN 5 color whole genome assay configuration, shown in grayscale. The left side of FIG. 4 shows a metaphase spread. The center of FIG. 4 shows the chromosomes after sorting according to size, centromere position and fluorescent label color scheme, as in FIG. 3B. The right side of FIG. 4 shows a karyogram of the sorted chromosomes.

FIG. 5A is an image showing an inversion in the Chromosome 16q arm in cells of passage 18 of the GM24385 cell line.

FIG. 5B is an image showing an inversion in the Chromosome 16p arm in cells of passage 18 of the GM24385 cell line.

FIG. 5C is an image showing translocations in Chromosome 16 in cells of passage 18 of the GM24385 cell line.

FIG. 5D is an image showing a whole arm deletion in Chromosome 16 in cells of passage 18 of the GM24385 cell line.

FID. 5E is an image showing a multi-radial chromosome 16 in cells of passage 18 of the GM24385 cell line.

FIG. 5F is an image showing decondensed (i.e., elongated) centromeres and isochromosomes in Chromosome 16 in cells of passage 18 of the CM24385 cell line.

FIG. 6A - FIG. 6B show size difference between chromosome homologs. FIG. 6A is a karyogram of a metaphase cell (passage 18, GM 24385) exhibiting gross karyotypic size differences between homologs of Chromosomes 1 and 3, indicating intrachromosomal CNV and/or condensation defect. FIG. 6B is a graph showing the rate of this observation, in percentage of cells per chromosome across a total of 54 (p18) cells.

FIG. 7 is an image of a modified cell showing a single inverted insert. The image is shown in black and white, with colors labeled, where target site is green, and insert is yellow.

FIG. 8 is a histogram showing the number of integration events in each cell.

FIG. 9 is a histogram showing the estimated copy number per integration event. Solid bars represent on-target events, and dashed bars represent off target events.

FIG. 10A and FIG. 10B are standard curve analysis graphs showing Sum Intensity values ($I$) for 30, 10 and 5 kb ladder spots plotted as a linear graph with $R^2$ values displayed for two fluorophores, ATTO643N and ATTO550.

FIG. 11 is a standard curve analysis graph for Cell 19. Sum Intensity values (I) are plotted for 30, 10 and 5 kb ladder spots as a linear graph with the $R^2$ value listed.

FIGs. 12A and 12B are cartoons depictions of the p53 gene on Chromosome 17 of HEK293T cells. FIG. 12A, is the normal karyotype for the cell line, demonstrating the Ch17 tetraploidy of the cell line, where one of the tetraploid chromosomes is missing a copy of the p53 gene. FIG. 12B shows the experimental CRISPR target cut sites located concurrently within the p53 gene.

FIG. 13 is a graph demonstrating the off-target effects of CRISPR edits as a percent of HEK293T cells scored with an inversion in the p53 probe region.

FIG. 14 is a graph demonstrating the off-target effects of CRISPR edits as a percent of HEK293T cells scored with a translocation in the p53 probe region.

**DEFINITIONS**

As used herein, "band" refers to a chromosomal region hybridized with probes labeled with a similar light emission signature (e.g., probes of the same color). A band includes one or more consecutive target DNA sequences on a single-stranded chromatid, that are bound by one or more probes labeled with a same or similar light emission signature.

As used herein, "aneuploidy" refers to having an unbalanced chromosome number, such as missing or extra chromosomes.

As used herein, "bleeding" refers to the light emission signature of one band partially overlapping or otherwise partially appearing on at least one other band.

As used herein, "centromere abnormality" refers to aberrant rearrangements (e.g., SCE or SCR) of long tandem DNA sequences at the centromere that can lead to chromosome fusions and genetic abnormalities.

As used herein, "change in chromosome number" refers to a chromosome variant having a change in the number of chromosomes, or an insertion or deletion of at least 100 kilobases in length.

As used herein, "chromoplexy" refers to a class of complex DNA rearrangement, wherein multiple strands of DNA are broken and ligated to each other in a new configuration, effectively scrambling the genetic material from one or more chromosomes. Chromoplexy often involves segments of DNA from multiple chromosomes (e.g., five or more).

As used herein, "chromatid-type break" refers to a gap in the chromatid due to a double strand break (DSB) in the DNA, where the break and re-joining affect only one of the sister-chromatids at any one locus.

As used herein, "chromosome condensation" refers to the reorganization or compaction of the chromatin strands into compact short chromosome structures that occurs in mitosis and meiosis.

As used herein, "chromosome condensation defect" refers to aberrant chromosome condensation. Defects in chromosome condensation may lead to improper segregation of chromosomes.

As used herein, "chromosomal fragmentation" refers to progressive degradation of condensed chromosomes during metaphase that may lead to cell death.

As used herein, "chromosome segment" refers to a region of DNA defined by start and end coordinates in a genome (e.g., bp 12900-14900 in Human Chromosome 2) or known sequence content (e.g., the sequence of a gene or mobile element). A chromosomal segment can be as small as a two base pairs, or as large as an entire chromosome.

As used herein, "chromosome variants" encompasses both structural variants and numerical variants. The term chromosomal variant is used as an operational demarcation between, for example, single nucleotide variants and insertion-deletion mutations (INDELs), which are not considered chromosomal variants herein, and segmental copy number variants, which are considered chromosomal variants herein if they are over 1,000 bp in length. Exemplary changes that are considered chromosomal variants herein include chromosomal changes that are deletions, novel sequence insertions, mobile element insertions, tandem and interspersed segmental duplications, inversions, truncations and translocations in a test genome as it compares to a reference genome.

As used herein, "chromothrypsis" or "chromothripsis" refers to a process by which dozens to up to thousands of chromosomal rearrangements occur in localized regions of one or a few chromosomes.

As used herein, "color channel" refers to a region of the light spectrum, including visible light, infrared light and ultraviolet light. A color channel may be specified to be as broad a set of wavelengths or as narrow a set of wavelengths as useful to an individual practicing the methods disclosed herein.

As used herein, "complex chromosomal rearrangement" or "CCR" refers to genomic structural rearrangements that involve at least two chromosomes and three breakpoints with varied outcomes (simple or 3-break insertions are excluded).

As used herein, "complex translocation" refers to a combination of translocation events that result in complex chromosomal rearrangement.

As used herein, "complex chromosomal rearrangement" or "CCR" refers to genomic structural rearrangements that involve at least two chromosomes and three breakpoints with varied outcomes (simple or 3-break insertions are excluded).

As used herein, "deletion" refers to loss of genetic material. The size of deletions varies widely and may be as small as a single base pair of the DNA, or as large as loss of an entire chromosome.

As used herein, "directional genomic hybridization" or "dGH" refers to a method of sample preparation, such that the sister chromatids of a metaphase spread become single-stranded, combined with a method of hybridization with a probe made up of a pool of uni-directional single-stranded oligonucleotides before chromosome visualization using fluorescent microscopy. It is noteworthy that libraries of probes can be designed to bind each strand, typically in different colors to permit a bidirectional analysis. Further details regarding dGH and a dGH reaction are provided herein.

As used herein, a "dGH probe" refers to a pool, number, group or plurality of single-stranded oligonucleotides used in a dGH reaction. For example, a probe can include 10, 20, 30, 50, 100, 200, 300, 500, 1,000, 10,000, 100,000 or more individual oligos, depending upon the size and sequence of the target DNA. Each of the oligos, of the dGH probe, typically includes a nucleic acid that binds a complementary DNA sequence of a target DNA sequence, typically a single-stranded chromatid, and a fluorescent label. Various features of such probes, and sets of such probes, are provided herein.

As used herein, "enrich" refers to increasing the proportion of a component in a mixture. In certain aspects, enrichment of metaphase cells refers to increasing the proportion of cells in metaphase present in a population of cells.

As used herein, "episome" or "episomal DNA" refers to a segment of DNA that can exist and replicate autonomously in the cytoplasm of a cell.

As used herein, "extrachromosomal DNA" or "ECDNA" refers to any DNA that is not part of a chromosome, either inside or outside the nucleus of a cell.

As used herein, "feature lookup table" refers to a table of numerical values which represents one or more feature node.

As used herein, "feature nodes" and "nodes" are used interchangeably to refer to numerical values, including sets of numerical values, representing any region of analytical interest on an oligonucleotide or polynucleotide strand. Nodes can be a specific locus, a string of loci, a gene, multiple genes, bands, or whole chromosomes. Nodes can be configurable and variable in size to allow different levels of granularity during analysis. By way of non-limiting example, nodes can represent normal features or abnormal features of a subject DNA strand. Also, by non-limiting example, nodes can provide numerical values for spectral profile data from labeled probe hybridization to control DNA strands, where nodes represent either normal structural features or abnormal structural features of the control DNA strand.

As used herein, "fragments" refers to the breaking of a strand of DNA, such as a chromosome. DNA fragmentation can occur as a part of normal cellular processes, such as apoptosis. Alternatively, DNA fragmentation may occur due to misrepair of chromosomal breaks.

As used herein, "genomic instability" refers to a spectrum of genetic aberrations ranging from subtle nucleotide changes to extreme genomic changes, including numerical and structural chromosome defects, which are potentially toxic and may lead to the development of cancer and disease. A range of genetic mutations may occur, including mutations, insertions, deletions, chromosome rearrangements and aneuploidy.

As used herein, "genotoxicity" refers to heritable and potentially toxic or deleterious effects on a cell's genetic material, and can be self-perpetuating, via induction of genomic instability and further mutations, often culminating in transformation to malignancy. Genotoxic agents include but are not limited to radiation, chemical compounds, and integrating genetic elements such as retroviruses.

As used herein, "grid" refers to a two-dimensional, regularly spaced arrangement of partitions such that lines connecting the partitions along an x-axis or along a y-axis form right angles at their intersection.

As used herein, "insertion" refers to the addition of genetic material to a chromosome. Such an insertion can be small, involving a single extra DNA base pair, or large, involving a piece of a chromosome.

As used herein" iso-chromosome" refers to supernumerary marker chromosomes made up of two copies of the same arm of a chromosome.

As used herein, "inversion" refers to an abnormality in chromosome structure that can result from the misrepair of two double-stranded breaks occurring at different points along a portion of the chromosome, such that this interstitial portion of the chromosome becomes effectively rotated through 180° after a "mis-rejoining" among the broken ends of the chromosome.

As used herein, a "metaphase spread" is a set of metaphase chromosomes from a single cell's nucleus prepared on a sample support matrix such as a glass slide.

As used herein, "micronuclei" or "MN" refers to extra-nuclear bodies that contain damaged chromosome fragments and/or whole chromosomes that were not incorporated into the nucleus after cell division.

As used herein, "mitosis" is a biological process that typically entails perfect duplication and segregation of chromosomes. In various cancers, chromosome mis-segregations, as well as changes in structural chromosomes (deletions or translocations), are known to occur.

As used herein, "multi-radial chromosome" refers to bulky multi-chromosome structures joined by at the centromeres by bridges. Multi-radial chromosomes can be formed due to misrepair of unrepaired one-ended double-strand breaks (DSBs) among non-homologous chromosomes and unequal chromosome segregation.

As used herein "numerical variants," "numeric variants," "chromosome numerical variants," or "chromosome numeric variants" are variants in which the number of chromosomes has increased or decreased. Numeric variants include chromosome variants in which an extra physical copy of a chromatid or chromosome is present that is not found on a corresponding wild type mitotic or meiotic cell depending on the context. Numerical variants include, for example, total chromosome copy number variants (i.e., genome ploidy), and chromosome numerical variants (i.e., gain or loss of a chromosome).

As used herein, "partition" refers to a structure or action which divides cells or chromosomes such that some level of containment and/or separation is provided for the cells and/or chromosomes. While a partition can be provided by a physical barrier providing for some level of containment, partition can also be provided by a selected separation distance. In certain aspects, cells can be partitioned into separated groups of cells. In certain aspects, chromosomes can be partitioned into separated groups of chromosomes. In certain aspects, a partition may be a well of a multi-well plate. In certain aspects, cells and/or chromosomes can be partitioned at selected, separated locations on a microscope slide.

As used herein, "pinpoint FISH" or "PPF" refers to is a synthetic oligonucleotide-based FISH assay designed to provide the highest resolution, lowest background, and lowest limit of detection available. PPF assays are optimized for detecting targets as small as 5-10kb in metaphase spreads, including specific breakpoints, and transgene inserts.

As used herein, "probe" refers to a number, group, quantity and/or plurality of labeled oligonucleotides designed to be complimentary to a target DNA sequence of interest, such as a portion of a target DNA sequence of interest, such that when combined with a hybridization reaction it will bind to and detect the target.

As used herein, "ring" or "ring chromosome" refers to a circular chromosome structure.

As used herein, "single-stranded chromatid" refers to the product of the process in which a DNA analog (e.g., BrdU/C) is provided to an actively dividing cell for a single replication cycle, which is then incorporated selectively into the newly synthesized daughter strand, a metaphase spread is prepared, the incorporated analog is targeted photolytically to achieve DNA nicks which are used to selectively to enzymatically digest and degrade the newly synthesized strand, resulting in a single-stranded product. If we use the terms Watson and Crick to describe the 5' to 3' strand and 3' to 5' strand of a double-stranded DNA complex, an untreated metaphase chromosome will have one sister chromatid with a parental Watson/ daughter Crick, one sister chromatid with a daughter Watson/parental Crick. In the chromosomes prepared according to the method above, one sister chromatid will consist of the parental Watson strand only, and the other sister chromatid will consist of the parental Crick strand only.

As used herein, "sister chromatid exchange" or "SCE" refers to an error-free swapping (cross-over) of precisely matched and identical DNA strands. Sister chromatid exchanges, while not structural variants, are associated with elevated rates of genomic instability due to an increased probability that alternative template sites such as repetitive elements adjacent to the break site will produce an unequal exchange resulting a structural variant.

As used herein, "sister chromatid recombination" or "SCR" refers to the homologous recombination process involving identical sister chromatids that results in a uni-directional non-crossover repair event, otherwise known as a gene conversion event.

As used herein, "spectral profile" refers to the graphic representation of the variation of light intensity of a material or materials at one or more wavelengths.

As used herein, "stretched" refers to reducing the level of DNA compaction. In certain aspects, stretching of DNA refers to separation of DNA from the nucleus and DNA packaging proteins *via* protease digestion, releasing the DNA molecules into solution, and then stretching the chromosomes along a solid surface through a dewetting process. In other aspects stretching refers to using a pressure-driven microfluidic flow to extract and stretch chromosomal DNA from individually isolated cell nuclei immobilized in microchannels.

As used herein, "structural feature" refers broadly to any aspect of a sequence of bases within an oligonucleotide or polynucleotide, including normal features or abnormal features of a sequence. For example, structural features include but are not limited to genetic elements selected from a protein coding region, a region which affects transcription, a region which affects translation, a region which affects post-translational modification and any combination thereof. By way of further non-limiting example, structural features include genetic elements selected from an exon, an intron, a 5' untranslated region, a 3' untranslated region, a promotor, an enhancer, a silencer, an operator, a terminator, a Poly-A tail, an inverted terminal repeat, an mRNA stability element, and any combination thereof.

As used herein, "structural variant" (SV) or "chromosomal structural variant" refers to a region of DNA that has experienced a genomic alteration resulting in copy, structure and content changes over 50bp in length. SVs are changes to the arrangement or order of segments of a genome as compared to a "normal" genome.

As used herein, "target DNA" refers to a region of DNA defined by start and end coordinates of a reference genome (e.g., bp 12900-14900 in Human Chromosome 2) or known sequence content (e.g., the sequence of a gene or mobile element) that is being detected.

As used herein, "target enrichment" refers to utilization of additional probes, beyond those probes used for banding, to a targeted area of interest, in order to track any changes to that specific region.

As used herein, "translocation" refers to a chromosomal abnormality in which a chromosome breaks and a portion of the broken chromosome reattaches to a different chromosome, thereby creating a fusion product that may lead to disease.

As used herein, "vector incorporated DNA" refers to any vectors which act as vehicles for a DNA insert. These may be cloning vectors, expression vectors or plasmid vectors introduced into the cell, including but not limited to artificial chromosome vectors, phage and phagemid vectors, shuttle vectors, and cosmid vectors.

It is to be understood that the present disclosure and the aspects and embodiments provided herein, are not limited to particular examples disclosed, as such may, of course, vary. It is also to be understood that the terminology used herein is for the purpose of disclosing particular example and embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention. When multiple low and multiple high values for ranges are given that overlap, a skilled artisan will recognize that a selected range will include a low value that is less than the high value. All headings in this specification are for the convenience of the reader and are not limiting.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

It must be noted that as used herein and in the appended claims, the singular forms "a," "an," and "the" include plural referents unless the context clearly dictates otherwise. Thus, for example, reference to "a chimeric antigen receptor" includes a plurality of such chimeric antigen receptors and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

## DETAILED DESCRIPTION

Methods, kits, and compositions are disclosed herein to overcome the long-standing problem of improved methods for determining a cell's and a cell population's distribution of structural variation, the stability of the cells and the cell population chromosomes and genotoxicity risk arising from structural variation, genomic instability, clonality and other specific risk factors, such as oncogene activation by genomic rearrangements. Thus, utilizing methods, compositions, and kits of the present disclosure, safe and effective cutting-edge biotherapeutics such as, for example, a gene edit, gene therapy, or CAR-T can be efficiently developed with a better understanding of genotoxic risks and a better defined safety profile. Methods, kits, and compositions herein can be used in the development of such biotherapeutics, can be used in pre-clinical assessment of such biotherapeutics, can be used in the quality control for example during manufacture of such biotherapies, can be used to assess the suitability of patients for treatment, can be used to diagnose the effects of genotoxicity in patients, and can be used to differentiate genotoxicity risks of treatment from pre-existing structural variation in patients. [0088] More specifically, the present disclosure harnesses the power of dGH to provide information on a more comprehensive set of chromosomal variants and repair events that can be combined with additional stability-related measurements, to provide information on a cell's chromosome stability, that can be combined across a population of cells, to provide valuable and comprehensive information regarding the chromosome and overall stability of a population of cells. This disclosure also harnesses the power of dGH to provide comprehensive information about other mechanisms of genotoxicity, such as damage to specific genotoxic sites (e.g., oncogenes), degree of cellular engineering (e.g., number and location of lenti-viral inserts), chromothrypsis and clonal outgrowth of potentially genotoxic populations.

In illustrative embodiments, this information is used to provide valuable information regarding the genotoxic risk of the population of cells. Accordingly, dGH is typically performed as part of methods herein. dGH provides the ability to multiplex, in a single assessment, measurements of many types of chromosome variants or repair events on a per-cell basis to provide a more accurate picture of chromosome and genomic instability. dGH provides an enhanced measurement of classical chromosome instability (CIN) including for example, small inversion events, as well as repair events such as sister chromatid exchange rates, which is another potential hallmark of instability, that are missed by other fluorescent micrographic techniques, sequencing techniques, PCR techniques and staining methods such as g-banding. Furthermore, dGH provides the ability to measure virtually any type of chromosome variant, provide a measure of chromothrypsis, and differentiate chromatid-type aberrations, as well as many other chromosomal variants and event types as those disclosed herein. These measurements can be coupled with population-based calculations of variation across individual genomes, longitudinally from passage to passage or in relationship to the reference genome in a sample of cells to provide a robust, high resolution measurement of chromosome or genome instability, which can also inversely be reported as chromosome stability.

Thus, in illustrative embodiments provided herein, chromosome/genome stability is analyzed on a more detailed and comprehensive level than prior methods, by utilizing the ability of directional genomic hybridization ("dGH") to provide information on numerous chromosome numeric and structural variants and repair events for individual cells from a population of cells, in other words on a per-cell or cell-by-cell basis. Furthermore, this is accomplished in certain illustrative embodiments by utilizing the ability of dGH to provide information on chromosomal structural variants and repair events that are not possible for, and/or at higher resolution than, other fluorescent microscopic techniques. Therefore, methods and compositions herein can detect the presence or absence of one, and in illustrative embodiments, two, three, four, or more chromosome numerical and structural variants and repair events in multiple individual cells of a population of cells, in further illustrative embodiments as compared to a control cell population or reference genome. Thus, dGH provides in some embodiments, a multiplex assessment of two or more chromosome variant(s) and/or repair event(s). In some embodiments, a dGH analysis can be combined with other techniques for assessing chromosome variants or other information about a cell, its chromosomes, or its genome.

Accordingly, chromosome variants and outcomes of chromosome repair events such as chromosome replication and/or repair, for example sister-chromatid exchanges, are detected in illustrative embodiments on a per-cell basis across a population of cells. Furthermore, in certain illustrative embodiments quantitative analysis of chromosome variants and/or chromosome repair events are used in a weighted analysis to provide further information regarding chromosome instability for a population of cells, and genotoxicity risk of the population. More specifically, in certain illustrative embodiments multiplex chromosome analysis is performed such that increased weight is given to certain chromosome numerical and structural variants and/or certain repair events, and such weighting is specific for different types of cells (i.e., cell-type specific), to provide even further refined predictions of genotoxicity risk of a population of cells.

Disclosed herein is a method of determining a chromosome stability value for a cell or typically a population of cells that includes the following steps:

performing a dGH reaction on the cell or population of cells;
assessing the presence of one or more chromosomal variants and/or repair events in each of the at least two cells of the population of cells using the results of the dGH reaction; and determining the chromosome stability value or score.

Such chromosome stability values can be used to determine the genotoxicity risk of a population of cells.

Disclosed herein is a method of determining a genotoxicity risk for a population of cells that includes the following steps:

a) performing a dGH reaction on the population of cells;
b) assessing the presence of one or more chromosomal variants and/or repair events in each of the at least two cells of the population of cells by detecting one or more fluorescent labels on dGH probes used in the dGH reaction;
c) determining a chromosome stability value or score for each of the at least two cells; and
d) determining the genotoxicity risk for the population of cells using a combined chromosome stability values from step c.

## dGH REACTION

Illustrative methods, kits, and compositions herein involve performing a dGH reaction and compositions used therein. In illustrative embodiments, results of the dGH reaction are used to assess the presence of two or more types of chromosome variants and/or repair events in individual cells of population of cells. Briefly, for a dGH reaction, samples of cells are prepared whereby (1) a nucleotide analog (e.g., BrdU/C) is provided to an actively dividing cell for one-replication cycle and is incorporated selectively into the newly synthesized daughter strand; (2) a metaphase spread is prepared; (3) the incorporated DNA analog is targeted photolytically to achieve DNA nicks which are used selectively to enzymatically digest and degrade the newly synthesized strand to produce single-stranded sister chromatids in the metaphase chromosomes; (4) at least one of the single-stranded metaphase chromosomes is hybridized *in situ* with single-stranded and typically uni-directional probes (e.g., a pool, plurality, group, number and/or quantity of single-stranded oligonucleotides comprised of fluorescently labeled DNA, RNA or chimeric oligonucleotides comprised of mixtures of DNA, RNA and / or synthetic, non-natural nucleotide bases) that in illustrative embodiments are designed against unique sequences of a reference genome such that typically only one single-stranded sister chromatid of the metaphase chromosome is labeled at a target site or sites (i.e., target DNA sequence(s)). In illustrative embodiments, a probe having single stranded oligonucleotides that are designed to bind repeat free sequences of a reference genome such that typically only one single-stranded sister chromatid of the metaphase chromosome is labeled at a target site or sites (i.e., target DNA sequence(s)) is bound. In illustrative embodiments, the single stranded oligonucleotides are designed to bind unique repetitive sequence (specific target repeat) such that typically only one single-stranded sister chromatid of the metaphase chromosome is labeled at a target site or sites (i.e., target DNA sequence(s)). In certain embodiments, a target site(s) can encompass an entire chromosome or chromosomes (See e.g., Ray et al., Chrom.(2013) 21: 165-174).

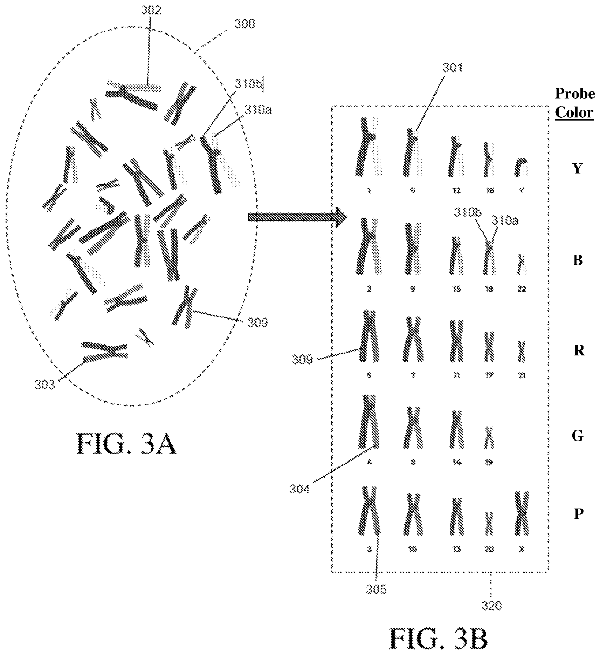

With reference to FIGS. 1 and 2A-2B, a dGH reaction typically includes contacting one or more pairs of single-stranded sister chromatids 110, 210 with a probe 118, 218 made up of a pool of single-stranded oligonucleotides (120, 126 and 130), each of which comprises a nucleic acid, typically a single-stranded synthetic oligonucleotide and a fluorescent label that is observable by fluorescent microscopic techniques. Such contacting is typically performed using metaphase spreads prepared from individual cells (see FIGS. 3A-3B, below), from a sample that typically includes a population of cells. Furthermore, each single-stranded oligonucleotide of the probe 118, 218 typically binds a different complementary DNA sequence within the same target DNA sequence (117, 217).

In an exemplary embodiment shown in FIG. 1, a probe 118 made up of a pool of single-stranded oligonucleotides includes a first, a second and a third single-stranded oligonucleotide, 120, 126 and 130, respectively. Each of the first, second and third single-stranded oligonucleotides, 120, 126, 130, is labeled with a fluorescent label, depicted as stars 122, 128 and 132, respectively. The first, second and third single-stranded oligonucleotides, 120, 126, 130, each hybridize with a complementary DNA sequence within the target DNA sequence 117. In this embodiment, the first single-stranded oligonucleotide 120, hybridizes with a first complementary DNA sequence 114 within the target DNA sequence 117. Similarly, the second single-stranded oligonucleotide 126 hybridizes with a second complementary DNA sequence 116 within the target DNA sequence 117. Further, the third single-stranded oligonucleotide 130 hybridizes with a third complementary DNA sequence 134 within the target DNA sequence 117. As illustrated, the fluorescent labels 122, 128 and 132, of the single-stranded oligonucleotides, 120, 126, 130 of the probe 118, are the same color. As shown in FIG. 1, a target DNA sequence 117 typically runs from a first complementary DNA sequence 114 to a last complementary DNA sequence 116 of a series of tiled complementary DNA sequences that span all or a portion of a chromosome.

dGH reactions typically involve the generation of single-stranded chromatids. Such single-stranded chromatids can be generated by any means known in the art. In illustrative embodiments, single-stranded chromatids are generated using CO-FISH techniques, as discussed elsewhere herein. dGH is distinguished from traditional FISH techniques, wherein double-stranded DNA oligonucleotide probes are denatured and hybridized with double-stranded sister chromatids as is appreciated by one skilled in the art, when preparing cells in a dGH reaction.

dGH reactions typically include probing more than one pair of single-stranded sister chromatids. In some embodiments, dGH reaction include probing 2, 3, 4, 5, 6, 7 ,8, 9, 10, 11, 12, 13, 14, 15, 16, 17, 18, 19, 20, 21, 22, or 23 or more pairs of single-stranded sister chromatids. In some embodiments, dGH reaction include probing 2 or more pairs of single-stranded sister chromatids.

Oligonucleotides of a plurality of single-stranded oligonucleotides that make up a probe are capable of hybridizing to single-stranded chromatids and can be of any functional length. Without limitation to any particular embodiment, the single-stranded oligos can be, for example, 10 to 100 nucleotides in length, 15 to 90 nucleotides in length, 25 to 75 nucleotides in length, 30 to 50 nucleotides in length, or 37 to 43 nucleotides in length, or any combination thereof.

In certain embodiments, sets of labeled probes for the methods disclosed herein can range in number of oligonucleotides from small probe sets directed to one or more than one gene of interest or larger probe sets that target all known genes on a single single-stranded chromatid, on several single-stranded chromatids, on a group of single-stranded chromatids, on all single-stranded sister chromatids, on a single chromosome, on a group of chromosomes, or on all the chromosomes in the organism under study. In some embodiments, a probe can include for example, between 10 and $2 \times 10^6$, 1,000 and $2 \times 10^6$, 10-10,000, 100-5,000, 100-1,000, 100-500, 200-1,000, 200-500 single-stranded oligonucleotides, each with a different nucleic acid sequence.

In some embodiments, the complementary sequences of the probe may be relatively equally dispersed throughout a genome. In another aspect, the complementary sequences of the probe can be more concentrated in certain regions of a genome and more dispersed in other regions of a genome. In certain aspects, a pool of labeled single-stranded oligonucleotides in each probe for the methods disclosed herein can range in number of oligonucleotides from smaller numbers of single-stranded oligonucleotides directed to specific target DNA sequences such as specific chromosomal regions on one chromosome, providing for example, locus specific banding on a limited number of chromosomal regions (e.g., one or more chromosomal regions), to a probe having a larger number of single-stranded oligonucleotides, for example that in some embodiments can detect larger target DNA sequences, such as larger chromosomal regions.

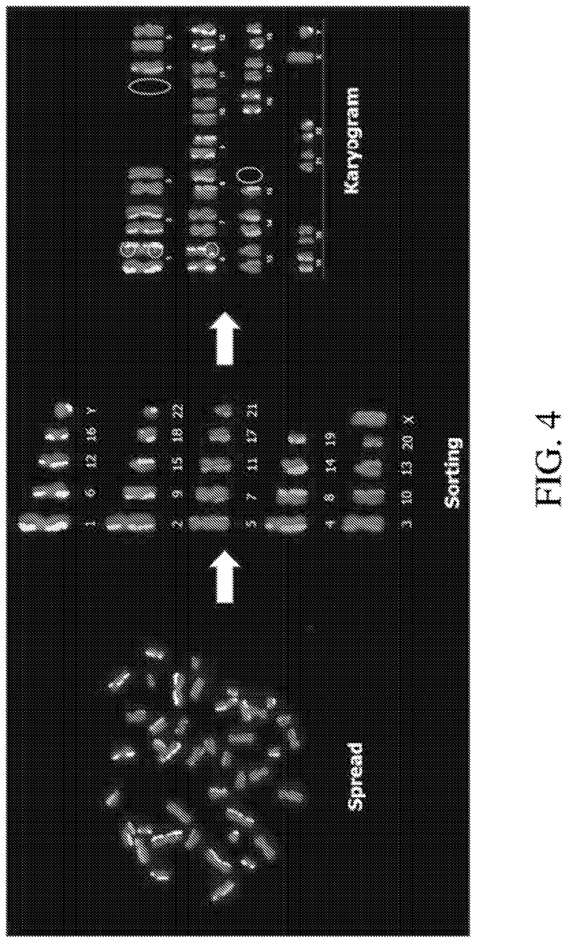

FIGS. 3A-3B illustrate results of an exemplary method using sets of probes, wherein each probe is made up of a pool of single-stranded oligonucleotides, including a cartoon of a metaphase spread 300 and a cartoon of a karyogram 320 from the metaphase spread 300. A plurality of chromosomes 309 from a cell are included in the metaphase spread 300, the images of which are rearranged into the karyogram 320 by chromosome size, centromere location and according to the color of the pool of single-stranded oligonucleotides used for each chromosome. Each of the chromosomes 309 includes a pair of single-stranded sister chromatids, such as for example sister chromatids 310a and 310b. In the illustrative method herein, five sets of probes are used, wherein each set of probes is labeled with a differently colored fluorescent label. In illustrative embodiments as shown in FIGS. 3A-3B, single-stranded oligonucleotides of each set of probes bind a different target DNA sequence of the same single-stranded sister chromatid, such as sister chromatid 310a. In some embodiments, a set of probes comprising oligonucleotides can bind to a target DNA sequence that spans at least 25%, 50%, 75%, 90%, or in illustrative embodiments as shown in FIGS. 3A-3B, at least 95% of a chromosome. Such embodiments, wherein at least 95% of a chromosome is bound by a set of probes having a same-colored fluorescent label, can be referred to as whole chromosome, single color painting of a chromosome. Thus, for example as shown in FIGS. 3A-3B, the Hoechst blue dye colored sister chromatids 310a of chromosomes 1, 6, 12, 16 and Y are hybridized with a first set of probes, wherein each probe is made up of a pool of single-stranded oligonucleotides labelled with a first color of fluorescent label 301, such as but not limited to yellow. As a result, the chromatid 310a becomes painted with yellow fluorescence, while the opposite sister chromatid 310b remains unpainted with yellow fluorescence. By painting the chromosome with a set of probes directed specifically to only one of the sister chromatids, it becomes possible to compare the pattern of fluorescent signals from the labelled set of probes, in this case yellow, to the pattern of fluorescence observed when a known control chromosome (e.g., a normal chromosome) is hybridized with the same set of probes. This is described in greater detail with reference to FIG. 4.

Still referring to FIGS. 3A-3B, chromosomes 2, 9, 15, 18 and 22 are hybridized with a second set of probes, wherein each probe is made up of a pool of single-stranded oligonucleotides labeled with a second colored fluorescent label 302, such as but not limited to light blue, such that the sister chromatids 310a are painted with a blue fluorescence. Chromosomes 5, 7, 11, 17 and 21 are hybridized with a third set of probes, wherein each probe is made up of a pool of single stranded oligonucleotides labeled with a third colored fluorescent label 303, such as but not limited to red, such that the sister chromatids 310a are painted with a red fluorescence. Chromosomes 4, 8, 14 and 19 are hybridized with a fourth set of probes, wherein each probe is made up of a pool of single stranded oligonucleotides labeled with a fourth colored fluorescent label 304, such as but not limited to green, such that the sister chromatids 310a are painted with a green fluorescence. Further, chromosomes 3, 10, 13, 20 and X are hybridized with a fifth set of probes, wherein each probe is made up of a pool of single-stranded oligonucleotides labeled with a fifth colored fluorescent label 305, such as but not limited to purple, such that the sister chromatids 310a are painted with a purple fluorescence. As shown in FIGS. 3A-3B, the opposite sister chromatids 310b of the chromosomes 309 are stained with a fluorescent dye, such as Hoechst dye, so as to enable visualization of the entire chromosome *via* fluorescent microscopy and facilitate detection of changes to chromosome structure and/or number.

Typically, as discussed above, a probe is made up of one pool of single-stranded oligonucleotides. Table 1 provides an exemplary set of dGH probes for dGH banding, discussed herein. Table 1 provides the order of the DNA binding site for the probe provided (1-19), the start and end nucleotide of the DNA binding site on the chromosome for each probe, the size of the DNA binding sequence, the number of oligos per probe (i.e., per band in this example) and the rest of the characteristics for this exemplary set of dGH probes.

The remainder of this page is intentionally left blank.

Table 1. Exemplary set of dGH probes for dGH banding.

| Feature and Colored Band Number (pàq arm) | Start (bp) | End (bp) | Size (kb) | Number of oligos per band | Band Color/ DNA label (Watson) | Band color/ DNA label (Crick) | average fluorescence density (target size/# of fluors) |
|---|---|---|---|---|---|---|---|
| Telomere p-arm | | | | | Blue | Blue | |
| Subtelomere p-arm | | | | | Blue | Blue | |
| 1 | 14497 | 9199710 | 9185213 | 27390 | Blue and Red | Blue | 1 fluor per 335 bp |
| 2 | 9199917 | 19417428 | 1E+07 | 27390 | Green | Blue | 1 fluor per 373 bp |
| 3 | 19417468 | 29156419 | 9738951 | 27390 | Red | Blue | 1 fluor per 355 bp |
| 4 | 29157122 | 40996360 | 1.2E+07 | 27390 | Green | Blue | 1 fluor per 432 bp |
| 5 | 40998055 | 52053266 | 1.1E+07 | 27390 | Red | Blue | 1 fluor per 404 bp |
| 6 | 52054602 | 65033440 | 1.3E+07 | 27390 | Green | Blue | 1 fluor per 473 bp |
| 7 | 65033522 | 75198573 | 1E+07 | 27390 | Magenta | Blue | 1 fluor per 371 bp |
| 8 | 75198607 | 96577268 | 2.1E+07 | 27390 | Yellow | Blue | 1 fluor per 780 bp |
| Centromere | | | | | | Blue | |
| 9 | 96577275 | 1.07E+08 | 1E+07 | 27390 | Magenta | Blue | 1 fluor per 382 bp |
| 10 | 107055871 | 1.2E+08 | 1.3E+07 | 27390 | Yellow | Blue | 1 fluor per 484 bp |
| 11 | 120339115 | 1.33E+08 | 1.3E+07 | 27390 | Magenta | Blue | 1 fluor per 476 bp |
| 12 | 133399786 | 1.46E+08 | 1.3E+07 | 27390 | Yellow | Blue | 1 fluor per 466 bp |
| 13 | 146189670 | 1.6E+08 | 1.4E+07 | 27390 | Green | Blue | 1 fluor per 503 bp |
| 14 | 159967656 | 1.73E+08 | 1.3E+07 | 27390 | Orange | Blue | 1 fluor per 484 bp |
| 15 | 173217075 | 1.87E+08 | 1.4E+07 | 27390 | Green | Blue | 1 fluor per 511 bp |
| 16 | 187214412 | 2.02E+08 | 1.5E+07 | 27390 | Orange | Blue | 1 fluor per 552 bp |
| 17 | 202327998 | 2.16E+08 | 1.3E+07 | 27390 | Green | Blue | 1 fluor per 491 bp |

(continued)

| Feature and Colored Band Number (pàq arm) | Start (bp) | End (bp) | Size (kb) | Number of oligos per band | Band Color/ DNA label (Watson) | Band color/ DNA label (Crick) | average fluorescence density (target size/# of fluors) |
|---|---|---|---|---|---|---|---|
| 18 | 215789917 | 2.25E+08 | 9443602 | 27390 | Orange | Blue | 1 fluor per 344 bp |
| 19 | 225233538 | 2.42E+08 | 1.7E+07 | 44561 | Magenta | Blue | 1 fluor per 371 bp |
| Subtelomere q-arm | | | | | Blue | Blue | |
| Telomere q-arm | | | | | Blue | Blue | |

Methods herein typically include detecting the fluorescent labels on probes used in dGH reactions in metaphase spreads using fluorescent microscopy. Methods are known in the art for detecting fluorescent labels of such probes. Typically, such probes, sometimes called dGH probes herein, are a pool of single-stranded oligonucleotides used in a dGH reaction that are labeled with the same fluorescent label. In some embodiments, with reference to Table 1, the oligonucleotides can be labelled with fluorescent labels that result in bands on a single-stranded sister chromatid that are different colors (e.g., blue, green, red, magenta, yellow, orange, etc.). In some embodiments herein, this is referred to as banded dGH. A wide variety of fluorophores are commercially available for use as fluorescent labels to label oligonucleotides. These fluorophores absorb and emit light at a wide variety of wavelengths and can be selected for labeling the oligonucleotide of various probes, such that the single-stranded sister chromatids are specifically colored with one or more bands. For example, with reference to Table 1, 27390 single-stranded oligonucleotides directed to the p arm of chromosome 2 are labeled with a red fluorophore, so as to generate a red band from base pairs 14497 to 9199710. This red band is observable via fluorescent microscopy, as described elsewhere herein. In some embodiments, all of the single-stranded oligonucleotides from one set of probes that bind target DNA segments on the same sister chromatid are fluorescently labeled with a single color, so as to paint substantially the entire, or the entire sister chromatid that single color.

In some embodiments, the dGH reaction is a dGH screening reaction, which in some embodiments is dGH Screen. In other embodiments, the dGH reaction is a targeted dGH reaction. In some embodiments, a method herein includes first performing a dGH screening reaction and the performing a targeted dGH reaction using the results of the dGH screening reaction to identify target DNA sequences for the targeted dGH reaction.

Some aspects herein are directed to compositions comprising the dGH ladder calibrant probe (e.g., internal control dGH probe ladders) disclosed herein. Thus, control dGH probes can have any of the characteristics and properties disclosed herein for dGH probes, including that they are typically designed to be complementary to unique sequences in the genome whose chromosome is being analyzed, such as the human genome. In some embodiments, the dGH probes of the internal control dGH probe ladder have the same label. In other embodiments the set of control dGH probes that makes up an internal control dGH probe ladder have multiple colors. Some aspects herein are directed to kits comprising one or more tubes or other containers containing an internal control dGH probe ladder, which are typically premade and predesigned internal control dGH probe ladders and other containers containing any of the components provided herein for performing a dGH reaction or analyzing the results thereof. For example, such a kit can include a container/tube with a solution of nucleotide analogs or a container/tube with a set of dGH probes that are complementary to target DNA sequences on an on-test chromosome. In some embodiments, such a kit can be ordered and/or shipped together although the components may not arrive within the same box. However, in some embodiments the kit components are contained within a box that can be labeled for, and include instructions for performing a dGH assay/method.

## CHROMOSOME VARIANTS AND REPAIR EVENTS

Illustrative methods, kits, and compositions herein involve or are used to detect, assess the presence or absence of, and/or quantity chromosome variants and/or event, for example repair events, in some embodiments as compared to a reference genome. Virtually any type (also called category herein) of chromosomal variant or repair event can be included in the analysis.

Chromosome variants can include chromosome numerical or structural variants. Chromosome variants and other outcomes of DNA replication and repair, such as sister-chromatid exchanges, are detected on a per-cell basis across a sample or a population of cells and in some embodiments are used to calculate an instability score which in some embodiments is a weight-based score used to determine genotoxicity risk. Chromosome structural variants and repair events included in the assessment can include some or all of those listed in Table 2, below. Thus, Table 2 provides examples of chromosome variants and repair events that can be detected in methods provided herein, for example using kits and compositions provided herein.

Table 2. Exemplary chromosome structural variants and repair events.

| Variant/Repair Type | Example | | |
|---|---|---|---|
| A. Chromosome Numeric Variants | 1. Total Chromosome Copy Number (genome ploidy) | | |
| | 2. Chromosome Numerical Variants (gain or loss of individual chromosomes) | | |
| | 3. Deletions and Insertions | | |
| B. Chromosome Structural Variants | 1. Translocations | | |
| | | | a. Unbalanced Translocations (dicentric/ acentric) |
| | | | b. Balanced Translocations |
| | | | c. Complex Translocations (involving 3 or more breakpoints) |
| | | | d. Symmetrical Translocations |
| | | | e. Asymmetrical Translocations |
| | 2. Inversions | | |
| | 3. Insertions | | |
| | 4. Marker Chromosomes | | |
| | 5. Chromothrypsis | | |
| | 6. Chromatid-Type Breaks | | |
| | 7. Sister Chromatid Recombination | | |
| C. Complex Chromosome Events | 1. Micronuclei | | |
| | 2. Fragments | | |
| | 3. Extra-chromosomal DNA (ecDNA) | | |
| | 4. Multi-Radial Chromosome | | |
| | 5. Iso-chromosomes | | |
| | 6. Chromoplexy | | |
| | 7. Rings (i.e., centric and acentric) | | |
| | 8. Centromere Abnormalities (e.g., spindling) | | |
| | 9. Chromosome Condensation Defects | | |
| D. Chromosome Repair Events | 1. Sister Chromatid Exchanges | | |

Structural variants may be simple or complex. Simple structural variants include single occurrences of unbalanced translocations, balanced translocations, homologous translocations, inversions, duplications, insertions, and deletions. Complex structural variants include multiple simple variants in a single cell, simple variants combined with the loss or gain of genomic material, loss or gain of entire chromosomes and more general DNA damage, in illustrative the more general DNA damage variant known as chromothrypsis. Heterogeneity of variants, defined as different structural variants appearing in the genomes of individual cells of the same organism, cell culture or batch of cells can involve simple or complex structural variants. A mosaic of structural variants occurs when dividing cells spontaneously develop a structural variant and both the variant free parent and the daughter containing the variant continue to propagate. In some embodiments, chromosome variant or repair events can include one or more of somatic mosaicism, a deletion, a copy number variation, or a structural rearrangement at a target genotoxic locus selected from 2q31.2 (PRKRA gene), 5q35.2 (BOD1 gene) and/or 7p15.2 (CBX3 gene). In some embodiments, chromosome variant or repair events, can include 2, 3, or 4 or more of somatic mosaicism, a deletion, a copy number variation, or a structural rearrangement at a target genotoxic locus selected from 2q31.2 (PRKRA gene), 5q35.2 (BOD1 gene) and/or 7p15.2 (CBX3 gene).

Structural variants are distinguished from base level changes such as single nucleotide polymorphisms (SNPs) or short insertions and deletions (INDELs). Structural variants occur when the ends of multiple double strand breaks are incorrectly rejoined or mis-repaired. Depending on the subsequent reproductive viability of the cell bearing the rearrangement the consequence of a resulting structural variant can be limited to a single cell, affect a sub-set of the tissues in an organism, or if it occurs in a germ cell, may even be inherited, and affect the lineage of the organism.

The potential for DNA mis-repair that leads to chromosome structural variants, numerical variants, and/or other events such as repair events exists whenever DNA double-strand breaks (DSBs) occur. DSBs can arise endogenously during normal cellular metabolic processes, such as replication and transcription. It has been estimated that DSBs occur naturally at a rate of 50 or more per cell, per cell cycle in actively metabolizing cells, and repair occurs both during replication and through replication-independent pathways. Double strand breaks are of particular concern when induced by exogenous factors above spontaneous rates either through radiation exposure, medical interventions such as chemotherapy with certain agents, exposures to toxins or during cellular engineering processes. Of particular note are processes employed to edit or correct a genetic aberration that intentionally employ DNA double strand breaks as a step in the engineering process, such as CRISPR CAS-9. While nominally targeted, nucleases used in CRISPR processes show a measurable degree of off-target cleavage. Formation of a structural variant during an editing process requires at least to concurrent double strand breaks, and since a normal human genome has two homologs of each chromosome, a single CRISPR edit can potentially have two concurrent double strand breaks, the mis-repair of which would yield a translocation between the two homologs. Multiple edits, for instance a triple knock-out would have proportionally more double strand breaks and thus a proportionally larger opportunity for DSB mis-repair. The number of double strand breaks in any given cell chosen from a batch of edited cells will be a function of 1) the degree and type of editing process 2) the rate of off target editing for the given editing system 3) the degree of DSBs from active metabolism. A fourth factor, the ability of the cell to functionally repair its own DSBs can vary and several disease states are known to detrimentally impact DNA repair.

If we then consider the normal rate of DSBs in actively metabolizing and dividing cells and the off-target nuclease cleavage, it is possible to have batches of cells with distributions of double strand breaks ranging from none (no editing, no metabolic breaks) to a maximum of 2 times the number of edits + the number of off-target edits + the number of concurrent random DSBs. Since a structural variant requires the mis-repair of at least two double strand breaks (yielding a simple translocation or inversion), the distribution of structural variants in the above example can range from 0 (no-misrepair) to ½ of the total number of double strand breaks.

Most DSBs are repaired by Non-Homologous End Joining (NHEJ) which operates throughout the cell cycle. In this process the broken ends are detected, processed, and ligated back together. This is an "error-prone" process because the previously existing base-pair sequence is not always restored with high fidelity. Nevertheless, this rejoining process (restitution) restores the linear continuity of the chromosome and does not lead to structural abnormalities. However, if two or more DSBs occur in close enough spatial and temporal proximity the broken end of one break-pair may mis-rejoin with an end of another break-pair, along with the same for the other two loose ends, resulting in a structural abnormality from the exchange. Examples include balanced and unbalanced translocations, inversions, or deletions. There is also a DSB repair process involving Homologous Recombination (HR) sometimes referred to as Homology Directed Repair (HDR). Homology directed repair (HDR) occurs post-replication when an identical homologous sequence becomes available and is near one another. The HDR pathway does not operate in G1 or G0 cells where the level of rad51 protein, necessary for HDR is very low or absent. However, as part of the process of gene editing (such as in the CRISPR system) the sequence to be edited is targeted and one or more DSBs are introduced to insert the desired sequence using HDR. Thus, any time DSBs are introduced, there is always a real chance that mis-rejoining among spontaneous or other DSBs form a structural variant.

Chromosomal instability (CIN) is a form of genomic instability (GIN) that involves frequent cytogenetic changes leading to changes in chromosome copy number (aneuploidy). Chromosomal instability is the predominant form of genomic instability that leads to changes in both chromosome numbers and structure. Numerical CIN is a high rate of either gain or loss of whole chromosomes, also called aneuploidy. Normal cells make errors in chromosome segregation in about 1% of cell divisions, whereas cells with CIN increase the error rate to 20% of cell divisions. By contrast, structural CIN is the rearrangement of parts of chromosomes and amplifications or deletions within a chromosome. Almost all solid tumors show CIN, and about 90% of human cancers exhibit chromosomal abnormalities and aneuploidy. The features of CIN tumor include global aneuploidy, loss of heterozygosity, homozygous deletions, translocation, and chromosomal changes such as deletions, insertions, inversions, and amplifications.

In certain illustrative embodiments, the one or more chromosome variants and/or repair events that are detected, assessed, and/or quantitated in methods herein include those that are not detectable by traditional FISH but are detectable by dGH. Thus, in certain illustrative embodiments the one or more chromosome variants and/or repair events include one or more of the following:

a) a sister chromatid exchange, a sister chromatid recombination,
b) a genomic inversion of a size of 5 megabases or less, or less than 5 megabases (e.g., between 2 kilobases and 4.9 megabases), and
c) a genomic insertion of a size 5 megabases or less, or less than 5 megabases (e.g., between 2 kilobases and 4.9 megabases).

The chromosome variants and repair events listed in Table 2 can be informative not only for the chromosome stability of a cell, but the genotoxic risk associated with that cell, or a population of cells, such as the genotoxic risk involved with administering that cell or population of cells to a subject. Further discussion of these chromosome variants and repair events, and their association with chromosome stability and genotoxicity risk are provided in the following paragraphs.

A chromosome numeric variant refers to a chromosome variant having a change in the number of chromosomes, or an insertion or deletion of at least 100 kilobases in length. Thus, this change in total chromosome copy number (genome ploidy) can occur by the addition of all or part of a chromosome (aneuploidy), the loss of an entire set of chromosomes (monoploidy) or the gain of one or more complete sets of chromosomes (euploidy). Chromosome numerical aberrations may occur, involving the gain or loss of an entire chromosome. In some cases, more than one pair of homologous chromosomes may be involved. Triploidy (3N) is related to poor prognosis, particularly in cancers with higher mortality such as gastric cancer, and colon cancer. Tetraploid (4N) cells are considered important in cancer because they can display increased tumorigenicity, resistance to conventional therapies, and are believed to be precursors to whole chromosome aneuploidy. Tetraploidy and chromosomal instability (CIN) combined are a dangerous combination. By virtue of having higher P53 gene copy number, activation may inadvertently promote formation of therapy-resistant tetraploid cells. In an example, disruption of the tumor suppression gene P53, due to loss or inactivation of chromosome 17p13 is a genotoxic event that impacts tumorigenesis and leads to development of lymphoma and leukemia. Some of the most common genetic disorders are associated with chromosome number variants, such as but not limited, Down's Syndrome (trisomy 21), Edward's Syndrome (trisomy 18), Patau Syndrome (trisomy 13), Cri du chat Syndrome or 5p Minus Syndrome (partial deletion of short arm of chromosome 5), Wolf-Hirschhorn Syndrome or Deletion 4p Syndrome, Jacobsen Syndrome or 11q Deletion Disorder, Klinefelter's Syndrome (presence of an additional X chromosome in males), and Turner Syndrome (presence of only a single X chromosome in females).

A translocation occurs when a chromosome breaks and a portion of the broken chromosome reattaches to a different chromosome, thereby creating a fusion product that may lead to disease. For example, chromosomal translocations are observed in acute myeloid leukemia, where a portion of Chromosome 8 will break off and fuse with part of Chromosome 11, thereby creating an 8/11 translocated product, or a fusion gene. Translocations can be balanced or unbalanced (i.e., dicentric or acentric), complex (i.e., involving three or more breakpoints), symmetrical or asymmetrical. The occurrence of translocations observed by dGH are indicative of chromosome instability.

A chromosomal inversion is a chromosome structure abnormality that can result from the misrepair of two double-stranded breaks occurring at different points along a portion of the chromosome, such that this interstitial portion of the chromosome becomes effectively rotated through 180° after a "mis-rejoining" among the broken ends of the chromosome. Importantly, this mis-rejoining must occur in such a way as to maintain the same 5' to 3' polarity of the strands of the chromosome and that of the inverted segment. While the backbone polarity is maintained, the DNA sequence of the nitrogenous bases within the segment is reversed. Genetic material may or may not be lost because of the chromosome breaks. A paracentric inversion occurs when both breaks occur in the same arm of the chromosome. A pericentric inversion occurs when one break occurs in the short arm and the other in the long arm of the chromosome. A chromosome 9 inversion is one of the most common structural balanced chromosomal variants and has been observed in congenital anomalies, growth retardation, infertility, recurrent pregnancy loss, and cancer. It is a particular problem to detect small inversions, such as those under 5MB with most techniques. dGH is particularly suited to detecting these small structural variants and has been demonstrated to routinely detect inversions of below 10kB.

Chromosomal insertions are the addition of genetic material to a chromosome. Such an insertion can be small, involving a single extra DNA base pair, or large, involving a piece of a chromosome. The effect of the insertion depends upon its location and size. For example, the insertion of one base pair could lead to a shift in the reading frame (i.e., a frameshift) during translation, resulting in synthesis of a defective protein that could lead, for example, to a birth defect. In another example, the insertion of three base pairs, though slightly larger, would not throw off the reading frame, and potentially would be less harmful than having the insertion of just one base pair. In another example, a large portion of one chromosome is inserted into another chromosome. Gain of chromosome 8q24.21 is a well-known insertion structural variant that causes the amplification of the oncogene, cMYC. Gain of this locus can increase gene expression or lead to uncontrolled activity of the onco-encoded proteins, and is observed in several cancers, including but not limited to colorectal carcinoma. It is very difficult to detect small insertions with most techniques. dGH can detect insertion 5MB and smaller.

Chromosomal deletions, sometimes known as partial monosomies, occur when a piece or section of chromosomal material is missing. Deletions can be just a base pair, part of a gene, an entire gene, or part of the chromosome. For example, DiGeorge syndrome (22q11.2 deletion syndrome) is a disorder caused when a small part of chromosome 22 is missing. Similar to small insertions, deletions smaller than 5 MB are difficult to detect with techniques other than dGH.

A number of marker chromosomes are known and can be identified using dGH in methods herein. Isochromosomes are supernumerary marker chromosomes made up of two copies of the same arm of a chromosome. The presence of an isochromosome in addition to the normal chromosome pair leads to a tetrasomy of the arm involved. The accurate description of such a marker chromosome using only conventional cytogenetic techniques is often difficult. Illustrative methods herein utilize dGH to identify marker chromosomes.

A marker chromosome is a small fragment of a chromosome that is distinctive, that is present in a cell as a separate structure from the rest of the chromosomes, and generally cannot be identified without specialized genomic analysis due to the size of the fragment. The significance of a marker is variable as it depends on what material is contained within the marker. A marker can be composed of inactive genetic material and have little or no effect, or it can carry active genes and cause genetic conditions such as iso(12p), which is associated with Pallister-Killian syndrome, and iso(18p), which is associated with mental retardation and syndromic facies. Chromosome 15 has been observed to contribute to a high number of marker chromosomes, but the reason has not been determined.

Chromothrypsis is a process by which dozens to up to thousands of chromosomal rearrangements occur in localized regions of one or a few chromosomes. When chromothrypsis occurs, essentially one or a few chromosomes (or a chromosome arm) is shattered, leading to the simultaneous creation of many double strand breaks. Most of the shattered fragments are stitched back together though Non-Homologous End Joining (NHEJ), which leads to the creation of a chromosome with complex, highly localized chromosomal rearrangements (e.g., chromoanagenesis). Broken DNA fragments may also be joined together to form circular, extrachromosomal double minute chromosomes. Chromothrypsis has been observed in the development of cancers. For example, *de novo* rearrangements caused by chromothrypsis can trigger chromosome instability in subsequent cell divisions.

Chromatid-type breaks refers to a break in the chromosome, where the break and re-joining affect only <u>one</u> of the sister-chromatids at any one locus. This differs from "chromosome-type" breaks, where the breaks and re-joins always affect both sister-chromatids at any one locus. Unrepaired DNA strand breaks contribute to genomic instability. Unrepaired chromatid breaks representing DNA strand breaks can result in chromosome deletions, translocations and gene amplifications seen in human cancers.

Sister chromatid recombination (SCR) is a structural variant that occurs during meiosis and promotes genomic integrity among cells and tissues through double-strand break repair. SCR refers to the homologous recombination process involving identical sister chromatids that results in a uni-directional non-crossover event, otherwise known as a gene conversion event. It is thought to occur when the homologous recombination intermediate known as the double Holliday junction is resolved in such a way that it results in a non-crossover. SCR can be employed by the cell to resolve both single-stranded DNA lesions (which involve a corresponding replication fork collapse) and double-stranded breaks. Gene conversion between sister chromatids is not usually associated with reciprocal exchange and is differentiated from an SCE for that reason. Aberrant SCR is associated with congenital defects and recurrent structural abnormalities. Mutations affecting genes involved in SCR have been linked to infertility and cancer. SCR is associated with chromosome instability, particularly with large structural rearrangements, aneuploidies and infertility. It is important to note that SCEs are detected by dGH but missed in all other karyotype assessment methods.

A number of complex events, such as repair events, produce structural variants that are listed in Table 2. Complex events produce complex chromosomal rearrangements (CCR) or complex genomic structural rearrangements that involve at least two chromosomes and three breakpoints with varied outcomes, except for simple or 3-break insertions. These CCRs may involve distal segments causing reciprocal translocation, or interstitial segments leading to insertion, inversion, deletion, or duplication, or they may involve a combination of both distal and interstitial segments. One chromosome may also have more than one aberration such as an inversion and a translocation that can coexist on the same chromosome.

The structural variants include micronuclei, chromosome fragments, extra-chromosomal DNA (i.e., ecDNA), multi-radial chromosomes, iso-chromosomes, chromoplexy, rings, centromere abnormalities and chromosome condensation defects. Several of these structural variants arise due to defects in the normal metabolism of the chromosomal DNA. These structures are described in greater detail in the paragraphs below.

Micronuclei (MN) are extra-nuclear bodies that contain damaged chromosome fragments and/or whole chromosomes that were not incorporated into the nucleus after cell division. Micronuclei can be induced by defects in the cell repair machinery and accumulation of DNA damages and chromosomal aberrations. A variety of genotoxic agents may induce micronuclei formation leading to cell death, genomic instability, or cancer development.

Multi-radial chromosomes are complex aberrant chromosomal structures that appear, in karyotype analysis, as a fusion of more than two sister chromatids, and are a hallmark of chromosomal instability. Multi-radial chromosomes are observed in several cancer predisposition syndromes, including Ataxia Telangiectasia, Nijmegen Breakage Syndrome, Bloom Syndrome, Werner Syndrome and Fanconi Anemia.

Extra-Chromosomal DNA (ecDNA) is any DNA found outside the chromosomes. In certain cases, ecDNA can be deleterious and can carry amplified oncogenes. In some aspects, deleterious ecDNA can be 100-1,000 times larger than kilobase size circular DNA found in healthy somatic tissues. In certain aspects, ecDNA includes episomal DNA and vector-incorporated DNA. ecDNA amplification promotes intratumoral genetic heterogeneity and accelerated tumor evolution. For example, ecDNA amplification has been observed in many cancer types but not in blood or normal tissue. Some of the most common recurrent oncogene amplifications have been observed on ecDNA. EcDNA amplifications resulted in higher levels of oncogene transcription compared to copy number-matched linear DNA, coupled with enhanced chromatin accessibility, and more frequently resulted in transcript fusions. Patients whose cancers carried ecDNA had significantly shorter survival, even when controlled for tissue type, than patients whose cancers were not driven by ecDNA-based oncogene amplification.

Chromosomal fragmentation occurs when the condensed chromosomes are rapidly degraded during metaphase, and results in cell death. Chromosome fragmentation is a major form of mitotic cell death which is identifiable during common cytogenetic analysis by its unique phenotype of progressively degraded chromosomes. Chromosome fragmentation is a non-apoptotic form of mitotic cell death and is observed from an array of cell lines and patient tissues. Its occurrence is associated with various drug treatment or pathological conditions.

Chromoplexy is a complex DNA rearrangement, wherein multiple strands of DNA are broken and ligated to each other in a new configuration, effectively scrambling the genetic material from one or more chromosomes. Chromoplexy often involves segments of DNA from multiple chromosomes (e.g., five or more). In one example of chromoplexy, homologous repeated sequences (i.e., HSRs) may become expanded by homologous recombination events in which a break induced in a palindromic sequence promotes homologous strand invasion and repair synthesis. Chromoplexy can account for many of the known genomic alterations found in prostate cancer by generation of oncogenic fusion genes (e.g., *BRAF* and *MAPK1* fusion) as well as by disruption or deletion of genes located near rearrangement breakpoints (e.g., tumor suppressor genes *PTEN, NKX3.1, TP53,* and *CDKN1B*).

Ring structures are circular chromosomal DNA that, in some instances, result from two terminal breaks in both chromosome arms, of a chromosome followed by fusion of the broken ends, or from the union of one broken chromosome end with the opposite telomere region, leading to the loss of genetic material. Alternatively, rings can be formed by fusion of subtelomeric sequences or telomere-telomere fusion with no deletion, resulting in complete ring chromosomes. Ring chromosomes may be dicentric (i.e., with more than one centromere) or acentric (i.e., no centromere). Ring chromosomes are associated with a variety of genetic diseases. In one example, r(20) syndrome is a rare genetic disorder characterized by a ring chromosome 20 replacing a normal chromosome 20.

Centromere abnormalities, such as "spindling," are aberrant chromosome rearrangements, such as from SCE or SCR, of long tandem DNA sequences at the centromere that can lead to chromosome fusions and genetic abnormalities. In some instances, intrachromatid recombination occurs, leading to the formation of a circle, such as a ring, and a deletion of a portion of the chromatid. In other instances, recombination leads to unequal exchange, thereby introducing instability in the total size of the centromeric array. In still other instances, homologous recombination at identical centromere sequences between different chromosomes can lead to the formation of dicentric and acentric chromosomes (i.e., two centromeres and no centromere, respectively). Chromosomal structural variants due to centromere abnormalities have been observed in a wide variety of cancers, including but not limited to breast cancer (chromosomes 12, 8, 7), colorectal cancer (chromosome 18), pancreatic cancer (chromosomes 18, 8), and melanomas (chromosomes 1, 18).

Chromosome condensation defects are defects in the reorganization or compaction of the chromatin strands into compact short chromosome structures that occurs in mitosis and meiosis. Generally, defects in chromosome condensation are caused by defects in one or more of the structures in is mediated by the condensin complex and other proteins and is necessary to prevent chromosomes from being entangled during chromosome segregation. In an example, Gulf War Illness (GWI) impacts 25-30% of gulf war veterans and is associated with a variety of condensation defects.

Sister chromatid exchanges are error-free swapping or cross-over event involving precisely matched and identical DNA strands of the sister chromatids of a condensed chromosome during mitosis. Sister chromatid exchanges, while not structural variants, are associated with elevated rates of genomic instability due to an increased probability that alternative template sites such as repetitive elements adjacent to the break site will produce an unequal exchange resulting a structural variant. SCE frequency is a commonly used index of chromosomal stability in response to environmental or genetic mutagens. A wide range of human diseases have been linked to SCE, including but not limited to lung cancer, leukemias, hearing loss, thyroid tumors, xeroderma pigmentosum and diffuse gastric cancer. SCEs are detectable by dGH, but not by any other karyotyping assessment methods.

Although sister chromatid exchange (SCE) events are not in themselves structural variants, they can be used as an indicator of chromosomal instability (De Pascalis *et al.,* 2015). SCE levels are increased in patients with various cancers associated with genomic instability (Salawu *et al.,* 2018; Soca-Chafre *et al.,* 2019; Xu *et al.,* 2015). Unlike translocations, inversions, and ring structures that are produced *via* NHEJ-mediated mis-joining of DSBs, SCEs arise during DNA replication and require HDR (Wilson and Thompson 2007). SCEs are non-recurrent repair events that appear as a random distribution within a population, while inversions, as true structural rearrangements, are stable and are passed on to daughter cells over many cell generations (i.e., they are recurrent within a population). While dGH can distinguish between recurrent and non-recurrent repair events in a population of cells, localized dGH assays can be helpful to identify these repair events as true inversions or SCEs. Other proxies of genomic instability, such as chromatid breaks and gaps, can arise only as a result of an event that occurred during the cell cycle immediately prior to the mitosis where it is observed.

## ADDITIONAL STABILITY-RELATED FACTORS

In some embodiments, additional stability-related factors, characteristics and/or measurements are made and used as part of a method herein. As a non-limiting example of an additional stability-related factor, results of a dGH reaction in methods herein can be used to detect a sub-clonal population in a population of cells, comprising substantially identical or identical chromosomal variant and/or repair events. Such sub-clonal population can include at least 10, 20, 30, 40, 50, 100, 250, 500, 1,000 or more cells of a cell population, or at least 0.1%, 1%, 2.5%, 5%, 10%, 20%, 25%, 50%, 75%, 90% but less than all cells of a cell population. In some embodiments, such sub-clonal population can be an aberrant outgrowth have an aberrant chromosome structure. In some embodiments, such sub-clonal population includes an integration of a recombinant insert sequence at or near an instability locus, a destabilizing locus, a genotoxic locus, an increased-risk locus, or a high-risk locus. Such embodiments can detect, measure and overweight outgrowth of chromosomally aberrant clones. As another non-limiting example of an additional factor, which in some embodiments can be considered a stability-related factor, results of a dGH reaction in methods herein can be used to detect mosaicism in a population of cells.

As another non-limiting example of an additional stability-related factor, specific chromosomal rearrangements involving, near, in or of known instability loci, destabilizing loci, increased risk loci, or high-risk loci, such as high-risk oncogenic loci, can be factored into genotoxicity risk aspects provided herein. Thus, chromosomal rearrangement around these loci can be used, considered, and factored into calculations and given weights much as is done for categories of chromosomal variants and event. For example, rearrangements involving, at, near, in, or of the following genomic coordinates could be considered in genotoxicity analysis and in illustrative embodiments confer a higher genotoxicity potential, risk, score and/or index: rearrangements that lead to loss or inactivation of chromosome 17p13 (p53) or chromosome 8p12-p23, or that lead to gain or amplification of chromosome 8q24.21 (amplification of cmyc) or that lead to homogeneously staining regions (hsrs) of marker chromosomes comprising chromosome 11q23 (kmt2a). Additional exemplary instability loci/genotoxic loci are provided in other sections herein.

Loss or inactivation of 17p13 (p53): p53 is a known tumor suppressor gene and key regulator of tetraploidy. Accordingly, translocations and inversions involving chromosome 17p13 can be considered in a genotoxicity analysis and for certain embodiments can be weighted in a potential or risk score and/or index.

Loss or inactivation Chromosome 8p12-p23: This locus is associated with BNIP3L, a known tumor suppressor gene that is lost or inactivated in many tumors, such as breast and ovarian cancers, contributing to abnormal proliferation.

Gain or amplification of chromosome 8q24.21 (amplification of cMYC): cMYC is a known oncogene and gain of this locus can increase gene expression or lead to uncontrolled activity of the onco-encoded proteins.

HSR 11q23 (KMT2A): The presence of homogeneously staining regions (hsr) on marker chromosomes has been correlated with amplification of specific proto-oncogenes. In this case, the homogeneously staining region on chromosome 11 is highly specific for KMT2A.

## CHROMOSOME STABILITY AND CHROMOSOME STABILITY SCORE/VALUE

As disclosed herein, illustrative methods, kits, and compositions herein involve analyzing the number of, and in illustrative embodiments, the variability of chromosomal variants and repair events in a population of cells, also referred to herein as a cell population. In illustrative embodiments, variability can be quantified to determine the chromosome stability or instability of the population. In some embodiments, a chromosome stability or instability value or score can be determined. In general, a population of cells with more chromosome stability has a lower chromosome stability value or score than a population of cells with less chromosome stability (i.e., more chromosome instability).

An advantage of fluorescent microscopy methods like dGH is that they allow analysis of many chromosomal variants and repair events (i.e., multiplex analysis) in the same cell (i.e., on a per-cell basis), including hallmarks of DNA damage, instability and genetic diseases, such as small inversion, for which there are no competing single cell techniques of analysis. Thus, methods herein in illustrative embodiments, are multiplex reactions that involve analyzing more than one chromosomal variant or repair event in a dGH reaction or analysis on a per-cell basis in a population of cells. In illustrative methods herein, dGH analysis results are quantitated by counting the chromosomal variants and repair events. Such quantitation can be done using the counted chromosomal variants and repair events using many different mathematical formula/strategies. For example, an averaging of chromosome variants or repair events on a per cell basis can be calculated for a population of cells. Generally, the more chromosome variants or repair events per cell, the lower the chromosome stability and the higher the chromosome instability (a high chromosome stability score).

In some embodiments, the heterogeneity and cell-to-cell variance for a specific locus (genomic or insertional) or loci is calculated in a sample or a population of cells. In some embodiments, a method of calculating total count of breakpoints is used to count total breakpoints, which can be used in the stability or genotoxicity score. And, in fact, in some embodiments, interpretation of scores can lead to understanding a cellular mechanism, such as a mechanism for causing instability.

In some embodiments, an averaged karyotype for a sample of cells from a population of cells coupled with a cell-to-cell variance calculation across a locus or loci of interest is used to derive a weighted numerical score describing the overall genome integrity of the population as compared to a reference genome, as well as the relative genomic stability of the sample or population of cells. This numerical score, or instability index can be tracked over time to provide data for example about cell transformation in culture, clonal outgrowth, and genomic stability post-treatment. As discussed elsewhere herein, chromosome variants and events can be weighted differently, and a weighted chromosome stability score can include different weighted factors. In some embodiments, a weighted chromosome stability score can include adding two or more weighted factors of the series of weighted factors, multiplying two or more weighted factors of the series of weighted factors, using one of the weighted factors as an exponent of another weighted factor, or by a combination of the adding, the multiplying, and/or the using the exponent.

In illustrative embodiments, a chromosome stability value or score is calculated using the counted chromosomal variants and/or repair events on a per-cell basis calculated for a population of cells. In illustrative embodiments the chromosomal stability value or score is a measure of variability across a population of cells. Such chromosome stability score can be calculated using an individual type (i.e., category) of chromosomal variant or event (e.g., SCE) on a per-cell basis across a population of cells. Typically, a multiplex analysis is performed for more than one individual type of chromosome variant or event (e.g., SCE and presence of marker chromosomes) across the cell population. Furthermore, additional stability-related measurements as provided in more detail herein, can be measured and included in the analysis. The analysis can combine values across multiple types of chromosomal variants and repair events per cell and then combine the combined value across the population of cells, or in illustrative embodiments, the analysis is performed on each individual type of chromosomal variant or event and analyzed across the population of cells for that particular type of chromosomal event. And then the individual combined scores, for the particular type of chromosomal variants and repair events across the population, are combined for all of the types of chromosomal variants and repair events that are analyzed. Whether the counts of chromosomal variants and repair events are analyzed as combined numbers per cell before being analyzed across the population, or individual types of chromosomal variants and repair events are analyzed across the population before the values are combined, the combining can be for example, multiplying values or subtracting values, or in certain illustrative embodiments it is by summing values.

In a general sense, the stability score for a population of cells is a weighted average of individual structural variation where the weights are chosen to highlight specific areas of interest or concern. Furthermore, the formula for the weighted average can be described so as to take into account multiplicative effects as well as exponential effects. Scores may be calculated for a single population based on variation from a presumed "normal" reference genome, or various strategies to capture variance between the scores of distinct populations may be employed. In some embodiments, scores can be calculated for two or more populations of cells. A variance strategy may be appropriate, for instance to compare two arms of a study, to distinguish between stable and unstable cell lines, to characterize instability of tumors, to distinguish between closely related species, such as chimpanzee and orangutang, to characterize clonal outgrowth of an oncogenic sub-clonal population in a gene therapy or in any case where distinguishing, differentiating of characterizing two or more populations of cells is meaningful. In general, the formula for a score for a single population of cells can be written as the summation of the terms from 1 to the number of distinctly measurable variants of the term.

In one illustrative embodiment, variance is calculated across cells from each population of cells being analyzed, for each of the numerical values from each of the categories of chromosomal variants or repair events being analyzed as well as some other additional stability-related categories, in a particular experiment, commercial services run, or other performance of the method, using a statistical formula that calculates variance. For example, a population variance can be calculated using the following formula (Formula 1):

$$\sigma^2 = \frac{\sum_{i=1}^{n}(x_i - \mu)^2}{n} \qquad (1)$$

where $\mu$ is the average value of $x$ for the entire population and $n$ is the number of cells whose value was used for the calculation.

Accordingly, as a non-limiting specific example, 20-100 cells from a cell line can be analyzed using dGH. An assessment can be performed, which measures the copy number of each chromosome, the number of translocations (per chromosome), the total chromosome count, presence of marker chromosomes, and repair events present on the secondary sister chromatid (inversions and sister chromatid exchanges). Variance can be calculated across the 20-100 cells of the cell line for each of the numerical values from each of the categories specified above using the above population variance formula.

In some embodiments, a chromosome stability value or score is determined for an on-test cell or typically cell population relative to a control or reference genome, cell, or cell population. Such control or reference genome can be a reference human genome, such as a wild-type human genome or a consensus human genome. A control or reference cell or cell population can be a wild-type cell or cell-population. Furthermore, a control or reference cell population can be a cell population whose chromosomal stability value or score has previously been determined or established and in certain illustrative embodiments is of the same cell-type of a cell population that is being analyzed. In illustrative embodiments, a control or reference cell population is of the same cell type as a cell population that is being analyzed. For example, if a CAR-T cell population is being analyzed, a control or reference cell population can be a T cell population from a healthy subject, or an immortalized T cell line whose chromosome stability value or score is known/has been determined. In other embodiments, when a cancer cell population is being analyzed, a control or reference cell or cell population can be, for example, another cancer cell line with a known/predetermined chromosome stability score. In another embodiment, when a genetically-modified population of cells is being analyzed, a reference or control cell can be a population of cells from the same type of cell or descendants of the same cells that are not genetically modified, as those that were genetically modified. In other embodiments, two or more populations of cells that are of the same cell type and in illustrative embodiments, descendants of the same cell population, are analyzed together. In such embodiments, chromosome stability values can be compared between the different populations of cells.

In further embodiments, a control or reference cell population can be the same population of cells under analysis at an earlier timepoint. Thus, for example, a chromosome stability score can be determined at a first time point, and then a chromosome stability score can be determined for descendants of the same population of cells at a later time point using the first time point or an earlier time point as the control sample/values. Such a later time point can be, for example, 1, 2, 4, 7, 14, 21, 28 days, or 1, 2, 3, 6, 12 months, or 1, 2, 3, 4, 5, 6 or more years after the initial timepoint. Furthermore, in some embodiments a chromosome stability score can be calculated at multiple time points and compared to an initial time point and/or to one or more earlier timepoints. Thus, a stability score or index, or instability score or index, or genotoxicity risk or score numerical score, or instability index can be tracked over time to provide data for example, about cell transformation in culture, clonal outgrowth, and genomic stability post-treatment.

For analysis that compares different cell populations, a variance from another cell population (e.g., the reference human genome (diploid- 46 chrs) can be analyzed. For example, average values from each sample of a set of samples can be analyzed for sample variance. For example, the following formula (Formula 2) can be used for the sample variance calculation:

$$s^2 = \frac{\sum_{i=1}^{n}(x_i - \bar{x})^2}{n-1} \tag{2}$$

where $\bar{x}$ is the average value of $x$ for the sample population and $n$ is the population size. In such calculations, the value for a particular chromosome variant or event or additional stability-related measurement for a control or reference sample or population is subtracted from an on-test population to obtain values of $X$ to enter into the calculation.

Thus, in the non-limiting specific example above, a set of cell lines can be analyzed, which are all descendants of a same parent cell line, and known or newly calculated values for the parent cell line can be used as the control or reference measurements and subtracted from those of each cell line (i.e., cell population for this analysis).

The higher the variance value for population variance, the more "unstable" the line is. The higher the variance value for sample variance, the more rearranged it is as compared to the reference genome. Thus, results of the variance calculations can be used to inform and calculate or otherwise determine a stability or instability index to assessment an on-test population of cells.

As a non-limiting example, grossly rearranged but stable cancer cell lines can have a relative high score for sample variance, and a relatively low score for population variance. A grossly rearranged and highly unstable cancer cell lines can for example, have high scores for both population variance and sample variance. Relatively normal diploid cell lines, with high cell-to-cell variability (high potential for transformation) can have a high score for population variance, and a low score for sample variance. Stable, normal cell lines are expected to have low scores for both population variance and sample variance. The Examples section herein provides a specific example of such analysis and calculations.

**GENOTOXICITY RISK**

Chromosome stability and chromosome stability scores or values as discussed herein, for example, as discussed above, can be used to assess, analyze, calculate and/or determine the genotoxicity risk of a cell or typically a population of cells. Genotoxicity risk in illustrative embodiments is the risk that a particular genetic change in the genome of a cell is detrimental to an organism having that cell and can sometimes lead to a disease. Genotoxicity of a cell or a population of cells can be assessed and/or reported in methods herein as a genotoxicity index or genotoxicity potential in addition to genotoxicity risk. Accordingly, provided herein in some aspects, are methods of determining a genotoxicity risk for a population of cells. Such methods include a step of determining a chromosome stability value as discussed herein and then using that value to determine genotoxicity risk. In general, the higher the chromosome stability value (sometimes called chromosome instability value) the higher the genotoxic risk associated with the population of cells.

Information and/or knowledge about a particular cell population being analyzed can be used to modify/manipulate a chromosomal stability value for that cell population to determine a genotoxic risk of that population of cells. For example, depending on a type of cell for a population of cells, different types of chromosome variants and/or repair events and different additional stability-related characteristics and/or measurements are more or less important in determining the genotoxic risk for that particular population of cells. For example, for a CAR-T cell population, certain types of chromosome variants and repair events are more or less important than for a genetically-modified liver cell or a cancer cell line, for example. Thus, weighting can be used and applied to chromosome stability values, to determine genotoxicity risk for a population of cells. In some embodiments, genotoxicity risk can be determined for two or more populations of cells.

In analyzing genotoxicity risk two or more factors, which typically include at least one type of chromosome variant and/or event determined using dGH, are considered in certain illustrative embodiments, and weighted based on the particular cell type. The value of a weighted factor can be added to another weighted factor(s), can be multiplied by the value of another weighted factor(s), and/or can be used as an exponent for a value from another factor. The value can be an average or mean value of individual cell counts for a type of factor across a cell population, or it can be a variability measure of the individual cell counts.

In analyzing genotoxicity risk two or more factors, which typically include at least one type of chromosome variant and/or event determined using dGH, are considered in certain illustrative embodiments, and weighted based on the particular cell type. The value of a weighted factor can be added to another weighted factor(s), can be multiplied by the value of another weighted factor(s), and/or can be used as an exponent for a value from another factor. The value can be an average or mean value of individual cell counts for a type of factor across a cell population, or it can be a variability measure of the individual cell counts.

Many types of genotoxic risks can be analyzed using methods herein. In fact, the methods herein are especially valuable because they are flexible and can be used to measure many types of genotoxicity risk. Thus, methods herein can be used in modeling a risk associated with genomic changes, especially those that include chromosomal variant or events, in a population of cells leading to virtually any disease when such population is present in an organism, for example a mammal, in illustrative embodiments a domestic animal or a human. Such modeling can analyze population stability wherein markers of instability can be weighted greater than other factors, for example with a factor that is greater than 1 (e.g., between greater than 1 (e.g., 1.1) and 100, or between 1.1 and 10). Furthermore, methods herein can be used in disease risk predictions or modeling, by analyzing and in illustrative weighing more heavily (e.g., greater than other factors, or with a weighting factor of between greater than 1 (e.g., 1.1) to 100 or 1.1 to 10) inserts and/or clonal outgrowth. In certain aspects, genotoxicity risk can be measured as oncogenic risk when analyzing a population of cancer cells.

## WEIGHTING OF CHROMOSOME VARIANTS AND EVENTS

In some embodiments, one or more types of chromosomal variants and/or repair events provide information that has greater value than other types of chromosomal variants and/or repair events especially with respect to determining genotoxic risk of a particular cell population. More specifically, in illustrative embodiments, methods for determining genotoxicity risk, score, index, or potential, are provided herein that include combining weighted chromosomal stability scores. Furthermore, in illustrative embodiments control cells are included in the analysis such that in some embodiments, an increased number of one or more chromosomal variant and/or event in a population of cells compared to a control population is an indication of an increased genotoxicity of the cell population. In illustrative embodiments, an increased variance in the number of one or more chromosomal variant and/or event in a population of cells compared to a control population is an indication of an increased genotoxicity of the cell population. Weighting of the values for types of chromosomal variants or events and in some embodiments, other characteristics (e.g., mutation status at a genotoxic locus, results of an orthogonal cell analysis) of a cell in a population of cells or of the cell population overall, in illustrative embodiments is set based on the cell type of a cell population being analyzed and based on the type of chromosomal variant and/or event or additional stability-related measurement. Furthermore, the weighting in illustrative embodiments is performed on each type of chromosomal variant and/or event independently before weighted values for each type of chromosomal variant and/or event are combined to arrive at or otherwise determine a genotoxicity risk assessment or score.

For example, in some embodiments, an increased numerical chromosome variant in a test cell population, in illustrative embodiments relative to the control cell population or reference and/or other cell populations, suggests or otherwise provides an indication of decreased chromosomal stability (increased chromosomal instability). In illustrative embodiments, in increased variance in the number of chromosomes in a test population of cells compared to a control population of cells suggests and/or is an indication of an increased genotoxicity risk of the test cell population. As such, some embodiments include an increased weight on the number of chromosomes detected, or the variance of the number of chromosomes detected in the test population compared to a control population of cells suggests and/or is an indication of an increased genotoxicity risk of the test cell population. By way of example only, in some embodiments, the number of chromosomes detected, or in illustrative embodiments, the variance of the number of chromosomes detected, in a population of cells, such as for example in a diploid or a complex genomic cell population is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the number of chromosomes detected, or in illustrative embodiments a variance of the number of chromosomes detected, in a cell population is weighted by a factor of between 10x and 1,000x, 500x, 250x, 125x, or 100x, or between 25x and 250x, between 50x and 150x, or between 90x and 110x. In some embodiments, such increased number of chromosomes is triploidy and/or tetraploidy.

For example, in some embodiments, an increased number of SCEs in a test cell population, in illustrative embodiments relative to a control or reference cell population and/or other cell population, suggests or otherwise provides an indication of decreased chromosomal stability (increased chromosomal instability). An increase in the number of SCEs is an indication of an increase in the number of chromosomal repair events and can indicate an increased cell stress. In illustrative embodiments, an increased variance in the number of SCEs in a test population of cells compared to a control population suggests and/or is an indication of an increased genotoxicity risk of the test cell population. As such, some embodiments include an increased weight on the number of SCEs detected, or the variance of the number of SCEs detected in the test population compared to the control population. By way of example only, in some embodiments, the number of SCEs detected, or in illustrative embodiments, the variance of the number of SCEs detected, in a population of cells, such as a diploid or a complex genomic cell population (having test or control cells) is weighted by a factor of 1.0x, 1.1x, 1.5x, 2.0x, 2.5x, 3.0x, or greater, or between 1.5x and 2x, 3x, 4x, 5x, 10x, or 100x, or between 2x and 3x, 4x, 5x, 10x, or factor of between 10x and 1,000x, 500x, 250x, 125x, or 100x, or between 25x and 250x, between 50x and 150x, or between 90x and 110x. In some embodiments, the number of the SCEs detected, or in illustrative embodiments a variance of the number SCEs detected in the population of cells, such as a diploid or a complex genomic cell population is weighted by a factor of 2.5x to 3.5x, 2.7x to 3.3x or 3.0x. In some embodiments, the diploid or complex genomic cell population is a genetically-modified cell population.

In another example, in some embodiments, an increased number of translocations in a test cell population, in illustrative embodiments relative to the control cell population or reference and/or other cell populations, suggests or otherwise provides an indication of decreased chromosomal stability (increased chromosomal instability). In illustrative embodiments, an increased variance in the number of translocations in a test population of cells compared to a control population of cells suggests and/or is an indication of an increased genotoxicity risk of the test cell population. As such, some embodiments include an increased weight on the number of translocations detected, or the variance of the number of translocations detected in the test population compared to the control population. Some embodiments include an increased weight on the types of translocations detected (i.e., unbalanced, balanced, complex, symmetrical and/or asymmetrical translocations), or the variance of the type(s) of translocations detected in the test population compared to the control population. By way of example only, in some embodiments, the number of translocations detected, or in illustrative embodiments, the variance of the number and/or types of translocations detected, in a population of cells such as for example a diploid or a complex genomic cell population is weighted by a factor of 1.0x, 1.1x, 1.5x, 2.0x, 2.5x, 3.0x, 4.0x, 5.0x, 6.0x, 7.0x, 8.0x, 9.0x, 10.0x, 11.0x, 12.0x, 13.0x, 14.0x, 15.0x, 16.0x, 17.0x, 18.0x, 19.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, or greater, or between 1.5x and 2x, 3x, 4x, 5x, 10x, 15x, 20x, 25x, 30x, 40x, 50x, 75x, or 100x, or between 2x and 3x, 4x, 5x. 10x, 15x, 20x, 25x, 30x, 40x, 50x, 75x, or 100x. In some embodiments, the number and/or types of the translocations detected, or in illustrative embodiments a variance of the number and/or types of translocations detected, in the diploid or complex genomic cell population is weighted by a factor of 2.0x. In some embodiments, the population of cells such as for example a diploid or a complex genomic cell population comprises at least a subpopulation comprising a genetically-modified genome and the number and/or types of the translocations detected, or in illustrative embodiments a variance of the number and/or types of the translocations detected is weighted by a factor of 1.0x - 2.0x, including any weighted values therebetween.

In another example, in some embodiments, an increased number of dicentric chromosomes detected in a test cell population, in illustrative embodiments relative to the control cell population and/or other cells in the diploid or complex genomic cell population, suggests or otherwise provides an indication of decreased chromosomal stability (increased chromosomal instability). In illustrative embodiments, in increased variance in the number dicentric chromosomes in a test population of cells compared to a control population of cells suggests and/or is an indication of an increased genotoxicity risk of the test cell population. As such, some embodiments include an increased weight on the number of dicentric chromosomes detected, or the variance of the number of dicentric chromosomes detected in the test population compared to a control population of cells suggests and/or is an indication of an increased genotoxicity risk of the test cell population. By way of example only, in some embodiments, the number of dicentric chromosomes detected, or in illustrative embodiments, the variance of the number of dicentric chromosomes detected, in the population of cells such as for example a diploid or a complex genomic cell population may be weighted by values including 1.0x, 1.1x, 1.5x, 2.0x, 2.5x, 3.0x, 4.0x, 5.0x, 6.0x, 7.0x, 8.0x, 9.0x, 10.0x, 11.0x, 12.0x, 13.0x, 14.0x, 15.0x, 16.0x, 17.0x, 18.0x, 19.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x or greater, or between 1.5x and 2x, 3x, 4x, 5x, 10x, 15x, 20x, 25x, 30x, 40x, 50x, 75x, or 100x, or between 2x and 3x, 4x, 5x, 10x, 15x, 20x, 25x, 30x, 40x, 50x, 75x, or 100x, factor of between 10x and 1,000x, 500x, 250x, 125x, or 100x, or between 25x and 250x, between 50x and 150x, or between 90x and 110x. In some embodiments, the value of the dicentric chromosomes detected in the population of cells such as for example a diploid or a complex genomic cell population may be weighted by 2.0x. In some embodiments, the population of cells such as for example a diploid or a complex genomic cell population comprises at least a subpopulation comprising a genetically-modified genome and the value of the dicentric chromosomes detected can be weighted by 2.0x.

In another example, in some embodiments, an increased number of inversions in a test cell population, in illustrative embodiments relative to the control cell population or reference and/or other cell populations, suggests or otherwise provides an indication of decreased chromosomal stability (increased chromosomal instability). In illustrative embodiments, an increased variance in the number of inversions in a test population of cells compared to a control population of cells suggests and/or is an indication of an increased genotoxicity risk of the test cell population. As such, some embodiments include an increased weight on the number of inversions detected, or the variance of the number of inversions detected in the test population compared to the control population. By way of example only, in some embodiments, the number of inversions detected, or in illustrative embodiments, the variance of the number and/or types of inversions detected, in a population of cells such as for example a diploid or a complex genomic cell population is weighted by a factor of 1.0x, 1.1x, 1.5x, 2.0x, 2.5x, 3.0x, 4.0x, 5.0x, 6.0x, 7.0x, 8.0x, 9.0x, 10.0x, 11.0x, 12.0x, 13.0x, 14.0x, 15.0x, 16.0x, 17.0x, 18.0x, 19.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, or greater, or between 1.5x and 2x, 3x, 4x, 5x, 10x, 15x, 20x, 25x, 30x, 40x, 50x, 75x, or 100x, or between 2x and 3x, 4x, 5x, 10x, 15x, 20x, 25x, 30x, 40x, 50x, 75x, or 100x, or between 10x and 1,000x, 500x, 250x, 125x, or 100x, or between 25x and 250x, between 50x and 150x, or between 90x and 110x. In some embodiments, the number of the inversions detected, or in illustrative embodiments a variance of the number inversions detected, in the population of cells such as for example a diploid or a complex genomic cell population is weighted by a factor of 1.0x.

In another example, in some embodiments, an increased number of insertions in a test cell population, in illustrative embodiments relative to the control cell population or reference and/or other cell populations, suggests or otherwise provides an indication of decreased chromosomal stability (increased chromosomal instability). In illustrative embodiments, in increased variance in the number of insertions in a test population of cells compared to a control population of cells suggests and/or is an indication of an increased genotoxicity risk of the test cell population. As such, some embodiments include an increased weight on the number of insertions detected, or the variance of the number of insertions detected in the test population compared to a control population of cells suggests and/or is an indication of an increased genotoxicity risk of the test cell population. By way of example only, in some embodiments, the number of insertions detected, or in illustrative embodiments, the variance of the number of insertions detected, in a population of cells such as for example a diploid or a complex genomic cell population is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the number of the insertions detected, or in illustrative embodiments a variance of the number insertions detected, in the population of cells such as for example a diploid or a complex genomic cell population is weighted by a factor of between 10x and 1,000x, 500x, 250x, 125x, or 100x, or between 25x and 250x, between 50x and 150x, or between 90x and 110x.

Using a dGH ladder calibrant probe set, the fluorescent signal level at each insertion can be used to estimate the number of copies of a recombinant nucleic acid insert that were inserted at each site. Sites with a single full recombinant nucleic acid insert can be determined using the calibrant ladder dGH probe and the amount of fluorescence at a site with a single recombinant nucleic acid insert can be set as the unit signal (i.e., equal to 1). To allow for variations in fluorescence between chromosomes, slides, and cells, insertions with a range of signal, for example, a range of 0.6-1.5 unit signals, can be classified as having a single recombinant nucleic acid insert ($ICN_1$: Insert Copy Number with a single recombinant nucleic acid insert). In some embodiments, the range of unit signals for insertions classified as having a single recombinant nucleic acid insert can be between 0.2, 0.3, 0.4, 0.5, 0.6, 0.7, 0.8, or 0.9 unit signals on the low end of the range and 1.1, 1.2,

EP 4 347 876 B1

1.3, 1.4, 1.5, 1.6, 1.7, 1.8, 1.9, 2, 2.25, or 2.5 unit signals on the high end of the range. In some embodiments, the range of unit signals for insertions classified as having a single recombinant nucleic acid insert can be between 0.2, 0.3, 0.4, 0.5, 0.6, 0.7, 0.8, or 0.9 unit signals on the low end of the range and 2.5 unit signals on the high end of the range. In some embodiments, the range of unit signals for insertions classified as having a single recombinant nucleic acid insert can be between 0.2, 0.3, 0.4, 0.5, 0.6, 0.7, 0.8, or 0.9 unit signals on the low end of the range and 1.5 unit signals on the high end of the range. In some embodiments, the range of unit signals for insertions classified as having a single recombinant nucleic acid insert can be between 0.2 unit signals on the low end of the range and 1.1, 1.2, 1.3, 1.4, 1.5, 1.6, 1.7, 1.8, 1.9, 2, 2.25, or 2.5 unit signals on the high end of the range. In some embodiments, the range of unit signals for insertions classified as having a single recombinant nucleic acid insert can be between 0.5 unit signals on the low end of the range and 1.1, 1.2, 1.3, 1.4, 1.5, 1.6, 1.7, 1.8, 1.9, 2, 2.25, or 2.5 unit signals on the high end of the range. In some embodiments, the range of unit signals for insertions classified as having a single recombinant nucleic acid insert can be between 0.2 and 2.5, 0.3 and 2, 0.4 and 1.75, 0.5 and 1.5, 0.7 and 1.3, or 0.8 and 1.2 unit signals. Insertions with a unit signal lower than the signal for a single recombinant nucleic acid insert can be classified as having a fractional recombinant nucleic acid insert ($ICN_f$: Insert Copy Number with a fractional recombinant nucleic acid insert). In some embodiments, the range of unit signals for insertions classified as having a single recombinant nucleic acid insert can be less than 0.2, 0.3, 0.4, 0.5, 0.6, 0.7, 0.8, or 0.9 unit signals. Insertions with a unit signal higher than the signal for a single recombinant nucleic acid insert can be classified as having a concatemer recombinant nucleic acid insert ($ICN_c$: Insert Copy Number with a concatemer recombinant nucleic acid insert). In some embodiments, the range of unit signals for insertions classified as having a single recombinant nucleic acid insert can be greater than 1.1, 1.2, 1.3, 1.4, 1.5, 1.6, 1.7, 1.8, 1.9, 2, 2.25, or 2.5 unit signals. A skilled artisan understands that the $ICN_1$, $ICN_f$, and $ICN_c$ are selected to not overlap with each other. For example, insertions with 0.5 to 1.5 unit signals can be classified as $ICN_1$, insertions with less than 0.5 unit signals can be classified as $ICN_f$, and insertions with greater than 1.5 unit signals can be classified as $ICN_c$. Depending on the cell line, insertion, etc. these insertion classifications can be weighted differently and, along with the number of each type of insertion, used to determine chromosome stability or genotoxicity risk. For example, if the potential risk of a bare promoter, which would be classified as the $ICN_f$, inserting randomly at an undesired location and initiating oncogenesis has a more dangerous effect in a cell or cell line, it can be weighted higher than $ICN_c$. If concatemers are more dangerous, which would be classified as the $ICN_c$, it can be weighted higher than $ICN_c$.

The weights can also be used to include whether the cells meet a regulatory guideline, for example, how many insertions are present on average per cell. In some embodiments, the regulatory guideline can be the current allowable insertions per cell based on the governmental guidelines. In some embodiments, an average number of insertions per cell if 3, 4, 5, 6, or 7 or can meet the regulatory guideline. If the average number of insertions per cell does not meet the regulatory guideline, then the average number of insertions can be weighted higher. For example, if the average number of insertions per cell meets the regulatory guideline, then it can be given a weight of 0, and if the average number of insertions per cell does not meet the regulatory guideline, then it can be given a weight of 1. With these weights, and given a population of cells with an average number of insertions of 8 that does not meet the regulatory guideline, the weighted average number of insertions factor would be 8. A skilled artisan understands that these weights can vary depending on the cell type, cell line, insertion, etc., and that the weights can even be negative, for example, where a specific factor shows increased chromosome stability or reduced genotoxicity risk. Using weights in this manner allows for data to be analyzed in multiple ways when the relative risks are unknown and also to look at binary classifications of risk where guidelines have been established.

Similarly, total copy number per cell ($ICN_T$) (e.g., total number of inserted recombinant nucleic acid inserts per cell) can be analyzed and weighted. The total copy number per cell can be calculated by multiplying the average number of insertions per cell (ICN) (determined by averaging the number of insertion signals per cell, which does not take into account the level of signal at each insertion) times the average size of the insertion ($ICN_e$). Thus, $ICN_1 = ICN * ICN_e$.

In another example, in some embodiments, an increased number of marker chromosomes in a test cell population, in illustrative embodiments relative to the control cell population or reference and/or other cell populations, suggests or otherwise provides an indication of decreased chromosomal stability (increased chromosomal instability). In illustrative embodiments, in increased variance in the number of marker chromosomes in a test population of cells compared to a control population of cells suggests and/or is an indication of an increased genotoxicity risk of the test cell population. As such, some embodiments include an increased weight on the number of marker chromosomes detected, or the variance of the number of marker chromosomes detected in the test population compared to a control population of cells suggests and/or is an indication of an increased genotoxicity risk of the test cell population. By way of example only, in some embodiments, the number of marker chromosomes detected, or in illustrative embodiments, the variance of the number of marker chromosomes detected, in a population of cells such as for example a diploid or a complex genomic cell population is weighted by a factor of, or at least 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x.

In another example, in some embodiments, the presence of, or an increased number of chromothrypsis events in a test cell population, in illustrative embodiments relative to the control cell population and/or other cells in the population of cells such as for example a diploid or a complex genomic cell population, suggests or otherwise provides an indication of decreased chromosomal stability (increased chromosomal instability). In illustrative embodiments, an increased variance in the number of chromothrypsis events in a test population of cells compared to a control population suggests and/or is an indication of an increased genotoxicity risk of the test cell population. As such, some embodiments include an increased weight on the number of chromothrypsis events detected, or the variance of the number of chromothrypsis events detected in the test population compared to the control population. By way of example only, in some embodiments, the number of chromothrypsis events detected, the variance of the number of chromothrypsis events detected, in the population of cells such as for example a diploid or a complex genomic cell population is weighted by a factor of 1.0x, 1.1x, 1.5x, 2.0x, 2.5x, 3.0x, 4.0x, 5.0x, 6.0x, 7.0x, 8.0x, 9.0x, 10.0x, 11.0x, 12.0x, 13.0x, 14.0x, 15.0x, 16.0x, 17.0x, 18.0x, 19.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, or greater, or between 1.5x and 2x, 3x, 4x, 5x, 10x, 15x, 20x, 25x, 30x, 40x, 50x, 75x, or 100x, or between 2x and 3x, 4x, 5x, 10x, 15x, 20x, 25x, 30x, 40x, 50x, 75x, or 100x, or between 10x and 1,000x, 500x, 250x, 125x, or 100x, or between 25x and 250x, between 50x and 150x, or between 90x and 110x. In some embodiments, the number of the chromothrypsis events detected, or in illustrative embodiments a variance of the number chromothrypsis events detected, in the population of cells such as for example a diploid or a complex genomic cell population is weighted by a factor of 3.0x. In some embodiments, the population of cells such as for example a diploid or a complex genomic cell population is a genetically-modified cell population and the number of the chromothrypsis events detected, or in illustrative embodiments a variance of the number of chromothrypsis events detected is weighted by a factor of 3.0x.

In another example, in some embodiments, an increased number of chromatid-type breaks in a test cell population, in illustrative embodiments relative to the control cell population or reference and/or other cell populations, suggests or otherwise provides an indication of decreased chromosomal stability (increased chromosomal instability). In illustrative embodiments, in increased variance in the number of chromatid-type breaks in a test population of cells compared to a control population of cells suggests and/or is an indication of an increased genotoxicity risk of the test cell population. As such, some embodiments include an increased weight on the number of chromatid-type breaks detected, or the variance of the number of chromatid-type breaks detected in the test population compared to a control population of cells suggests and/or is an indication of an increased genotoxicity risk of the test cell population. By way of example only, in some embodiments, the number of chromatid-type breaks detected, or in illustrative embodiments, the variance of the number of chromatid-type breaks detected, in a population of cells such as for example a diploid or a complex genomic cell population is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x, or between 10x and 1,000x, 500x, 250x, 125x, or 100x, or between 25x and 250x, between 50x and 150x, or between 90x and 110x.

In another example, in some embodiments, an increased number of sister chromatid recombination events (SCRs) in a test cell population, in illustrative embodiments relative to the control cell population or reference and/or other cell populations, suggests or otherwise provides an indication of decreased chromosomal stability (increased chromosomal instability). In illustrative embodiments, in increased variance in the number of SCRs in a test population of cells compared to a control population of cells suggests and/or is an indication of an increased genotoxicity risk of the test cell population. As such, some embodiments include an increased weight on the number of SCRs detected, or the variance of the number of SCRs detected in the test population compared to a control population of cells suggests and/or is an indication of an increased genotoxicity risk of the test cell population. By way of example only, in some embodiments, the number of SCRs detected, or in illustrative embodiments, the variance of the number of SCRs detected, in a population of cells such as for example a diploid or a complex genomic cell population is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the number of the SCRs detected, or in illustrative embodiments a variance of the number SCRs detected, in the population of cells such as for example a diploid or a complex genomic cell population is weighted by a factor of between 10x and 1,000x, 500x, 250x, 125x, or 100x, or between 25x and 250x, between 50x and 150x, or between 90x and 110x.

In another example, in some embodiments, an increased number of micronuclei in a test cell population, in illustrative embodiments relative to the control cell population or reference and/or other cell populations, suggests or otherwise provides an indication of decreased chromosomal stability (increased chromosomal instability). In illustrative embodiments, in increased variance in the number of micronuclei in a test population of cells compared to a control population of cells suggests and/or is an indication of an increased genotoxicity risk of the test cell population. As such, some embodiments include an increased weight on the number of micronuclei detected, or the variance of the number of micronuclei detected in the test population compared to a control population of cells suggests and/or is an indication of an increased genotoxicity risk of the test cell population. By way of example only, in some embodiments, the number of micronuclei detected, or in illustrative embodiments, the variance of the number of micronuclei detected, in a population of cells such as for example a diploid or a complex genomic cell population is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x, or between 10x and 1,000x, 500x, 250x, 125x, or 100x, or between 25x and 250x, between 50x and 150x, or between 90x and 110x. In some embodiments, the number of the micronuclei detected, or in illustrative embodiments a variance of the number micronuclei detected, in the population of cells such as for example a diploid or a complex genomic cell population is weighted by a factor of between 10x and 1,000x, 500x, 250x, 125x, or 100x, or between 25x and 250x, between 50x and 150x, or between 90x and 110x.

In another example, in some embodiments, an increased number of chromosomal fragments and/or an increased amount of chromosomal fragmentation in a test cell population, in illustrative embodiments relative to the control cell population or reference and/or other cell populations, suggests or otherwise provides an indication of decreased chromosomal stability (increased chromosomal instability). In illustrative embodiments, in increased variance in the number of chromosomal fragments and/or an increased amount of chromosomal fragmentation in a test population of cells compared to a control population of cells suggests and/or is an indication of an increased genotoxicity risk of the test cell population. As such, some embodiments include an increased weight on the number of chromosomal fragments and/or an increased amount of chromosomal fragmentation detected, or the variance of the number of chromosomal fragments and/or an increased amount of chromosomal fragmentation detected in the test population compared to a control population of cells suggests and/or is an indication of an increased genotoxicity risk of the test cell population. By way of example only, in some embodiments, the number of chromosomal fragments and/or an increased amount of chromosomal fragmentation detected, or in illustrative embodiments, the variance of the number of SCRs detected, in a population of cells such as for example a diploid or a complex genomic cell population is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the number of the chromosomal fragments and/or an increased amount of chromosomal fragmentation detected, or in illustrative embodiments a variance of the number chromosomal fragments and/or an increased amount of chromosomal fragmentation detected, in the population of cells such as for example a diploid or a complex genomic cell population is weighted by a factor of between 10x and 1,000x, 500x, 250x, 125x, or 100x, or between 25x and 250x, between 50x and 150x, or between 90x and 110x.

In another example, in some embodiments, increased amounts of extra-chromosomal DNA (ecDNA), or in the ecDNA structures, in a test cell population, in illustrative embodiments relative to the control cell population or reference and/or other cell populations, suggests or otherwise provides an indication of decreased chromosomal stability (increased chromosomal instability). In illustrative embodiments, in increased variance in the amount of ecDNA in a test population of cells compared to a control population of cells suggests and/or is an indication of an increased genotoxicity risk of the test cell population. As such, some embodiments include an increased weight on the amount of ecDNA detected, or the variance of the amount of ecDNA detected in the test population compared to a control population of cells suggests and/or is an indication of an increased genotoxicity risk of the test cell population. By way of example only, in some embodiments, the amount of ecDNA detected, or in illustrative embodiments, the variance of the amount of ecDNA detected, in a population of cells such as for example a diploid or a complex genomic cell population is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the amount of ecDNA detected, or in illustrative embodiments a variance of the amount of ecDNA detected, in the population of cells such as for example a diploid or a complex genomic cell population is weighted by a factor of between 10x and 1,000x, 500x, 250x, 125x, or 100x, or between 25x and 250x, between 50x and 150x, or between 90x and 110x.

In another example, in some embodiments, increased numbers of multi-radial chromosomes, or multi-radial chromosome structures, in a test cell population, in illustrative embodiments relative to the control cell population or reference and/or other cell populations, suggests or otherwise provides an indication of decreased chromosomal stability (increased chromosomal instability). In illustrative embodiments, in increased variance in the number of multi-radial chromosomes in a test population of cells compared to a control population of cells suggests and/or is an indication of an increased genotoxicity risk of the test cell population. As such, some embodiments include an increased weight on the number of multi-radial chromosomes detected, or the variance of the number of multi-radial chromosomes detected in the test population compared to a control population of cells suggests and/or is an indication of an increased genotoxicity risk of the test cell population. By way of example only, in some embodiments, the number of multi-radial chromosomes detected, or in illustrative embodiments, the variance of the number of multi-radial chromosomes detected, in a population of cells such as for example a diploid or a complex genomic cell population is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the number of multi-radial chromosomes detected, or in illustrative embodiments a variance of the number of multi-radial chromosomes detected, in the population of cells such as for example a diploid or a complex genomic cell population is weighted by a factor of between 10x and 1,000x, 500x, 250x, 125x, or 100x, or between 25x and 250x, between 50x and 150x, or between 90x and 110x.

In another example, in some embodiments, an increased number of iso-chromosomes, or isochromosome structures, in a test cell population, in illustrative embodiments relative to the control cell population or reference and/or other cell populations, suggests or otherwise provides an indication of decreased chromosomal stability (increased chromosomal instability). In illustrative embodiments, in increased variance in the number of iso-chromosomes in a test population of cells compared to a control population of cells suggests and/or is an indication of an increased genotoxicity risk of the test cell population. As such, some embodiments include an increased weight on the number of iso-chromosomes detected, or the variance of the number of iso-chromosomes detected in the test population compared to a control population of cells suggests and/or is an indication of an increased genotoxicity risk of the test cell population. By way of example only, in some embodiments, the number of iso-chromosomes detected, or in illustrative embodiments, the variance of the number of iso-chromosomes detected, in a population of cells such as for example a diploid or a complex genomic cell population is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the number of iso-chromosomes detected, or in illustrative embodiments a variance of the number of iso-chromosomes detected, in the population of cells such as for example a diploid or a complex genomic cell population is weighted by a factor of between 10x and 1,000x, 500x, 250x, 125x, or 100x, or between 25x and 250x, between 50x and 150x, or between 90x and 110x.

In some embodiments, a number of chromoplexy events detected, relative to the control cell population and/or other cells in the population of cells such as for example a diploid or a complex genomic cell population, may be weighted. As such, some embodiments may include an increased weight on the value assigned for any chromoplexy events detected. By way of example only, in some embodiments, the value of chromoplexy events detected in the population of cells such as for example a diploid or a complex genomic cell population may be weighted by values including 1.0x, 1.1x, 1.5x, 2.0x, 2.5x, 3.0x, or greater, or between 1.5x and 2x, 3x, 4x, 5x, 10x, or 100x, or between 2x and 3x, 4x, 5x, 10x, or 100x, or between 10x and 1,000x, 500x, 250x, 125x, or 100x, or between 25x and 250x, between 50x and 150x, or between 90x and 110x. In some embodiments, the value of the chromoplexy events detected in the population of cells such as for example a diploid or a complex genomic cell population may be weighted by 3.0x. In some embodiments, the population of cells such as for example a diploid or a complex genomic cell population comprises at least a subpopulation comprising a genetically-modified genome and the value of the chromoplexy events detected can be weighted by 3.0x.

In another example, in some embodiments, an increased number of ring chromosomes in a test cell population, in illustrative embodiments relative to the control cell population or reference and/or other cell populations, suggests or otherwise provides an indication of decreased chromosomal stability (increased chromosomal instability). In illustrative embodiments, in increased variance in the number of ring chromosomes in a test population of cells compared to a control population of cells suggests and/or is an indication of an increased genotoxicity risk of the test cell population. As such, some embodiments include an increased weight on the number of ring chromosomes detected, or the variance of the number of ring chromosomes detected in the test population compared to a control population of cells suggests and/or is an indication of an increased genotoxicity risk of the test cell population. By way of example only, in some embodiments, the number of ring chromosomes detected, or in illustrative embodiments, the variance of the number of ring chromosomes detected, in a population of cells such as for example a diploid or a complex genomic cell population is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or

EP 4 347 876 B1

between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the number of ring chromosomes detected, or in illustrative embodiments a variance of the number of ring chromosomes detected, in the population of cells such as for example a diploid or a complex genomic cell population is weighted by a factor of between 10x and 1,000x, 500x, 250x, 125x, or 100x, or between 25x and 250x, between 50x and 150x, or between 90x and 110x.

In another example, in some embodiments, an increased number of chromosomes with centromere abnormalities (centromere abnormalities) in a test cell population, in illustrative embodiments relative to the control cell population or reference and/or other cell populations, suggests or otherwise provides an indication of decreased chromosomal stability (increased chromosomal instability). In illustrative embodiments, in increased variance in the number of centromere abnormalities in a test population of cells compared to a control population of cells suggests and/or is an indication of an increased genotoxicity risk of the test cell population. As such, some embodiments include an increased weight on the number of centromere abnormalities detected, or the variance of the number of centromere abnormalities detected in the test population compared to a control population of cells suggests and/or is an indication of an increased genotoxicity risk of the test cell population. By way of example only, in some embodiments, the number of centromere abnormalities detected, or in illustrative embodiments, the variance of the number of centromere abnormalities detected, in a population of cells such as for example a diploid or a complex genomic cell population is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the number of centromere abnormalities detected, or in illustrative embodiments a variance of the number of centromere abnormalities detected, in the population of cells such as for example a diploid or a complex genomic cell population is weighted by a factor of between 10x and 1,000x, 500x, 250x, 125x, or 100x, or between 25x and 250x, between 50x and 150x, or between 90x and 110x.

In another example, in some embodiments, an increased number of chromosome condensation defects (condensation defects) in a test cell population, in illustrative embodiments relative to the control cell population or reference and/or other cell populations, suggests or otherwise provides an indication of decreased chromosomal stability (increased chromosomal instability). In illustrative embodiments, in increased variance in the number of condensation defects in a test population of cells compared to a control population of cells suggests and/or is an indication of an increased genotoxicity risk of the test cell population. As such, some embodiments include an increased weight on the number of condensation defects detected, or the variance of the number of condensation defects detected in the test population compared to a control population of cells suggests and/or is an indication of an increased genotoxicity risk of the test cell population. By way of example only, in some embodiments, the number of condensation defects detected, or in illustrative embodiments, the variance of the number of condensation defects detected, in a population of cells such as for example a diploid or a complex genomic cell population is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the number of condensation defects detected, or in illustrative embodiments a variance of the number of condensation defects detected, in a cell population is weighted by a factor of between 10x and 1,000x, 500x, 250x, 125x, or 100x, or between 25x and 250x, between 50x and 150x, or between 90x and 110x

In another example, in some embodiments, a change in a total number of chromosomes detected (i.e., aneuploidy events), in illustrative embodiments relative to the control cell population and/or other cells in a cell population, suggests or otherwise provides an indication of decreased chromosomal stability (increased chromosomal instability). In illustrative embodiments, changes in variance in the number of aneuploidy events in a test population of cells compared to a control population of cells suggests and/or is an indication of an increased genotoxicity risk of the test cell population. As such, some embodiments include an increased weight on the change in a total number of aneuploidy events in the test population compared to a control population of cells suggests and/or is an indication of an increased genotoxicity risk of the test cell population. By way of example only, in some embodiments, the number of aneuploidy events, or in illustrative embodiments, the variance of the number of aneuploidy events detected in a cell population, for example a diploid or a complex genomic cell population is weighted by a factor of 1.0x, 1.1x, 1.5x, 2.0x, 2.5x, 3.0x, 4.0x, 5.0x, 6.0x, 7.0x, 8.0x, 9.0x, 10.0x, 11.0x, 12.0x, 13.0x, 14.0x, 15.0x, 16.0x, 17.0x, 18.0x, 19.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, or greater, or between 1.5x and 2x, 3x, 4x, 5x, 10x, 15x, 20x, 25x, 30x, 40x, 50x, 75x, or 100x, or between 2x and 3x, 4x, 5x. 10x, 15x, 20x, 25x, 30x, 40x, 50x, 75x, or 100x, or between 10x and 1,000x, 500x, 250x, 125x, or 100x, or between 25x and 250x, between 50x and 150x, or between 90x and 110x, or between 0.5x and 1.5x, 0.9x and 1.1x, or 1.0x, or between 10x and 1,000x, 500x, 250x, 125x, or 100x, or between 25x and 250x, between 50x and 150x, or between 90x and 110x. In some embodiments, the number of the aneuploidy events detected, or in illustrative embodiments a variance of the number aneuploidy events detected, in a cell population is weighted by a factor of between 0.5x and 1.5x, or between 0.9x and 1.1x, or 1.0x, or between 10x and 1,000x, 500x, 250x, 125x, or 100x, or between 25x and 250x, between 50x and 150x, or between 90x and 110x, or between 0.5x and 1.5x, 0.9x and 1.1x, or 1.0x, or between 10x and 1,000x, 500x, 250x, 125x, or 100x, or between 25x and 250x, between 50x and 150x, or between 90x and 110x. In some embodiments, the population of cells, such as for example in a diploid or a complex genomic cell population, is a genetically-modified population of cells and the number of the aneuploidy events detected in the cell population is weighted by a factor of between 0.5x and 1.5x, 0.9x and 1.1x, or 1.0x, or between 10x and 1,000x, 500x, 250x, 125x, or 100x, or between 25x and 250x, between 50x and 150x, or between 90x and 110x, or between 0.5x and 1.5x, 0.9x and 1.1x, or 1.0x, or between 10x and 1,000x, 500x, 250x, 125x, or 100x, or between 25x and 250x, between 50x and 150x, or between 90x and 110x.

In another example, in some embodiments, a chromosomal rearrangement in, near, of or involving a known instability locus, increased-risk and/or high-risk locus, such as a known destabilizing and/or high-risk oncogenic locus, in illustrative embodiments relative to a control cell population and/or other cells in a cell population, suggests a higher genotoxicity, for example oncogenicity risk. Thus, the presence, number, and/or variance of the number of rearrangements involving an instability, destabilizing or high risk genomic locus (i.e., genomic coordinates), such as those provided herein as non-limiting examples, can be considered and factored or otherwise weighted more highly in genotoxicity (e.g., oncogenicity) analysis and in illustrative embodiments confer a higher genotoxicity (e.g., oncogenicity) potential, risk, score and/or index. By way of example only, in some embodiments, the presence, number, or variance in the number of rearrangements in, near, or of known high risk oncogenic loci in a cell population, for example a diploid or a complex genomic cell population is weighted by a factor greater than 1, greater than some or all other factors, or of 1.0x, 1.1x, 1.5x, 2.0x, 2.5x, 3.0x, 4.0x, 5.0x, 6.0x, 7.0x, 8.0x, 9.0x, 10.0x, 11.0x, 12.0x, 13.0x, 14.0x, 15.0x, 16.0x, 17.0x, 18.0x, 19.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, or greater, or between 1.5x and 2x, 3x, 4x, 5x, 10x, 15x, 20x, 25x, 30x, 40x, 50x, 75x, or 100x, or between 2x and 3x, 4x, 5x. 10x, 15x, 20x, 25x, 30x, 40x, 50x, 75x, or 100x, or between 10x and 1,000x, 500x, 250x, 125x, or 100x, or between 25x and 250x, between 50x and 150x, or between 90x and 110x, or between 0.5x and 1.5x, 0.9x and 1.1x, or 1.0x. In illustrative embodiments such rearrangement involving, at, near or of a high-risk locus are rearrangements that lead to loss or inactivation of chromosome 17p13 (p53) or chromosome 8p12-p23, or that lead to gain or amplification of chromosome 8q24.21 (amplification of cMYC) or that lead to homogeneously staining regions (hsrs) of marker chromosomes comprising chromosome 11q23 (KMT2A). Additional instability, destabilizing, increased-risk, and/or high-risk loci are provided herein.

In certain embodiments, rearrangements that lead to loss or inactivation of chromosome 17p13 (p53) are weighted greater than 1.0 such as any of the values and ranges immediately above or in illustrative examples of 1.0x, 1.1x, 1.5x, 2.0x, 2.5x, 3.0x, 4.0x, 5.0x, 6.0x, 7.0x, 8.0x, 9.0x, 10.0x, 11.0x, 12.0x, 13.0x, 14.0x, 15.0x, 16.0x, 17.0x, 18.0x, 19.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, or greater, or between 1.5x and 2x, 3x, 4x, 5x, 10x, 15x, 20x, 25x, 30x, 40x, 50x, 75x, or 100x, or between 2x and 3x, 4x, 5x. 10x, 15x, 20x, 25x, 30x, 40x, 50x, 75x, or 100x, or between 10x and 1,000x, 500x, 250x, 125x, or 100x, between 10x and 200x, between 25x and 250x, between 50x and 150x, or between 90x and 110x, or between 0.5x and 1.5x, 0.9x and 1.1x, or 1.0x, or between 10x and 1,000x, 500x, 250x, 125x, or 100x, or between 25x and 250x, between 50x and 150x, in a population of cancer cells, such as those known to harbor a loss or inactivation of P53. In certain embodiments, rearrangements that lead to loss or inactivation of chromosome 8p12-p23, are weighted greater than 1.0 such as any of the values and ranges immediately above, or in illustrative examples of 1.0x, 1.1x, 1.5x, 2.0x, 2.5x, 3.0x, 4.0x, 5.0x, 6.0x, 7.0x, 8.0x, 9.0x, 10.0x, 11.0x, 12.0x, 13.0x, 14.0x, 15.0x, 16.0x, 17.0x, 18.0x, 19.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, or greater, or between 1.5x and 2x, 3x, 4x, 5x, 10x, 15x, 20x, 25x, 30x, 40x, 50x, 75x, or 100x, or between 2x and 3x, 4x, 5x. 10x, 15x, 20x, 25x, 30x, 40x, 50x, 75x, or 100x, or between 10x and 1,000x, 500x, 250x, 125x, or 100x, or between 25x and 250x, between 50x and 150x, or between 90x and 110x, or between 0.5x and 1.5x, 0.9x and 1.1x, or 1.0x, or between 10x and 1,000x, 500x, 250x, 125x, or 100x, or between 25x and 250x, between 50x and 150x in a population of breast or ovarian cancer cells,

In certain embodiments, rearrangements that lead to gain or amplification of chromosome 8q24.21 (amplification of cMYC), are weighted greater than 1.0 such as any of the values and ranges immediately above, or in illustrative examples of 1.0x, 1.1x, 1.5x, 2.0x, 2.5x, 3.0x, 4.0x, 5.0x, 6.0x, 7.0x, 8.0x, 9.0x, 10.0x, 11.0x, 12.0x, 13.0x, 14.0x, 15.0x, 16.0x, 17.0x, 18.0x, 19.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, or greater, or between 1.5x and 2x, 3x, 4x, 5x, 10x, 15x, 20x, 25x, 30x, 40x, 50x, 75x, or 100x, or between 2x and 3x, 4x, 5x. 10x, 15x, 20x, 25x, 30x, 40x, 50x, 75x, or 100x, or between 10x and 1,000x, 500x, 250x, 125x, or 100x, or between 25x and 250x, between 50x and 150x, or between 90x and 110x, or between 0.5x and 1.5x, 0.9x and 1.1x, or 1.0x, or between 10x and 1,000x, 500x, 250x, 125x, or 100x, or between 25x and 250x, between 50x and 150x in a population of breast cancer cells. In certain embodiments, rearrangements that lead to gain or amplification of chromosome 11q23 (KMT2A), are weighted greater than 1.0 such as any of the values and ranges immediately above, or in illustrative examples of 1.0x, 1.1x, 1.5x, 2.0x, 2.5x, 3.0x, 4.0x, 5.0x, 6.0x, 7.0x, 8.0x, 9.0x, 10.0x, 11.0x, 12.0x, 13.0x, 14.0x, 15.0x, 16.0x, 17.0x, 18.0x, 19.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, or greater, or between 1.5x and 2x, 3x, 4x, 5x, 10x, 15x, 20x, 25x, 30x, 40x, 50x, 75x, or 100x, or between 2x and 3x, 4x, 5x. 10x, 15x, 20x, 25x, 30x, 40x, 50x, 75x, or 100x, or between 10x and 1,000x or between 25x and 250x, between 50x and 150x, or between 90x and 110x, or between 0.5x and 1.5x, 0.9x and 1.1x, or 1.0x, or between 10x and 1,000x, 500x, 250x, 125x, or 100x, or between 25x and 250x, between 50x and 150x in a population of breast cancer cells.

Genotoxic Risk and Orthogonal Factors

Characteristics of a cell or population thereof in addition to those that are identified and optionally measured using dGH, are used in a determination of genotoxicity in some embodiments herein. As a non-limiting example, the presence of a specific sequence variant at a target genetic locus, e.g., a target gene mutation, can be considered and factored into methods and analysis herein. For example. The mutation status of a target gene can be included in a chromosomal stability score, and in illustrative embodiments, a weighted chromosomal stability score, for example, wherein a weighted factor value for the target gene mutation is used. In some embodiments, a weighted factor value for a target gene mutation can be 0 or 1, depending on a presence or absence of the mutation. In some embodiments, a weighted factor value for the target gene mutation is multiplied by one or more other weighted factors.

Thus, in some embodiments, a mutation of a target proto-oncogene or tumor suppressor gene can be used in methods herein. Accordingly, Orthogonal analysis and/or results thereof, by other methods, for instance a mutation or SNP analysis of an oncogene, can be included in a method provided herein. In some illustrative embodiments, results of orthogonal analysis, which in illustrative embodiments are one or more characteristics, such as genetic characteristics, of a population of cells, can be used in methods provided herein. For example, in a genotoxicity risk, index or score determination, such results or characteristics can be weighted by applying an appropriate weighting factor. In this manner, second order effects - which may or may not be structural - and that increase (or decrease) genotoxicity risk arising from structural changes can be accounted for. As a non-limiting example, a single oncogene activated by an inverted sequence would be measured by dGH and the presence of that activated oncogene given a weight, for instance:

a) Oncogenic Genotoxicity Risk Factor. OCF = W1(Prevalence of Inversion)
Where the weight is assigned appropriately according to the relative genotoxicity. If the presence or absence of a mutation or SNP in the oncogene would impact the relative genotoxicity, then the above could be modified as:
b) Oncogenic Genotoxicity Risk Factor. OCF = W1(Prevalence of Inversion) + W2 (Prevalence of SNP or mutation)

If the effect is simply additive. If, however, the effect of the SNP is binary or multiplicative the effect could be:

$$\text{Oncogenic Genotoxicity Risk Factor. OCF} = W1 * W2(\text{Prevalence of Inversion}).$$

In the above non-limiting example, W2 could be 0 if the SNP or mutation is not present and the baseline risk of the genotoxic inversion prevails. W2 could be between 0 and 1, if the SNP or mutation reduces the genotoxicity risk, and greater than 1 if it increases the risk. Multiple weights can be combined in other fashions to fit actual genotoxic risk data, or judgements about relative risks and best rank risk factors. For instance, if, in the above non-limiting illustration, the additional risk due to the SNP or mutation is dramatically increased, an exponential relationship might better represent risk. For instance:

a. Oncogenic Genotoxicity Risk Factor. OCF = W1^W2(Prevalence of Inversion)

In a non-limiting example, the equation for the variance score S, can be written in a number of forms. One general formula for the variance score S, that has the advantage of accounting for outside factors and second order effects when necessary and also is easily reducible to a straight-forward weighted average when appropriate, is as written in the following equation (Formula 3):

$$S_t = \sum_1^n [\![(W)]\!]_n W_f)[\![(P)]\!]_n)^{x_m} \qquad (1)$$

where n is the number of all possible variants that can be considered, such as but not limited to the variants described in Table 2. $W_n$ can change depending upon the importance of the variant. For example, for important variants, $W_n$ would be a

non-zero positive or negative number. For unimportant, unmeasured, or ignored variants, $W_n$ would be zero. $W_n$ is the weight given to a specific structural variant, typically ranging from -1000 to plus 1000. In a non-limiting example, $W_n$ of zero indicates that the variant $n$ does not contribute to the stability score. $W_f$ is a multiplicative factor based on other types of variants, other factors or judgements. As a non-limiting specific example, the lack of HER2 amplification can reduce the relative risk of breast cancer and $W_f$ for a variant $n$ is set at 0.25 for a particular cell population, thus reducing the overall weight of one or more scores. In another non-limiting specific example, in HER2 positive cells, $W_f$ for a variant $n$ is set at 10 or even 100, so as to dramatically increase the weight given to the presence of that variant $n$. $P_n$ is the prevalence of the variant $n$ in the cell population. In a non-limiting example, when the prevalence of the variant $n$ is 0, the variant $n$ makes no contribution to $S$. $X_m$ is the exponential weighting factor. The presence of specific variants, conditions or other factors can exponentially amplify the risk from variant $n$ and that amplified risk can be accounted for with this term. Accordingly, $X_m$ typically has a defined set of values. In a non-limiting example, a BRCA mutation in breast cancer can indicate that variant $n$ is of particular concern and thus $X_m$ is set at, for example, 3 indicating the presence of a BRCA mutation or it is set at 1 to indicate no BRCA mutation.

Formula 3 is general and will apply to any situation where a population-based score of variation can be applied, to population variance, to risk assessment, to degrees of DNA damage, tumor instability, and the like. Other formulations of the variance score $S$ can include additive terms, additional multiplicative terms or additional exponential terms. For example, the prevalence terms $P_n$ includes all variation types that can be measured by dGH, but $P_n$ can also include types of variants, such as for example chromosome count, that can be measured by dGH and other techniques as well as variants that are preferentially measured by other techniques. In this manner, a variant that is important to a specific situation, such as for example a low prevalence knockout of a gene, can be included in the calculation of a variance score S and in the calculation of variance score of S between two populations of cells.

It should also be noted that, for a perfectly organized genome with 100 percent correspondence to the reference genome, a normal number of undamaged chromosomes, and no measurable aberrations of any type, variance scores S calculated in this manner are by definition zero, since all variant prevalence $P_n$ would be zero by definition.

The weighting of different variants using the $W_n$ terms can be adjusted by any justifiable method, including but not limited to, based upon data, judgement, historical precedent and the like. The same or similar data sets can be analyzed using differing weighting methodologies as appropriate. For example, an analysis of a data set can be performed using one set of weights to highlight and rank general risks of genotoxicity. A differing set of weights can then be used to elucidate the potential for specific genotoxic effects (i.e., such as but not limited to insertional mutagenesis) and a third set of weights can then be used to indicate a propensity to chromothrypsis.

In many cases, the general form of the variant score $S$ will be reduced to a simple equation based on the specific research, clinical or other goals. Individual variant score $S_i$, such as for example for inversions in a cell population, has the form of the following formula (Formula 4):

$$S_i = \left(W_i W_f\right)\llbracket (P)\rrbracket_i{}^{Xm} \qquad (2)$$

In the common case where there are no additional multiplicative or exponential effects to consider $W_f$ and $X_m$ are 1 and the individual variant score would reduce to the following formula (Formula 5):

$$S_n = W_n P_n \qquad (3)$$

In a non-limiting example, in the case of biodosimetry, where the effect of ionizing radiation dosage can be calibrated using specific types of chromosomal aberrations, the general formula for $S_t$ can be reduced to the following formula (Formula 6):

$$S_t = W_i P_t + W_t P_t + W_{dC} P_{dC} \qquad (4)$$

where $I$ indicates inversions, $t$ indicates translocations and $dC$ indicates di-centrics, which are three types of variations that have been shown to arise from exposure of cell populations to ionizing radiation. Depending upon the specific situation, other types of variation can be included or ignored (i.e., their weight set to zero).

For instance, in this formulation of a score $S_t$ a biodosimetry data set, the relative decay rates of inversion, translocations and di-centrics may be used to set relative weights. For example, $W_i$ can be set to 1 (i.e., slowest decay rate), $W_t$ can be set to three (i.e., decaying in a of a matter of years) and $W_{dc}$ can be set to 10 (i.e., decays over a matter of months). In this example, then a relatively higher score for the dicentric term indicates a near term exposure to ionizing radiation, the total score for any one population of cells indicates the relative damage at the time of the measurement, while at the same time the total variance from time point to time point indicates the total difference between cell populations in damage at the time of the measurement. Alternative weighting schemes can easily be designed to meet the additional and/or alternative goals of the analysis of a biodosimetry data set.

In a non-limiting example, small random inversions (e.g., those between 2 kb and less than 5 mb) only detectable in cell populations by dGH, can act to knockdown or knockout genes and thus can pose a significant genotoxicity or oncogenic risk. Thus, a weighting scheme that emphasizes this risk can be appropriate for a study investigating previously cryptic initiators of radiation induced diseases. For example, the $S_n$ terms and the summation term $S_t$ provide useful information about a specific cell population's deviation from the reference genome. And, in of themselves, properly weighted scores can be used to provide a measure of genotoxicity risk, instability, oncogenesis, DNA damage and other factors. The power of this approach, particularly when combined with the unique data from dGH measurements is compounded when combined with various variance calculations to show the relative differences between cell populations.

Variances of the score $S_t$, is applicable to any variable that captures the differences between two or more) cell populations. However, of particular note are variances in $S_t$ between the following types of cells:

- Wild type and edited cell populations, where the variance in the score $S_t$ can be indicative of relative editing efficiency, relative genotoxicity risk, or decreased genomic stability.
- Different passages of the same cell culture in longitudinal studies of cell populations as they grow and expand where the variance in the score $S_t$ can be indicative of decreased genomic stability, clonal outgrowth, or differentiation, for instance of IPSCs.
- Cells of different types from the same original donor where the variance in the score $S_t$ can be indicative of can be indicative of localized disease states (e.g., tumors, Alzheimer's), localized DNA damage, aging, smoking history, cancer risk, and the like.
- Cell lines prepared by different methods where the where the variance in the score $S_t$ can be indicative of relative instability arising, for instance from different immortalization techniques.

## MEASURING INTEGRATION EVENTS

In some aspects, provided herein are methods for detecting and in illustrative embodiments, counting, measuring, or determining an insert number of a recombinant nucleic acid insert in a population of genetically-modified cells. Such aspects can include the following steps:

a) performing a directional genomic hybridization (dGH) reaction by contacting one or more pairs of single-stranded sister chromatids on cells from the population with a dGH insert probe that binds to the recombinant nucleic acid insert in metaphase spreads prepared from each of the cells, wherein the insert is integrated into the genome of the genetically-modified cells; and
b) measuring an intensity of a fluorescence signal generated by the dGH insert probe in the metaphase spreads using fluorescence microscopy.

Such methods provided herein for determining and/or measuring copy number of an insert also called integration events, provide clean integration data, even from complex or heterogeneous cell populations. Furthermore, in illustrative embodiments such methods provide a unique, an in illustrative embodiments whole genome, orthogonal method of direct visualization of inserts, without bioinformatic prediction of outcomes. Furthermore, multi-channel fluorescence can be used for flexible and multiplex assay design. Such methods, like all or virtually all methods herein can be performed on human, murine, canine, non-human primate and CHO cells.

Accordingly, in another aspect, provided herein is a method of determining an insert number of a recombinant nucleic acid insert at each insertion loci (sometimes referred to herein as integration events) in two or more genetically-modified cells of a population of genetically-modified cells, the method comprising the steps of:

a) performing a directional genomic hybridization (dGH) reaction on metaphase spreads prepared from each of the two or more cells by contacting one or more pairs of single-stranded sister chromatids from the two or more cells with a calibrant ladder dGH probe set and a dGH insert probe that binds a dGH insert probe target DNA sequence on the recombinant nucleic acid insert, wherein the recombinant nucleic acid insert is integrated into the genome of the genetically-modified cells, and wherein each dGH calibrant probe of the calibrant ladder dGH probe set binds a single-stranded sister chromatid of the one or more pairs, at a different target DNA sequence of a known size of a set of known sizes that encompass the size of the one or more dGH insert probe target DNA sequences of the recombinant nucleic acid insert; and
b) measuring a fluorescence intensity of a fluorescence signal at each calibrant loci on the one or more pairs of single-stranded sister chromatids bound by each dGH calibrant probe and each insert loci on the one or more pairs of single-stranded sister chromatids bound by the dGH insert probe, in the metaphase spreads using fluorescence microscopy; and

c) comparing the measured fluorescence intensity of the fluorescence signal generated for two or more of the calibrant loci each bound by one of the dGH calibrant probes to the measured fluorescence intensity of the fluorescence signal generated for each insertion loci bound by the dGH insert probe, to determine the insert number at each insertion loci of the recombinant nucleic acid insert in the two or more genetically-modified cells.

In any of the embodiments herein that include a calibrant ladder dGH probe set, the calibrant ladder dGH probe set can bind to one single-stranded sister chromatid. In some embodiments, the calibrant ladder dGH probe set can include one calibrant ladder dGH probe, also referred to herein as a single point internal calibrant. In some embodiments, the calibrant ladder dGH probe set can include 2, 3, 4, 5, 6, 7, 8, 9, 10, 11, 12, 13, 14, 15, 16, 17, 18, 19, or 20 calibrant ladder dGH probes.

In some embodiments, the comparing comprises using the fluorescence signals generated at the two or more calibrant loci and the sizes of the corresponding calibrant target DNA sequences to generate a standard curve and comparing the fluorescence signal generated at each insertion loci to the standard curve taking into account the size of the dGH insert probe target DNA sequence.

In some embodiments, the comparing comprises using the fluorescence signals generated at the two or more calibrant loci and the sizes of the corresponding calibrant target DNA sequences, and the fluorescence signal generated at each insertion loci to the standard curve taking into account the size of the dGH insert probe target DNA sequence, using Formula 11 herein.

In embodiments disclosed herein, the estimation of copy number per integration event can be evaluated in cell samples for the effects of recombinant nucleic acid insert integration on genotoxicity. In some embodiments, the copy number for each integration event present in genetically-modified cell lines can be estimated using the following formulae on a per metaphase spread basis, and assuming these numbers come from the same cell, a "Sum Intensity" (I) can be calculated by the following formula:

$$\mathbf{I = S \times A} \qquad\qquad (77)$$

wherein the units are RFU/px$^2$, "Signal" S = count of electrons/px (in RFU/px, arbitrary units), px = pixels, and A = area (px).

In some embodiments disclosed herein, the "Control Normalization Constant" ($N_{cnc}$) is the ratio of Fluorescence between Control and full size Target (Formula 8). In some embodiments, the $I_{insert}$ (full) is calculated using Formula 9. In embodiments, the "Size in kb" of the insert, ($K_{insert}(m)$), can be calculated using Formula 10, and the "Insert Copy Number" (ICN), can be calculated using Formula 11:

$$N_{cnc} = F_{insert} \,/\, F_{control} \qquad\qquad (8)$$

$$I_{insert}\,(full) = I_{control} \times F_{insert} = N_{cnc} \times I_{control}\ \mathrm{RFU/px} \qquad\qquad (9)$$

$$\text{``Size in kb''}\ K_{insert}(m) = I_{insert}(m) \,/\, I_{insert}(full) \times K(full) \Rightarrow K_{insert}(m) - \mathrm{RFU/RFU}/K(full) \qquad (10)$$

$$\mathbf{ICN_e = I_{insert}(m) \,/\, I_{insert}(full)} \qquad\qquad (11)$$

In embodiments herein, Formula 11 can correct for the difference in size and coverage of a single point internal calibrant dGH probe, where, in some embodiments, can be 90 oligos/23 kb, and a dGH insert probe, where in some embodiments, can be 76 oligos/10 kb. In some embodiments, the single point internal calibrant dGH probe and the dGH insert probe are labeled using the same fluorescent moiety, excited by the same wavelengths of light, and detected by the same detection method, and thus, in such embodiments, no additional correction factors are required.

In another aspect, provided here in a method for preparing a plurality of metaphase spreads from a test population of genetically-modified cells useful for determining an insert number of a recombinant nucleic acid insert at each insertion loci in two or more genetically-modified cells of the population of genetically-modified cells, comprising:

a) obtaining the test population of genetically-modified cells;
b) treating genetically-modified cells in the test population of genetically-modified cells with a metaphase-arresting composition;
c) preparing the plurality of metaphase spreads comprising one or more single-stranded sister chromatids;
d) performing a directional genomic hybridization (dGH) reaction on at least two of the plurality of metaphase spreads to label one or both single-stranded sister chromatids of a pair of single-stranded sister chromatids, wherein the dGH reaction comprises contacting the one or more pairs of single-stranded sister chromatids with a calibrant ladder dGH probe set and a dGH insert probe that binds a dGH insert probe target DNA sequence on the recombinant nucleic acid insert;

e) analyzing at least one pair of the labeled single-stranded sister chromatids in four or more metaphase spreads, wherein the analyzing comprises detecting the dGH calibrant probe and each insert loci on the one or more pairs of single-stranded sister chromatids bound by the dGH insert probe; and

f) determining the insert number of a recombinant nucleic acid insert at each insertion loci in two or more genetically-modified cells of the population of genetically-modified cells using the dGH calibrant probe.

Thus, using the above methods, and similar methods employing similar strategies insert copy number can be calculated. Such methods such as those provided immediately above, can be considered methods for measuring integration events, wherein the following can be measured:

Integrational copy number per cell and integrational copy number per event;

Integrational copy number per chromosome, arm of chromosome, band location (g-band, dGH band, Alu band); and

Integrational copy number per defined loci, which includes validation of the negative, i.e., an integrational copy number of zero at a genotoxic site.

In relating these to Genotoxicity and to weighting and scoring, it is important to consider the following considerations: Because random insertions can hit any loci and cannot be located precisely in every cell, there is a desire to moderate the risk that a random insertion will be genotoxic by limiting the number allowable insertions per cell. So, in the case of 10 genotoxic targets in a genome, you would have approximately 1/300.000,000 chance per insertion of hitting a genotoxic site. Some art suggests that 5 is an acceptable per cell average Insertional Copy Number (ICN) for lentiviral insertions, for instance. However, that is based on an average using methods that do not measure insertions of individual cells, such as next-generation sequencing and droplet digital PCR.

Methods provided herein however, in illustrative embodiments those using exponential terms, can move from these fuzzy risks based on odds using prior art methods, to weighting random insertions at known genotoxic sites higher than random insertions in the bulk of the genome. Weights can be assigned for up to 3 billion different possible insertion sites. However, in illustrative embodiments weights can be assigned to 100-500 or 100-300 different possible insertion sites to provide an informative risk model.

In certain illustrative embodiments, a sub-clonal outgrowth or population term/factor can be used as a weighted factor, but in illustrative embodiments, sub-clonal outgrowth is used as an exponent term. Thus, one insertion at a genotoxic site would get a weight above 1, for example between 5 and 20 or as a non-limiting example, a weight of 10. Thus, a factor for a sub population containing the insert at a genotoxic site in some embodiments can be calculated with the following formula:

$$S = W_{genotoxicinsert}(P_{genotoxicinsert}) \text{ X subclonal} \tag{12}$$

S = 10 * 1^1 = 10. For the case of a single hit to the genotoxic site

S = 10 * 100^2 = 100,000 For the case where the genotoxic sub-clonal population has taken over half the culture (100 of 200 cells).

If an even stronger signal of genotoxicity is desired, a larger exponent can be assigned to sub-clonal population outgrowth. For example, such methods can detect a sub-clonal outgrowth phenomenon early when it is less apparent. Thus, a non-limiting specific exemplary formula could be the following:

$$S = 10 * 5 \wedge 7 = 781,250$$

Other components of a total genotoxicity risk analysis or other instability score in illustrative can be included in the analysis as well. A similar strategy could be applied for mosaics, pre-existing variants, or any other factor that a particular context indicates is very important/relevant. In some embodiments, the above embodiments, considerations and formula can be applied to genetically-modified cells that have been genetically modified using a lentiviral vector or one or more transposons, since integration events using these technologies is believed to be random. However, in other embodiments the considerations, formula and embodiments above apply in the negative to any technique that is targeted. Such methods could be used, for example to support that a perfectly targeted insertion method has no such risk. The embodiments, considerations, and formula also apply to anything that might have some random integration characteristics such as methods that employ an AAV vector.

In some embodiments, on-target and off-target events in gene editing processes can be detected and measured using methods herein, including in certain embodiments, those provided in this section. Accordingly, in some embodiments, the population of genetically-modified cells are a population of genome-edited cells. In illustrative embodiments of such methods, the method can further comprise including a bystander dGH probe in the dGH reaction, wherein the bystander dGH probe recognizes a DNA binding sequence at an intended target genome editing loci. The bystander dGH probe may also be referred to herein as a babysitter probe, marker probe, loci-marker probe, bracketing probe, or target probe. In some embodiments, which in illustrative embodiments are subembodiments wherein the cells are genome-edited cells, an off-target insert number and an on-target insert number are analyzed and/or calculated. Such analysis and calculation can be done for example, by comparing the location of a fluorescent signal on the one or more pairs of single-stranded sister chromatids generated by the bystander dGH probe to location of the fluorescence signal generated at each insertion loci. The bystander dGH probe can be used to determine which of the sister chromatids of a pair of sister chromatids the insert has inserted. Such methods can be used to analyze cells whose genomes were edited by any gene editing technology including for example, CRISPR, TALENs, and zinc fingers.

In some embodiments, the recombinant nucleic acid insert can encode a transgene. In some embodiments, the recombinant nucleic acid insert can encode a chimeric antigen receptor. In some embodiments, the recombinant nucleic acid insert can encode a recombinant T cell receptor.

Methods herein for determining an insert number can be useful in assessing the safety and effectiveness of compositions and methods used to produce genetically-modified cells whose genome comprises the one or more copies of the insert, which provide separate aspects herein. Thus, such methods can be used to compare the results of different compositions and methods used to produce different populations of genetically-modified cells by comparing the genotoxicity risk or score or other instability score between the different populations determined using a method provided herein. Thus, methods for determining an insert number or methods of assessing the safety and effectiveness of compositions and methods used to produce genetically-modified cells, can include any or all of the steps, and in certain embodiments, are combined with methods herein for determining chromosomal stability of a population of cells, and in illustrative embodiments are combined with methods for determining genotoxicity of a population of cells. The population of cells in such examples is typically a genetically-modified population of cells. In some embodiments, the compositions used to produce different populations of genetically-modified cells can include any gene editing technology, for example, CRISPR, TALENs, and/or zinc fingers and/or nucleic acids encoding any gene editing technology. In some embodiments, the compositions can include any vector known in the art. In some embodiments, the compositions can include lentiviruses, transposons, or adenovirus associated vector (AAV). In some embodiments, the methods used to produce different populations of genetically-modified cells can include any method known in the art for genetically modifying cells, for example, transfection or transduction. In some embodiments, the methods can include microinjection, gene gun, or electroporation of cells. In some embodiments, the methods can include any vector known to transduce cells, for example, a lentiviral vector or adenovirus associated vector (AAV). In some embodiments, the methods can include transposons and/or transposases or nucleic acids encoding transposases.

Thus, since cellular reagent delivery can result in unintended genomic consequences, methods herein provide affective methods for detecting and quantifying structural damage associated with experimental delivery conditions. As indicated herein, such methods can include detection of on-target/off-target integrations.

## NON-LIMITING EXEMPLARY CELL TYPES AND WEIGHTING

As indicated herein, weighting of types of chromosomal variants and/or events and other characteristics, and which types are considered and weighted more heavily, are typically set based on the type of cells of a population of cells being analyzed, or other characteristics and knowledge regarding the population of cells and the context for the analysis, for example. Provided in subsections below, are some non-limiting examples for some specific cell types.

Neurodegenerative Diseases and Cell Types and Weighting

In some embodiments, significant chromosome rearrangements can trigger a higher genotoxic risk score, such as described in the following exemplary embodiments. As indicated herein, weighting of types of chromosomal variants and/or events and other characteristics are typically set based on the type of cells of a population of cells being analyzed, or other characteristics and knowledge regarding the population of cells and the context for the analysis, for example.

In an example of chromosome structural changes and/or repair event that can trigger a higher genotoxic risk score, in some embodiments, the test cell population is derived from a patient suffering from, or suspected to be suffering from, or being tested for a neurodegenerative disease or disorder. For example, in some embodiments, the patient, from whom the test cells are derived, such as but not limited blood cells or neuronal cells, suffers from, or is suspected of suffering from, or is being tested for a neurodegenerative disease/disorder such as but not limited to schizophrenia, Alzheimer's, autism or epilepsy. In some embodiments, the patient, from whom the test cells are derived, such as but not limited blood cells or neuronal cells, suffers from, or is suspected to be suffering from, or is being tested for a different neurodegenerative disease or disorder. In some embodiments, the test cells are a population of blood cells. In some embodiments, the blood cells are cultured blood cells. In an illustrative embodiment, the test cell population is a population of neural cells. For example, in some embodiments, the neural cells are a population of cells from a neural cell line. In another exemplary embodiment, the neural cells are a population of cells from a culture of primary neural cells, such as but not limited to primary neural cells grown in a 2-D neural cell culture or grown in a 3-D neural organoid. The population of neural cells can include, in some embodiments for example, a population of neural cells that are cultured primary cells isolated from the central nervous system, or cell lines arising therefrom. In some illustrative embodiments, the population of test cells are neurons, astrocytes, oligodendrocytes, and/or neural endothelial cells, such as brain endothelial cells. In some embodiments, the test cells derived from the patient are non-blood cells or non-neuronal cells. For example, in some embodiments, the population of test cells are muscle cells, skin cells, cells from the digestive tract, or other cells from the patient. In some embodiments, the population of test cells are cultured muscle cells, skin cells, cells from the digestive tract, or other cells from the patient. In certain illustrative embodiments, chromosomal rearrangements of high risk loci that lead to a neurodegenerative disease/disorder are weighted greater than 1.0 such as any of the values and ranges immediately above, or in illustrative examples of 1.0x, 1.1x, 1.5x, 2.0x, 2.5x, 3.0x, 4.0x, 5.0x, 6.0x, 7.0x, 8.0x, 9.0x, 10.0x, 11.0x, 12.0x, 13.0x, 14.0x, 15.0x, 16.0x, 17.0x, 18.0x, 19.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, or greater, or between 1.5x and 2x, 3x, 4x, 5x, 10x, 15x, 20x, 25x, 30x, 40x, 50x, 75x, or 100x, or between 2x and 3x, 4x, 5x. 10x, 15x, 20x, 25x, 30x, 40x, 50x, 75x, or 100x, or between 10x and 1,000x or between 25x and 250x, between 50x and 150x, or between 90x and 110x, or between 0.5x and 1.5x, 0.9x and 1.1x, or 1.0x, or between 10x and 1,000x, 500x, 250x, 125x, or 100x, or between 25x and 250x, between 50x and 150x in a population of test cells. In illustrative certain embodiments, the populations of cells are an outgrowth of a chromosomally aberrant clone, such as but not limited to as is described elsewhere herein, wherein the genome of the cells are identical.

In illustrative embodiments, increased variance in the amount of somatic mosaicism, the number of deletions, copy number variation or other structural rearrangements in a test population of cells, such as but not limited blood cells, neural cells, and/or other types of cells, compared to a control population of cells suggests and/or is an indication of an increased genotoxicity risk of the test cell population. As such, some embodiments include an increased weight on the amount of somatic mosaicism, the number of deletions, copy number variation or other structural rearrangements detected, or the variance of the amount of somatic mosaicism, the number of deletions, copy number variation or other structural rearrangements detected in the test population compared to a control population of cells suggests and/or is an indication of an increased genotoxicity risk of the test cell population. By way of example only, in some embodiments, the amount of somatic mosaicism, the number of deletions, copy number variation or other structural rearrangements detected, or in illustrative embodiments, the variance of the amount of somatic mosaicism, the number of deletions, copy number variation or other structural rearrangements detected, in a population of test cells, such as but not limited to, in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the amount of somatic mosaicism, the number of deletions, copy number variation or other structural rearrangements detected, or in illustrative embodiments a variance of the amount of somatic mosaicism, the number of deletions, copy number variation or other structural rearrangements, in a population of test cells, such as but not limited to, in the population of cells, such as for example in a diploid or a complex genomic cell population, such as but not limited to a population of neural cells is weighted by a factor of between 10x and 1,000x, 500x, 250x, 125x, or 100x, or between 25x and 250x, between 50x and 150x, or between 90x and 110x. In illustrative certain embodiments, the populations of cells are an outgrowth of a chromosomally aberrant clone, such as but not limited to as is described elsewhere herein, wherein the genome of the cells are identical.

In certain embodiments, the amount of somatic mosaicism, the number of deletions, copy number variation, and/or structural rearrangements of chromosome 2q31.2 (PRKRA gene) are weighted greater than 1.0 such as any of the values and ranges immediately above, or in illustrative examples between 10x and 1,000x, 500x, 250x, 125x, or 100x, or between 25x and 250x, between 50x and 150x, or between 90x and 110x in a population of blood and/or neuronal cells. In certain embodiments, the amount of somatic mosaicism, the number of deletions, copy number variation and/or structural rearrangements of chromosome 5q35.2 (BOD1 gene) are weighted greater than 1.0 such as any of the values and ranges immediately above, or in illustrative examples between 10x and 1,000x, 500x, 250x, 125x, or 100x, or between 25x and 250x, between 50x and 150x, or between 90x and 110x in a population of blood and/or neuronal cells, or another type of cells. In certain embodiments, the amount of somatic mosaicism, the number of deletions, copy number variation and/or structural rearrangements of chromosome 7p15.2 (CBX3 gene) are weighted greater than 1.0 such as any of the values and ranges immediately above, or in illustrative examples between 10x and 1,000x, 500x, 250x, 125x, or 100x, or between 25x and 250x, between 50x and 150x, or between 90x and 110x in a population of blood and/or neuronal cells, or in another type of cells. In certain embodiments, the amount of somatic mosaicism, the number of deletions, copy number variation and/or structural rearrangements of the for the chromosomal locus of NEGRI1 gene, the PTBP2 gene, the CADPS gene, KMT2 gene, the E KCNN2 gene, the MACROD2 gene, the MMP12 gene, the NTM gene, the ANTXRL gene, the CHST9 gene, the DNM3 gene, the NDST3 gene, the SDK1 gene, the STRC gene, the SKY gene, the SCN1A gene, the SCN2A gene, the SETD2 gene, the ARID1B gene, the AKT1 gene, the AKT3 gene, the MTOR gene, the PIK3CA gene, the TSC1 gene, the TSC2 gene, the mTOR gene, the PI3K-Akt gene, the p53, and/or the PTEN gene are weighted greater than 1.0 such as any of the values and ranges immediately above, or in illustrative examples between 10x and 1,000x, 500x, 250x, 125x, or 100x, or between 25x and 250x, between 50x and 150x, or between 90x and 110x in a population of blood and/or neuronal cells, or in another type of cells. In certain embodiments, the amount of somatic mosaicism, the number of deletions, copy number variation and/or structural rearrangements of a cluster of genes on chromosome 17, especially cluster that includes one, two, three, or in illustrative embodiments all of the KANSL1, WNT3, MAPT and CRHRI genes, such as for example in a population of blood and/or neuronal cells, or in another type of cells, are weighted greater than 1.0 such as any of the values and ranges immediately above, or in illustrative examples between 10x and 1,000x, 500x, 250x, 125x, or 100x, or between 25x and 250x, between 50x and 150x, or between 90x and 110x in a population of blood and/or neuronal cells or in another type of cells. In illustrative certain embodiments, the populations of cells are an outgrowth of a chromosomally aberrant clone, such as but not limited to as is described elsewhere herein, wherein the genome of the cells are identical.

In another example, in some embodiments, an increased number of instability markers, such as but not limited to general genome hyperploidy, in a test cell population, such as but not limited to a population of blood or neuronal cells, or another type of cells, in illustrative embodiments relative to the control cell population or reference and/or other cell populations, suggests or otherwise provides an indication of decreased chromosomal stability (increased chromosomal instability). In illustrative embodiments, in increased variance in the number of instability markers, such as but not limited to general genome hyperploidy, in a test population of cells, such as but not limited to a population of blood or neuronal cells, or another type of cells, compared to a control population of cells suggests and/or is an indication of an increased genotoxicity risk of the test cell population. As such, some embodiments include an increased weight on the number of instability markers, such as but not limited to general genome hyperploidy, detected, or the variance of the number of instability markers, such as but not limited to general genome hyperploidy, detected in the test population, such as but not limited to a population of blood or neuronal cells, or another type of cells, compared to a control population of cells suggests and/or is an indication of an increased genotoxicity risk of the test cell population. By way of example only, in some embodiments, the number of instability markers, such as but not limited to general genome hyperploidy, detected, or in illustrative embodiments, the variance of the number of instability markers, such as but not limited to general genome hyperploidy, detected, in a population of test cells, such as but not limited to, in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the number of instability markers, such as but not limited to general genome hyperploidy, detected, or in illustrative embodiments a variance of the number of instability markers, such as but not limited to general genome hyperploidy, detected, in a population of test cells, such as but not limited to, in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x.

In another example, in some embodiments, an increased number of instability markers, such as but not limited to aneuploidy of any one of chromosomes 12, 18, 21 or X, in a test cell population, such as but not limited to a population of blood or neuronal cells, or another type of cells, in illustrative embodiments relative to the control cell population or reference and/or other cell populations, suggests or otherwise provides an indication of decreased chromosomal stability (increased chromosomal instability). In illustrative embodiments, in increased variance in the number of instability markers, such as but not limited to aneuploidy of any one of chromosomes 12, 18, 21 or X, in a test population of cells, such as but not limited to a population of blood or neuronal cells, or another type of cells, compared to a control population of cells suggests and/or is an indication of an increased genotoxicity risk of the test cell population. As such, some embodiments include an increased weight on the number of instability markers, such as but not limited to aneuploidy of any one of chromosomes 12, 18, 21 or X, detected, or the variance of the number of instability markers, such as but not limited to aneuploidy of any one of 12, 18, 21 or X, detected in the test population of cells, such as but not limited to a population of blood or neuronal cells, or another type of cells, compared to a control population of cells suggests and/or is an indication of an increased genotoxicity risk of the test cell population. By way of example only, in some embodiments, the number of instability markers, such as but not limited to aneuploidy of any one of chromosomes 12, 18, 21 or X, detected, or in illustrative embodiments, the variance of the number of instability markers, such as but not limited to aneuploidy of any one of chromosomes 12, 18, 21 or X, detected, in a population of test cells, such as but not limited to, in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the number of instability markers, such as but not limited to any one of chromosomes 12, 18, 21 or X, detected, or in illustrative embodiments a variance of number of instability markers, such as but not limited to aneuploidy of any one of chromosomes 12, 18, 21 or X, detected, in a population of test cells, such as but not limited to, in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x. In illustrative certain embodiments, the populations of cells are an outgrowth of a chromosomally aberrant clone, such as but not limited to as is described elsewhere herein, wherein the genome of the cells are identical.

In another example, in some embodiments, an increased number of micronuclei, or micronuclei structures, in a test cell population, such as but not limited to a population of blood cells or neuronal cells, or another type of cells, in illustrative embodiments relative to the control cell population or reference and/or other cell populations, suggests or otherwise provides an indication of decreased chromosomal stability (increased chromosomal instability). In illustrative embodiments, in increased variance in the number of micronuclei in a test population of cells, such as but not limited to blood cells or neuronal cells, or another type of cells, compared to a control population of cells suggests and/or is an indication of an increased genotoxicity risk of the test cell population. As such, some embodiments include an increased weight on the number of micronuclei detected, or the variance of the number of micronuclei detected in the test population of cells, such as but not limited to a population of blood or neuronal cells, or another type of cells, compared to a control population of cells suggests and/or is an indication of an increased genotoxicity risk of the test cell population. By way of example only, in some embodiments, the number of micronuclei detected, or in illustrative embodiments, the variance of the number of micronuclei detected, in a population of test cells, such as but not limited to, in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the number of micronuclei detected, or in illustrative embodiments a variance of the number of micronuclei detected, in a population of test cells, such as but not limited to, in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x. In illustrative certain embodiments, the populations of cells are an outgrowth of a chromosomally aberrant clone, such as but not limited to as is described elsewhere herein, wherein the genome of the cells are identical.

In another example, in some embodiments, an increased number/amount of somatic mosaicism, deletions, copy number variation or other structural rearrangements involving any one of chromosome 21 (21q21.3), PS1, PS2, MAPT, APP, NPC1, and/or the SNCA genes, in a test cell population, such as but not limited to a population of blood cells or neuronal cells, or another type of cell, in illustrative embodiments relative to the control cell population or reference and/or other cell populations, suggests or otherwise provides an indication of decreased chromosomal stability (increased chromosomal instability). In illustrative embodiments, in increased variance in the number/amount of somatic mosaicism, deletions, copy number variation or other structural rearrangements involving any one of chromosome 21 (21q21.3), PS1, PS2, MAPT, APP, NPC1, and/or the SNCA genes in a test population of cells, such as but not limited to blood cells or neuronal cells, or another type of cell, compared to a control population of cells suggests and/or is an indication of an increased genotoxicity risk of the test cell population. As such, some embodiments include an increased weight on the number/amount of somatic mosaicism, deletions, copy number variation or other structural rearrangements involving any one of chromosome 21 (21q21.3), PS1, PS2, MAPT, APP, NPC1, and/or the SNCA genes detected, or the variance of the number/amount of somatic mosaicism, deletions, copy number variation or other structural rearrangements involving any one of chromosome 21 (21q21.3), PS1, PS2, MAPT, APP, NPC1, and/or the SNCA genes detected in the test population of cells, such as but not limited to blood cells or neuronal cells, or another type of cell, compared to a control population of cells suggests and/or is an indication of an increased genotoxicity risk of the test cell population. By way of example only, in some embodiments, the number/amount of somatic mosaicism, deletions, copy number variation or other structural rearrangements involving any one of chromosome 21 (21q21.3), PS1, PS2, MAPT, APP, NPC1, and/or the SNCA genes detected, or in illustrative embodiments, the variance of the number of number/amount of somatic mosaicism, deletions, copy number variation or other structural rearrangements involving any one of chromosome 21 (21q21.3), PS1, PS2, MAPT, APP, NPC1, and/or the SNCA genes detected, in a population of test cells, such as but not limited to a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the number/amount of somatic mosaicism, deletions, copy number variation or other structural rearrangements involving any one of chromosome 21 (21q21.3), PS1, PS2, MAPT, APP, NPC1, and/or the SNCA genes detected, or in illustrative embodiments a variance of the number/amount of somatic mosaicism, deletions, copy number variation or other structural rearrangements involving any one of chromosome 21 (21q21.3), PS1, PS2, MAPT, APP, NPC1, and/or the SNCA genes detected, in a population of test cells, such as but not limited to the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x. In illustrative certain embodiments, the populations of cells are an outgrowth of a chromosomally aberrant clone, such as but not limited to as is described elsewhere herein, wherein the genome of the cells are identical.

CAR-T Cells and Cell Types and Weighting

Due to the nature of the methods by which CAR-T cells are created, CAR-T cells are subject to an increased instability risk, which in turn creates a risk that CAR-T cells can present safety issues in patients receiving these therapies. For example, significant chromosomal rearrangements have been observed in CAR-T based therapies. Such chromosomal rearrangements can trigger a higher genotoxic risk for these therapeutics. Markers, or high risk loci, are known to be associated with such chromosomal variants, whether due to repair events or not. For example, markers are known for Angioimmunoblastic T-cell lymphoma (AILT), peripheral T-cell lymphoma unspecified (PTCL-u) and other lymphomas. These markers can be evaluated during creation of the CAR-T based therapies and then at later time points after a patient has received the therapy, so as to evaluate the risk of the patient developing cancer due to the therapy received.

In an illustrative example, in some embodiments, markers of increased genotoxic risk include, but are not limited to a gain of chromosomes 22q, 19, and/or 11p11-q14 (11q13). An additional marker of increased genotoxic risk includes, but is not limited to, loss of chromosome 13q. Accordingly, in CAR-T cells and cells from patients treated with CAR-T based therapies, chromosomal loci 22q, 19, 11p11-q14 (11q13) and 13q are considered to be High-risk loci. Thus, in some exemplary embodiments, gains of chromosomes 22q, 19, and 11p11-q14 (11q13) or losses of chromosome 13q, in a test cell population, such as but not limited to a population of CAR-T cells or cells from a patient treated with a CAR-T based therapy, in illustrative embodiments relative to the control cell population or reference and/or other cell populations, suggests or otherwise provides an indication of decreased chromosomal stability (increased chromosomal instability). In illustrative embodiments, increased variance in the gains of chromosomes 22q, 19, and 11p11-q14 (11q13) or losses of chromosome 13q, in a test cell population, such as but not limited to a population of CAR-T cells or cells from a patient treated with a CAR-T based therapy, compared to a control population of cells suggests and/or is an indication of an increased genotoxicity risk of the test cell population. As such, some embodiments include an increased weight on the gains of chromosomes 22q, 19, and 11p11-q14 (11q13) or losses of chromosome 13q, in a test cell population, such as but not limited to a population of CAR-T cells or cells from a patient treated with a CAR-T based therapy, detected in the test population of cells compared to a control population of cells suggests and/or is an indication of an increased genotoxicity risk of the test cell population. By way of example only, in some embodiments, the gains of chromosomes 22q, 19, and 11p11-q14 (11q13) or losses of chromosome 13q, or in illustrative embodiments, the variance gains of chromosomes 22q, 19, and 11p11-q14 (11q13) or the losses of chromosome 13q, in a population of test cells, such as but not limited to, in a population of CAR-T cells or cells from a patient treated with a CAR-T based therapy, or in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the gains of chromosomes 22q, 19, and 11p11-q14 (11q13) or losses of chromosome 13q detected, or in illustrative embodiments a variance of the gains of chromosomes 22q, 19, and 11p11-q14 (11q13) or losses of chromosome 13q detected, in a population of test cells, such as but not limited to, in a population of CAR-T cells or cells from a patient treated with a CAR-T based therapy, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x. In illustrative certain embodiments, the populations of cells are an outgrowth of a chromosomally aberrant clone, such as but not limited to as is described elsewhere herein, wherein the genome of the cells are identical.

In another illustrative example, in some embodiments, markers of increased genotoxic risk, in CAR-T cells and/or patients that have received CAR-T based therapies, include, but are not limited to gains of high risk loci on chromosome 17 (17q11-q25), chromosome 8 (involving the MYC locus at 8q24), chromosome 22q, chromosome 4q (4q28-q31 and 4q34-qtel), chromosome 11q13, and chromosome 17. In another illustrative example, in some embodiments, markers of increased genotoxic risk, in CAR-T cells and/or patients that have received CAR-T based therapies, include, but are not limited to loss of chromosome 13q, chromosome 6q (6q16-q22), chromosome 11p11, and chromosome 9 (9p21-q33). Accordingly, in some exemplary embodiments, gains of one of chromosomes 17 (17q11-q25), 8 (8q24), 22q, 4q (4q28-q31 and 4q34-qtel), 11q13, and 17, and losses of one of chromosomes 13q, 6q (6q16-q22), 11p11, and 9 (9p21-q33), in a test cell population, such as but not limited to a population of CAR-T cells or cells from a patient treated with a CAR-T based therapy, in illustrative embodiments relative to the control cell population or reference and/or other cell populations, suggests or otherwise provides an indication of decreased chromosomal stability (increased chromosomal instability). In illustrative embodiments, increased variance in the gains of one of chromosomes 17 (17q11-q25), 8 (8q24), 22q, 4q (4q28-q31 and 4q34-qtel), 11q13, and 17, and losses of one of chromosomes 13q, 6q (6q16-q22), 11p11, and 9 (9p21-q33), in a test cell population, such as but not limited to a population of CAR-T cells or cells from a patient treated with a CAR-T based therapy, compared to a control population of cells suggests and/or is an indication of an increased genotoxicity risk of the test cell population. As such, some embodiments include an increased weight on the gains of one of chromosomes 17 (17q11-q25), 8 (8q24), 22q, 4q (4q28-q31 and 4q34-qtel), 11q13, and 17, and losses of one of chromosomes 13q, 6q (6q16-q22), 11p11, and 9 (9p21-q33), in a test cell population, such as but not limited to a population of CAR-T cells or cells from a patient treated with a CAR-T based therapy, detected in the test population of cells compared to a control population of cells suggests and/or is an indication of an increased genotoxicity risk of the test cell population. By way of example only, in some embodiments, the gains of one of chromosomes 17 (17q11-q25), 8 (8q24), 22q, 4q (4q28-q31 and 4q34-qtel), 11q13, and 17, and losses of one of chromosomes 13q, 6q (6q16-q22), 11p11, and 9 (9p21-q33), or in illustrative embodiments, the variance in gains of one of chromosomes 17 (17q11-q25), 8 (8q24), 22q, 4q (4q28-q31 and 4q34-qtel), 11q13, and 17, and losses of one of chromosomes 13q, 6q (6q16-q22), 11p11, and 9 (9p21-q33), in a population of test cells, such as but not limited to, in a population of CAR-T cells or cells from a patient treated with a CAR-T based therapy, or in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the gains of one of chromosomes 17 (17q11-q25), 8 (8q24), 22q, 4q (4q28-q31 and 4q34-qtel), 11q13, and 17, and losses of one of chromosomes 13q, 6q (6q16-q22), 11p11, and 9 (9p21-q33) detected, or in illustrative embodiments a variance of the gains of one of chromosomes 17 (17q11-q25), 8 (8q24), 22q, 4q (4q28-q31 and 4q34-qtel), 11q13, and 17, and losses of one of chromosomes 13q, 6q (6q16-q22), 11p11, and 9 (9p21-q33) detected, in a population of test cells, such as but not limited to, in a population of CAR-T cells or cells from a patient treated with a CAR-T based therapy, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x. In illustrative certain embodiments, the populations of cells are an outgrowth of a chromosomally aberrant clone, such as but not limited to as is described elsewhere herein, wherein the genome of the cells are identical.

In another illustrative example, in some embodiments, markers of increased genotoxic risk, in CAR-T cells and/or patients that have received CAR-T based therapies, include, but are not limited to trisomy of chromosomes 3, 5 and/or 8. Accordingly, in some exemplary embodiments, trisomy of chromosomes 3, 5 and/or 8, in a test cell population, such as but not limited to a population of CAR-T cells or cells from a patient treated with a CAR-T based therapy, in illustrative embodiments relative to the control cell population or reference and/or other cell populations, suggests or otherwise provides an indication of decreased chromosomal stability (increased chromosomal instability). In illustrative embodiments, increased variance in trisomy of chromosomes 3, 5 and/or 8 in a test cell population, such as but not limited to a population of CAR-T cells or cells from a patient treated with a CAR-T based therapy, compared to a control population of cells suggests and/or is an indication of an increased genotoxicity risk of the test cell population. As such, some embodiments include an increased weight on trisomy of chromosomes 3, 5 and/or 8, in a test cell population, such as but not limited to a population of CAR-T cells or cells from a patient treated with a CAR-T based therapy, detected in the test population of cells compared to a control population of cells suggests and/or is an indication of an increased genotoxicity risk of the test cell population. By way of example only, in some embodiments, trisomy of chromosomes 3, 5 and/or 8, or in illustrative embodiments, the variance in trisomy of chromosomes 3, 5 and/or 8, in a population of test cells, such as but not limited to, in a population of CAR-T cells or cells from a patient treated with a CAR-T based therapy, or in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, trisomy of chromosomes 3, 5 and/or 8 detected, or in illustrative embodiments a variance of trisomy of chromosomes 3, 5 and/or 8 detected, in a population of test cells, such as but not limited to, in a population of CAR-T cells or cells from a patient treated with a CAR-T based therapy, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x. In illustrative certain embodiments, the populations of cells are an outgrowth of a chromosomally aberrant clone, such as but not limited to as is described elsewhere herein, wherein the genome of the cells are identical.

In another illustrative example, in some embodiments, markers of increased genotoxic risk, in CAR-T cells and/or patients that have received CAR-T based therapies, include, but are not limited to isochromosome 7q and monosomy of chromosome 7. Accordingly, in some exemplary embodiments, isochromosome 7q and monosomy of chromosome 7, in a test cell population, such as but not limited to a population of CAR-T cells or cells from a patient treated with a CAR-T based therapy, in illustrative embodiments relative to the control cell population or reference and/or other cell populations, suggests or otherwise provides an indication of decreased chromosomal stability (increased chromosomal instability). In illustrative embodiments, increased variance in the number of isochromosome 7q and monosomy of chromosome 7 in a test cell population, such as but not limited to a population of CAR-T cells or cells from a patient treated with a CAR-T based therapy, compared to a control population of cells suggests and/or is an indication of an increased genotoxicity risk of the test cell population. As such, some embodiments include an increased weight on the number of isochromosome 7q and monosomy of chromosome 7, in a test cell population, such as but not limited to a population of CAR-T cells or cells from a patient treated with a CAR-T based therapy, detected in the test population of cells compared to a control population of cells suggests and/or is an indication of an increased genotoxicity risk of the test cell population. By way of example only, in some embodiments, the number of isochromosome 7q and monosomy of chromosome 7, or in illustrative embodiments, the variance in the number of isochromosome 7q and monosomy of chromosome 7, in a population of test cells, such as but not limited to, in a population of CAR-T cells or cells from a patient treated with a CAR-T based therapy, or in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the number of isochromosome 7q and monosomy of chromosome 7 detected, or in illustrative embodiments a variance in the number of isochromosome 7q and monosomy of chromosome 7 detected, in a population of test cells, such as but not limited to, in a population of CAR-T cells or cells from a patient treated with a CAR-T based therapy, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x. In illustrative certain embodiments, the populations of cells are an outgrowth of a chromosomally aberrant clone, such as but not limited to as is described elsewhere herein, wherein the genome of the cells are identical.

In another illustrative example, in some embodiments, markers of increased genotoxic risk, in CAR-T cells and/or patients that have received CAR-T based therapies, include, but are not limited to disruption of known oncogenes and/or tumor suppressors that are associated with lymphomas. These disruptions include but are not limited to somatic mosaicism, deletions, copy number variation and/or structural rearrangements of these known oncogenes and/or tumor suppressor genes. In some embodiments, or in illustrative embodiments, the oncogenes and/or tumor suppressors include but are not limited to TCR, IRF4, ALK, NPM, TP53, EVI1, LMO1, LMO2, CDKN2A, SIL, TAL1, USP44, CRADD. Accordingly, in some exemplary embodiments, somatic mosaicism, deletions, copy number variation and/or structural rearrangements of an oncogene and/or tumor suppressor, such as but not limited to TCR, IRF4, ALK, NPM, TP53, EVI1, LMO1, LMO2, CDKN2A, SIL, TAL1, USP44, CRADD, in a test cell population, such as but not limited to a population of CAR-T cells or cells from a patient treated with a CAR-T based therapy, in illustrative embodiments relative to the control cell population or reference and/or other cell populations, suggests or otherwise provides an indication of decreased chromosomal stability (increased chromosomal instability). In illustrative embodiments, increased variance in somatic mosaicism, deletions, copy number variation and/or structural rearrangements of an oncogene and/or tumor suppressor, such as but not limited to TCR, IRF4, ALK, NPM, TP53, EVI1, LMO1, LMO2, CDKN2A, SIL, TAL1, USP44, CRADD, in a test cell population, such as but not limited to a population of CAR-T cells or cells from a patient treated with a CAR-T based therapy, compared to a control population of cells suggests and/or is an indication of an increased genotoxicity risk of the test cell population. As such, some embodiments include an increased weight on somatic mosaicism, deletions, copy number variation and/or structural rearrangements of an oncogene and/or tumor suppressor, such as but not limited to TCR, IRF4, ALK, NPM, TP53, EVI1, LMO1, LMO2, CDKN2A, SIL, TAL1, USP44, CRADD, in a test cell population, such as but not limited to a population of CAR-T cells or cells from a patient treated with a CAR-T based therapy, detected in the test population of cells compared to a control population of cells suggests and/or is an indication of an increased genotoxicity risk of the test cell population. By way of example only, in some embodiments, the number (or amount) of somatic mosaicism, deletions, copy number variation and/or structural rearrangements of an oncogene and/or tumor suppressor, such as but not limited to TCR, IRF4, ALK, NPM, TP53, EVI1, LMO1, LMO2, CDKN2A, SIL, TAL1, USP44, CRADD, in a population of test cells, such as but not limited to, in a population of CAR-T cells or cells from a patient treated with a CAR-T based therapy, or in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the number (or amount) of somatic mosaicism, deletions, copy number variation and/or structural rearrangements of an oncogene and/or tumor suppressor, such as but not limited to TCR, IRF4, ALK, NPM, TP53, EVI1, LMO1, LMO2, CDKN2A, SIL, TAL1, USP44, CRADD detected, or in illustrative embodiments a variance in the number (or amount) of somatic mosaicism, deletions, copy number variation and/or structural rearrangements of an oncogene and/or tumor suppressor, such as but not limited to TCR, IRF4, ALK, NPM, TP53, EVI1, LMO1, LMO2, CDKN2A, SIL, TAL1, USP44, CRADD detected, in a population of test cells, such as but not limited to, in a population of CAR-T cells or cells from a patient treated with a CAR-T based therapy, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x. In illustrative certain embodiments, the populations of cells are an outgrowth of a chromosomally aberrant clone, such as but not limited to as is described elsewhere herein, wherein the genome of the cells are identical.

In another illustrative example, in some embodiments, markers of increased genotoxic risk, in CAR-T cells and/or patients that have received CAR-T based therapies, include but are not limited to disruption of additional high risk loci on chromosomes 6p25.3, 17 and 14, by somatic mosaicism, deletions, copy number variation and other structural rearrangements, such as but not limited to translocations and inversions. Accordingly, in some exemplary embodiments, somatic mosaicism, deletions, copy number variation and/or structural rearrangements, such as but not limited to translocations and inversions, such as but not limited to at chromosomes 6p25.3, 17 and 14, in a test cell population, such as but not limited to a population of CAR-T cells or cells from a patient treated with a CAR-T based therapy, in illustrative embodiments relative to the control cell population or reference and/or other cell populations, suggests or otherwise provides an indication of decreased chromosomal stability (increased chromosomal instability). In illustrative embodiments, increased variance in somatic mosaicism, deletions, copy number variation and/or structural rearrangements, such as but not limited to translocations and inversions, such as but not limited to at chromosomes 6p25.3, 17 and 14, in a test cell population, such as but not limited to a population of CAR-T cells or cells from a patient treated with a CAR-T based therapy, compared to a control population of cells suggests and/or is an indication of an increased genotoxicity risk of the test cell population. As such, some embodiments include an increased weight on somatic mosaicism, deletions, copy number variation and/or structural rearrangements, such as but not limited to translocations and inversions, such as but not limited to at chromosomes 6p25.3, 17 and 14, in a test cell population, such as but not limited to a population of CAR-T cells or cells from a patient treated with a CAR-T based therapy, detected in the test population of cells compared to a control population of cells suggests and/or is an indication of an increased genotoxicity risk of the test cell population. By way of example only, in some embodiments, the number (or amount) of somatic mosaicism, deletions, copy number variation and/or structural rearrangements, such as but not limited to translocations and inversions, such as but not limited to at chromosomes 6p25.3, 17 and 14, in a population of test cells, such as but not limited to, in a population of CAR-T cells or cells from a patient treated with a CAR-T based therapy, or in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the number (or amount) of somatic mosaicism, deletions, copy number variation and/or structural rearrangements, such as but not limited to translocations and inversions, such as but not limited to at chromosomes 6p25.3, 17 and 14, detected, or in illustrative embodiments a variance in the number (or amount) of somatic mosaicism, deletions, copy number variation and/or structural rearrangements, such as but not limited to translocations and inversions, such as but not limited to a chromosomes 6p25.3, 17 and 14, detected, in a population of test cells, such as but not limited to, in a population of CAR-T cells or cells from a patient treated with a CAR-T based therapy, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x. In illustrative certain embodiments, the populations of cells are an outgrowth of a chromosomally aberrant clone, such as but not limited to as is described elsewhere herein, wherein the genome of the cells are identical.

Oncogenic Risks, and Cell Types and Weighting

In another non-limiting example, chromosome rearrangements of oncogenes and/or tumor suppressor genes and/or tumor activator genes, can trigger a higher genotoxic risk score, such as described in the following exemplary embodiments.

In another illustrative example of bladder cancer, in some embodiments, markers of increased genotoxic risk, such as for example in patients who have bladder cancer, who are suspected of having bladder cancer, and/or have or who are receiving therapy for bladder cancer, include, but are not limited to loss of heterozygosity (LOH) at one or more of chromosomes 11p, 3p, 13q, and 17p, or of another chromosome. Accordingly, in some exemplary embodiments, LOH of chromosome 11P, 3P, 13Q, 17p, or another chromosome, in a test cell population, such as but not limited to a population of bladder cells from a patient with bladder cancer, a patient suspected of having bladder cancer, or a patient receiving therapy for bladder cancer, or cultured cells from such a patient, in illustrative embodiments relative to the control or reference and/or other cell populations, suggests or otherwise provides an indication of decreased chromosomal stability (increased chromosomal instability). In illustrative embodiments, increased variance in LOH of chromosome 11P, 3P, 13Q, 17p, or another chromosome, in a test cell population, such as but not limited to a population of bladder cells from a patient with bladder cancer, a patient suspected of having bladder cancer, or a patient receiving therapy for bladder cancer, or cultured cells from such a patient, compared to a control population of cells suggests and/or is an indication of an increased genotoxicity risk of the test cell population. As such, some embodiments include an increased weight on LOH of chromosome 11P, 3P, 13Q, 17p, or another chromosome, in a test cell population, such as but not limited to a population of bladder cells from a patient with bladder cancer, a patient suspected of having bladder cancer, or a patient receiving therapy for bladder cancer, or cultured cells from such a patient, detected in the test population of cells compared to a control population of cells suggests and/or is an indication of an increased genotoxicity risk of the test cell population. By way of example only, in some embodiments, LOH of chromosome 11P, 3P, 13Q, 17p, or another chromosome, or in illustrative embodiments, the variance in LOH of chromosome 11P, 3P, 13Q, 17p, or another chromosome, in a population of test cells, such as but not limited to, in a population of bladder cells from a patient with bladder cancer, a patient suspected of having bladder cancer, or a patient receiving therapy for bladder cancer, or cultured cells from such a patient, or in a population of cells, such as for example in a diploid or a complex genomic cell population, Is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, LOH of chromosome 11P, 3P, 13Q, 17p, or another chromosome, detected, or in illustrative embodiments a variance of LOH of chromosome 11P, 3P, 13Q, 17p, or another chromosome, detected, in a population of test cells, such as but not limited to, in a population of bladder cells from a patient with bladder cancer, a patient suspected of having bladder cancer, or a patient receiving therapy for bladder cancer, or cultured cells from such a patient, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x. In certain illustrative embodiments, the cells of the patient with bladder cancer, a suspected of having bladder cancer, or receiving therapy for bladder cancer, or cultured cells from the patient are non-bladder cells from the patient. In illustrative certain embodiments, the populations of cells are an outgrowth of a chromosomally aberrant clone, such as but not limited to as is described elsewhere herein, wherein the genome of the cells are identical.

In another illustrative example of bladder cancer, in some embodiments, markers of increased genotoxic risk, such as for example in patients who have bladder cancer, who are suspected of having bladder cancer, and/or have or who are receiving therapy for bladder cancer, include, but are not limited to deletion of one or more of the 8p, 9p, 11p, 11q and Y chromosome arms, or another chromosome arm. Accordingly, in some exemplary embodiments, deletion of one or more of the 8p, 9p, 11p, 11q and Y chromosome arms, or another chromosome arm, in a test cell population, such as but not limited to a population of bladder cells from a patient with bladder cancer, a patient suspected of having bladder cancer, or a patient receiving therapy for bladder cancer, or cultured cells from such a patient, in illustrative embodiments relative to the control cell population or reference and/or other cell populations, suggests or otherwise provides an indication of decreased chromosomal stability (increased chromosomal instability). In illustrative embodiments, increased variance in deletion of one or more of the 8p, 9p, 11p, 11q and Y chromosome arms, or another chromosome arm, in a test cell population, such as but not limited to a population of bladder cells from a patient with bladder cancer, a patient suspected of having bladder cancer, or a patient receiving therapy for bladder cancer, or cultured cells from such a patient, compared to a control population of cells suggests and/or is an indication of an increased genotoxicity risk of the test cell population. As such, some embodiments include an increased weight on deletion of one or more of the 8p, 9p, 11p, 11q and Y chromosome arms, or another chromosome arm, in a test cell population, such as but not limited to a population of bladder cells from a patient with bladder cancer, a patient suspected of having bladder cancer, or a patient receiving therapy for bladder cancer, or cultured cells from such a patient, detected in the test population of cells compared to a control population of cells suggests and/or is an indication of an increased genotoxicity risk of the test cell population. By way of example only, in some embodiments, deletion of one or more of the 8p, 9p, 11p, 11q and Y chromosome arms, or another chromosome arm, or in illustrative embodiments, the variance in deletion of one or more of the 8p, 9p, 11p, 11q and Y chromosome arms, or another chromosome arm, in a population of test cells, such as but not limited to, in a population of bladder cells from a patient with bladder cancer, a patient suspected of having bladder cancer, or a patient receiving therapy for bladder cancer, or cultured cells from such a patient, or in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, deletion of one or more of the 8p, 9p, 11p, 11q and Y chromosome arms, or another chromosome arm, detected, or in illustrative embodiments a variance of deletion of one or more of the 8p, 9p, 11p, 11q and Y chromosome arms, or another chromosome arm, detected, in a population of test cells, such as but not limited to, in a population of bladder cells from a patient with bladder cancer, a patient suspected of having bladder cancer, or a patient receiving therapy for bladder cancer, or cultured cells from such a patient, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x. In certain illustrative embodiments, the cells of the patient with bladder cancer, a suspected of having bladder cancer, or receiving therapy for bladder cancer, or cultured cells from the patient are non-bladder cells from the patient. In illustrative certain embodiments, the populations of cells are an outgrowth of a chromosomally aberrant clone, such as but not limited to as is described elsewhere herein, wherein the genome of the cells are identical.

In another illustrative example of bladder cancer, in some embodiments, markers of increased genotoxic risk, such as for example in patients who have bladder cancer, who are suspected of having bladder cancer, and/or have or who are receiving therapy for bladder cancer, include, but are not limited to gain of one or more of 1q, 8q, 17q, and 20q chromosome arms, or other chromosome arms. Accordingly, in some exemplary embodiments, gain of one or more of 1q, 8q, 17q, and 20q chromosome arms, or other chromosome arms, in a test cell population, such as but not limited to a population of bladder cells from a patient with bladder cancer, a patient suspected of having bladder cancer, or a patient receiving therapy for bladder cancer, or cultured cells from such a patient, in illustrative embodiments relative to the control cell population or reference and/or other cell populations, suggests or otherwise provides an indication of decreased chromosomal stability (increased chromosomal instability). In illustrative embodiments, increased variance in gain of one or more of 1q, 8q, 17q, and 20q chromosome arms, or other chromosome arms, in a test cell population, such as but not limited to a population of bladder cells from a patient with bladder cancer, a patient suspected of having bladder cancer, or a patient receiving therapy for bladder cancer, or cultured cells from such a patient, compared to a control population of cells suggests and/or is an indication of an increased genotoxicity risk of the test cell population. As such, some embodiments include an increased weight on gain of one or more of 1q, 8q, 17q, and 20q chromosome arms, or other chromosome arms, in a test cell population, such as but not limited to a population of bladder cells from a patient with bladder cancer, a patient suspected of having bladder cancer, or a patient receiving therapy for bladder cancer, or cultured cells from such a patient, detected in the test population of cells compared to a control population of cells suggests and/or is an indication of an increased genotoxicity risk of the test cell population. By way of example only, in some embodiments, gain of one or more of 1q, 8q, 17q, and 20q chromosome arms, or other chromosome arms, or in illustrative embodiments, the variance in gain of one or more of 1q, 8q, 17q, and 20q chromosome arms, or other chromosome arms, in a population of test cells, such as but not limited to, in a population of bladder cells from a patient with bladder cancer, a patient suspected of having bladder cancer, or a patient receiving therapy for bladder cancer, or cultured cells from such a patient, or in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, gain of one or more of 1q, 8q, 17q, and 20q chromosome arms, or other chromosome arms, detected, or in illustrative embodiments a variance of gain of one or more of 1q, 8q, 17q, and 20q chromosome arms, or other chromosome arms, detected, in a population of test cells, such as but not limited to, in a population of bladder cells from a patient with bladder cancer, a patient suspected of having bladder cancer, or a patient receiving therapy for bladder cancer, or cultured cells from such a patient, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x. In certain illustrative embodiments, the cells of the patient with bladder cancer, a suspected of having bladder cancer, or receiving therapy for bladder cancer, or cultured cells from the patient are non-bladder cells from the patient. In illustrative certain embodiments, the populations of cells are an outgrowth of a chromosomally aberrant clone, such as but not limited to as is described elsewhere herein, wherein the genome of the cells are identical.

In another illustrative example of bladder cancer, in some embodiments, markers of increased genotoxic risk, such as for example in patients who have bladder cancer, who are suspected of having bladder cancer, and/or have or who are receiving therapy for bladder cancer, include, but are not limited to insertions, deletions, copy number variation and/or other structural rearrangements, including translocations and inversions involving one or more of the FHIT, CDKN2A, PTCH, DBCCR1, TSC1, TP53, RB, HRAS, GPR126, PLEKHS 1, CCND1 and APOBEC3B genes, wherein the insertions and deletions are at least 1kb to 2 kb in size. Accordingly, in some exemplary embodiments, insertions, deletions, copy number variation and/or other structural rearrangements, including translocations and inversions involving one or more of the FHIT, CDKN2A, PTCH, DBCCR1, TSC1, TP53, RB, HRAS, GPR126, PLEKHS1, CCND1 and APOBEC3B genes, wherein the insertions and deletions are at least 1kb to 2 kb in size, in a test cell population, such as but not limited to a population of bladder cells from a patient with bladder cancer, a patient suspected of having bladder cancer, or a patient receiving therapy for bladder cancer, or cultured cells from such a patient, in illustrative embodiments relative to the control cell population or reference and/or other cell populations, suggests or otherwise provides an indication of decreased chromosomal stability (increased chromosomal instability). In illustrative embodiments, increased variance in insertions, deletions, copy number variation and/or other structural rearrangements, including translocations and inversions involving one or more of the FHIT, CDKN2A, PTCH, DBCCR1, TSC1, TP53, RB, HRAS, GPR126, PLEKHS1, CCND1 and APOBEC3B genes, wherein the insertions and deletions are at least 1kb to 2 kb in size, in a test cell population, such as but not limited to a population of bladder cells from a patient with bladder cancer, a patient suspected of having bladder cancer, or a patient receiving therapy for bladder cancer, or cultured cells from such a patient, compared to a control population of cells suggests and/or is an indication of an increased genotoxicity risk of the test cell population. As such, some embodiments include an increased weight on insertions, deletions, copy number variation and/or other structural rearrangements, including translocations and inversions involving one or more of the FHIT, CDKN2A, PTCH, DBCCR1, TSC1, TP53, RB, HRAS, GPR126, PLEKHS1, CCND1 and APOBEC3B genes, wherein the insertions and deletions are at least 1kb to 2 kb in size, in a test cell population, such as but not limited to a population of bladder cells from a patient with bladder cancer, a patient suspected of having bladder cancer, or a patient receiving therapy for bladder cancer, or cultured cells from such a patient, detected in the test population of cells compared to a control population of cells suggests and/or is an indication of an increased genotoxicity risk of the test cell population. By way of example only, in some embodiments, insertions, deletions, copy number variation and/or other structural rearrangements, including translocations and inversions involving one or more of the FHIT, CDKN2A, PTCH, DBCCR1, TSC1, TP53, RB, HRAS, GPR126, PLEKHS1, CCND1 and APOBEC3B genes, wherein the insertions and deletions are at least 1kb to 2 kb in size, or in illustrative embodiments, the variance in insertions, deletions, copy number variation and/or other structural rearrangements, including translocations and inversions involving one or more of the FHIT, CDKN2A, PTCH, DBCCR1, TSC1, TP53, RB, HRAS, GPR126, PLEKHS1, CCND1 and APOBEC3B genes, wherein the insertions and deletions are at least 1kb to 2 kb in size, in a population of test cells, such as but not limited to, in a population of bladder cells from a patient with bladder cancer, a patient suspected of having bladder cancer, or a patient receiving therapy for bladder cancer, or cultured cells from such a patient, or in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, insertions, deletions, copy number variation and/or other structural rearrangements, including translocations and inversions involving one or more of the FHIT, CDKN2A, PTCH, DBCCR1, TSC1, TP53, RB, HRAS, GPR126, PLEKHS1, CCND1 and APOBEC3B genes, wherein the insertions and deletions are at least 1kb to 2 kb in size, detected, or in illustrative embodiments a variance of insertions, deletions, copy number variation and/or other structural rearrangements, including translocations and inversions involving one or more of the FHIT, CDKN2A, PTCH, DBCCR1, TSC1, TP53, RB, HRAS, GPR126, PLEKHS1, CCND1 and APOBEC3B genes, wherein the insertions and deletions are at least 1kb to 2 kb in size, detected, in a population of test cells, such as but not limited to, in a population of bladder cells from a patient with bladder cancer, a patient suspected of having bladder cancer, or a patient receiving therapy for bladder cancer, or cultured cells from such a patient, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x. In certain illustrative embodiments, the cells of the patient with bladder cancer, a suspected of having bladder cancer, or receiving therapy for bladder cancer, or cultured cells from the patient are non-bladder cells from the patient. In illustrative certain embodiments, the populations of cells are an outgrowth of a chromosomally aberrant clone, such as but not limited to as is described elsewhere herein, wherein the genome of the cells are identical.

In another illustrative example of bladder cancer, in some embodiments, markers of increased genotoxic risk, such as for example in patients who have bladder cancer, who are suspected of having bladder cancer, and/or have or who are receiving therapy for bladder cancer, include, but are not limited to outgrowth of chromosomally aberrant clones, such as but not limited to wherein the genomes of the clones are identical, such as but not limited to wherein the clones include, have or comprise any of the chromosomal structural or numerical variants, and/or chromosomal variations due to chromosome repair events described elsewhere herein. Accordingly, in some exemplary embodiments, outgrown of chromosomally aberrant clones, in a test cell population, such as but not limited to a population of bladder cells from a patient with bladder cancer, a patient suspected of having bladder cancer, or a patient receiving therapy for bladder cancer, or cultured cells from such a patient, in illustrative embodiments relative to the control cell population or reference and/or other cell populations, suggests or otherwise provides an indication of decreased chromosomal stability (increased chromosomal instability). In illustrative embodiments, increased variance in outgrown of chromosomally aberrant clones, in a test cell population, such as but not limited to a population of bladder cells from a patient with bladder cancer, a patient suspected of having bladder cancer, or a patient receiving therapy for bladder cancer, or cultured cells from such a patient, compared to a control population of cells suggests and/or is an indication of an increased genotoxicity risk of the test cell population. As such, some embodiments include an increased weight on outgrown of chromosomally aberrant clones, in a test cell population, such as but not limited to a population of bladder cells from a patient with bladder cancer, a patient suspected of having bladder cancer, or a patient receiving therapy for bladder cancer, or cultured cells from such a patient, detected in the test population of cells compared to a control population of cells suggests and/or is an indication of an increased genotoxicity risk of the test cell population. By way of example only, in some embodiments, outgrown of chromosomally aberrant clones, or in illustrative embodiments, the variance in outgrown of chromosomally aberrant clones, in a population of test cells, such as but not limited to, in a population of bladder cells from a patient with bladder cancer, a patient suspected of having bladder cancer, or a patient receiving therapy for bladder cancer, or cultured cells from such a patient, or in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, outgrown of chromosomally aberrant clones, detected, or in illustrative embodiments a variance of outgrown of chromosomally aberrant clones, detected, in a population of test cells, such as but not limited to, in a population of bladder cells from a patient with bladder cancer, a patient suspected of having bladder cancer, or a patient receiving therapy for bladder cancer, or cultured cells from such a patient, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x. In certain illustrative embodiments, the cells of the patient with bladder cancer, a suspected of having bladder cancer, or receiving therapy for bladder cancer, or cultured cells from the patient are non-bladder cells from the patient.

In another illustrative example of bladder cancer, in some embodiments, markers of increased genotoxic risk, such as for example in patients who have bladder cancer, who are suspected of having bladder cancer, and/or have or who are receiving therapy for bladder cancer, include, but are not limited to insertions, deletions, changes in copy number or other structural rearrangements such as but not limited to translocations and inversions involving chromosome 5q31 and/or 1q32, or of another chromosome, wherein the insertions and deletions are greater than 1kb or greater than 2 kb. Accordingly, in some exemplary embodiments, insertions, deletions, changes in copy number or other structural rearrangements such as but not limited to translocations and inversions involving chromosome 5q31 and/or 1q32, or of another chromosome, wherein the insertions and deletions are greater than 1kb or greater than 2 kb, in a test cell population, such as but not limited to a population of bladder cells from a patient with bladder cancer, a patient suspected of having bladder cancer, or a patient receiving therapy for bladder cancer, or cultured cells from such a patient, in illustrative embodiments relative to the control cell population or reference and/or other cell populations, suggests or otherwise provides an indication of decreased chromosomal stability (increased chromosomal instability). In illustrative embodiments, increased variance in insertions, deletions, changes in copy number or other structural rearrangements such as but not limited to translocations and inversions involving chromosome 5q31 and/or 1q32, or of another chromosome, wherein the insertions and deletions are greater than 1kb or greater than 2 kb, in a test cell population, such as but not limited to a population of bladder cells from a patient with bladder cancer, a patient suspected of having bladder cancer, or a patient receiving therapy for bladder cancer, or cultured cells from such a patient, compared to a control population of cells suggests and/or is an indication of an increased genotoxicity risk of the test cell population. As such, some embodiments include an increased weight on insertions, deletions, changes in copy number or other structural rearrangements such as but not limited to translocations and inversions involving chromosome 5q31 and/or 1q32, or of another chromosome, wherein the insertions and deletions are greater than 1kb or greater than 2 kb, in a test cell population, such as but not limited to a population of bladder cells from a patient with bladder cancer, a patient suspected of having bladder cancer, or a patient receiving therapy for bladder cancer, or cultured cells from such a patient, detected in the test population of cells compared to a control population of cells suggests and/or is an indication of an increased genotoxicity risk of the test cell population. By way of example only, in some embodiments, insertions, deletions, changes in copy number or other structural rearrangements such as but not limited to translocations and inversions involving chromosome 5q31 and/or 1q32, or of another chromosome, wherein the insertions and deletions are greater than 1kb or greater than 2 kb, or in illustrative embodiments, the variance in insertions, deletions, changes in copy number or other structural rearrangements such as but not limited to translocations and inversions involving chromosome 5q31 and/or 1q32, or of another chromosome, wherein the insertions and deletions are greater than 1kb or greater than 2 kb, in a population of test cells, such as but not limited to, in a population of bladder cells from a patient with bladder cancer, a patient suspected of having bladder cancer, or a patient receiving therapy for bladder cancer, or cultured cells from such a patient, or in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, insertions, deletions, changes in copy number or other structural rearrangements such as but not limited to translocations and inversions involving chromosome 5q31 and/or 1q32, or of another chromosome, wherein the insertions and deletions are greater than 1kb or greater than 2 kb, detected, or in illustrative embodiments a variance of insertions, deletions, changes in copy number or other structural rearrangements such as but not limited to translocations and inversions involving chromosome 5q31 and/or 1q32, or of another chromosome, wherein the insertions and deletions are greater than 1kb or greater than 2 kb, detected, in a population of test cells, such as but not limited to, in a population of bladder cells from a patient with bladder cancer, a patient suspected of having bladder cancer, or a patient receiving therapy for bladder cancer, or cultured cells from such a patient, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x. In certain illustrative embodiments, the cells of the patient with bladder cancer, a suspected of having bladder cancer, or receiving therapy for bladder cancer, or cultured cells from the patient are non-bladder cells from the patient.

In another illustrative example of bladder cancer, in some embodiments, markers of increased genotoxic risk, such as for example in patients who have bladder cancer, who are suspected of having bladder cancer, and/or have or who are receiving therapy for bladder cancer, include, but are not limited to chromatid-type breaks and gaps. Accordingly, in some exemplary embodiments, the number of chromatid-type breaks and gaps, in a test cell population, such as but not limited to a population of bladder cells from a patient with bladder cancer, a patient suspected of having bladder cancer, or a patient receiving therapy for bladder cancer, or cultured cells from such a patient, in illustrative embodiments relative to the control cell population or reference and/or other cell populations, suggests or otherwise provides an indication of decreased chromosomal stability (increased chromosomal instability). In illustrative embodiments, increased variance in the number of chromatid-type breaks and gaps, in a test cell population, such as but not limited to a population of bladder cells from a patient with bladder cancer, a patient suspected of having bladder cancer, or a patient receiving therapy for bladder cancer, or cultured cells from such a patient, compared to a control population of cells suggests and/or is an indication of an increased genotoxicity risk of the test cell population. As such, some embodiments include an increased weight on the number of chromatid-type breaks and gaps, in a test cell population, such as but not limited to a population of bladder cells from a patient with bladder cancer, a patient suspected of having bladder cancer, or a patient receiving therapy for bladder cancer, or cultured cells from such a patient, detected in the test population of cells compared to a control population of cells suggests and/or is an indication of an increased genotoxicity risk of the test cell population. By way of example only, in some embodiments, the number of chromatid-type breaks and gaps, or in illustrative embodiments, the variance in the number of chromatid-type breaks and gaps, in a population of test cells, such as but not limited to, in a population of bladder cells from a patient with bladder cancer, a patient suspected of having bladder cancer, or a patient receiving therapy for bladder cancer, or cultured cells from such a patient, or in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the number of chromatid-type breaks and gaps, detected, or in illustrative embodiments a variance of the number of chromatid-type breaks and gaps, detected, in a population of test cells, such as but not limited to, in a population of bladder cells from a patient with bladder cancer, a patient suspected of having bladder cancer, or a patient receiving therapy for bladder cancer, or cultured cells from such a patient, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x. In certain illustrative embodiments, the cells of the patient with bladder cancer, a suspected of having bladder cancer, or receiving therapy for bladder cancer, or cultured cells from the patient are non-bladder cells from the patient.

In another illustrative example of bladder cancer, in some embodiments, markers of increased genotoxic risk, such as for example in patients who have bladder cancer, who are suspected of having bladder cancer, and/or have or who are receiving therapy for bladder cancer, include, but are not limited to gain of one or more of chromosomes 3, 7, and 17, or of another chromosome. Accordingly, in some exemplary embodiments, gain of one or more of chromosomes 3, 7, and 17, or of another chromosome, in a test cell population, such as but not limited to a population of bladder cells from a patient with bladder cancer, a patient suspected of having bladder cancer, or a patient receiving therapy for bladder cancer, or cultured cells from such a patient, in illustrative embodiments relative to the control cell population or reference and/or other cell populations, suggests or otherwise provides an indication of decreased chromosomal stability (increased chromosomal instability). In illustrative embodiments, increased variance in gain of one or more of chromosomes 3, 7, and 17, or of another chromosome, in a test cell population, such as but not limited to a population of bladder cells from a patient with bladder cancer, a patient suspected of having bladder cancer, or a patient receiving therapy for bladder cancer, or cultured cells from such a patient, compared to a control population of cells suggests and/or is an indication of an increased genotoxicity risk of the test cell population. As such, some embodiments include an increased weight on gain of one or more of chromosomes 3, 7, and 17, or of another chromosome, in a test cell population, such as but not limited to a population of bladder cells from a patient with bladder cancer, a patient suspected of having bladder cancer, or a patient receiving therapy for bladder cancer, or cultured cells from such a patient, detected in the test population of cells compared to a control population of cells suggests and/or is an indication of an increased genotoxicity risk of the test cell population. By way of example only, in some embodiments, gain of one or more of chromosomes 3, 7, and 17, or of another chromosome, or in illustrative embodiments, the variance in gain of one or more of chromosomes 3, 7, and 17, or of another chromosome, in a population of test cells, such as but not limited to, in a population of bladder cells from a patient with bladder cancer, a patient suspected of having bladder cancer, or a patient receiving therapy for bladder cancer, or cultured cells from such a patient, or in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, gain of one or more of chromosomes 3, 7, and 17, or of another chromosome, detected, or in illustrative embodiments a variance of gain of one or more of chromosomes 3, 7, and 17, or of another chromosome, detected, in a population of test cells, such as but not limited to, in a population of bladder cells from a patient with bladder cancer, a patient suspected of having bladder cancer, or a patient receiving therapy for bladder cancer, or cultured cells from such a patient, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x. In certain illustrative embodiments, the cells of the patient with bladder cancer, a suspected of having bladder cancer, or receiving therapy for bladder cancer, or cultured cells from the patient are non-bladder cells from the patient.

In another illustrative example of bladder cancer, in some embodiments, markers of increased genotoxic risk, such as for example in patients who have bladder cancer, who are suspected of having bladder cancer, and/or have or who are receiving therapy for bladder cancer, include, but are not limited to loss of chromosome 9p21, or of another chromosome, or a portion of another chromosome. Accordingly, in some exemplary embodiments, loss of chromosome 9p21, or of another chromosome, or a portion of another chromosome, in a test cell population, such as but not limited to a population of bladder cells from a patient with bladder cancer, a patient suspected of having bladder cancer, or a patient receiving therapy for bladder cancer, or cultured cells from such a patient, in illustrative embodiments relative to the control cell population or reference and/or other cell populations, suggests or otherwise provides an indication of decreased chromosomal stability (increased chromosomal instability). In illustrative embodiments, increased variance in loss of chromosome 9p21, or of another chromosome, or a portion of another chromosome, in a test cell population, such as but not limited to a population of bladder cells from a patient with bladder cancer, a patient suspected of having bladder cancer, or a patient receiving therapy for bladder cancer, or cultured cells from such a patient, compared to a control population of cells suggests and/or is an indication of an increased genotoxicity risk of the test cell population. As such, some embodiments include an increased weight on loss of chromosome 9p21, or of another chromosome, or a portion of another chromosome, in a test cell population, such as but not limited to a population of bladder cells from a patient with bladder cancer, a patient suspected of having bladder cancer, or a patient receiving therapy for bladder cancer, or cultured cells from such a patient, detected in the test population of cells compared to a control population of cells suggests and/or is an indication of an increased genotoxicity risk of the test cell population. By way of example only, in some embodiments, loss of chromosome 9p21, or of another chromosome, or a portion of another chromosome, or in illustrative embodiments, the variance in loss of chromosome 9p21, or of another chromosome, or a portion of another chromosome, in a population of test cells, such as but not limited to, in a population of bladder cells from a patient with bladder cancer, a patient suspected of having bladder cancer, or a patient receiving therapy for bladder cancer, or cultured cells from such a patient, or in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, loss of chromosome 9p21, or of another chromosome, or a portion of another chromosome, detected, or in illustrative embodiments a variance of loss of chromosome 9p21, or of another chromosome, or a portion of another chromosome, detected, in a population of test cells, such as but not limited to, in a population of bladder cells from a patient with bladder cancer, a patient suspected of having bladder cancer, or a patient receiving therapy for bladder cancer, or cultured cells from such a patient, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x. In certain illustrative embodiments, the cells of the patient with bladder cancer, a suspected of having bladder cancer, or receiving therapy for bladder cancer, or cultured cells from the patient are non-bladder cells from the patient.

In another illustrative example of bladder cancer, in some embodiments, markers of increased genotoxic risk, such as for example in patients who have bladder cancer, who are suspected of having bladder cancer, and/or have or who are receiving therapy for bladder cancer, include, but are not limited to the presence of double minutes with amplification of the CCND1 gene, or with the amplification or another gene, or the presence of double minutes alone. Accordingly, in some exemplary embodiments, the presence of double minutes with amplification of the CCND1 gene, or with the amplification or another gene, or the presence of double minutes alone, in a test cell population, such as but not limited to a population of bladder cells from a patient with bladder cancer, a patient suspected of having bladder cancer, or a patient receiving therapy for bladder cancer, or cultured cells from such a patient, in illustrative embodiments relative to the control cell population or reference and/or other cell populations, suggests or otherwise provides an indication of decreased chromosomal stability (increased chromosomal instability). In illustrative embodiments, increased variance in the presence of double minutes with amplification of the CCND1 gene, or with the amplification or another gene, or the presence of double minutes alone, in a test cell population, such as but not limited to a population of bladder cells from a patient with bladder cancer, a patient suspected of having bladder cancer, or a patient receiving therapy for bladder cancer, or cultured cells from such a patient, compared to a control population of cells suggests and/or is an indication of an increased genotoxicity risk of the test cell population. As such, some embodiments include an increased weight on the presence of double minutes with amplification of the CCND1 gene, or with the amplification or another gene, or the presence of double minutes alone, in a test cell population, such as but not limited to a population of bladder cells from a patient with bladder cancer, a patient suspected of having bladder cancer, or a patient receiving therapy for bladder cancer, or cultured cells from such a patient, detected in the test population of cells compared to a control population of cells suggests and/or is an indication of an increased genotoxicity risk of the test cell population. By way of example only, in some embodiments, the presence of double minutes with amplification of the CCND1 gene, or with the amplification or another gene, or the presence of double minutes alone, or in illustrative embodiments, the variance in the presence of double minutes with amplification of the CCND1 gene, or with the amplification or another gene, or the presence of double minutes alone, in a population of test cells, such as but not limited to, in a population of bladder cells from a patient with bladder cancer, a patient suspected of having bladder cancer, or a patient receiving therapy for bladder cancer, or cultured cells from such a patient, or in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, the presence of double minutes with amplification of the CCND1 gene, or with the amplification or another gene, or the presence of double minutes alone, detected, or in illustrative embodiments a variance of the presence of double minutes with amplification of the CCND1 gene, or with the amplification or another gene, or the presence of double minutes alone, detected, in a population of test cells, such as but not limited to, in a population of bladder cells from a patient with bladder cancer, a patient suspected of having bladder cancer, or a patient receiving therapy for bladder cancer, or cultured cells from such a patient, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x. In certain illustrative embodiments, the cells of the patient with bladder cancer, a suspected of having bladder cancer, or receiving therapy for bladder cancer, or cultured cells from the patient are non-bladder cells from the patient. In certain illustrative embodiments, the cells of the patient with bladder cancer, a suspected of having bladder cancer, or receiving therapy for bladder cancer, or cultured cells from the patient are non-bladder cells from the patient.

Relative Insertional Mutagenesis Risk in Gene Editing System Involving a Genomic Insert Cassette and Weighting

Due to the nature of the methods by which gene editing system function, when inserting a genomic cassette recombinant nucleic acid insert into the genome of an organism, cells that have been modified using such methods are subject to an increased instability risk, which in turn creates a risk that such cells can present safety issues in patients receiving these therapies, and/or a risk that cell lines created by these methods can change or mutate, such as but not limited to by clonal outgrowth. For example, significant chromosomal rearrangements have been observed in some gene editing systems that use/involve genomic cassette inserts. Such chromosomal rearrangements can trigger a higher genotoxic risk for cells modified by these methods. Markers, or high-risk loci, are known to be associated with such chromosomal variants, whether due to repair events or not. For example, the EVI1, LMO1, LMO2, CDKN2A, TP53, cMYC, CCNA2, TERT, CCNE1, TNFSF10, KMT2B, DLK1, GTL2, PTEN, CSF1, BRCA1, BRCA2, EP300, CD44, NF1, HER2, WAS, FBXW7, MATR3, TNKS, SFI1, MYST3, PUM1, BMPR1A, TCF12, PIK3R1, POLD3, POLD4, RAD52, RAD51, SMARCAL1, ATM, P16 and PPP1R12A genes, the RTL1, RIAN, MIRG and/or DIO3 region of the AAV-HCC locus on chromosome 12, changes at fragile sited (i.e., FRA2G, FRA3B, FRA4F, FRA6E, FRA6F, FRA7E, FRA7G, FRA7H, FRA7I, FRA8C, FRA9E, FRA11E, FRA16D, FRAXB), gains of chromosomes 12, 17 and X, gains of 17q11-q12, 17q25-ter, trisomy 12, 14, 15 and 17, duplications of 12p and 20q, monosomy or other loss involving chromosomes 9, 13, and 14, and translocations of 1q, and/or clonal outgrowth of cells having such chromosomal variants are known to be markers in these gene editing systems. These markers can be evaluated during modification of cells using these systems, and then at later points in time, such as but not limited to prior to and/or after the cells are delivered to a patent as a therapy, and /or at any time point during with such modified cells are grown in culture, and the like.

In another illustrative example of gene editing systems involving the use of genomic insert cassettes, in some embodiments, markers of increased genotoxic risk include but are not limited to, insertions, deletions, copy number variation and/or other structural rearrangements including translocations and/or inversions involving the EVI1, LMO1, LMO2, CDKN2A, TP53, cMYC, CCNA2, TERT, CCNE1, TNFSF10, KMT2B, DLK1, GTL2, PTEN, CSF1, BRCA1, BRCA2, EP300, CD44, NF1, HER2, WAS, FBXW7, MATR3, TNKS, SFI1, MYST3, PUM1, BMPR1A, TCF12, PIK3R1, POLD3, POLD4, RAD52, RAD51, SMARCAL1, ATM, P16 and PPP1R12A genes, or of another gene, wherein the insertion(s) and/or deletion(s) is at least 1kb or at least 2 kb in size, or in a clonal outgrowth of a cell that has received such a cassette and has such a chromosomal variation, wherein the cells of the clonal outgrowth have an identical genome. Accordingly, in some exemplary embodiments, insertions, deletions, copy number variation and/or other structural rearrangements including translocations and/or inversions involving the EVI1, LMO1, LMO2, CDKN2A, TP53, cMYC, CCNA2, TERT, CCNE1, TNFSF10, KMT2B, DLK1, GTL2, PTEN, CSF1, BRCA1, BRCA2, EP300, CD44, NF1, HER2, WAS, FBXW7, MATR3, TNKS, SFI1, MYST3, PUM1, BMPR1A, TCF12, PIK3R1, POLD3, POLD4, RAD52, RAD51, SMARCAL1, ATM, P16 and PPP1R12A genes, or of another gene, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population, such as but not limited to a population of cells, in illustrative embodiments relative to the control cell population or reference and/or other cell populations, suggests or otherwise provides an indication of decreased chromosomal stability (increased chromosomal instability). In illustrative embodiments, increased variance in insertions, deletions, copy number variation and/or other structural rearrangements including translocations and/or inversions involving the EVI1, LMO1, LMO2, CDKN2A, TP53, cMYC, CCNA2, TERT, CCNE1, TNFSF10, KMT2B, DLK1, GTL2, PTEN, CSF1, BRCA1, BRCA2, EP300, CD44, NF1, HER2, WAS, FBXW7, MATR3, TNKS, SFI1, MYST3, PUM1, BMPR1A, TCF12, PIK3R1, POLD3, POLD4, RAD52, RAD51, SMARCAL1, ATM, P16 and PPP1R12A genes, or of another gene, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population, compared to a control population of cells suggests and/or is an indication of an increased genotoxicity risk of the test cell population. As such, some embodiments include an increased weight on the insertions, deletions, copy number variation and/or other structural rearrangements including translocations and/or inversions involving the EVI1, LMO1, LMO2, CDKN2A, TP53, cMYC, CCNA2, TERT, CCNE1, TNFSF10, KMT2B, DLK1, GTL2, PTEN, CSF1, BRCA1, BRCA2, EP300, CD44, NF1, HER2, WAS, FBXW7, MATR3, TNKS, SFI1, MYST3, PUM1, BMPR1A, TCF12, PIK3R1, POLD3, POLD4, RAD52, RAD51, SMARCAL1, ATM, P16 and PPP1R12A genes, or of another gene, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population, detected in the test population of cells compared to a control population of cells suggests and/or is an indication of an increased genotoxicity risk of the test cell population. By way of example only, in some embodiments, the insertions, deletions, copy number variation and/or other structural rearrangements including translocations and/or inversions involving the EVI1, LMO1, LMO2, CDKN2A, TP53, cMYC, CCNA2, TERT, CCNE1, TNFSF10, KMT2B, DLK1, GTL2, PTEN, CSF1, BRCA1, BRCA2, EP300, CD44, NF1, HER2, WAS, FBXW7, MATR3, TNKS, SFI1, MYST3, PUM1, BMPR1A, TCF12, PIK3R1, POLD3, POLD4, RAD52, RAD51, SMARCAL1, ATM, P16 and PPP1R12A genes, or of another gene, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population, or in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, insertions, deletions, copy number variation and/or other structural rearrangements including translocations and/or inversions involving the EVI1, LMO1, LMO2, CDKN2A, TP53, cMYC, CCNA2, TERT, CCNE1, TNFSF10, KMT2B, DLK1, GTL2, PTEN, CSF1, BRCA1, BRCA2, EP300, CD44, NF1, HER2, WAS, FBXW7, MATR3, TNKS, SFI1, MYST3, PUM1, BMPR1A, TCF12, PIK3R1, POLD3, POLD4, RAD52, RAD51, SMARCAL1, ATM, P16 and PPP1R12A genes, or of another gene, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population detected, in a population of test cells, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x.

In another illustrative example of gene editing systems involving the use of genomic insert cassettes, in some embodiments, markers of increased genotoxic risk include but are not limited to, insertions, deletions, copy number variation and/or other structural rearrangements including translocations and/or inversions involving the RTL1, RIAN, MIRG, or DIO3 region of the AAV-HCC locus on chromosome12, or of another gene on chromosome 12 or on any other chromosome, wherein the insertion(s) and/or deletion(s) is at least 1kb or at least 2 kb in size, or in a clonal outgrowth of a cell that has received such a cassette and has such a chromosomal variation, wherein the cells of the clonal outgrowth have an identical genome. Accordingly, in some exemplary embodiments, insertions, deletions, copy number variation and/or other structural rearrangements including translocations and/or inversions involving the RTL1, RIAN, MIRG, or DIO3 region of the AAV-HCC locus on chromosome12, or of another gene on chromosome 12 or on any other chromosome, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population, such as but not limited to a population of cells, in illustrative embodiments relative to the control cell population or reference and/or other cell populations, suggests or otherwise provides an indication of decreased chromosomal stability (increased chromosomal instability). In illustrative embodiments, increased variance in insertions, deletions, copy number variation and/or other structural rearrangements including translocations and/or inversions involving the RTL1, RIAN, MIRG, or DIO3 region of the AAV-HCC locus on chromosome 12, or of another gene on chromosome 12 or on any other chromosome, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population, compared to a control population of cells suggests and/or is an indication of an increased genotoxicity risk of the test cell population. As such, some embodiments include an increased weight on the insertions, deletions, copy number variation and/or other structural rearrangements including translocations and/or inversions involving the RTL1, RIAN, MIRG, or DIO3 region of the AAV-HCC locus on chromosome 12, or of another gene on chromosome 12 or on any other chromosome, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population, detected in the test population of cells compared to a control population of cells suggests and/or is an indication of an increased genotoxicity risk of the test cell population. By way of example only, in some embodiments, the insertions, deletions, copy number variation and/or other structural rearrangements including translocations and/or inversions involving the RTL1, RIAN, MIRG, or DIO3 region of the AAV-HCC locus on chromosome12, or of another gene on chromosome 12 or on any other chromosome, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population, or in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, insertions, deletions, copy number variation and/or other structural rearrangements including translocations and/or inversions involving the RTL1, RIAN, MIRG, or DIO3 region of the AAV-HCC locus on chromosome12, or of another gene on chromosome 12 or on any other chromosome, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population detected, in a population of test cells, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x.

In another illustrative example of gene editing systems involving the use of genomic insert cassettes, in some embodiments, markers of increased genotoxic risk include but are not limited to, insertions, deletions, copy number variation and/or other structural rearrangements, such as but not limited to including translocations and/or inversions, involving the one or more fragile sites, such as but not limited to FRA2G, FRA3B, FRA4F, FRA6E, FRA6F, FRA7E, FRA7G, FRA7H, FRA7I, FRA8C, FRA9E, FRA11E, FRA16D, and/or FRAXB, wherein the insertion(s) and/or deletion(s) is at least 1kb or at least 2 kb in size, or in a clonal outgrowth of a cell that has received such a cassette and has such a chromosomal variation, wherein the cells of the clonal outgrowth have an identical genome. Accordingly, in some exemplary embodiments, insertions, deletions, copy number variation and/or other structural rearrangements including translocations and/or inversions involving FRA2G, FRA3B, FRA4F, FRA6E, FRA6F, FRA7E, FRA7G, FRA7H, FRA7I, FRA8C, FRA9E, FRA11E, FRA16D, and/or FRAXB, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population, such as but not limited to a population of cells, in illustrative embodiments relative to the control cell population or reference and/or other cell populations, suggests or otherwise provides an indication of decreased chromosomal stability (increased chromosomal instability). In illustrative embodiments, increased variance in insertions, deletions, copy number variation and/or other structural rearrangements including translocations and/or inversions involving FRA2G, FRA3B, FRA4F, FRA6E, FRA6F, FRA7E, FRA7G, FRA7H, FRA7I, FRA8C, FRA9E, FRA11E, FRA16D, and/or FRAXB, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population, compared to a control population of cells suggests and/or is an indication of an increased genotoxicity risk of the test cell population. As such, some embodiments include an increased weight on the insertions, deletions, copy number variation and/or other structural rearrangements including translocations and/or inversions involving FRA2G, FRA3B, FRA4F, FRA6E, FRA6F, FRA7E, FRA7G, FRA7H, FRA7I, FRA8C, FRA9E, FRA11E, FRA16D, and/or FRAXB, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population, detected in the test population of cells compared to a control population of cells suggests and/or is an indication of an increased genotoxicity risk of the test cell population. By way of example only, in some embodiments, the insertions, deletions, copy number variation and/or other structural rearrangements including translocations and/or inversions involving FRA2G, FRA3B, FRA4F, FRA6E, FRA6F, FRA7E, FRA7G, FRA7H, FRA7I, FRA8C, FRA9E, FRA11E, FRA16D, and/or FRAXB, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population, or in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, insertions, deletions, copy number variation and/or other structural rearrangements including translocations and/or inversions involving FRA2G, FRA3B, FRA4F, FRA6E, FRA6F, FRA7E, FRA7G, FRA7H, FRA7I, FRA8C, FRA9E, FRA11E, FRA16D, and/or FRAXB, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population detected, in a population of test cells, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x.

In another illustrative example of gene editing systems involving the use of genomic insert cassettes, in some embodiments, markers of increased genotoxic risk include but are not limited to, gains of chromosomes 12, 17 X, 17q11-q12, and/or 17q25-ter, trisomy 12, 14, 15 and/or 17, duplications of 12p and/or 20q, monosomy or other loss involving chromosomes 9, 13, and/or 14 and/or any other chromosome, and translocations of chromosome 1q and/or of any other chromosome, wherein the insertion(s) and/or deletion(s) is at least 1kb or at least 2 kb in size, or in a clonal outgrowth of a cell that has received such a cassette and has such a chromosomal variation, wherein the cells of the clonal outgrowth have an identical genome. Accordingly, in some exemplary embodiments, gains of chromosomes 12, 17 X, 17q11-q12, and/or 17q25-ter, trisomy 12, 14, 15 and/or 17, duplications of 12p and/or 20q, monosomy or other loss involving chromosomes 9, 13, and/or 14 and/or any other chromosome, and translocations of chromosome 1q and/or of any other chromosome, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population, such as but not limited to a population of cells, in illustrative embodiments relative to the control cell population or reference and/or other cell populations, suggests or otherwise provides an indication of decreased chromosomal stability (increased chromosomal instability). In illustrative embodiments, increased variance in gains of chromosomes 12, 17 X, 17q11-q12, and/or 17q25-ter, trisomy 12, 14, 15 and/or 17, duplications of 12p and/or 20q, monosomy or other loss involving chromosomes 9, 13, and/or 14 and/or any other chromosome, and translocations of chromosome 1q and/or of any other chromosome, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population, compared to a control population of cells suggests and/or is an indication of an increased genotoxicity risk of the test cell population. As such, some embodiments include an increased weight on the gains of chromosomes 12, 17 X, 17q11-q12, and/or 17q25-ter, trisomy 12, 14, 15 and/or 17, duplications of 12p and/or 20q, monosomy or other loss involving chromosomes 9, 13, and/or 14 and/or any other chromosome, and translocations of chromosome 1q and/or of any other chromosome, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population, detected in the test population of cells compared to a control population of cells suggests and/or is an indication of an increased genotoxicity risk of the test cell population. By way of example only, in some embodiments, the gains of chromosomes 12, 17 X, 17q11-q12, and/or 17q25-ter, trisomy 12, 14, 15 and/or 17, duplications of 12p and/or 20q, monosomy or other loss involving chromosomes 9, 13, and/or 14 and/or any other chromosome, and translocations of chromosome 1q and/or of any other chromosome, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population, or in a population of cells, such as for example in a diploid or a complex genomic cell population, is weighted by a factor of 1.0x, 1.1x, 1.5x, 2.0x, 2.5x, 3.0x, 4.0x, 5.0x, 10.0x, 20x, 25x, 30x, 35x, 40x, 45x, 50.0x, 55.0x, 60.0x, 65.0x, 70.0x, 75.0x, 80.0x, 85.0x, 90.0x, 95.0x, 100.0x, 150.0x, 200.0x, 250.0x, 300.0x, 350.0x, 400.0x, 450.0x, 500.0x or greater, or between 1.5x and 2x, 3x, 4x, 5x, 10x, 15x, 20x, 25x, 50x, 75x, 100x, 150x, 200x, 250x or 300x, or between 2x and 3x, 4x, 5x, 10x, 15x, 20x, 25x, 30x, 40x, 50x, 75x, or 100x, 150x, 200x, 250x or 300x, or between 10x and 20x, 30x, 40x, 50x, 75x, or 100x, 150x, 200x, 250x or 300x. In some embodiments, gains of chromosomes 12, 17 X, 17q11-q12, and/or 17q25-ter, trisomy 12, 14, 15 and/or 17, duplications of 12p and/or 20q, monosomy or other loss involving chromosomes 9, 13, and/or 14 and/or any other chromosome, and translocations of chromosome 1q and/or of any other chromosome, wherein the insertion(s) and/or deletion(s) is at least 1kb or at least 2 kb in size, or in a clonal outgrowth of a cell that has received such a cassette and/or that has such a chromosomal variation, wherein the cells of the clonal outgrowth have an identical genome, in a test cell population detected, in a population of test cells, or in the population of cells, such as for example in a diploid or a complex genomic cell population, is weighted by a factor of between 10x and 1,000x, 500x, 250x, 125x, or 100x, or between 25x and 250x, between 50x and 150x, or between 90x and 110x.

## ADDITIONAL dGH REACTION COMPONENTS, EMBODIMENTS AND CONSIDERATIONS

As disclosed herein, illustrative methods, kits, and compositions herein involve a dGH reaction or components thereof. dGH reactions typically involve the generation of single-stranded chromatids. Such single-stranded chromatids can be generated by any means known in the art. In illustrative embodiments, single-stranded chromatids are generated using the CO-FISH technique. As disclosed in "Strand-Specific Fluorescence in situ Hybridization: The CO-FISH Family" by S. M. Bailey et al., Cytogenet. Genome Res. 107: 11-14 (2004), chromosome organization can be studied using strand-specific FISH (fluorescent or fluorescence in situ hybridization), which is often referred to as CO-FISH or Chromosome Orientation-FISH. The CO-FISH technique requires cultivation of cells in the presence of bromodeoxyuridine (BrdU) and/or bromodeoxycytidine (BrdC) for a single round of replication (a single S phase).

Each newly replicated double helix contains one parental DNA strand plus a newly synthesized strand in which the nucleotide analogs have partially replaced thymidine and/or deoxycytidine. Following preparation of metaphase chromosomes on microscope slides by standard cytogenetic techniques, the cells are exposed to UV light in the presence of the photosensitizing DNA dye Hoechst, which results in numerous strand breaks that occur preferentially at the sites of BrdU incorporation. Nicks produced in the chromosomal DNA by this treatment then serve as selective substrates for enzymatic digestion by Exo III. This results in the specific removal of the newly replicated strands while leaving the original (parental) strands largely intact. Thus, for the purposes of subsequent hybridization reactions, the two sister chromatids of a chromosome are rendered single stranded, and complementary to one another, without the need for thermal denaturation. The unresected parental strands then serve as single stranded target DNA for hybridization with single-stranded probes. CO-FISH was designed to determine the orientation of tandem repeats within centromeric regions of chromosomes. Mammalian telomeric DNA consists of tandem repeats oriented in 5'->3' towards the termini of all vertebrate organisms. In CO-FISH, single-stranded oligonucleotides are directed to tandem repeat sequences in the telomeres, so as to detect the telomers of the single-stranded sister chromatids, thereby enabling the determination of the 5' -> 3' orientation of the sister chromatids.

Methods for performing dGH typically involve analyzing chromosomes using light microscopy on metaphase spreads. Metaphase is a stage in the cell cycle (in mitosis or meiosis) where all the genetic material is condensing into chromosomes. These chromosomes become visible during metaphase. Metaphase chromosomes are used in various microscopic methods to analyze chromosomes, for example for chromosomal abnormalities. To prepare a metaphase spread, cells of a population are cultured for at least 1 cell cycle in the presence of a DNA analog which, in illustrative embodiments, can be BrdU/C to prepare hemi-substituted chromosomes/hemi-substituted sister chromatids. In some nonlimiting embodiments, cells are then arrested in the metaphase stage to obtain a metaphase-enriched cell population by adding one or more metaphase arresting agents such as colchicine, demecolchin, vinblastine, nocodazole, etc., at a concentration ranging from 0.05 $\mu$g/ml to 0.3 $\mu$g/ml, for example, from 0.05$\mu$g/ml to 0.1$\mu$g/ml, from 0.05$\mu$g/ml to 0.15$\mu$g/ml, from 0.05 to 0.2, from 0.05 to 0.25, or from 0.05 to 0.3$\mu$g/ml, to obtain a metaphase-enriched cell population. In some embodiments, the metaphase arresting agent is added at a concentration ranging from 0.1$\mu$g/ml to 0.3$\mu$g/ml, from 0.15 to 0.3, from 0.20 to 0.3, or from 0.25 to 0.3$\mu$g/ml. In illustrative embodiments, the metaphase arresting agent, N-methyl-N-deacetyl-colchicine (Colcemid™), is added to the cell culture. Cells are typically incubated at around 25 °C or around 37 °C, for example, between 22 °C and 30 °C, 23 °C and 27 °C, 33 °C and 41 °C, or 35 °C and 39 °C. Cells are then incubated for approximately 4 hours to accumulate mitotic cells. In some embodiments, cells can be incubated for at least 15, 30, or 45 minutes or 1, 2, 3, 4, 5, 6, 7, 8, 9, 10, 11, or 12 hours to accumulate mitotic cells. In some embodiments, cells can be incubated for between 15, 30, or 45 minutes or 1, 2, 3, 4, 5, 6, 7, 8, 9, or 10 hours on the low end of the range and 11 or 12 hours on the high end of the range. In some embodiments, cells can be incubated for between 15, 30, or 45 minutes or 1, 2, 3, 4, 5, or 6 hours on the low end of the range and 7, 8, 9, 10, 11, or 12 hours on the high end of the range. In some embodiments, cells can be incubated for between 15, 30, or 45 minutes or 1, 2, or 3 hours on the low end of the range and, 4, 5, 6, 7, 8, 9, 10, 11, or 12 hours on the high end of the range. In some embodiments, cells can be incubated for between 15 minutes and 6 hours, for example, between 1 hour and 6 hours, 3 hours and 6 hours, or between 3 hours and 5 hours.

The cells are then harvested, wherein, in some non-limiting embodiments, the cells can be harvested by a dGH harvest procedure, which in illustrative embodiments include incubating the cells, in some non-limiting embodiments, for a period of 1-20, 2-20, 5-20, in illustrative embodiments, 5-10 minutes in the presence of a hypotonic solution, which in illustrative embodiments can be a hypotonic KCl solution. In some non-limiting embodiments, the cells are further subjected to fixation by exposing the cells to a fixative, which in some non-limiting embodiments, can comprise methanol and acetic acid, in a volume ratio of 1:1 to 5:1, in illustrative embodiments, 3:1 to form processed cells. In some embodiments, the processed cells are deposited (dropped, or placed) onto slides (e.g., Drop slides) to form metaphase spreads. The metaphase spreads are contacted with one or more dGH probes, in illustrative embodiments, two or more dGH probes that are capable of hybridizing to one or more target DNA, in illustrative embodiments, two or more target DNA found on one of the single-stranded sister chromatids generated from the metaphase spreads (i.e., dGH hybridization procedure). In some non-limiting embodiments, a single un-sorted sample enriched for metaphase cells, spotted onto a slide is probed with oligonucleotide probes. In some non-limiting embodiments, the exemplary workflow includes 10 to 500 spreads per sample, and 1-5 oligonucleotide probes, in illustrative embodiments, dGH probes per assay. In some embodiments, at least one non-enriched sample can be included.

## CELL POPULATIONS

Cells and in illustrative embodiments, populations of cells are analyzed in methods provided herein. Accordingly, in some embodiments of any of the methods disclosed herein, the method may include assessing 1 cell or more than 1 cell, for example, at least 1, 2, 3, 4, 5, 6, 7, 8, 9, 10, 12, 15, 20, 25, 30, 35, 40, 45, 50, 55, 60, 65, 70, 75, 80, 85, 90, 95, or 100 cells from a population of cells. In some embodiments, the method can include assessing 4 or more cells. In some embodiments, the method may include assessing 10 or more cells. In some embodiments, the method may include assessing 20 or more cells. In some embodiments, the method can include assessing 50 or more cells. For example, the method may include assessing between 2 cells and 20, 25, 30, 35, 40, 45, 50, 55, 60, 65, 70, 75, 80, 85, 90, 95, or 100 cells from the population of cells, or any number of cells there between the range. In other embodiments, the method includes assessing between 4 cells and 20, 25, 30, 35, 40, 45, 50, 55, 60, 65, 70, 75, 80, 85, 90, 95, or 100 cells from the population of cells. In other embodiments, the method includes assessing between 10 cells and 20, 25, 30, 35, 40, 45, 50, 55, 60, 65, 70, 75, 80, 85, 90, 95, or 100 cells from the population of cells. In some embodiments, the method includes assessing between 1, 2, 3, 4, 5, 6, 7, 8, 9, 10, 12, 15, 20, 25, 30, 35, 40, 45, 50, 55, 60, 65, 70, 75, 80, 85, 90, or 95 cells and 100 cells from the population of cells. In other embodiments, the method includes assessing more than 100 cells from the population of cells. Some embodiments include assessing the same or a substantially similar number of cells from a control or reference cell population.

In some embodiments, the population of cells, e.g., test population of cells, or test population of cancer cells, can be any type of cell population for which chromosomal stability or genotoxic risk can be measured. In some embodiments, the population of cells can be a cell line. In some embodiments, the cell population can be blood cells, such as B cells, T cells, or NK cells, liver cells, kidney cells, iPSCs, neural or neuronal cells, or cells whose genomes have or have not been edited. In some embodiments, the neural or neuronal cells can include cells from a neural cell line or primary neural cells. In some embodiments, primary neural cells can be in a 2-D culture. In some embodiments, primary neural cells can be in a 3-D neural culture such as in 3-D neural organoids. In some embodiments, the neural or neuronal cells can include neurons, glial cells, astrocytes, oligodendrocytes, and/or neural endothelial cells. In some embodiments, the population of cells comprise cells from a cell line or primary cell culture of cells isolated from a central nervous system. In some embodiments, the population of cells comprise cells from a cell line or primary cell culture of cells isolated from a central nervous system. In some embodiments the population of cells can be a diploid population of cells. In some embodiments, the population of diploid cells can include: 1) a lymphocyte having a chimeric antigen receptor, 2) a cell having a transgene, 3) a cell having a gene-edited insert sequence, 4) a cell comprising a recombinant nucleic acid insert, 5) a cell comprising a DNA sequence insert for binding guide RNA, 6) a cell comprising a transcription activator-like effector binding sequence, 7) a cell having a zinc finger binding sequence, 8) one cell in a population of cells, wherein the population of cells is a substantially homogeneous population of cells, or 9) one or more genetically-modified cell. In some embodiments, the diploid cell can include 1) a T cell having a chimeric antigen receptor, 2) a cell having a transgenic sequence, 3) a cell having a gene-edited insert sequence, 4) a cell having a DNA sequence insert for binding guide RNA, 5) a cell having a transcription activator-like effector binding sequence, 6) a cell having a zinc finger binding sequence, 7) a substantially homogeneous population of cells, 8) one or more cells that have been previously genetically modified, or 9) at least one lymphocyte that has been genetically modified and includes a chimeric antigen receptor. In some embodiments, a substantially homogeneous population of cells can include at least 70%, 75%, 80%, 85%, 90%, 95%, 96%, 97%, 98%, or 99% of the cells in the population having genomes that are at least 70%, 75%, 80%, 85%, 90%, 95%, 96%, 97%, 98%, or 99% identical. In some embodiments, a substantially homogeneous population of cells can include at least 70% of the cells in the population having genomes that are at least 70%, 75%, 80%, 85%, 90%, 95%, 96%, 97%, 98%, or 99% identical. In some embodiments, a substantially homogeneous population of cells can include at least 80% of the cells in the population having genomes that are at least 70%, 75%, 80%, 85%, 90%, 95%, 96%, 97%, 98%, or 99% identical. In some embodiments, a substantially homogeneous population of cells can include at least 90% of the cells in the population having genomes that are at least 70%, 75%, 80%, 85%, 90%, 95%, 96%, 97%, 98%, or 99% identical. In some embodiments, a substantially homogeneous population of cells can include at least 95% of the cells in the population having genomes that are at least 70%, 75%, 80%, 85%, 90%, 95%, 96%, 97%, 98%, or 99% identical. In some embodiments, a substantially homogeneous population of cells can include at least 70%, 75%, 80%, 85%, 90%, 95%, 96%, 97%, 98%, or 99% of the cells in the population having genomes that are at least 70% identical. In some embodiments, a substantially homogeneous population of cells can include at least 70%, 75%, 80%, 85%, 90%, 95%, 96%, 97%, 98%, or 99% of the cells in the population having genomes that are at least 80% identical. In some embodiments, a substantially homogeneous population of cells can include at least 70%, 75%, 80%, 85%, 90%, 95%, 96%, 97%, 98%, or 99% of the cells in the population having genomes that are at least 90% identical. In some embodiments, a substantially homogeneous population of cells can include at least 70%, 75%, 80%, 85%, 90%, 95%, 96%, 97%, 98%, or 99% of the cells in the population having genomes that are at least 95% identical. In other embodiments, the population of cells can be a complex population of cells having a complex karyotype. Cells with a complex karyotype do not have a wild-type number of chromosomes and have at least 2, 3, 4, 5, 6 or more chromosomal variants. In some embodiments, the cell population can be any population of mammalian cells, such as human, murine, canine, non-human primate and CHO cells. In some embodiments, the cell population can be genetically-engineered. For example, a population of genetically-modified blood cells, such as B cells, T cells, or NK cells, liver cells, kidney cells, iPSCs, neuronal cells, or cells whose genome has been edited. In some embodiments, genetically-modified cells are gene-edited cells. Complex cell populations are typically cancer cell populations. Examples of cancer cells include cells with 1Q deletions, translocations, bladder cancer, cells with translocations on the chromosomes, cells having chromosomes with random inversions, cells with high copy number of chromosomes such as numerical variants or non-normal numbers, cells with chromosomal structural variations, cells having genomes with mutations or single nucleotide polymorphisms (SNPs), cells having large inserts on the chromosomes, cells from different cancers/subsets, cells having chromatin condensation defects (detectable - long spindly centromeres), cells having chromatin remodeling and chromatin changes, or chromatin rearrangements, cells with PARPI knockout, cells from any known disease having any known genetic markers for genetic diseases such as cells having ALK-EML4 inversion, cells having BRCA1 (repair protein in breast cancer), cells having HER2 activation (which is initially virulence - then later a stability-driven cancer). Chromatin changes occur in response to DNA damage and involve histone modifications, chromatin remodeling, recruiting histone variants and histone chaperones. Chromatin modulation through PARylation initiates the DNA damage response and promotes DNA repair. PARylation is mediated by poly-(ADP-ribose) polymerases (PARPs). PARP1 transfers ADP-ribose from NAD+ to protein acceptor sites. Increased PARP1 activity leads to reduced NAD+ levels and cell death. The activation of PARP1 and accumulation of PAR have been demonstrated in post-mortem brains of Alzheimer's disease patients, particularly in neurons of the frontal and temporal lobes. Together, these observations indicate that chromatin modifications play a major role after DNA damage in Alzheimer's disease and suggest that interventions aimed at inhibiting chromatin modification such as PARP1 inhibition, may slow down the progression of Alzheimer's disease.

In some embodiments, the population of cells is a population of control or reference cells (or a control population of cells), such as but not limited to a population of T cells from a healthy subject, or a population of cells from an immortalized T cell line whose chromosome stability value or score is known/has been determined. In some embodiments, the control population of cells is largely similar to a test population of cells. In some embodiments, the control population of cells can be derived from the same tissue as the test population of cells. In some embodiments, the control population of cells can be the same cell type as the test population of cells. In some embodiments, the control population of cells can be age matched, tissue matched, and/or condition matched to the test population of cells. In some embodiments, the control population of cells can be a different passage number of the same cell line as the test population of cells. In some embodiments, the population of control or reference cells is a population of cells of a cancer cell line with a known/predetermined chromosome stability score. In some embodiments, such as but not limited to when a genetically-modified population of cells is being analyzed, the population of reference or control cells is a population of cells from the same type of cell or descendants of the same cells that are not genetically modified, as those that were genetically modified. In some embodiments, two or more populations of cells that are of the same cell type and in illustrative embodiments, descendants of the same cell population, are analyzed together.

CAR-T cell therapy is a type of treatment in which a patient's T cells (a type of immune system cell) are changed in the laboratory so they will attack cancer cells. T cells are taken from a patient's blood. Then the gene for a special receptor that binds to a certain protein on the patient's cancer cells is added to the T cells in the laboratory. The special receptor is called a chimeric antigen receptor (CAR). Large numbers of the CAR T cells are grown in the laboratory and given to the patient by infusion. CAR T-cell therapy is used to treat certain blood cancers, and it is being studied in the treatment of other types of cancer. Also called chimeric antigen receptor T-cell therapy. CAR-T cells are T cells which are genetically modified such that they express a chimeric antigen receptor (CAR) that is specific for a predetermined antigen.

In some embodiments, the population of cells comprise a chimeric antigen receptor. In some embodiments, the cells comprise a recombinant T cell receptor.

In some embodiments, the population of cells is a population of induced pluripotent stem cells (iPSCs).

In some embodiments, the population of cells has any genetic insertion by means of lentiviruses, transposons, or adenovirus associated vector (AAV).

In some embodiments, the population of cells is from a Downs syndrome patient, such as having trisomy 21, or a Downs syndrome patient developing Alzheimer's, which indicates that genotoxicity is happening in the Downs syndrome patient. Some embodiments have population of cells from subjects with autism spectrum. Some embodiments have populations of cells from subjects having rare diseases, patients undergoing gene therapy treatment for muscle wasting disorders, or patients having any condition that leads to genotoxicity. Populations of cells can be from neurodegenerative disease including schizophrenia, Alzheimer's, autism, and epilepsy.

In some embodiments, populations of cells exhibit mosaicism in blood or cell cultures, which shows as heterogeneous chromosomes. For example, blood from an irradiated person treated with stem cells, may have many genomes, e.g., a heterogeneous cell population. In cases of clonal outgrowth where a few take over and faster and after a few rounds of multiplication the cells lose heterogeneity. Significant rearrangements which would trigger a higher genotoxicity risk score for these diseases include the following genes and loci, such as, somatic mosaicism, deletions, copy number variation or other structural rearrangements involving 2q31.2 (PRKRA gene), 5q35.2 (BOD1 gene) and 7p15.2 (CBX3 gene) in brain cells, and somatic mosaicism, deletions, copy number variation, or other structural rearrangements involving the NEGRI1, PTBP2, CADPS, KMT2,E KCNN2, MACROD2, MMP12, NTM, ANTXRL,CHST9, DNM3, NDST3, SDK1, STRC, SKY, SCN1A, SCN2A, SETD2, ARID1B, AKT1, AKT3, MTOR, PIK3CA, TSC1,TSC2, mTOR, PI3K-Akt, p53, and PTEN genes (various chromosomal loci), and a cluster of genes on Chr17 (KANSL1, WNT3, MAPT and CRHRI) in blood and neuronal cells.

Populations of cells include cells for instability testing where marker for instability are tested such as general genome hyperploidy, aneuploidy at chromosome 21, aneuploidy chromosome X, aneuploidy chromosome 18 and presence of micronuclei.

Genome editing, or genome engineering, or gene editing, is a type of genetic engineering in which DNA is inserted, deleted, modified or replaced in the genome of a living organism. Unlike early genetic engineering techniques that randomly inserts genetic material into a host genome, genome editing targets the insertions to site specific locations.

CRISPR (an acronym for clustered regularly interspaced short palindromic repeats) is a family of DNA sequences found in the genomes of prokaryotic organisms such as bacteria and archaea. Cas9 (or "CRISPR-associated protein 9") is an enzyme that uses CRISPR sequences as a guide to recognize and cleave specific strands of DNA that are complementary to the CRISPR sequence. Cas9 enzymes together with a guide RNA CRISPR sequence form the basis of a technology known as CRISPR-Cas9 that can be used to edit genes within organisms.

Transcription activator-like effector nucleases (TALEN) are restriction enzymes that can be engineered to cut specific sequences of DNA. They are made by fusing a TAL effector DNA-binding domain to a DNA cleavage domain (a nuclease which cuts DNA strands). Transcription activator-like effectors (TALEs) can be engineered to bind to a desired DNA sequence, so when combined with a nuclease, DNA can be cut at specific locations.

Zinc-finger nucleases (ZFNs) are artificial restriction enzymes generated by fusing a zinc finger DNA-binding domain to a DNA-cleavage domain. Zinc finger domains can be engineered to target specific desired DNA sequences and this enables zinc-finger nucleases to target unique sequences within complex genomes. By taking advantage of endogenous DNA repair machinery, these reagents can be used to alter the genomes of higher organisms.

Banded dGH and dGH Paints

In some embodiments, illustrated in a non-limiting manner by the dGH probes of Table 1, the oligonucleotides can be labelled with fluorescent labels that result in bands on a single-stranded sister chromatid that are different colors (e.g., blue, green, red, magenta, yellow, orange, etc.). In some embodiments herein, this is referred to as banded dGH or multi-color banded dGH and the corresponding probes can be referred to as a set of multi-colored banding dGH probes or a set of multi-color dGH probes used for multi-color banding. Methods herein that include a dGH reaction can in some embodiments include a multi-color banded dGH reaction. For such methods a dGH reaction can be performed by contacting a pair of single-stranded sister chromatids in a metaphase spread with two or more dGH probes, each dGH probe comprising a fluorescent label of a set of fluorescent labels, wherein at least two of the two or more dGH probes each binds to a different target DNA sequence on one of the single-stranded sister chromatids and each comprises a fluorescent label of a different color. Furthermore, such methods can include performing fluorescence analysis of one or both single-stranded sister chromatids by detecting fluorescence signals generated based on a hybridization pattern of the at least two dGH probes to one or both single-stranded sister chromatids. The fluorescence analysis can then be used to assess for the presence of chromosome variants and/or repair events. In some embodiments, performing fluorescence analysis comprises generating spectral measurements. In some embodiments, performing fluorescence analysis comprises generating a fluorescence pattern from one or both single-stranded sister chromatids.

In some embodiments, multi-color banded dGH can be performed by a) contacting a pair of single-stranded sister chromatids generated from the chromosome in a metaphase spread, with two or more dGH probes, each dGH probe comprising a fluorescent label of a set of fluorescent labels, wherein each dGH probe comprises a pool of single-stranded oligonucleotides that comprise a same fluorescent label of the set of fluorescent labels, wherein each single stranded oligonucleotide of a pool binds a different complementary DNA sequence within a same target DNA sequence found on one of the single-stranded sister chromatids, wherein at least two of the two or more dGH probes each binds to a different target DNA sequence on one of the single-stranded sister chromatids and each comprises a fluorescent label of a different color.

Such methods in some embodiments further include generating a fluorescence pattern from one or both single-stranded sister chromatids using fluorescence detection, wherein the fluorescence pattern is based on a hybridization pattern of the two or more dGH probes to one or both single-stranded sister chromatids of the pair. Furthermore, such methods can include assessing based on the fluorescence pattern, the presence of chromosome variants and/or repair events. In some embodiments, the assessing based the fluorescence pattern comprise:

(c) (i) comparing the fluorescence pattern of the one or both single-stranded sister chromatids to a reference fluorescence pattern representing a control sequence; and
(c) (ii) detecting at least one difference between the reference fluorescence pattern and the fluorescence pattern of the one or both single-stranded sister chromatids.

In some embodiments, dGH reactions herein utilize dGH paints. Such dGH paints in illustrative embodiments are a probe that is a pool of fluorescently labeled, single-stranded oligonucleotides that bind a unidirectional tiled set of complementary DNA sequences across a chromosome, for example spanning 75%, 80%, 85%, 90%, 95%, 96%, 97%, 98%, 99% or all of a chromosome or single-stranded sister chromatid. In some embodiments, whole genome dGH paints (e.g., dGH SCREEN™, KromaTiD, Inc., Longmont, CO, USA), also referred to as dGH whole chromatid paints (e.g., dGH paints, see Table 1), which are fluorescently-labelled single-stranded, unidirectional tiled oligonucleotides, for every chromosome of a genome, for example every human chromosome (i.e., autosomes 1-22, and sex chromosomes X and Y) can be hybridized to metaphase spreads and analyzed using fluorescence microscopy. Thus, dGH paints, for example, can be a set of colors, such as five distinct color panels such that chromosomes painted the same color can be differentiated by size, shape, and centromere position, such as is shown in Fig. 3B and the right side of FIG. 4.

Thus, in some embodiments, the single-stranded oligonucleotides that make up a probe are tiled across some or substantially all, or all of a chromatid. Accordingly, in some embodiments, each single-stranded oligonucleotide of a pool of single-stranded oligonucleotides that make up a probe binds to one of a series of target DNA sequences, wherein the 5' end of a target DNA sequence is the 5' end nucleotide of a complementary DNA sequence that is closest to the 5' end of a single-stranded chromatid that is bound by a single-stranded oligonucleotide of that probe, and the 3' end of that target DNA sequence is the 3' end nucleotide of the complementary DNA sequence that is closest to the 3' end of the single-stranded chromatid that is bound by a single-stranded oligonucleotide of that probe.

In some embodiments, methods and compositions are disclosed herein for the detection of chromosome numerical and structural variants and repair events by labeling of single-stranded chromatids with dGH probes of different colors. The hybridization pattern of the labeled probes produces a spectral profile which enables high-resolution detection of structural variants and repair events, facilitating distinction of benign variations from deleterious structural variations. Further, the spectral profile provides information regarding complex structural variations where more than one rearrangement of chromosomal segments may have occurred.

In certain aspects, sets of labeled probes can be designed to provide bands bracketing the centromere of one or more chromosome and such probes can be run as a single set or panel of probes or a plurality of (i.e., multiple) sets or panels of probes for chromosome identification and enumeration. In certain aspects, bands on either side of the centromere of each chromosome can be labeled in different colors for further differentiation of p and q arms.

In certain aspects, sets of labeled probes can be designed to provide bands which target the subtelomeric and/or telomeric regions of one or more chromosome. In some aspects, the p and q arm terminal bands of a set of probes can be run as a separate panel of probes or as multiple panels of probes for tracking the subtelomeric and/or telomeric regions of one or more chromosome. In certain aspects, probes directed to the subtelomeric and/or telomeric regions of one or more chromosomes provide structural information for the target chromosome as well as structural information for the particular arm of the target chromosome. Application of probes for bands to subtelomeric and/or telomeric regions provides information for detection of structural rearrangement events involving the targeted subtelomeric and/or telomeric regions.

Any individual band may cover part or all of a gene. Also, any particular gene may be covered by all or part of one or more than one band.

In certain aspects, a target enrichment strategy may be utilized wherein additional probes are utilized beyond those probes used for painting or banding entire or virtually entire chromosome(s), to a targeted area of interest, in order to detect features of the target area of interest. In certain aspects, the targeted area of interest may be smaller than a band. In certain aspects, the targeted area of interest may be limited to a portion of a band, cover one whole band, or span across portions of or the entirety of two or more bands. In certain aspects, probes used for target enrichment can be labeled with the same or different fluorophores as the band(s) within which the target enrichment probes hybridize. In aspects wherein the same fluorophore is used on the target enrichment probes the intensity of the fluorescent signal is boosted in that channel. In aspects wherein a different fluorophore is used on the target enrichment probes, a combinatorial fluorescent signal is produced.

In certain aspects, probes designed for target enrichment have the same or different design parameters as the probes used for banded paints. Using the same design parameters results in competitive hybridization, whereas using different design parameters results in a mixture of competitive and non-competitive hybridization. Target enrichment improves limit of detection and improves the ability to track specific chromosomal loci.

Any reference spectral profile may be used as a basis for comparison of the spectral profile of the chromosome under study. The reference spectral profile may be that of a chromosome with a known abnormality, a chromosome considered normal, the corresponding sister chromatid, a statistically determined normal profile, a database containing reference data for chromosomes considered to have normal or abnormal profiles, or any combination thereof. In addition, the distribution of probes designed against the reference genome or sequence (i.e., the density pattern of the probes across unique or repetitive sequences *in silico*) as it relates to a reference spectral profile (increased brightness in regions with more probes and reduced brightness in areas with less probes) may be used to identify and describe structural variation in a test sample when a deviation in the expected spectral profile of the target(s) is present.

The structural variations determined by the present methods can be of any type of structural variation from normal including but not limited to change in the copy number of a segment of the chromosome, an inversion, an insertion, a translocation, a truncation, a sister chromatid exchange, a sister chromatid recombination, a micronuclei formation, a chromothrypsis or fragmentation event or any combination thereof. Changes in the copy number of a segment may be deletions, amplifications, or any combination thereof.

The labeled probes may be labeled by any means known in the art. Probes can also comprise any number of different types of labels. Combinations of probes may also have any number of different types of labels, differing labels from one probe to another probe. The label on the probes may be fluorescent. The light emitted by the label on the probes may be detectable in the visible light spectrum, in the infra-red light spectrum, in the ultra-violet light spectrum, or any combination thereof. Light emitted from the probes may be detected in a pseudo-color or otherwise assigned a color different from the actual light emitted by the probe.

In one embodiment, a plurality of sets of probes used for hybridization comprises different sets of probes wherein each different set of probes are labeled with a different color. The plurality of sets of probes may comprise differently labeled probes, wherein the separate sets of probes are labeled with two different colors (i.e., one set of probes of a first color and a second set of probes of a second color), three different colors, four different colors, five different colors, six different colors, seven different colors, eight different colors, nine different colors, ten different colors, eleven different colors, twelve different colors, thirteen different colors, fourteen different colors, fifteen different colors, sixteen different colors, seventeen different colors, eighteen different colors, nineteen different colors, twenty different colors, twenty-one different colors, twenty-two different colors, twenty-three different colors, twenty-four different colors, twenty-five different colors, twenty-six different colors, twenty-seven different colors, twenty-eight different colors, twenty-nine different colors, thirty different colors, or more than thirty different colors. In some embodiments, each single-stranded sister chromatid can be assigned to a chromosome number based on the color of the set of one or more dGH probes that binds thereto and other visual features of the single-stranded sister chromatid.

In some embodiments, there can be a plurality of sets of probes in the range of 1-50, 1-40, 1-30, 1-20, 1-10, 10-50, 15-45, or 20-40 sets of probes. In some embodiments, there can be more than 50 sets of probes. In some embodiments, the number of sets of probes can depend on the total number of chromosome pairs in a subject whose chromosomes need to be analyzed. In some illustrative embodiments, there can be 24 sets of probes, wherein one set binds to only one human single-stranded sister chromatids including X and Y chromosome. In some embodiments, the number of probes in a particular set of probes can vary, starting from at least 1 probe in a set to more than 1, 10, 20, 30, 50, 75, or 100 probes in a set. In some embodiments, there is at least 1 probe in a set. In some embodiments, there can be 1-100, 1-90, 1-80, 1-70, 1-60, 1-50, 1-40, 1-30, 1-20, or 1-10 probes in a set. In some embodiments, there can be more than 100, 200, 300, 400, or 500 probes in a set.

The location of the label on the hybridization probe may be in any location on the probe that can support attachment of a label. The probe may be labeled on the end of the probe, labeled on the side of the probe, labeled in the body of the probe or any combination thereof. The label on the body of the probe may be on a sugar or amidite functional group of the probe.

Detection of the probes may be performed by any means known in the art. Any means may be used to filter the light signal from the probes, including but not limited to narrow band filters. Any means can be used to process the light signals from the probes, including but not limited to computational software. In some embodiments, only certain parts of the light signature from the probes are used for analysis of chromosomal structural variants.

The methods disclosed herein may be practiced in combination with other techniques for detecting chromosomal abnormalities. In one embodiment, the methods disclosed herein may be practiced in combination with chromosomal staining techniques, including but not limited to staining of chromosomes with DAPI, Hoechst 33258, actinomycin D or any combination thereof.

Directional genomic hybridization (dGH) is a technique that can be applied to measure both the rates of mis-repair and the identity of certain mis-repairs. This method can be employed to detect both *de novo* chromosomal variants in metaphase chromosomes in individual cells or can be utilized to assess chromosomal variants involving a particular genomic locus. In previous embodiments, the detection of orientation changes (inversions) sister chromatid exchanges and non-crossover sister chromatid recombination as well as a balanced allelic translocation would be visualized as the same signal pattern change in a single cell with a single method. These chromosomal variants are detected alongside and in addition to the chromosomal variants visible to standard chromosome-based cytogenetic methods of analysis (unbalanced and balanced non-allelic translocations, changes in ploidy, large inversions, large insertions, and large duplications). However, unless targeted methods are employed, differentiating the orientation change chromosomal variants (high risk) from transient repair intermediates resulting from SCE and SCR events (low risk), and balanced translocations between two homologous chromosomes (relatively low risk) is often not possible. In recent years, additional types of mis-repair and their relative contribution to oncogenesis and genomic Instability have been described, further illustrating the need for methods for determining the genotoxicity potential of a population of cells.

In certain embodiments, methods herein can utilize publicly available bioinformatic data about which genes, promotors and genomic regions to assess the risk of genotoxicity caused by the mis-repair or mis-repairs to individual cells as well as with proteome and transcriptome data to inform patient diagnosis.

Directional genomic hybridization (dGH) in aspects and embodiments herein, can be performed as either a *de novo* method which can detect structural variants against a reference (normal) genome or as a targeted method, assessing structural variants at a particular target region such as an edit site. In both embodiments, the dGH method provides definitive data on the prevalence or occurrence of one or more chromosome structural or numerical variants or repair events in individual cells. When using the targeted embodiment, the presence of a specific target can be inferred, as the assay is designed as a binary test for the target. However, the *de novo* embodiment, while able to detect almost any chromosomal variant without prior target hypothesis, can only provide a rough identity of a variant (e.g., a putative telomeric inversion of the p arm of C3, of approximately 7Mb) and cannot provide definitive data on the rearrangement type, orientation, size, location or sequence of the variant.

Banding chromosomes *via* differential staining of light and dark bands or multi-colored bands is a technique widely employed for distinguishing a normal karyotype from a structurally rearranged karyotype. Each method of banding has its strengths and weaknesses. G-banding and inverted (or R-banding with DAPI) and chromomycin staining are the most broadly used techniques for producing differential light and dark banding of chromosomes and are adequate for detecting a subset of simple structural variants including numerical variants (variations in the number of whole chromosomes or large parts of chromosomes), simple translocations, and some large inversions (depending on the degree of band pattern disruption). They are rapid and cost effective DNA-staining methods and are the current industry standard for karyotyping in clinical diagnostics. Though they provide basic karyotype information, these techniques have very limited utility for detecting smaller numerical variants (deletions and insertions) and small inversions, and often cannot be used to describe complex rearrangements. They do not provide any locus-specific information other than to describe an observed light/dark band disruption involving the general region of interest. In the case of translocations, they also have significant blind spots. If chromosome banding patterns present as alternating "...light-dark-light-dark..." sequences, as in G-banding, the resolution of exchange breakpoint locations will be inherently inferior to the same pattern presenting as alternating color sequences, say, "... R-G-B-Y...". These staining based methods are subject to "Three-band Uncertainty" in localization of translocation breakpoints (Savage 1977) that applies to the first (light-dark) situation. In addition, these methods do not detect balanced translocations that are equivalent exchanges between two homologous chromosomes with breakpoints at the same loci or nearby loci, nor will they detect sister chromatid exchanges/ sister chromatid recombination (gene conversion) events.

Whole chromosome FISH painting techniques such as SKY and MFISH can be used to provide a more precise description of observed structural variants, because each chromosome (2 copies of each chromosome per normal cell) is labeled in a different color. These techniques identify which chromosomes are involved in an observed rearrangement, but they cannot provide breakpoint coordinates nor identify the genomic segments of the chromosomes included or missing as a product of the rearrangement. For example, much like with the monochrome dGH paints, a deletion or an amplification cannot be attributed to any particular region or locus of a specific chromosome via SKY, MFISH, or similar methods.

Band-specific multicolor labeling strategies (the most well-known method is mBAND) can provide a more resolved picture of certain complex events, including identification of which segments of a particular chromosome are involved in a rearrangement, limited to the resolution of the assay. The resolution of the mBAND assay is determined by how discrete (small) the band size is in any given region, and how suitable the sample is for resolving the bands both for their presence, and their relative order (e.g., how long and stretched out the chromosomes are). But like all the other FISH-based techniques, mBAND cannot detect balanced translocations between homologous chromosomes, small inversions, or sister chromatid exchange/sister chromatid recombination events (gene conversion) events, no matter how high the resolution is. Furthermore, the bands are created by amplifying and differentially labeling portions of needle micro-dissected chromosomes through DOP-PCR to create overlapping libraries of probes and assessing these bands in a normal karyotype against high-resolution G-banding and/or inverted DAPI-banding in order to deduce the position of each band. Therefore, the precise start and end coordinates of each band are unknown and can only be inferred by comparison to the highest resolution G-banding of metaphase cells with a normal karyotype.

"Oligopainting", as referred to in the U.S. Patent Application Publication No. 2010/0304994, would have an advantage over mBAND in that the bands could be precisely designed against known genomic coordinates with synthetic oligos. The precise start and end of each band would be known genomic coordinates, and not an estimation based on comparison to light-dark banding on a normal karyotype. But like all the other FISH-based techniques, "oligopainting" would not be able to detect balanced translocations between homologous chromosomes, small inversions, or sister chromatid exchange/sister chromatid recombination events (gene conversion) events.

The presently disclosed methods for detecting structural variations provide the missing elements from the monochrome dGH paints: providing specific genomic coordinates and differentiating true inversion events (which involve a re-ordering of the genomic segments) from sister chromatid exchange events (which do not change the order of genomic segments, but which cannot be differentiated from inversions using the monochrome dGH paints). The risk associated with these 2 events (inversions are high risk, SCEs are low risk because they are essentially a "correct repair" and does not result in a change in order or copy number of genomic segments) is important for clinicians to understand. There is a risk for a loss of heterozygosity (one good copy of a gene is replaced with the bad copy- resulting in a disease phenotype) associated with sister chromatid exchange, but it should be distinguished from true inversion events in the context of risk and patient outcomes. CRISPR Cas9 and other gene editing systems which rely on DNA breaks and DNA break repair need accurate risk profiles. Differentiating these SCE/SCR "false positives" from potentially genotoxic events (inversions) is possible with the presently disclosed methods. The order of the genomic segments is visible, as well as the orientation of the signal on either the primary sister chromatid or the opposite sister chromatid (see schematics). K- Band is differentiated as a technique from the other multi-colored banding methods because of the sample preparation method required, which involves the removal of the newly synthesized DNA daughter strand from a sister chromatid complex, providing a single-stranded template that allows for chromatid- specific labeling.

In the context of gene editing, the detection and identification of structural variants produced during the manipulation and alteration of a genome is a priority for patient health. The need to measure inversions and sister chromatid exchanges as a significant piece of the repair equation alongside deletions, amplifications, and translocations at a high resolution in single cells is widely recognized by the diagnostics community as a need- as well as among regulators. The presently disclosed methods are able to deliver structural variant data that is missed by sequencing and inaccessible using other differential banding or FISH based banding methods. As outlined in the previous description, the sample preparation component of the assay in combination with the uni-directionality of the oligonucleotide probes enables an assessment of repair events that are not detectable by other banding techniques and provide and important additional level of structural variant data. Because enzyme-directed gene editing processes hijack and harness cellular synthesis and repair machinery they introduce a level of additional complexity to an otherwise very complex process. Sequencing approaches for confirming the edit, as well as for assessing the rest of the genome for un-intended effects frequently rely on the presence of an intact target sequence to generate data. However, if a resection and deletion has occurred in the region of the target sequence then amplification of the region for sequence analysis is not possible. And in a pooled DNA format, this information will be missing- which is a concern when screening for structural variations that include copy number variation and carry an increased risk for genotoxicity. Complex structural variants are also very difficult to assess via sequencing. In this way, the most genomically unstable and dangerous structural variants are the most likely to be missed by sequencing. In the context of a metaphase spread, the entire genome of each cell is available to be measured and assessed for the presence of structural variation without any amplification and sequence analysis. *De novo* rearrangements as well as rearrangements to the target of interest can be measured, and populations of edited cells can be monitored over time for both unintentional spontaneous and stable structural changes that could be of concern (like cancer-driving fusion genes) as well as the stability of the desired edit over time. With the genomic coordinate specifics offered by the presently disclosed methods, sequencing can be employed to take a deeper base-pair specific look at the structural variants observed. The two techniques can be used in concert to enable more precise detection and characterization of an edited genome.

## INDEXING SEQUENCE

In certain aspects an indexing sequence or nucleic acid tag, which is a non-functional nucleic acid tag in terms of cellular function, is provided for example as part of a recombinant nucleic acid insert. The indexing sequence or non-functional nucleic acid tag can be attached to a vector, for example a CAR-T vector for instance, and will get inserted into a transduced cell, for example a transduced CAR-T cell along with the CAR. In some embodiments, the indexing sequence is large enough such that a recombinant nucleic acid insert including for example a transgene and the indexing sequence, or the indexing sequence itself is at least 1kb, 2kb, 3kb, 4kb, or 5kb, or between 1 kb and 1.5 kb, 2 kb, 3 kb, 4 kb, or 5 kb, or between 1.5 kb and 2 kb, 3 kb, 4 kb, or 5 kb, or between 2 kb and 3 kb, 4 kb, or 5 kb, for example. This method is helpful in situations where CAR-T sequences of less than 1 kb cannot be detected easily. The indexing sequence is a unique DNA sequence for detection and is not found in any wild-type human chromosome. In such embodiments an indexing dGH probe or a tag dGH probe that binds a target DNA sequence that is complementary to all or a part of the indexing sequence, is used in a dGH reaction to detect an associated recombinant nucleic acid insert. Such embodiments can improve the ability to detect insert sites for a recombinant nucleic acid insert.

## LOCALIZED dGH

In certain embodiments, a set of labeled probes can be designed to target loci within a genome which are known to influence or cause a disease state. In one aspect, probes can be designed to target genes known to be associated with the development or presence of lung cancer. Similarly probes can be designed and utilized with the methods disclosed herein for any disease or condition of interest.

In certain embodiments, dGH probes can be designed to target loci within a genome which are known to be correlated with different states of a particular disease. In one aspect, probe sets or a plurality of probe sets can be designed to indicate the state of disease progression, for instance in a neurodegenerative disease.

In certain embodiments, probe sets can be designed to target loci within a genome which are known to be correlated with genetic disorders. In one aspect, probe sets or a plurality of probe sets can be designed as a prenatal diagnostic tool for genetic disorders.

In certain embodiments, probe sets or a plurality of probe sets can be designed to target loci within a genome to provide diagnostic tools for any disease or health condition of interest. In certain aspects, the disease or condition may be selected from diseases of the respiratory tract, musculoskeletal disorders, neurological disorders, diseases of the skin, diseases of the gastrointestinal tract and various types of cancers.

In certain embodiments, probe sets or a plurality of probe sets can be designed to target specific classes of genes within a genome. In one aspect, probes can be designed to target genes for different types of kinases.

In certain aspects, probe sets or a plurality of probe sets can be designed to focus on research areas of interest. In one aspect, probes can be designed to test almost any hypotheses relating to genomic DNA sequences in the biomedical sciences. The dGH platform differs from traditional FISH as a result of its unique chromosomal preparation technique. Analog nucleotide incorporation during the DNA replication phase of the cell cycle enables exonuclease daughter strand degradation that leaves dGH prepped chromosomes single-stranded. While the preparation conditions differ, dGH does not require additional laboratory equipment. dGH probes can be visualized using standard FISH equipment and fluorescence microscopes found in many labs, making visualization and analysis as simple as possible.

Single-stranded chromosomes allow for unidirectional dGH probe binding which enables the detection of orientation information that traditional FISH assays cannot provide.

Gene editing (or genome editing) is the process of intentionally modifying an organism's genome through the insertion, deletion, or replacement of DNA. Editing is dependent upon creating a double-strand break (DSB) at a particular point within the genome. This is accomplished with engineered nucleases that are targeted to specific genomic loci with guide molecules, or with sequence specifications programmed into the nuclease itself. Gene editing has been carried out with a variety of recognized methods. Widely used editing systems include CRISPR/Cas9, ZFNs, TALENs, and meganucleases. Each of these systems operate by targeting an engineered nuclease to an exact location within the genome where they bind and create sequence-specific DSBs. A target DNA sequence can be deleted, modified or replaced using the cell's endogenous repair machinery. Insertions and deletions at the edit site can range in size from a large sequence to a single base pair. Nuclease engineering, optimized delivery conditions and cellular repair mechanisms enable researchers to manipulate segments of DNA and the genes they encode for.

Editing-associated errors, both on- and off-target, result in genomic variants which could impact patient safety. In order to realize the clinical potential of gene editing treatments, all editing associated errors must be identified and quantified. Editing-associated errors can be broadly classified into three categories: mis-edits, mis-repairs, and mis-edit/mis-repair combinations. Mis-edits occur when the editing enzyme creates off-target DSBs at homologous or random sites in the genome. Mis-edits typically result in small insertions or deletions (indels) of nucleotides at unintended genomic loci.

Mis-repairs occur when a cell's endogenous machinery incorrectly repairs on-target nuclease-induced DSBs. Mis-repairs result in unintended changes to the edit site that can vary from single base pair insertions/deletions to large genomic rearrangements.

Additionally, combinations of these errors can take place in which a mis-repair occurs at an off-target site. While less frequent, this can result in genomic changes that are particularly complex and difficult to identify. All editing-associated errors can result in genomic variants that are potentially harmful and represent risk for the patient. Measuring nuclease-induced changes at the edit site and throughout the genome is necessary, since it is possible for even low-frequency, heterogeneous, rearrangements to have serious consequences. Understanding the existing heterogeneity and spontaneous rate or rearrangements that exist pre-editing is essential for measuring editing effects.

Disclosed directional Genomic Hybridization (dGH) methods provide an efficient and practical technique for measuring genomic baselines, on- and off-target editing effects and the persistence of genomic variants over time. By measuring structural variation in many single cells, disclosed methods can be used to quantitate individual on- and off-target variants, including those that are present in less than one percent of the edited cells. dGH utilizes an innovative chromosome preparation technique, that incorporates analog nucleotides, daughter strand degradation and synthetic TAG-oligos. This design enables single assay de novo identification of sequence, location, and orientation for each genomic structural change. Synthetic TAG-oligos are bioinformatically designed to be repeat-free. dGH TAG-oligos bind single-stranded DNA in a unidirectional manner, allowing for the de novo visualization of complex structural rearrangements which are undetectable with any other method. dGH provides a straightforward method for obtaining quantifiable structural genomic data at the single cell level. dGH fits into the gene editing workflow as an orthogonal and complementary analytical technique to sequencing.

dGH can be employed at multiple time points to monitor chromosomal structure throughout the lifetime of the editing process.

Pre-edit: dGH provides a measurement of pre-existing structural variation which is unique to each model, person, and cell type. In addition to establishing a genomic baseline, dGH can be used for process optimization. dGH can use the on-and off-target rates of structural variation to monitor the effects of differing process variables including engineered-nuclease variants, guide strand choice and delivery method, and direct comparisons within the cell lines of interest.

Post-edit: dGH can measure structural rearrangements at multiple time points immediately following editing. In addition, dGH can be used to track the persistence of measured variants over time. From establishing a baseline structural variation rate, to measuring editing-associated changes, to tracking genomic integrity after treatment, dGH provides a comprehensive structural genomic profiling technique which helps to ensure that any genomic rearrangements resulting from editing are accounted for. In contrast to the many sequencing-based analysis approaches available, dGH provides direct visualization of structural variants at the single cell level. Unlike sequencing, dGH does not rely on pooled DNA and it provides rearrangement data within the context of the cell. Fundamental differences between dGH and sequencing result in fundamental differences between the data that each platform provides.

Sequencing based approaches inherently provide data based on isolated and pooled DNA from edited cell populations. This means that the cellular context in which genetic aberrations occur is entirely lost. The dGH process, however, gathers data from actively dividing cells, resulting in a cellular 'snapshot' of the genetic structural variation that has taken place within that cell up until the point of arrest and fixation.

Additionally, sequencing approaches rely on PCR amplification of a specific genomic region of interest. This type of amplification subjects results to PCR biases and makes de novo analysis and analysis of regions high in repeats challenging.

Ultimately sequencing is ideal for high throughput analysis of specific regions of the genome, while dGH can provide whole genome, de novo characterization of low-frequency or heterogeneous variants. Each method has different strengths that provide unique data and both methods are required in order to gain a comprehensive understanding of editing and its associated risks.

In certain aspects, sets of probes of single stranded oligonucleotides can be designed to directly visualize and characterize rearrangements at edit sites. By using synthetic single stranded oligonucleotides and repeat-free bioinformatic design, disclosed methods can track edit site translocations, inversions, copy number variants, viral integrations and other complex rearrangements within an approximate 2 kB resolution. The disclosed methods allow examination of edits on genome one cell at a time without PCR biases or misrepresentative sequencing averages from heterogeneous cell populations. Disclosed methods based on orthogonal and complementary techniques determine a chromosome stability score and genotoxicity risk to address the safety of edits introduced into a genome.

If a specific site on the genome is to be edited, a set of custom single stranded oligonucleotides to that specific site on the genome can be developed so that the specific site on the genome can be detected and analyzed by dGH methods. For example, if a wild-type gene is to be introduced for gene therapy, then a probe having single stranded oligonucleotides for that sequence are prepared and the section of the edited gene can be analyzed for chromosome stability and genotoxicity risk. Another example is if a chimeric antigen receptor containing T cell or gene-edited cell is designed, then a CAR probe having single stranded oligonucleotides that target the CAR sequence or the gene-edited sequence are designed for detection and for analysis of chromosomal structural variants introduced because of inserting the CAR sequence or performing the gene-editing.

In certain aspects, a plurality of sets of probes made up of single stranded oligonucleotides whose complementary sequences are tiled across a target DNA sequence spanning an entire chromosome and covering all chromosomes allow direct visualization of structural rearrangements anywhere in the genome, making it possible to discover previously unseen or unsuspected rearrangements without knowing where to look in the first place. Such methods provide a discovery tool in patients with undiagnosed diseases to detect chromosomal structural variants. Disclosed methods led to detection of a previously unknown inversion in both an undiagnosed disease patient and one of their family members.

In some embodiments, a probe having a pool of 10 to 10,000 single stranded oligonucleotides labeled with one fluorescent label (for example, blue color) is designed to target a specific gene-edited sequence or CAR-T sequence, whereas the rest of the single stranded sister chromatid is detected with a probe having single stranded oligonucleotides labeled with a different colored fluorescent label (for example, red), so that a localized dGH and a generalized SCREEN analysis can be performed and analyzed simultaneously.

In certain aspects, chromosomal stability of gene-edited cells is analyzed.

In certain aspects directional genome hybridization in-Site provides whole genome tracking inserting DNA cassettes as small as 2kb.

Unidirectional dGH probes provide a single cell, genome-wide perspective of cellular engineering outcomes, including tracking of viral and non-viral mediated insert integration (CRISPR/Cas9 and alternative systems).

As an orthogonal analysis to PCR/sequencing techniques, dGH in-Site™ assays enable, through direct visualization, definitive measurement of the presence or absence of inserts. This includes genome-wide distribution and orientation of transgenes or inserted segments. When used in combination with targeted probes and/or dGH dosimetry paints, dGH in-Site™ assays provide quantitative, single cell on-vs off-target outcomes of the delivery and editing process and can be used to track the stability of the edited genome in both clonal isolates and non-clonal populations.

Offering the lowest limit of detection of integrated or genomic DNA targets by fluorescence, dGH in-Site™ is the most comprehensive tool available for researchers interested in tracking engineering outcomes in a de novo fashion. Assay benefits of in-Site™ technology.

## ANALYSIS OF EXTRACHROMOSOMAL DNA (ECDNA)

Biological samples comprising the DNA of cells are prepared to facilitate contacting the sample with oligonucleotide probes which are single-stranded, unique and complementary to at least a portion of the DNA. In certain aspects, the biological sample comprising cellular DNA further comprises ecDNA. Both the ecDNA and the chromosomal DNA can be hybridized with probes having the same nucleic acid sequences and fluorescent light signatures. In aspects where ecDNA and chromosomal DNA are similarly labeled, a determination can be made from where on the chromosome the ecDNA originated.

Oligonucleotide probes used for banding a chromosome under examination can be selected to specifically locate the chromosomal source or origination of DNA found in ecDNA. In certain aspects, spectral analysis of the hybridization pattern of oligonucleotide probes to chromosomal DNA allows for identification of the chromosomal source of DNA in the ecDNA. The comparison of spectral signatures, in certain aspects the investigation of similarities in spectral signatures, between chromosomes and ecDNA provides for identification of particular chromosomal DNA as the source of amplified regions of DNA incorporated in ecDNA. In certain aspects, the analysis of banding patterns resulting from hybridization of probes to chromosomal DNA provides for identification of genes and regions of interest in the chromosome under study. In certain aspects, a band or bands identified as of interest in chromosomes under study can then be used to inform the design of a specific probe or panels of probes if multiple bands are identified as source material incorporated into ecDNA to further characterize sequences present in the ecDNA.

Methods for analysis for ecDNA can be applied to episomal DNA, vector-incorporated DNA as well as any other DNA within a cell which is not present on a chromosome.

Spectral Analysis

Methods provided herein that include performing spectral analysis for assessing, determining, detecting, or measuring in each of at least 2 cells, a number and/or variability of each of one or more chromosomal variants and/or repair events, in certain illustrative embodiments include detecting and analyzing spectral information, such as fluorescence images or measurements made therefrom, produced upon excitation of fluorescence labels and/or dyes by a light source. Such labels and/or dyes are found on probes and/or DNA stains associated with chromatids, which typically are analyzed on metaphase spreads in methods herein. Such labels and/or dyes can be detected in the visible, infrared or ultraviolet spectrum of light. Color channels can be selected to detect specific regions of the light spectrum based on the fluorescence labels and/or dyes selected. Thus, during a dGH analysis a hybridization pattern is generated upon binding of at least 2 dGH probes to one or both single-stranded sister chromatids that are under analysis. This hybridization pattern is used to generate one or more spectral measurements upon excitation of the labels on the hybridized dGH probes typically to generate a spectral pattern, which in illustrative embodiments is a fluorescent pattern. In some embodiments, the banding. In illustrative embodiments, the banding pattern on the at least one single-stranded sister chromatid comprises bands of between 2 and 10 different colors. Typically, this is performed using a fluorescence microscope and analysis software, which generates a spectral image representing the hybridization pattern. In some embodiments, the fluorescence pattern on the at least one single-stranded sister chromatid comprising bands of different colors that are detected using a fluorescence microscope system. In some embodiments, spectral measurements can include fluorescent wavelength intensities of the labels on the hybridized probes. In some embodiments, spectral measurements can include relative fluorescent units (RFU) of the labels on the hybridized probes. In some embodiments, spectral measurements can include representation of oligonucleotide density distribution across a chromosome. In some embodiments, spectral measurements can be a collection of different data points on fluorescent wavelength intensities, and RFU. In some embodiments, spectral measurements can include any form of comparison, such as, but not limiting to overlaying of one or more data points across fluorescent wavelength intensities, oligonucleotide density distribution, and/or a chromosome image, such as, but not limiting to an ideogram. In certain illustrative embodiments, spectral measurements can include overlaying wavelength intensities of the labels on hybridized probes with an oligonucleotide density distribution. In some embodiments, oligonucleotide density along the one or both single-stranded sister chromatids is used in the detecting the structural variations and/or the repair events. In certain illustrative embodiments, spectral measurements can include overlaying wavelength intensities of the labels on hybridized probes with an oligonucleotide density distribution, and a chromosome image. Furthermore, such analysis can include overlaying markers used to detect repeat sequences over any of the multi-color dGH fluorescence information. This layering of various sources of information increases the ability to detect, determine, and classify repair events and/or structural features such as structural variations. Furthermore, this layering of these various sources of information can be combined with methods herein to narrow down the chromosomal region of a particular structural feature or repair event.

In some embodiments of any embodiments or aspects that include spectral measurements, size of the band produced by the hybridizing dGH probes can be determined. In some embodiments, spectral measurements form a banding pattern comprising bands of different colors, and. each color refers to the wavelength of light emission that can be detected as a separate and distinct wavelength. In such embodiments, the bands can be as small as 1,000 bases. In some embodiments, bands can be as small as 2,000 bases. In some embodiments, spectral measurements can include spectral intensity measurements. In such embodiments, spectral intensity measurements are along one or both sister chromatids. In some embodiments, spectral intensity measurements can be used to create a spectral fingerprint of one or both of the sister chromatids. In some embodiments, spectral measurements of one or both sister chromatids can be compared to a reference spectral measurement. In some embodiments, reference spectral measurements can include spectral intensity measurements, and in some embodiments, such reference spectral intensity measurements can be used to normalize spectral intensity measurements of one or both sister chromatids under study.

In some embodiments, spectral measurements can be used to form a spectral pattern (e.g., a spectral profile). A spectral pattern (e.g., spectral profile) can be understood as a collection of data layers that can effectively assist in assessing, determining, detecting, or measuring in each of at least 2 cells, a number and/or variability of each of one or more chromosomal variants and/or repair events as disclosed herein. Obtaining a spectral profile of a particular chromosome can comprise: (a) detecting fluorophores by methods not limiting to staining a chromosome or a portion thereof, or any technique that involve staining a DNA; (b) detecting hybridization of probes that can be achieved by detecting signals from a specific color channel and combining it with data on chromosome location; (c) integrating the signal from total fluorophores across a chromosome, or a portion thereof to form a fluorescence pattern, for example to form a fingerprint pattern of the chromosome, or a portion thereof; and (d) obtaining a profile of the fluorophores from the fluorescence pattern (e.g., fingerprint pattern).

A spectral pattern, for example a fluorescence pattern, such as a spectral profile, for example a fluorescence profile, can include the variation of light intensity at a given wavelength. As such, the fluorescence pattern (e.g., spectral profile) of the fluorescently labeled spectral image can represent a banding pattern based on hybridization of differently colored dGH probes to one or more sister chromatids. In such embodiments, the fluorescence pattern (e.g., spectral profile) represents a banding pattern comprising bands of different colors.

In certain illustrative embodiments, spectral analysis captures information about all fluorophores and/or stains in one microscopic image. Such enhanced digitized version can be enhanced for example, such that different colors generated by the microscopic analysis are more apparent and/or appear as different colors in the digitized image. As understood by those of ordinary skill in the art, spectral measurements can be obtained and analyzed by any number of methods including, but not limited to fluorescence microscopy, laser scanning microscopy, fluorescence cytometry, analysis software, or other fluorescence analyzers, and any combination thereof. In some embodiments, a scanning microscope system (e.g., ASI scanning microscope system and analysis software, such as cytogenetics software (e.g., GenASIS cytogenetics software) can be used for imaging and analysis such that a spectral profile is generated from hybridized dGH probes and is analyzed for assessing, determining, detecting, or measuring in each of at least 2 cells, a number and/or variability of each of one or more chromosomal variants and/or repair events. In some embodiments, spectral profiles of single-stranded sister chromatids generated from target chromosomes or chromosome pairs from on-test cells, can be selected for analysis from metaphase spreads and compared to spectral profiles of corresponding control target chromosomes or chromosome pairs. In some embodiments, due to the close proximity of bands to each other, adjacent bands appear to bleed over into each other. The bleeding over can be used as an additional marker to improve localization of events within a band based on the presence of bleed over from adjacent bands and the ratio of bleed over signal to band signal.

Multi-color dGH banding analysis can achieve both detection of bands down to a 5 Kb, 2 Kb, or 1 Kb target DNA sequence as well as globalized visualization of genome-wide information. This increased resolution can achieve identification of small structural variations, and repair events in a localized area of a chromosome or single-stranded sister chromatid. In some embodiments, such dGH banding analysis is combined with techniques disclosed herein, such as, for example, staining or monochrome dGH painting.

In certain embodiments, spectral analysis of dGH bands can achieve high resolution information of a chromosome down to fractions of a band. For example, individual dGH bands can be subdivided to the North end of a band, with data layers (e.g., oligonucleotide density differences between bands or regions of bands and/or repetitive sequence markers) providing information to a finer point. Analysis of this information can be used to determine, localize, and/or map a region of a chromosome in which a repair event, or a structural feature such as a chromosomal structural variant occurred. In certain illustrative embodiments, using dGH probes designed to detect a 1 Mb band, dGH banding can locate structural information within 500 Kb, 400 Kb, 300 Kb, 200 Kb, or 100 Kb of a breakpoint, compared to 100 Mb when using painting techniques alone. Methods provided herein using a level of condensation and even selecting or generating less condensed chromosomes or single-stranded chromatids can improve such resolution with respect to a site or region of a repair event or structural feature.

In certain embodiments, methods disclosed herein can be used to identify deletions within a band. For example, a 1 Mb deletion on chromosome 1 would not be identified with single color painting techniques, but with multi-color dGH banding methods as disclosed herein, the missing chromosome segment can be identified as an omitted band or one-half of 2 bands when compared to a banded reference chromosome.

In certain embodiments, methods disclosed herein can be used to identify localized information of complex structural variants, such as a structural variation within a structural variation. In some embodiments, dGH banding can be used to identify a deletion within an inversion. For example, if an inversion is covered by 5 bands, and one band is missing, comparison with the banding number of and pattern of a reference chromosome will identify the deleted band within the inversion.

In certain embodiments, methods disclosed herein can be used to identify small copy number variations, such as an insertion of a band. For example, an insert resulting in an additional band of 1 Mb can be visualized using dGH banding, which would not be possible using painting techniques.

In some embodiments, dGH banding can be used to identify viral inserts (integration sites) as small as 5 Kb, 4 Kb, 3 Kb, 2Kb, or smaller in size, such as, 1 Kb, or 0.5 Kb. In some embodiments, dGH banding can be used to identify and differentiate two sequential inserts as small as 5 Kb, 4 Kb, 3 Kb, 2Kb, or 1 Kb each in size.

In certain embodiments, the bands created by a set of dGH probes can provide structural resolution of a chromosome that range in size from about 150 Mb to 1 Kb. In illustrative embodiments, a set of dGH probes can provide a structural resolution of a chromosome that range in size between 20 Mb and 1 Kb. In some embodiments, a set of dGH probes can provide a structural resolution of a chromosome that range in size below 20 Mb, 15 Mb, 10 Mb, 7.5 Mb, 5 Mb, 1 Mb, 750 Kb, 500 Kb, 250 Kb, 100 Kb, 75 Kb, 50 Kb, 25 Kb, 10 Kb, 5 Kb, 4 Kb, 3 Kb, 2 Kb, or 1 Kb. In some illustrative embodiments, a set of dGH probes can provide a structural resolution of a chromosome that is of 1Kb in size.

In some embodiments, the aspect of structural resolution of a chromosome provided by a set of dGH probes can also be interpreted in terms of size of bands that are formed on a chromosome by the set of dGH probes. Likewise, in certain embodiments, dGH probes can be designed to provide bands on a chromosome or a portion thereof, that range in size from 50 Kb to 2 Kb. In some embodiments, bands formed by dGH probes can range in size for example, between 50 Mb and 1 Kb, 30 Mb and 1 Kb, 10 Mb and 1 Kb, 1 Mb and 1 Kb, 100 Kb and 1 Kb, 50 Kb and 1 kb, 10 Kb and 2 Kb, 8 Kb and 2 Kb, 6 Kb and 2 Kb, or 4 Kb and 2 Kb.

In certain embodiments, a set of labeled dGH probes can be designed to target loci within a genome which are known to influence or cause a disease state. In one embodiment, a dGH probe set can be designed to target genes known to be associated with the development or presence of lung cancer. Similarly, a dGH probe set can be designed and utilized with the methods disclosed herein for any disease or condition of interest.

In certain embodiments, methods disclosed herein can achieve a resolution down to 10 Kb, 5 Kb, 2 Kb or 1 Kb. Thus, dGH probes can be designed to bind to target DNA sequences, and typically are complementary to a portion of a target DNA sequence, wherein the target DNA sequences have the same size as the ranges provided herein for bands.

In certain embodiments, a set of labeled dGH probes can be designed to target loci within a genome which are known to be correlated with different states of a particular disease. In one embodiment, a dGH probe set can be designed to indicate the state of disease progression, for instance in a neurodegenerative disease.

In certain embodiments, a set of labeled dGH probes can be designed to target loci within a genome which are known to be correlated with genetic disorders. In one embodiment, a dGH probe set can be designed as a prenatal diagnostic tool for genetic disorders.

In certain embodiments, a set of labeled dGH probes can be designed to target loci within a genome to provide diagnostic tools for any disease or health condition of interest. In certain embodiments, the disease or condition may be selected from diseases of the respiratory tract, musculoskeletal disorders, neurological disorders, diseases of the skin, diseases of the gastrointestinal tract and various types of cancers.

In certain embodiments, a set of labeled dGH probes can be designed to target specific classes of genes within a genome. In one embodiment, a dGH probe set can be designed to target genes for different types of kinases.

In certain embodiments, a set of labeled dGH probes can be designed to focus on research areas of interest. In one embodiment, a dGH probe set can be designed to test almost any hypotheses relating to genomic DNA sequences in the biomedical sciences.

Directional Genomic Hybridization (dGH) Expansion

In certain embodiments, expansion microscopy (Asano et al., (2018) Current Protocols in Cell Biology e56, Volume 80) can be applied to dGH samples to improve the spatial resolution of dGH. In certain embodiments, expansion microscopy involves embedding a sample in a swellable hydrogel, then chemically linking the sample to the hydrogel. The sample can then be labelled, swelled, and imaged. The process of swelling the sample increases the spatial (x, y, z) resolution to levels comparable to confocal or super resolution imaging on a non-expanded sample. Accordingly, improved ability to localize repair events, for example structural variations is achieved.

Nodal Analysis

Methods are disclosed herein for identifying one or more structural feature (e.g., chromosomal variation) and/or repair event of a subject DNA strand. In certain aspects, such methods are implemented in a processor. In one aspect, methods for identifying one or more structural features (e.g., chromosomal variations) and/or repair events comprise receiving spectral measurements representing at least one sequence of base pairs on a subject DNA strand, the spectral measurement can include frequency data corresponding to the sequence of bases of the subject DNA strand. The frequency data can be divided into at least two color channels. In different aspects, various data are contained in the color channels, including but not limited to positional data and intensity data. A spectral pattern (e.g., spectral profile) can be created from such data and be converted into a data table comprising positional data, intensity data as well as other data determined to be of interest in the at least two color channels. A data table thus produced for a subject DNA strand can be compared with a reference feature lookup table comprising one or more feature nodes representing normal and/or abnormal features of a corresponding control DNA strand to identify one or more normal and/or abnormal features of the subject DNA strand. In one aspect, the feature node is defined by a color band representing a sub- sequence of bases of the control DNA strand beginning at a start base and ending at an end base.

Nodal analysis, wherein spectral pattern (e.g., spectral profile) information of subject DNA sequences is converted to numeric form for comparison to control or references DNA sequences can be performed in conjunction with the directional genomic hybridization methods disclosed herein or can be utilized in the context of other methods which provide polynucleotide sequence data convertible to a numeric form. In certain aspects, the reference or control lookup tables are a single table of values or multiple tables of values. In some aspects, the different reference or control look up tables provide values which correspond to different genomic regions. In certain aspects, the comparison of the lookup tables from the subject DNA with the reference or control look up tables is performed by a machine learning and/or AI algorithm. The values of spectral pattern (e.g., spectral profile) data from subject DNA strands can be related to specific nodes through analysis of control or reference lookup tables. A set of nodes can then be run through nodal analysis to find related pathways or effected pathways, wherein relationships between nodes are previously known or determined by analysis.

In certain aspects, the spectral pattern (e.g., spectral profile) data from a subject DNA strand can be stored to a memory for later comparison and analysis to determine structural features (e.g., chromosomal variations) and/or repair events of interest. In some aspects, the spectral pattern (e.g., spectral profile data can be stored in a relational database, graph database, lookup tables, or any other bioinformatics database format.

In certain aspects, spectral pattern (e.g., spectral profile) data is analyzed from DNA regions which are not spatially collocated. In some aspects, spectral pattern (e.g., spectral profile) data originate from DNA regions in spatial proximity. In certain aspects, spectral pattern (e.g., spectral profile) data is linked by a series of keys based on oligonucleotide sequences of the pool of oligonucleotides in a dGH probe, spectrum, oligonucleotide density, chromosome, chromosome arm, band ID, band orientation, and band coverage (e.g., gene region). In some aspects, genomic features can be defined by band, band spectrum, band sequence, band orientation, and band nearest neighbors or by dGH probe, dGH probe spectrum, dGH probe orientation and dGH probe nearest neighbors.

In certain aspects, a sequence across a feature, a chromosome arm, or a chromosome can be defined by beginning at the 5' end on one of the plurality of single stranded oligonucleotides that comprise a dGH probe, , band, or region of interest, then analyzing the band spectrum, size, and coverage of each band consecutively moving toward the 3' end. In some aspects, these features are converted into keys which can be compared against a database to determine the location and features of an aberration or abnormality and, by extension, which nodes in the database are affected by those aberrations or abnormalities. Some combinations of aberrations or abnormalities indicate specific rearrangement events, e.g., a truncated band in one region combined with extra signal of the same spectrum in a different region would indicate a translocation event.

Spectral patterns (e.g., spectral profile) data can be analyzed or meta-analyzed with any statistical analysis tools including but not limited to: graph theory, nodal analysis, artificial intelligence, machine learning (including k-nearest neighbor, principal component analysis, etc.), and neural networks.

Spectral profile data can be analyzed or met-analyzed with any statistical analysis tools including but not limited to graph theory, nodal analysis, artificial intelligence, machine learning (including k-nearest neighbor, principal component analysis, etc.), and neural networks.

The methods disclosed herein can be combined with methods incorporating multiple types of data into a database for analysis. In certain aspects, data from other sources includes but is not limited to sequencing, genomics, transcriptomics, proteomics, and metabolomics. In certain aspects, inversions, sister chromatid exchanges, and other dGH specific data are analyzed against sequencing data.

Comparison can be performed against known, published sequencing data or against novel or unpublished data. In some embodiments, data generated by the methods disclosed herein are summarized on a report with automatically generated ideograms showing unique and recurring rearrangements and analysis, meta-analysis, or nodal analysis on both a sample level and a cohort or experiment level.

## ORTHOGONAL METHODS

As disclosed herein, illustrative methods, kits, and compositions herein include using results of analysis of an on-test population of cells using one or more additional methods combined with those of a dGH reaction. Such methods can include, as non-limiting examples, nucleic acid sequencing, array analysis, and/or PCR. These additional methods are typically molecular and cell biological techniques for analyzing a characteristic of cells, and are sometimes referred to herein as orthogonal methods.

More specifically, in some embodiments, one or more orthogonal methods that can be performed as part of a method herein, or whose results can be used herein, include sequencing, such as, for example Sanger sequencing, next-generation sequencing (NGS) / massively parallel sequencing, single molecule sequencing, or long-read sequencing, nucleic acid amplification methods such, for example, isothermal techniques, polymerase chain reaction (PCR), quantitative PCR (qPCR), digital PCR, digital droplet PCR, microarray analysis. Some embodiments include performing the one or more orthogonal methods on a cell population prior to, in parallel with, or after performing the dGH reaction. For example, in some embodiments, a method herein includes performing NGS on a subpopulation of cells of a cell population before performing a directional genomic hybridization reaction on a different subpopulation of cells from the cell population.

Results of orthogonal methods include for example, information about one or more genomes of the cells of the cell population that was generated for example, using sequencing or array analysis. In particular, some embodiments include designing a dGH probe (i.e., some or all of a pool of single-stranded oligonucleotides making up the probe, using sequence data generated by NGS. In such embodiments, the genomic sequence information can affect the specific chromosome variants or events considered/included, or in some embodiments, can affect the weighting of such variants or events.

Kits

In some aspects, provided herein are kits and instructions for determining and ranking the potential instability, oncogenicity, genotoxicity, and relative risks for cellular gene therapies and population of cells. Kits disclosed herein comprise two, three, four, five, six or more of the components provided for performing methods herein. Such components can include any of the components used in any of the methods disclosed herein. In some embodiments, such components include components used for dGH processing, such as used in dGH harvesting, as provided herein. As non-limiting examples, such components can include dGH probes, including dGH paints, or dGH probe sets provided herein; ladder(s) (e.g., internal control ladders, internal control dGH probe ladders, and/or calibrant ladders); buffer(s); enzyme(s) (such as for degrading chromatids that include nucleoside analogs); nucleotide analogs (e.g., BrdU/C); colcemid; a fixative solution; a DNA stain (e.g., a Hoechst stain); solid support matrices optionally with one or more mask, a flow cytometry reagent, and/or a support matrix having a two-dimensional, regularly spaced arrangement of spots/positions, probes (in illustrative embodiments dGH probes or dGH probe sets), cells, and/or chromosomes. Such regularly spaced arrangement of probes can be in dry format. Kit components can further include instructions for use. In some embodiments, a kit can include a calibrant ladder dGH probe set, as disclosed elsewhere herein.

In some embodiments, kit components (e.g., probes and probe sets) can include markers of increased genotoxic risk in one or more cell populations such as, for example, neurons, T cells, CAR-T cells, or any other cells disclosed herein. In illustrative embodiments, the kits include markers of increased genotoxic risk in CAR-T cells. In embodiments, kits include components to identify one or more of: gains of one of chromosomes 17 (17q11-q25), 8 (8q24), 22q, 4q (4q28-q31 and 4q34-qtel), 11q13, and 17, and losses of one of chromosomes 13q, 6q (6q16-q22), 11p11, and 9 (9p21-q33) in a test cell population. In some embodiments, a dGH insert probe can be included that binds a dGH insert probe target DNA sequence on a recombinant nucleic acid insert. In some embodiments, the dGH insert probe can bind any dGH insert probe target DNA sequence. In some embodiments, the recombinant nucleic acid insert can encode a chimeric antigen receptor. In some embodiments, the recombinant nucleic acid insert can encode recombinant T cell receptor.

Such kit components can be contained within one or more containers that can be ordered and/or shipped together, and can be included together on a virtual product area of a store, such as an online store.

**EXEMPLARY EMBODIMENTS**

Provided in this Exemplary Embodiments section are non-limiting exemplary aspects and embodiments provided herein and further discussed throughout this specification. For the sake of brevity and convenience, all of the aspects and embodiments disclosed herein and all of the possible combinations of the disclosed aspects and embodiments are not listed in this section. Additional embodiments and aspects are provided in other sections herein. Furthermore, it will be understood that embodiments are provided that are specific embodiments for many aspects, as discussed in this entire disclosure. It is intended in view of the full disclosure herein, that any individual embodiment recited below or in this full disclosure can be combined with any aspect recited below or in this full disclosure where it is an additional element that can be added to an aspect or because it is a narrower element for an element already present in an aspect. Such combinations are discussed more specifically in other sections of this detailed description.

Disclosed herein is a method of determining a chromosome stability, in exemplary embodiments a chromosome stability score, for a test population of cells, the method comprising the steps of:

a) performing a directional genomic hybridization reaction by contacting one or more pairs of single-stranded sister chromatids with a first set of probes, each probe comprising a fluorescent label of a set of fluorescent labels, in metaphase spreads prepared from each of at least 2, 3, 4, 5, 10, 15, 20, 25, 30, 40, 50, 75, 100, 250, 500 or 1,000 cells from the test population of cells, wherein each probe comprises a pool of single-stranded oligonucleotides that comprise a same fluorescent label of the set of fluorescent labels, and wherein each single stranded oligonucleotide of a pool binds a different complementary DNA sequence within the same target DNA sequence, and;

b) assessing the presence of two or more types of chromosome variants and/or repair events in each of the cells of the test population of cells individually by detecting the set of fluorescent labels,

wherein the two or more types of chromosome variants or repair events include at least one type of chromosome variant or repair event selected from the group consisting of a sister chromatid exchange, a sister chromatid recombination, a genomic inversion of a size between 1 kilobase or 2 kilobases and less than 5, 4, 3, or 2 megabases, for example 4.9 megabases and a genomic insertion of a size between 1 kilobase or 2 kilobases and less than 5, 4, 3, or 2 megabases, for example 4.9 megabases; and

c) determining the chromosome stability, in exemplary embodiments the chromosome stability score, for the test population of cells by measuring for each of the at least 2 cells, a number and variability of each of the two or more types of chromosomal variants and/or repair events detected in each of the at least 2 cells.

Disclosed herein is a method of determining chromosome stability for a test population of cells, the method comprising the steps of:

a) performing a directional genomic hybridization reaction by contacting one or more pairs of single-stranded sister chromatids with a first set of probes, each probe comprising a fluorescent label of a set of fluorescent labels, in metaphase spreads prepared from each of at least four cells from the test population of cells, wherein each probe comprises a pool of single-stranded oligonucleotides that comprise a same fluorescent label of the set of fluorescent labels, and wherein each single stranded oligonucleotide of a pool binds a different complementary DNA sequence within the same target DNA sequence;

b) assessing a presence or absence of two or more types of chromosome variants and/or repair events in each of the at least four cells of the test population of cells individually by detecting the set of fluorescent labels, wherein the two or more types of chromosome variants or repair events comprise at least one type of chromosome variant or repair event selected from the group consisting of a sister chromatid exchange, a sister chromatid recombination, a genomic inversion of a size between 2 kilobases and 4.9 megabases and a genomic insertion of a size between 2 kilobases and 4.9 megabases; and

c) determining the chromosome stability for the test population of cells based on the presence or absence of, and/or based on the prevalence of the two or more types of chromosomal variants and/or repair events in each of the at least four cells.

Disclosed herein is a method of determining chromosome stability for a test population of cells, the method comprising the steps of:

a) performing a directional genomic hybridization reaction by contacting one or more pairs of single-stranded sister chromatids with a first set of probes, each probe comprising a fluorescent label of a set of fluorescent labels, in metaphase spreads prepared from each of at least four cells from the test population of cells, wherein each probe comprises a pool of single-stranded oligonucleotides that comprise a same fluorescent label of the set of fluorescent labels, and wherein each single stranded oligonucleotide of a pool binds a different complementary DNA sequence within the same target DNA sequence;

b) assessing a presence or absence of two or more types of chromosome variants and/or repair events in each of the at least four cells of the test population of cells individually by detecting the set of fluorescent labels, wherein the two or more types of chromosome variants or repair events comprise at least one type of chromosome variant or repair event selected from the group consisting of a sister chromatid exchange, a sister chromatid recombination, a genomic inversion of a size between 2 kilobases and 4.9 megabases and a genomic insertion of a size between 2 kilobases and 4.9 megabases; and

c) determining the chromosome stability for the test population of cells based on the presence or absence of the two or more chromosomal variants and/or repair events in each of the at least four cells.

Disclosed herein is a method of determining chromosome stability for a test population of cells, the method comprising the steps of:

a) performing a directional genomic hybridization reaction by contacting one or more pairs of single-stranded sister chromatids with a first set of probes, each probe comprising a fluorescent label of a set of fluorescent labels, in metaphase spreads prepared from each of at least four cells from the test population of cells, wherein each probe comprises a pool of single-stranded oligonucleotides that comprise a same fluorescent label of the set of fluorescent labels, and wherein each single stranded oligonucleotide of a pool binds a different complementary DNA sequence within the same target DNA sequence;

b) assessing a presence or absence of two or more types of chromosome variants and/or repair events in each of the at least four cells of the test population of cells individually by detecting the set of fluorescent labels, wherein the two or more types of chromosome variants or repair events comprise at least 2, 3, 4, 5, 6, 7, or 8, or all types of chromosome variant or repair event selected from the group consisting of a change in genome ploidy, a change in total chromosome copy number, a gain of an individual chromosome, a loss of an individual chromosome, a deletion of a chromosome, an at least 1kb deletion of a portion of a chromosome, an at least 1 kb insertion of a portion of a chromosome, insertion of an entire chromosome, a balanced translocation, an acentric unbalanced translocation, a dicentric unbalanced translocation, a complex translocation involving three or more break points, a symmetrical translocation, an asymmetrical translocation, an inversion, an insertion, a marker chromosome, chromothrypsis, a chromatid-type break, a sister chromatid recombination, a complex chromosome event, a micronucleus, a chromosome fragment that is at least 1bkb, an extrachromosomal DNA (ecDNA) that is at least 1 kb, a multi-radial chromosome, an iso-chromosome, an acentric ring, a centric ring, a chromosome condensation defect, a centromere abnormality, and a sister chromatid exchange (SCE); and

c) determining the chromosome stability for the test population of cells based on the prevalence of the four or more chromosomal variants and/or repair events detected in each of the at least four cells.

In some embodiments, the method further comprises comparing the chromosome stability score for the test population to a chromosome stability score for a control cell or population of cells. In some embodiments, an increased number of chromosomal variants and/or repair events, and/or an increased variability of the number of chromosomal variants and/or repair events in the test population of cells compared to a control cell or population of cells is indicative of a decreased chromosome stability value for the test population of cells.

In some embodiments, an increased variability of the number of chromosomal variants and/or repair events in the test population of cells compared to the control cell or population of cells is indicative of a decreased chromosome stability value for the test population of cells.

In some embodiments, the first set of probes is a first set of a plurality of sets of probes, wherein each set binds to a target DNA sequence on a different single-stranded sister chromatid. In some embodiments, each probe of a set of probes binds a different target DNA sequence of the same single-stranded sister chromatid of the one or more pairs of single-stranded sister chromatids.

In some embodiments, each single-stranded sister chromatid can be assigned to a chromosome number based on the color of the set of dGH probes that binds thereto and other visual features of the single-stranded sister chromatid.

In some embodiments, each set of the plurality of sets of probes is a single probe, and wherein each probe binds a target DNA sequence that spans at least 75%, 80%, 90%, 95%, or 99% of the length of a same single-stranded sister chromatid.

In some embodiments, complementary DNA sequences for a pool of oligonucleotides for dGH probes included in the dGH reaction are spaced to achieve a fluorescent density of 1 fluor per 250 to 1,000 nucleotides of a single-stranded chromatid of the one or more single-stranded chromatids.

In some embodiments, wherein the method further comprises comparing the chromosome stability score for the test population to a chromosome stability score for a control cell or population of cells, wherein an increased number of chromosomal variants and/or repair events, and/or an increased variability of the number of chromosomal variants and/or repair events in the test population of cells compared to a control cell or population of cells is indicative of a decreased chromosome stability value for the test population of cells.

In some embodiments, an increased variability of the number of chromosomal variants and/or repair events in the test population of cells compared to the control cell or population of cells is indicative of a decreased chromosome stability value for the test population of cells.

In one aspect, provided herein is a method of determining a genotoxicity risk for a test population of cells, the method comprising the steps of:

a) performing a directional genomic hybridization (dGH) reaction by contacting one or more pairs of single-stranded sister chromatids on at least 4, cells from the test population, with a plurality of sets of dGH probes, wherein the dGH probes of each set of dGH probes binds to a different target DNA sequence on a same single-stranded sister chromatid, wherein each set of dGH probes binds to a corresponding set of target DNA sequences on a different single-stranded sister chromatid, and wherein each single-stranded sister chromatid can be assigned to a chromosome number based on the color of the set of dGH probes that binds thereto and other visual features of the single-stranded sister chromatid;

b) assessing the presence of two or more types of chromosome variants and/or repair events in each of the at least 4 cells of the test population of cells individually based on results of the dGH reaction;

c) measuring the number and variability of each of the two or more chromosomal variant and/or repair event types detected in each of the at least 4 cells of the test population of cells; and

d) comparing the number and/or variability of each of the two or more chromosomal variant and/or repair event types detected in the test population of cells to a number and/or variability for a corresponding type of chromosomal variant and/or repair event of a control cell or a population of control cells, wherein a weight is assigned to the number and/or variability for each type of chromosomal variant and/or repair event to provide a series of weighted factors, wherein the weight assigned to obtain each weighted factor of the series of weighted factors depends on a characteristic of and/or a type of cells of the test population of cells to determine a weighted chromosomal stability score, wherein the weighted chromosomal stability score is used to determine the genotoxicity risk of the test population of cells, and wherein an increased weighted chromosomal stability score in the test population of cells compared to the control cell or the population of control cells is indicative of an increased genotoxicity risk for the test population of cells. In some embodiments, the test population of cells comprises genetically-modified cells having a recombinant nucleic acid insert, and wherein the dGH reaction, or a parallel dGH reaction is performed using a dGH insert probe that binds to the recombinant nucleic acid insert.

In some embodiments, the recombinant nucleic acid insert comprises a chimeric antigen receptor sequence, a recombinant T cell receptor sequence, a transgenic sequence, a gene-edited sequence, a deleted gene sequence, an inserted gene sequence, a DNA sequence for binding guide RNA, a transcription activator-like effector binding sequence, or a zinc finger binding sequence.

In some embodiments, the method further comprises determining a number of recombinant nucleic acid inserts at each integration site of the recombinant nucleic acid insert.

Methods, compositions and kits herein can be used for determining, ranking or determining and ranking, the potential instability, oncogenicity, genotoxicity, and/or relative risks for cellular gene therapies or for populations of cells. In some embodiments, the recombinant nucleic acid insert comprises a transgene.

In some embodiments, the transgene is a chimeric antigen receptor sequence.

In some embodiments, the transgene is a recombinant T cell receptor sequence.

In some embodiments, the transgene is a gene-edited sequence.

Disclosed herein is a method of determining a genotoxicity risk for a test population of cancer cells, the method comprising the steps of:

a) performing a directional genomic hybridization (dGH) reaction by contacting one or more pairs of single-stranded sister chromatids on at least 2, 3, 4, 5, 10, 15, 20, 25, 30, 40, 50, 75, 100, 250, 500 or 1,000 cells from the test population, with a plurality of sets of dGH probes, wherein the dGH probes of each set of dGH probes binds to a different target DNA sequence on a same single-stranded sister chromatid, wherein each set of dGH probes binds to a corresponding set of target DNA sequences on a different single-stranded sister chromatid, and wherein each single-stranded sister chromatid can be assigned to a chromosome number based on the color of the set of dGH probes that binds thereto and other visual features of the single-stranded sister chromatid;

b) assessing the presence of two or more types of chromosome variants and/or repair events in each of the at least 2 cells of the test population of cells individually based on results of the dGH reaction;

c) measuring the number and variability of each of the two or more chromosomal variant and/or repair event types detected in each of the at least 2 cells of the test population of cells; and

d) comparing the number and/or variability of each of the two or more chromosomal variant and/or repair event types detected in the test population of cells to a number and/or variability for a corresponding type of chromosomal variant and/or repair event of a control cell or a population of control cells, wherein a weight is assigned to the number and/or variability for each type of chromosomal variant and/or repair event to provide a series of weighted factors, wherein the weight assigned to obtain each weighted factor of the series of weighted factors depends on a characteristic of and/or a type of cells of the test population of cells to determine a weighted chromosomal stability score, wherein the weighted chromosomal stability score is used to determine the genotoxicity risk of the test population of cells, and wherein an increased weighted chromosomal stability score in the test population of cells compared to the control cell or the population of control cells is indicative of an increased genotoxicity risk for the test population of cells.

Disclosed herein is a method of determining an insert number of a recombinant nucleic acid insert at each insertion loci in two or more genetically-modified cells of a population of genetically-modified cells, the method comprising the steps of:

a) performing a directional genomic hybridization (dGH) reaction on metaphase spreads prepared from each of the two or more cells by contacting one or more pairs of single-stranded sister chromatids from the two or more cells with a calibrant ladder dGH probe set and a dGH insert probe that binds a dGH insert probe target DNA sequence on the recombinant nucleic acid insert, wherein the recombinant nucleic acid insert is integrated into the genome of the genetically-modified cells, and wherein each dGH calibrant probe of the calibrant ladder dGH probe set binds a single-stranded sister chromatid of the one or more pairs, at a different calibrant target DNA sequence of a known size of a set of known sizes that encompass the size of the one or more dGH insert probe target DNA sequences of the recombinant nucleic acid insert; and

b) measuring a fluorescence intensity of a fluorescence signal at each calibrant loci on the one or more pairs of single-stranded sister chromatids bound by each dGH calibrant probe and each insert loci on the one or more pairs of single-stranded sister chromatids bound by the dGH insert probe, in the metaphase spreads using fluorescence microscopy; and

c) comparing the measured fluorescence intensity of the fluorescence signals generated at two or more of the calibrant loci each bound by one of the dGH calibrant probes to the measured fluorescence intensity of the fluorescence signal generated at each insertion loci bound by the dGH insert probe, to determine the insert number at each insertion loci of the recombinant nucleic acid insert in the two or more genetically-modified cells.

In some embodiments, the comparing comprises using the fluorescence signals generated at the two or more calibrant loci and the sizes of the corresponding calibrant target DNA sequences to generate a standard curve and comparing the fluorescence signal generated at each insertion loci to the standard curve taking into account the size of the dGH insert probe target DNA sequence.

In some embodiments, the comparing comprises using the fluorescence signals generated at the two or more calibrant loci and the sizes of the corresponding calibrant target DNA sequences, and the fluorescence signal generated at each insertion loci to the standard curve taking into account the size of the dGH insert probe target DNA sequence, using formula 11 herein.

In some embodiments, the population of genetically-modified cells are a population of genome-edited cells, and wherein the method further comprises including a bystander dGH probe in the dGH reaction, wherein the bystander dGH probe recognizes a DNA binding sequence at an intended target genome editing loci.

In some embodiments, an off-target insert number and an on-target insert number are calculated by comparing the location of a fluorescent signal on the one or more pairs of single-stranded sister chromatids generated by the bystander dGH probe to location of the fluorescence signal generated at each insertion loci.

In some embodiments, the method measures integrational copy number per cell, integrational copy number per event, integrational copy number per chromosome, arm of chromosome, band location, and/or integrational copy number per defined loci.

In some embodiments, the method measures integrational copy number per defined loci and the defined loci comprise an instability locus.

In some embodiments, the method is used to determine safety and efficacy of compositions and a method used to produce the population of genetically-modified cells.

Disclosed herein is a method for preparing a plurality of metaphase spreads from a test population of cells useful for analyzing chromosome stability, comprising:

a) obtaining the test population of cells;

b) treating cells in the test population of cells with a nucleotide analog for one cell cycle;

c) preparing the plurality of metaphase spreads;

d) treating the plurality of metaphase spreads to degrade the nucleotide analog;

e) treating the degraded metaphase spreads with a nuclease to produce one or more single-stranded sister chromatids;

f) performing a directional genomic hybridization (dGH) reaction on at least four of the plurality of metaphase spreads to label one or both single-stranded sister chromatids of a pair of single-stranded sister chromatids, wherein the dGH reaction comprises contacting the one or more pairs of single-stranded sister chromatids with a first set of probes;

g) analyzing at least one pair of the labeled single-stranded sister chromatids in four or more metaphase spreads, wherein the analyzing comprises detection of chromosome variants and/or repair events; and

h) determining the chromosome stability for the test population of cells based on the detection of chromosome variants and/or repair events.

In another aspect, provided herein is a method for preparing a plurality of metaphase spreads from a test population of cells useful for analyzing genotoxicity risk, comprising:

a) obtaining the test population of cells;

b) treating cells in the test population of cells with a nucleotide analog for one cell cycle;

c) preparing the plurality of metaphase spreads;

d) treating the plurality of metaphase spreads to degrade the nucleotide analog;

e) treating the degraded metaphase spreads with a nuclease to produce one or more single-stranded sister chromatids;

f) performing a directional genomic hybridization (dGH) reaction on at least four of the plurality of metaphase spreads to label one or both single-stranded sister chromatids of a pair of single-stranded sister chromatids, wherein the dGH reaction comprises contacting the one or more pairs of single-stranded sister chromatids with a first set of probes;

g) analyzing at least one pair of the labeled single-stranded sister chromatids in four or more metaphase spreads, wherein the analyzing comprises detection of chromosome variants and/or repair events; and

h) determining the genotoxicity risk for the test population of cells based on the detection of chromosome variants and/or repair events.

Disclosed herein is a method for preparing a plurality of metaphase spreads from a test population of genetically-modified cells useful for determining an insert number of a recombinant nucleic acid insert at each insertion loci in two or more genetically-modified cells of the population of genetically-modified cells, comprising:

a) obtaining the test population of cells;

b) treating cells in the test population of cells with a nucleotide analog for one cell cycle;

c) preparing the plurality of metaphase spreads;

d) treating the plurality of metaphase spreads to degrade the nucleotide analog;

e) treating the degraded metaphase spreads with a nuclease to produce one or more single-stranded sister chromatids;

f) performing a directional genomic hybridization (dGH) reaction on at least two of the plurality of metaphase spreads to label one or both single-stranded sister chromatids of a pair of single-stranded sister chromatids, wherein the dGH reaction comprises contacting the one or more pairs of single-stranded sister chromatids with a calibrant ladder dGH probe set and a dGH insert probe that binds a dGH insert probe target DNA sequence on the recombinant nucleic acid insert;

g) analyzing at least one pair of the labeled single-stranded sister chromatids in four or more metaphase spreads, wherein the analyzing comprises detecting the dGH calibrant probe and each insert loci on the one or more pairs of single-stranded sister chromatids bound by the dGH insert probe; and

h) determining the insert number of a recombinant nucleic acid insert at each insertion loci in two or more genetically-modified cells of the population of genetically-modified cells using the dGH calibrant probe.

Methods herein typically include a contacting step. Such contacting step utilizes a reaction mixture composition, which themselves provide separate aspects herein. In such contacting step, one, two, or more, or in illustrative embodiments a pool of single-stranded oligonucleotides and in illustrative embodiments, directional probes are contacted with single-stranded sister chromatids from one or more individual cells. Each probe is a pool of single stranded oligonucleotides that have been labeled with the same fluorescent label. Each single-stranded oligonucleotide of a pool binds a different complementary sequence within the same target DNA sequence.

In certain embodiments of any aspects or embodiments provided herein, a probe can include for example, between 10 and $2x10^6$, 10 and $1x10^6$, 10 and $1x10^5$, 10 and $1x10^4$, 10 and $1x10^3$, 10 and $1x10^2$, 100 and $2x10^6$, 100 and $1x10^6$, 100 and $1x10^5$, 100 and $1x10^4$, 100 and $1x10^3$, 1,000 and $2x10^6$, 1,000 and $1x10^6$, 1,000 and $1x10^5$, 1,000 and $1x10^4$, 10-10,000, 50-5,000, 100-5,000, 100-2,500, 100-1,000, 100-500, 150-5,000, 150-2,500, 150-1,000, 150-500, 150-500, 200-5,000, 200-2,500, 200-1,000, 200-500 of single-stranded oligonucleotides in one pool of the probe. And a method, contacting step, and/or reaction mixture can include a set of probes that can include, for example, between 1, 2, 3, 4, 5, 6, 7, 8, 9, 10 on the low end of the range, and 15 on the high end, or between 1, 2, 3, 4, 5, 6, 7, 8, 9, 10 on the low end of the range, and 20 on the high end probes.

Each single stranded oligonucleotide of can be between 4 and 250, 4 and 200, 4 and 150, 4 and 100, 4 and 50, 4 and 20, 4 and 10, 5 and 250, 5 and 200, 5 and 150, 5 and 100, 5 and 50, 5 and 20, 5 and 10, 10 and 250, 10 and 200, 10 and 150, 10 and 100, 10 and 50, 10 and 20, 10 and 15, 20 and 200, 20 and 100, 20 and 50, 30 and 50, 35 and 45, or 15 and 25 nucleotides, or about or exactly 4, 5, 6, 7, 8, 9, 10, 15, 20, 25, 30, 35, 40, 45, 50, 60, 70, 75, 80, 90, 100, 150, 200, or 250 nucleotides in length. In certain illustrative embodiments, such a method is a dGH assay, as illustrated in the Examples herein.

Upon such contacting, the pool of single-stranded oligonucleotides bind their complementary DNA sequence within a target DNA sequence, which can be, for example, a target chromatid region, a target chromatid site, a target chromosome region, a target chromosome site, a target DNA region, or a target insert cassette region. Furthermore, a probe or set of probes that bind one target, or a method utilizing such probes that bind one target can be referred to as an assay. Such probes are typically labeled with a color label that is detectable using fluorescence microscopy for example in a chromosomal imaging method. The target DNA sequence can be, for example, a 1,000 nucleotide region of a chromosome or of a chromosomal fragment, or it can be an entire chromosome or chromosome fragment.

Accordingly, a target DNA sequence, in any aspect or embodiment herein, can be between 100 and $500x10^6$, 100 and $250x10^6$, 100 and $200x10^6$, 100 and $100x10^6$, 100 and $10x10^6$, 100 and $1x10^6$, 100 and $1x10^5$, 100 and $1x10^4$, 100 and $1x10^3$, 1,000 and $500x10^6$, 1,000 and $250x10^6$, 1,000 and $200x10^6$, 1,000 and $100x10^6$, 1,000 and $10x10^6$, 1,000 and $1x10^6$, 1,000 and $1x10^5$, 1,000 and $1x10^4$, 2,000 and $500x10^6$, 2,000 and $250x10^6$, 2,000 and $200x10^6$, 2,000 and

$100x10^6$, 2,000 and $10x10^6$, 2,000 and $1x10^6$, 2,000 and $1x10^5$, 2,000 and $1x10^4$, 3,000 and $500x10^6$, 3,000 and $250x10^6$, 3,000 and $200x10^6$, 3,000 and $100x10^6$, 3,000 and $10x10^6$, 3,000 and $1x10^6$, 3,000 and $1x10^5$, 3,000 and $1x10^4$, 4,000 and $500x10^6$, 4,000 and $250x10^6$, 4,000 and $200x10^6$, 4,000 and $100x10^6$, 4,000 and $10x10^6$, 4,000 and $1x10^6$, 4,000 and $1x10^5$, 4,000 and $1x10^4$, 5,000 and $500x10^6$, 5,000 and $250x10^6$, 5,000 and $200x10^6$, 5,000 and $100x10^6$, 5,000 and $10x10^6$, 5,000 and $1x10^6$, 5,000 and $1x10^5$, 5,000 and $1x10^4$, 1,000 and 250,000, 1,000 and 200,000, 1,000 and 100,000, 1,000 and 50,000, 1,000 and 25,000, 1,000 and 20,000, 1,000 and 10,000, 1,000 and 5,000, 1,000 and 2,500, 1,000 and 2,000, 2,000 and 500,000, 2,000 and 250,000, 2,000 and 200,000, 2,000 and 100,000, 2,000 and 50,000, 2,000 and 25,000, 2,000 and 20,000, 2,000 and 10,000, 2,000 and 5,000, 2,000 and 2,500, 2,000 and 2,000, 2,500 and 500,000, 2,500 and 250,000, 2,500 and 200,000, 2,500 and 100,000, 2,500 and 50,000, 2,500 and 25,000, 2,500 and 20,000, 2,500 and 10,000, 2,500 and 5,000, 3,000 and 500,000, 3,000 and 250,000, 3,000 and 200,000, 3,000 and 100,000, 3,000 and 50,000, 3,000 and 25,000, 3,000 and 20,000, 3,000 and 10,000, 3,000 and 5,000, 4,000 and 500,000, 4,000 and 250,000, 4,000 and 200,000, 4,000 and 100,000, 4,000 and 50,000, 4,000 and 25,000, 4,000 and 20,000, 4,000 and 10,000, 4,000 and 5,000, 5,000 and 500,000, 5,000 and 250,000, 5,000 and 200,000, 5,000 and 100,000, 5,000 and 50,000, 5,000 and 25,000, 5,000 and 20,000, or 5,000 and 10,000 nucleotides in length and is typically part of a chromosome or chromosome fragment.

In some embodiments, of any of the aspects or embodiments as disclosed herein that include a probe or a dGH probe, said probe or dGH probe comprises a pool of single-stranded oligonucleotides such that each of the single-stranded oligonucleotides comprise a label, such as, but not limiting to, a fluorescent label, and are complementary to a different complementary DNA sequence within a same target DNA sequence on at least one of single-stranded sister chromatid. In some embodiments, the fluorescent label comprises one of two or more fluorescent dyes conjugated at the 5' end of the single-stranded oligonucleotide. In some embodiments, pools of single-stranded oligonucleotides comprise labels of at least 2, 3, 4, 5, 6, 7, 8, 9, 10, 11, 12, 13, 14, 15, 16, 17, 18, 19, 20, 25, or 30 different colors, thus capable of emitting light at least 2, 3, 4, 5, 6, 7, 8, 9, 1011, 12, 13, 14, 15, 16, 17, 18, 19, 20, 25, or 30 different colors. In some embodiments, pools of single-stranded oligonucleotides comprise labels of 2 to 30, 3 to 25, 3 to 20, 3 to 15, 3 to 10, 3 to 8, or 3 to 7 different colors, thus capable of emitting light at 2 to 30, 3 to 25, 3 to 20, 3 to 15, 3 to 10, 3 to 8, or 3 to 7 different colors. In some embodiments, dGH probes complementary to a target DNA sequence on each single-stranded sister chromatid comprise labels of, thus capable of emitting light at, between 2 to 30, 3 to 25, 3 to 20, 3 to 15, 3 to 10, 3 to 8, or 3 to 7 different colors. In some embodiments, the different colors as disclosed herein appear adjacent to each other in a banded pattern or a dGH banded pattern. In some embodiments, a banded pattern or a dGH banded pattern comprises consecutive bands of different colors along a chromosome or typically a single-stranded sister chromatid. In some embodiments, such a banded pattern or a dGH banded pattern can be banded dGH or multi-color banded dGH and the corresponding probes can be referred to as a set of multi-colored banding dGH probes or a set of multi-color dGH probes used for multi-color banding. In some embodiments of the methods as disclosed herein, a first set of probes is a set of multi-color dGH probes, or the plurality of sets of probes are sets of multi-color dGH probes. In some embodiments, the dGH reaction is a multi-color banded dGH reaction.

In some embodiments, oligonucleotide density is used to detect the structural variant and/or repair event. In some embodiments, an oligonucleotide density along the one or both single-stranded sister chromatids can be to detect the structural variation and/or the repair event, wherein, in some embodiments, the fluorescence pattern or the dGH fluorescence pattern can be a banding pattern on the at least one single-stranded sister chromatid comprising bands of different colors that are detected using a fluorescence microscope system, wherein in such embodiments, the banding pattern on the at least one single-stranded sister chromatid can comprise bands of between 2 and 10 different colors.

In some embodiments, a label is selected from the group consisting of a label on the end of the probe, a label on the side of the probe, one or more labels on the body of the probe, and any combination thereof. In some embodiments, a label is a body label on a sugar or amidite functional group of the probe. In some embodiments, a label is selected from the group consisting of a label detectable in the visible light spectrum, a label detectable in the infra-red light spectrum, a label detectable in the ultra violet light spectrum, and any combination thereof. In some illustrative embodiments, methods as disclosed herein comprise a label that is detectable in the visible light spectrum.

The following non-limiting examples are provided purely to more fully illustrate some embodiments, and in no way limit the scope and spirit of the present disclosure. They should in no way be construed, however, as limiting the broad scope of the invention.

## EXAMPLES

### Example 1. Determination of Chromosome Stability of a Cell Line at Different Passage Numbers

**[0391]** In this example, directional Genomic Hybridization reactions were performed and whole genomes analyzed for metaphase spreads from a cell line using cells from passage 12 or passage 18. The chromosome stability for the two passages were different, and the cells at passage 18 showed subclonal outgrowth (e.g., a subclonal population).

For the whole genome directional Genomic Hybridization reaction (dGH SCREEN™, KromaTiD, Inc., Longmont, CO, USA), a set of dGH probes, each probe comprising a fluorescent label of a set of fluorescent labels (i.e., dGH whole chromatid paints, see Table 1), which are each probe included a pool of single-stranded oligonucleotides that have the same fluorescent label of the set of fluorescent labels, and wherein each single stranded oligonucleotide of a pool binds a different complementary DNA sequence within the same target DNA sequence (i.e., unidirectional tiled oligonucleotides) for every human chromosome (i.e., autosomes 1-22, and sex chromosomes X and Y) were hybridized to metaphase spreads from the cells at passage 12 or passage 18, and then imaged on an Applied Spectral Imaging Harmony system using a 100x objective (FIG. 4). The dGH probes (i.e., paints) in this assay were of five distinct color panels such that chromosomes painted the same color could be differentiated by size, shape, and centromere position, such as is shown in Fig. 3B, where Y indicates the color yellow, B = blue, R= red, G= green, and P = purple), and FIG. 4. Images of dGH painted metaphase spreads, for each cell, were sorted into karyograms and analyzed for translocations, inversions, aneuploidy, insertions, large (i.e., whole chromatid arm) deletions, and other complex structural rearrangements, shown as a cartoon in FIGs 3A and FIG. 3B, and images shown in FIG. 4, and such as described elsewhere herein.

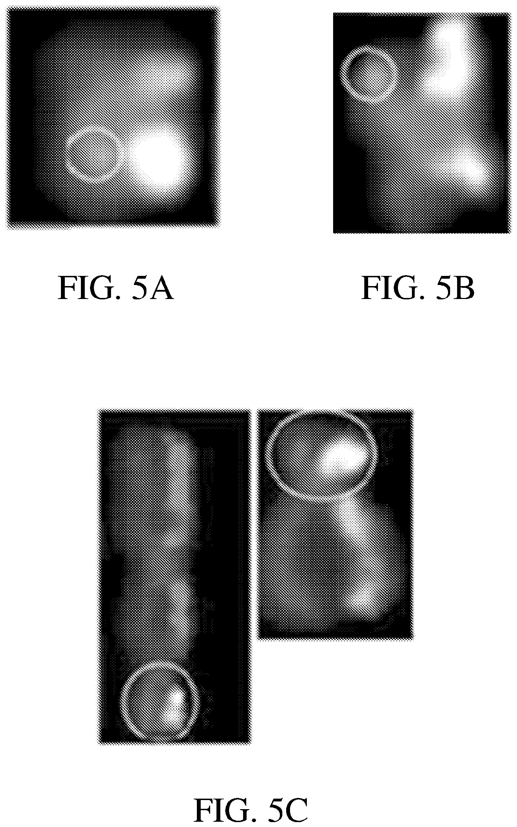

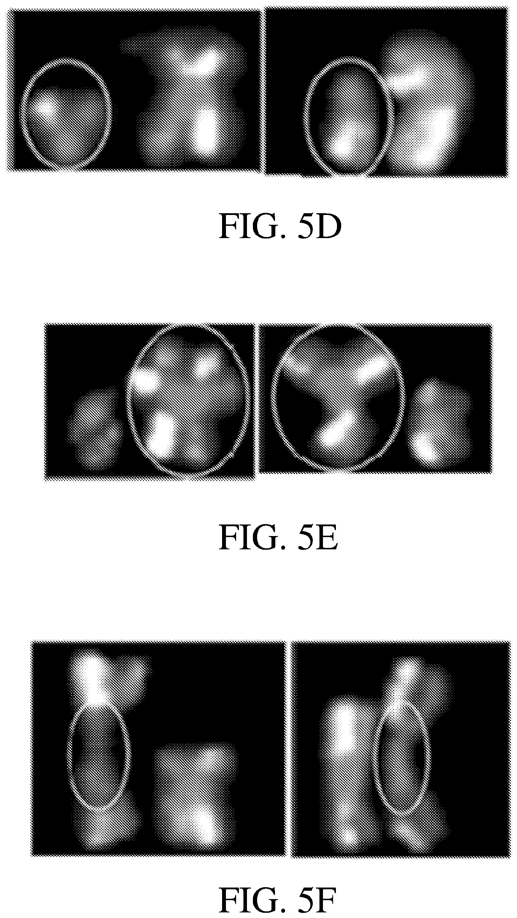

The dGH SCREEN assay (KromaTiD, Inc., Longmont, CO, USA) was performed on two separate passages of the GM24385 cell line, a human lymphoblastoid cell line GM24385 (labeled as HG-002) from the Coriell Institute for Medical Research (Camden, NJ) developed from a male of Eastern European Ashkenazi Jewish ancestry participating in the Personal Genome Project (ID huAA53E0) presenting with rare disease symptoms. For this experiment, 50 cells from passage 12 and 54 cells from passage 18 were analyzed for the presence of structural variants including translocations, inversions, large insertions (>200kb), aneuploidy and complex variants (i.e., complex structural rearrangements, and/or complex chromosome events). Substantial differences in both the prevalence of structural variants (e.g., chromosome variants) and the chromosomal variant profile between the two passages was observed, and markers of chromosome stability (i.e., markers indicative of genomic instability and, specifically, centromere instability) on Chromosome 16 (FIGs. 5A-5F) were observed in the later passage (p18) of this lymphoblastoid cell line. As can be seen in FIG. 5A, 37% of the cells contained an inversion in the q arm of Chr.16. The inversions were typically small, located in the midarm. Similarly, 19% of the late passage cells presented with a 16p arm inversion (FIG. 5B), and like the q arm inversions, the p arm inversions were also small and located in the mid-arm region. Both the inversion events could be associated with elevated chromosome instability. Other structural rearrangements identified included random Chr 16 translocations, which were found in 4% of the p18 lymphoblastic cells, where the translocations included non-reciprocal, balanced, and unbalanced variants with partner chromosomes Chr 7 and Chr 10. Further, whole arm deletions were observed in 11% of the cells.

Centromere abnormalities were also identified in the later passage cell line, including Chr 16 multi-radial associations (FIG. 5E) in 4% of the cells, as well as decondensed (i.e., elongated) centromeres and iso-chromosomes, as seen in FIG. 5F, which occurred in 22% of the cells.

Similar presentations of structural aberrations (e.g., structural rearrangements) on Chromosome 16 are seen in ICF syndrome patients (immunodeficiency, centromeric region instability), and have not been previously described for huAA53E0. ICF syndrome (immunodeficiency, centromeric region instability, facial anomalies) is a unique DNA methylation deficiency disease diagnosed by an extraordinary collection of chromosomal anomalies specifically in the vicinity of the centromeres of chromosomes 1 and 16 (Chr1 and Chr16) in mitogen-stimulated lymphocytes. These centromere abnormalities include chromosome condensation defects (e.g., chromosome decondensation of centromere-adjacent (qh) heterochromatin), multi-radial chromosomes with up to 12 arms, and whole-arm deletions. In a 2000 study (Tuck-Muller_Cell Genetics_2000), lymphoblastoid cell lines from two separate ICF patients exhibited Chr1 and Chr16 anomalies in 61% of the cells and were observed to continuously generate 1qh or 16qh breaks (i.e., chromatid-type break). No other consistent chromosomal variation was seen except for various telomeric associations, which had not been previously noted in ICF cells. Multi-radial chromosomes composed of arms of both Chr1 and Chr16 were favored over homologous associations and cells containing multi-radials (FIG. 5E) with 3 or 14 arms almost always displayed losses or gains of Chr1 or Chr16 arms from the metaphase. The data suggested that chromosome condensation defects, specifically decondensation of 1qh and 16qh, often leads to unresolved Holliday junctions, chromosome breakage (e.g., chromatid-type breaks), arm mis-segregation (e.g., isochromosome), and the formation of multi-radials that may yield more stable chromosomal variations, such as translocations.

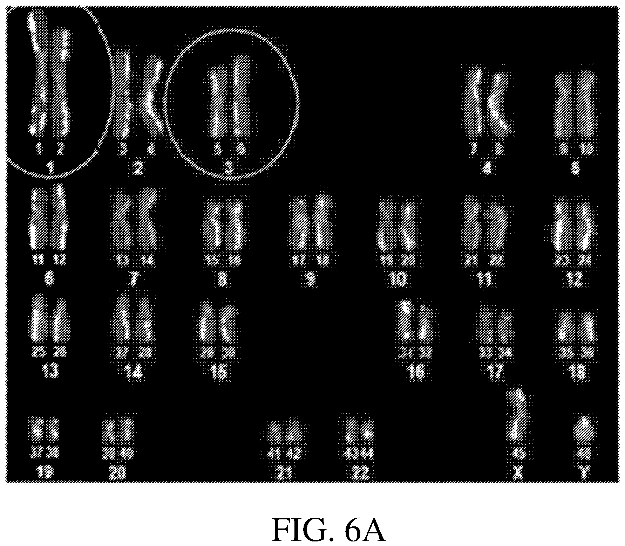

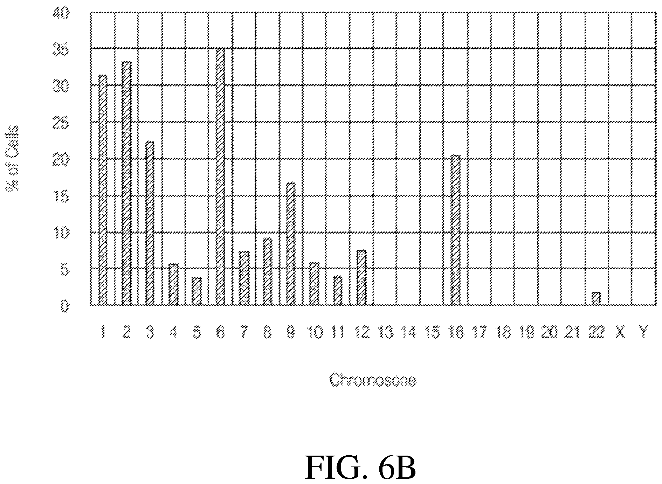

While the changes observed between the two passages indicate genomic instability (e.g., chromosome instability), transformation and a sub-clonal population in the population of cells (i.e., clonal outgrowth of the immortalized cell line), the chromosome 16 specific complex chromosome variants (i.e., abnormalities) have an elevated significance, and the rates of such events can weighted (i.e., weighted factor) in a dGH reaction analysis (contributing to an elevated stability score) for the purpose of genotoxicity risk assessment. Other passage 18 observations not present in passage 12 include substantial size and chromosome condensation defects, such as condensation differences between chromosome homologs (FIG. 6A and FIG. 6B), centromere abnormalities involving chromosomes 1 and 9 in addition to the chromosome 16 abnormalities, and copy number variations, such as monosomy observed in 41% of cells and trisomy in 5% of cells.

While the significance of these karyotypic changes are not fully understood, using a weighted chromosomal stability scoring to identify other samples presenting with these variants in a larger cohort longitudinally across passages would flag samples with potential increased genotoxicity risk for conditions associated with this genotoxic karyotypic presentation.

In addition to the karyotypic changes described previously, the rates and presentation of other structural variants (i.e., structural rearrangements) between the two passages varied significantly. Passage 12 had a low rate of random translocations, and 34 genomic inversions (events seen in >8% of cells). copy number variations, such as monosomy was observed in 18% of cells and trisomy in 4% of cells. Centromere abnormalities were largely absent, and there was a low level of complex events. Passage 18 also had a low rate of translocations, but Chromosome 16 was involved in 50% of the translocations. By passage 18 only 10 inversions (events seen in >8% of cells) were observed, and the copy number variations, specifically, monosomy was present in 41% of cells and trisomy in 5% of cells. Specifically, the number and prevalence of genomic inversions changed dramatically between the two passages, with 34 total inversions were observed in p12, and by p18 only 7 of those specific inversions were still detected. One "germ-line" inversion observed in p12 in 96% of cells, was present in only half of the cells by p18. Changes in inversion profile likely a byproduct of transformation and sub-clonal expansion, and also a hallmark of genomic instability (see, e.g., Porubsky D et al. Cell. 2022 May 26;185(11): 1986-2005.e26).

### Example 2. Chromosomal Instability and Genotoxicity of Cancer Cell Lines.

In this example, cancer cell lines were evaluated with a dGH whole genome SCREEN assay to investigate genomic instability (e.g., chromosome instability) and the overall structural variation in each line. Five cancer cell lines were cultured, harvested, and prepared for dGH as in Example 1. The whole genome dGH SCREEN assay was run on 20 metaphase spreads for each cell line.

The five cancer cell lines were DLD1 (a colorectal adenocarcinoma cell line), SNU-16 (ascites derived from a female patient with stomach cancer), COLO-HSR (isolated from a female patient with colorectal adenocarcinoma), COLO-DM, and KATO III (isolated from a male patient with stomach cancer). Each of the cancer cell lines exhibited characteristics of genome instability (e.g., chromosome instability), as evidenced by increased numbers of structural rearrangements (e.g., chromosome variants and/or repair events). and variable karyotypic presentation between cells of each cell line. While all the cancer cell lines varied from the reference human genome (hg38), the degree of variance was remarkably different between cell lines. Sample variance (variance within a cell line) and variance from the reference human genome were calculated by using the total chromosome copy number, genome ploidy (or total chromosome count per cell), number of translocations, number of homogeneously staining regions (HSRs), and number of "other" category events for each cell (in this example, complex translocation and amplification events, dicentric unbalanced translocations, isodicentrics), and ring structures); then calculating the variability between cells within the cell line, and between the cell line average and the reference genome.

The variance calculations were performed as either unweighted or weighted in this example. For the weighted variance calculation, variance in total chromosome count (ploidy) was weighted 1X, translocations 10X, HSRs 10X, and the "other" category 100X. This allowed for differentiation between the cancer cell lines according to the category of events measured. The "other" column captured events such as complex translocation and amplification events, dicentric unbalanced translocations (i.e., isodicentrics), and ring structures. which are all hallmarks of chromosome instability. Higher scores in this category, either in the variance from the reference genome or in the sample variance are particularly strong indicators of instability and were weighted accordingly. Table 3 provides a summary of scoring data for the cancer cell lines, Tables 4-5 provide unweighted and weight sample variance, respectively, and Tables 4-5 provide unweighted and weighted variance from the reference genome. "Objects in these tables show the total number of chromosomes and fragments.

Table 3: Summary of scoring data, describing average number of events per cell for each cancer cell line.

| Sample | Average Total Per Cell for: | | | |
|---|---|---|---|---|
| | Objects | Translocations | HSR | Other Events |
| DLD1 | 45 | 0.4 | 0 | 1.2 |
| SNU-16 | 83.6 | 2.1 | 0.8 | 4.8 |
| COLO-HSR | 60.2 | 12.6 | 1.3 | 1.6 |
| COLO-DM | 59.6 | 12.4 | 2 | 6.6 |
| KATO III | 82.8 | 3.2 | 2 | 4.4 |

Table 4: Summary of unweighted sample variance.

| Sample | Unweighted sample variance due to: | | | | Total Unweighted Sample Variance |
|---|---|---|---|---|---|
| | Objects | Translocations | HSR | Other Events | |
| DLD1 | 2 | 0.5 | 0 | 0.4 | 2.9 |
| SNU-16 | 11.3 | 0.1 | 0.4 | 1.5 | 13.2 |
| COLO-HSR | 480.7 | 26.4 | 0.3 | 1.1 | 508.5 |
| COLO-DM | 527.9 | 29.5 | 3 | 11.5 | 572 |
| KATO III | 1.7 | 0.2 | 0 | 0.8 | 2.8 |

Table 5: Summary of weighted sample variance.

| Sample | Weighted sample variance due to: | | | | Total Weighted Sample Variance |
|---|---|---|---|---|---|
| | Objects | Translocations | HSR | Other Events | |
| DLD1 | 2 | 5 | 0 | 40 | 47 |
| SNU-16 | 11.3 | 1 | 4 | 150 | 166.3 |
| COLO-HSR | 480.7 | 264 | 3 | 110 | 857.7 |
| COLO-DM | 527.9 | 295 | 30 | 1150 | 2002.9 |
| KATO III | 1.7 | 2 | 0 | 80 | 83.7 |

Table 6: Summary of unweighted variance from the control cell line.

| Sample | Unweighted variance from control cell line due to: | | | | Total Unweighted Variance from Control Cell Line Unweighted Chromosome Instability Score |
|---|---|---|---|---|---|
| | Objects | Translocations | HSR | Other Events | |
| DLD1 | 0.5 | 0.08 | 0 | 0.72 | 1.3 |
| SNU-16 | 708.76 | 2.2 | 0.32 | 12 | 723.3 |
| COLO-HSR | 100.82 | 79.38 | 0.845 | 1.201 | 182.4 |
| COLO-DM | 92.48 | 77.501 | 2 | 21.64 | 193.6 |
| KATO III | 675.281 | 5.28 | 2.1 | 9.68 | 692.3 |

Table 7: Summary of weighted variance from the control cell line.

| Sample | Weighted variance from control cell line due to: | | | | Total Weighted Variance from Control Cell Line Weighted Chromosome Instability Score |
|---|---|---|---|---|---|
| | Objects | Translocations | HSR | Other Events | |
| DLD1 | 0.5 | 0.8 | 0.0 | 72.0 | 73.3 |
| SNU-16 | 708.8 | 22.0 | 3.2 | 1200.0 | 1934.0 |
| COLO-HSR | 100.8 | 793.8 | 8.5 | 120.1 | 1023.2 |
| COLO-DM | 92.5 | 775.0 | 20.0 | 2164.0 | 3051.5 |
| KATO III | 675.3 | 52.8 | 21.0 | 968.0 | 1717.1 |

Discussion

Variability was measured as a function of the number of chromosome variants in a sample, differences between cells within a sample, and differences between the sample and the reference human genome. The DLD1 cell line is essentially diploid, whereas the COLO-HSR, COLO-DM, and SNU-16 cell lines have higher and variable ploidy. The chromosome stability score for COLO-DM was elevated (i.e., more unstable) as compared to the chromosome stability score for COLO-HSR. Ring structures were observed in COLO-DM, and that karyotype was very highly rearranged. The KATO III cells were highly rearranged from the reference genome but did not exhibit high variability within the cell line.

**Example 3. Quantitative Recombinant Nucleic Acid Insert Analysis Using a Single Point Internal Calibrant dGH Probe**

In this example, a whole-genome fluorescent *in-situ* hybridization--based assay comprised of dGH probes, similar to those disclosed in Table 1, designed for use in metaphase directional genomic hybridization (dGH), was used to assess on- and off-target integrations, the relative copy number of inserts present at each integration site, and any genomic structural rearrangements present in genetically-modified induced pluripotent stem cells (iPSCs). Metaphase spreads from cells that had been genetically-modified with a recombinant nucleic acid insert in their genomes were prepared as in Example 1. For the whole genome directional Genomic Hybridization (dGH) reaction, dGH insert probes designed to bind to the genomic sequence of a recombinant nucleic acid insert (also referred to herein as the insert cassette) (e.g., designed to bind to the dGH insert probe target DNA sequence), a dGH bystander (e.g., marker or babysitter) probe designed to bind the intended target genome editing locus, and a calibrant ladder dGH probe with a single point probe (e.g., a single point internal calibrant dGH probe) were hybridized to the metaphase spreads and imaged using a 100x objective.

The multiplexed design employed simultaneous measurement of recombinant nucleic inserts using dGH insert probes, along with bystander probes that showed the target loci ("on-target"), and implicitly defined the rest of the genome as "off-target". This design also included dGH paints on chromosomes 1, 2, and 3 covering approximately 1/3 of the genome to effectively measure non-specific DNA damage, for example, inversions, translocations (balanced and unbalanced), and chromothripsis. These paints also measured the rate of sister chromatid exchange (SCE) formation. At normal rates of SCE formation (defined as the rates of the wild-type un-modified cell), the rates are not concerning. However, elevated rates of SCE formation in a specific cell or sample is evidence of impaired DNA repair, which can be an early hallmark of instability.

The single point internal calibrant dGH probe was designed to hybridize to Chromosome 8 and provided a visually distinct signal of known size that served multiple functions. First, this calibrant ladder dGH probe, labeled with ATTO 550, served as a quality control check that the hybridization of small probes was successful. The single point internal calibrant dGH probe on Chr8 was also an internal size control calibrant signal from which to calculate or estimate the size of each of the unknown insertions of the recombinant nucleic acid insert. The full recombinant nucleic acid insert is ~10 kb long, but each individual insertion in a cell may be partial (<10 kb), full (~10 kb), or tandem (>10 kb). Relative sizes of inserts were calculated using Formula 11 below. This formula corrects for the difference in size and coverage of the calibrant dGH probe (90 oligos/23 kb) and the dGH insert probe (76 oligos/10 kb).

The single point internal calibrant dGH probe used in this example was an internal control, i.e., measured in each cell analyzed, to provide a cell-specific relationship between the single point internal calibrant dGH probe and any insertion (or insert) signal(s) observed in that cell. External controls are also possible, either using other cells or using non-biological fluorescence calibration tools. However, the relative presentation of the chromosomes in the metaphase spread could vary from cell to cell, even across a single slide, and this could cause variation in the signals. The signal for an internal control varies in proportion with the insert signals of a cell, while an external control would require the inclusion of a control correction factor. Thus, using an internal control, labeled and detected in the same manner as the insert signal, required correcting only for genomic differences and the resulting different number of fluorescent units in the probe.

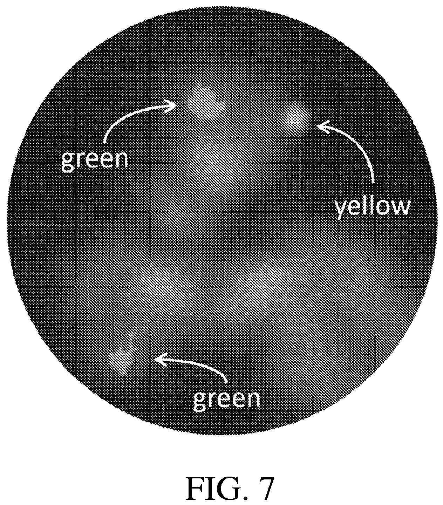

The bystander probes allowed for determination of which of the sister chromatids was the source of the insertion. For example, shown in FIG. 7, in black and white with colors indicated, an insert on the opposite sister chromatid had an inverted orientation at the target loci. Since there was no green signal co-located with the yellow insert signal, the inversion likely occurred upon insertion and was not a product of a complete or partial inversion of the target loci post insertion. Overall, 6 inverted insertions at the target loci were observed. Without a probe to mark the sister chromatids, it is not possible to determine if an off-target insertion is normally orientated or inverted. However, if on one chromosome, two insertions appear on opposite sisters, it can be assumed that one is normally oriented and the other is inverted.

Estimation of Copy Number Per Integration Event

To evaluate cell samples for the effects of recombinant nucleic acid insert integration on genotoxicity, the copy number for each integration event present in the genetically-modified cell lines was estimated using the following formulae on a per metaphase spread basis, and assuming these numbers come from the same cell. "Sum Intensity" was calculated using Formula 7:

$$\mathbf{I} = \mathbf{S} \times \mathbf{A} \qquad\qquad (7)$$

wherein the units are RFU/px$^2$, "Signal" S = count of electrons/px (in RFU/px, arbitrary units), px = pixels, and A = area (px).

The "Control Normalization Constant" ($N_{cnc}$) = Ratio of Fluorescence between Control and full size Target (Formula 8). $I_{insert}$ (full) was calculated using Formula 9. The $K_{insert}(m)$ was calculated using Formula 10, and the "Insert Copy Number" (ICN), was calculated using Formula 11, where:

"Control Normalization Constant" = Ratio of Fluorescence between Control and full-size Target

$$N_{cnc} = F_{insert} \,/\, F_{control} \qquad (8)$$

$$I_{insert}\,(full) = I_{control} \times F_{insert} = N_{cnc} \times I_{control} \; \text{RFU/px} \qquad (9)$$

$$\text{"Size in kb"} \; K_{insert}(m) = I_{insert}(m) \,/\, I_{insert}(full) \times K(full) => K_{insert}(m) - \text{RFU/RFU}/K(full) \qquad (10)$$

$$ICN_e = I_{insert}(m) \,/\, I_{insert}(full) \qquad (11)$$

Formula 11 corrects for the difference in size and coverage of the single point internal calibrant dGH probe (90 oligos/23 kb) and the dGH insert probe (76 oligos/10 kb). However, since the single point internal calibrant dGH probe and the dGH insert probe were labeled using the same fluorescent moiety, excited by the same wavelengths of light, and detected by the same detection method, no additional correction factors were required.

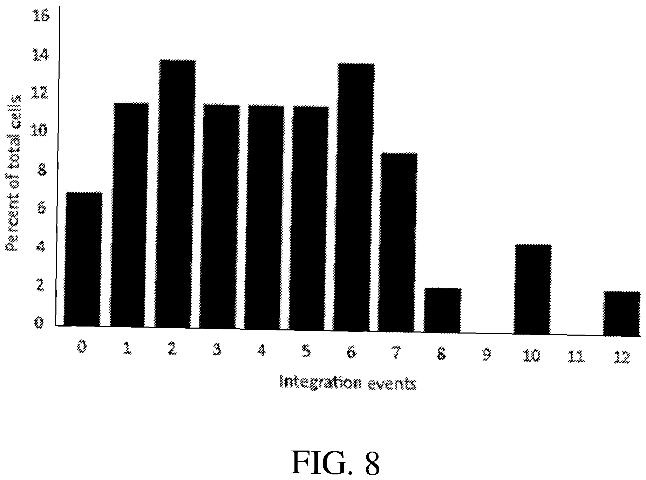

A total of 43 spreads were analyzed. Automated cytogenetics software was used for identification of insertion events and an outside boundary was drawn around each qualified signal to define the size and shape of the signals. The RFU of each signal was determined from within this boundary. Each individual insertion event was checked by an analyst to ensure that the boundaries were drawn correctly. Furthermore, each insertion signal was then classified as "on-target" or "off-target". On-target was defined as co-location with the bystander probe, which in this example was labeled in a distinct fluorescent label and detected on a separate channel. The bystander probes are typically, and in this example, were significantly larger than the dGH insert probe. Using this combination of size and fluorescence distinction allowed for an error-free identification of the bystander loci and unambiguous classification of a signal as "on" or "off" target. The size and fluorescence distinction of the bystander probes also allowed for detection of other phenomenon at the target loci, particularly structural variation (inversion, translocations, etc.) as well as sister chromatid exchanges. This dual function of the bystander probes provided results specifically valuable in genetically modified cells as the bystander probes were typically designed to mark an on-target insertion that could be directed to that specific loci by a purposeful cleavage of the DNA at the desired loci, for instance with CRISPR nucleases, which then in turn provide an opportunity for mis-repair and the generation of structural variation or the initiation of chromothrypsis. In this example, out of 43 spreads analyzed: 2% had on-target only insertions, 14% had on-target and off-target insertions, 77% had off-target insertions only, and 7% had no insertion event. On average, each cell had about 4 independent integration events. FIG. 8 shows the distribution of integration events per cell.

A summary of the probe data for the calibrant dGH probe (Chr8) and dGH insert probes is shown in the following tables. Columns marked as "measurement" are outputs from GenASIs software and are used to generate the columns marked as "calculation" as outlined in Formulas 7-11.

Table 8. Chr8 spot data. Values represent data collected from 43 spreads.

| | Background | Chr8 control spot | | | | |
|---|---|---|---|---|---|---|
| | A647 intensity | Area | A647 intensity | Chr8 Length | Signal - background | $I_{control}$ |
| | RFU | pixels | RFU | pixels | RFU | RFU x px |
| | Measurements | | | | Calculations | |
| Average | 532.9767442 | 28.7721519 | 1535.759494 | 73.40506329 | 1007.493671 | 30777.79747 |
| STDEV | 193.8674311 | 16.18324604 | 381.7760366 | 24.50084937 | 328.3412424 | 24918.9875 |
| STDEV / Avg | 0.363744635 | 0.562462137 | 0.248591031 | 0.333776013 | 0.325899062 | 0.809641675 |

Table 9. On-target insertion data. Values represent data collected from 43 spreads.

| | | On-target insert | | | | |
|---|---|---|---|---|---|---|
| | | A647 intensity | Area | Signal - background | $I_{insert}(m)$ | $I_{insert}(full)$ | $K_{insert}(m)$ |
| | | RFU | pixels | RFU | RFU x px | RFU x px | kb |
| | | Measurements | | Calculations | | | |
| Average | 1210.142857 | 14.42857143 | 840 | 12847.85714 | 9640.479469 | 25.15104113 |
| STDEV | 310.0458162 | 5.095015571 | 268.533052 | 8164.107307 | 5205.698761 | 17.96333271 |
| STDEV / Avg | 0.256205963 | 0.353119891 | 0.319682205 | 0.635445056 | 0.539983388 | 0.714218255 |

Table 10. Off-target insertion data. Values represent data collected from 43 spreads.

| | Off-target insert | | | | |
|---|---|---|---|---|---|
| | A647 intensity | Area | Signal-background | $I_{insert}(m)$ | $K_{insert}(m)$ |
| | RFU | pixels | RFU | RFU x pixel | kb |
| | Measurement | Measurement | Calculation | Calculation | Calculation |
| Average | 1283.545977 | 17.03448276 | 731.2126437 | 12620.16092 | 17.01096824 |
| STDEV | 257.6589246 | 14.98692785 | 225.9672076 | 12084.04075 | 14.50654651 |
| STDEV/Avg | 0.200739926 | 0.879799408 | 0.309030772 | 0.957518754 | 0.852776062 |

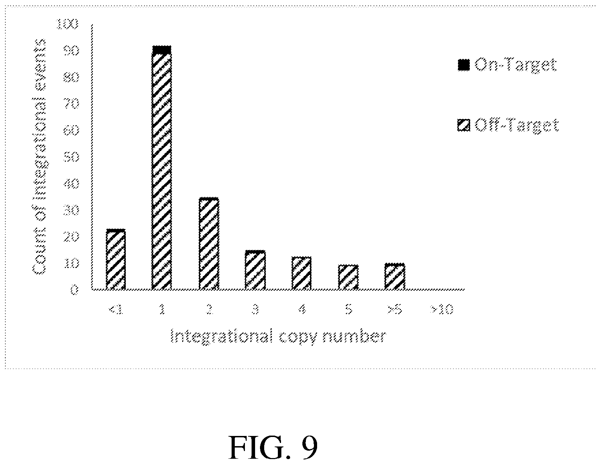

The copy number (ICN) of the inserts shown in FIG. 8 is the copy number per cell, which is determined by simply counting the raw number of signals. However, as can be seen from Tables 1 to 3, the size of those signals expressed in Relative Fluorescent Units (RFUs) varied significantly. By normalizing the RFUs for each integration event using Formulas 9-11 an insert copy number (ICN) per event ($ICN_e$) can be determined. Using this method, where the relative size of the signals is converted to a copy number defined in base units of 1 and by defining bins based on ICN as shown in Table 11, a histogram frequency of copy number per event can be estimated as shown in FIG. 9. This binning strategy reflects the inherent variability of fluorescence measurements performed on biological samples and is designed to highlight the large overall variation in raw fluorescence signal size.

Table 11. Insert copy number per event ($ICN_e$) bins of each of the inserts in FIG. 8.

| Bin | $ICN_e$ | Count |
|---|---|---|
| <0.5 | <1 | 22 |
| 0.6-1.5 | 1 | 89 |
| 1.6-2.5 | 2 | 34 |
| 2.6-3.5 | 3 | 14 |
| 3.6-4.5 | 4 | 12 |
| 4.6-5.5 | 5 | 9 |
| 5.6-10 | >5 | 9 |
| >10 | >10 | 0 |

Any integration event that was calculated to be $ICN_e$ = 0.6-1.5 was counted as a single integration event ($ICN_1$), resulting in 89/189 insertions scored as having a single copy of the recombinant nucleic acid insert. The average $ICN_e$ value was 1.7, with a range from 0.3-8.8.

The estimated average overall insertional copy number can be determined by multiplying the average of the insertional copy number per cell (ICN) (determined by counting signals) with the average insertional copy number per event ($ICN_e$) to give an estimate of the total number of successful and potentially functional insertions across all the individual genomes analyzed. Adjusting for integrations per events, the actual ICN is estimated by multiplying the ICN by

the $ICN_e$ For this data set, the ICN was estimated to increase from 4 (based on counting signals) to 6.8, a factor of nearly 70%.

FIG. 8 suggests that not only were there signals that were clearly 1 unit signal or multiple unit signals, but also signals that were smaller than a single unit signal (as shown in the <1 bin), which was defined as below 0.6 of a unit signal (as discussed above, a full insert was defined as 0.6 to 1.5 of unit signal). Signals larger than one unit signal could be tandem insertions, or concatemers of the insert.

The binning strategy was conservative in two ways. First, assuming that the structure of a recombinant nucleic acid insert is designed to include both a promotor and a transgene and thus form a fully functional insert (regardless of the genomic locus of insertion: Insert = (Promotor + Transgene), each unit increase in signal could yield multiple fully functional transgenes per insertion, even when the insertion does not target a locus with an inherent promoter. These concatemerized functional insertions could then yield enhanced, or potentially genotoxic, overexpression.

This binning strategy, including some signals less than 1 unit and greater than 1 unit, was designed to capture fully functional insertions, even in the case where a partial non-functional sequence remains attached. For instance, the $ICN_e$ =1 Bin ($ICN_1$)was purposefully designed to capture insertion events with 1 complete insertion, which was assumed to be functional, as well as functional insertions that include extra non-functional genomic sequence attached on either end of the insert. Thus, the totality of the 1 Bin should include three different genomic inserts with the same functionality and the same genotoxicity:

Exemplary Structures of Inserts in the Insertional Copy Number per event = 1 Bin ($ICN_1$)

$$Insert = (Promotor + Transgene)$$

$$Insert = Partial\ Transgene + (Promotor + Transgene)$$

or

$$Insert = (Promotor + Transgene) + Partial\ Promotor$$

The insertion of a partial sequence (the <1 Bin) could represent the insertion of a transgene without its desired promoter or alternatively the insertion of a promotor without its transgene. Inserting the bare promoter could generate potentially genotoxic results if the promotor designed to turn on a desired transgene initiated the expression of an undesired gene. By defining the Bins carefully in this manner, the <1 bin will also in part represent functional promotors which may be randomly inserted at undesirable locations within the modified genome.

Exemplary Structures of Inserts in the Insertional Copy Number per event <1 Bin ($ICN_f$)

Insert = Promotor
Insert = Transgene
Insert = Partial Promotor
Insert = Partial Transgene

Exemplary Structures of Inserts in the Insertional Copy Number per event >1 Bin ($ICN_c$)

$$Insert\ Signal = Sum\ of\ (Promotor - Transgene)\ Signals$$

In a similar manner, for this insert structure (promotor + transgene), the range of functional inserts within the bin can be defined to be within the range of: $ICN_e$-1 to $ICN_e$, for example, the $ICN_e$ = 2 bin could include signals of both 1 functional insert and 2 functional inserts, but cannot (without in situ recombination of fractional inserts sequences) contain 0 functional inserts or more than 2 functional inserts. Thus, by both counting, locating, and sizing the signals in this experiment, the signals were classified into distinct groups based on their intended functionality and potential for unexpected results, where $ICN_1$ is the number of insertional events with 1 functional insert (and a signal between 0.6 and 1.5 units), $ICN_i$ (inverted insertions) is the number of events with an inverted insert, $ICN_c$ (concatemer insertions) is the number of insertion events with more than 1 functional insert (and a signal greater than 1.5 units), and $ICN_f$ (fractional insertions) is the number of insertional events with less than 1 functional insert (and a signal less than 0.6 and units).

Table 12: Per cell rates of the various types of insertion events.

| | $ICN_1$ | $ICN_i$ | $ICN_c$ | $ICN_f$ |
|---|---|---|---|---|
| On-Target | 89 | 6 | | 22 |
| Off-Target | 3 | Not Measured | 78 | 1 |
| Total Inserts | 92 | 6 | 3 | 23 |
| ICN Per Cell | 2.14 | 0.14 | 1.88 | 0.53 |

Weights were assigned to each type of insertion event (which are classified by Bin) to estimate a genotoxicity score using Formula 13 below and the two weighting methods provided in Table 13:

$$G = W0*( ICN_a) + W1 * (ICN_1) + W2 * (ICN_i) + W3 * (ICN_c) + W4 * (ICN_f) \quad\quad (13)$$

where $ICN_a$ is the number of insertions per cell.

Table 13. Weighting scheme using Formula 13.

| Factor | Weight | Weighting Method 1 | Weighting Method 2 |
|---|---|---|---|
| $ICN_a$ | W0 | W0 = 0 if sample meets Regulatory Guideline for insertions per cell, and 1 if does not | W0 = 0 if sample meets Regulatory Guideline for insertions per cell, and 1 if does not |
| $ICN_1$ | W1 | 0 | 0 |
| $ICN_i$ | W2 | 0 | 5 |
| $ICN_c$ | W3 | 5 | 100 |
| $ICN_f$ | W4 | 100 | 5 |

Weighting Method 1 more heavily weights fractional insertions ($ICN_f$) while Weighting Method 2 more heavily weights concatemer insertions ($ICN_c$). Additionally, Weighting Method 1 does not include inverted insertions ($ICN_i$) in the genotoxicity risk. Depending on the cell type and insertion and other factors disclosed herein, the weighting method can vary.

The cells analyzed here had an average of 6.8 functional inserts per cell, so (assuming the regulatory guideline is 5 or less functional inserts per cell), W0 is 1. Thus, the two genotoxicity risks based on either weighting method 1 or weighting method 2 are:

Genotoxicity Risk (Weighting Method 1) = (6.8 * 1) + (2.14 * 0) + (0.14 * 0) + (1.88 * 5) + (0.53 * 100) = 62.4 Genotoxicity Risk (Weighting Method 2)= (6.8 * 1) + (2.14 * 0) + (0.14 * 5) + (1.88 * 100) + (0.53 * 5) = 198.15

A separate calculation of genotoxicity risk was performed using Formula 14 below, focusing on the presence of fractional insertions (the <1 Bin ($ICN_f$)). Fractional insertions could represent the insertion of a transgene without its desired promoter or the insertion of a promotor without its transgene. Inserting the promoter without the transgene could generate potentially genotoxic results if the promotor initiates expression of an undesired gene.

$$Genotoxicity\ Risk = INC_T\ (ICN_T\ Weight) + ICN_f\ (INC_f\ Weight) \quad\quad (14)$$

$ICN_1$ = Total copy number = $ICN*ICN_e$
ICN = Average Number of Insertions per cell
$ICN_e$ = Average size of insertion
$ICN_f$ = Fractional Copy Number
$ICN_1$ Weight = 0 if below regulatory guidelines; 10 if above regulatory guidelines
$ICN_f$ Weight = 2

Based on Formula 14, the Genotoxicity Risk for these cells was:

$$Genotoxicity\ Risk = 6.8 * 10 + 2 * 22 = 112$$

**Example 4. Quantitative Recombinant Nucleic Acid Insert Analysis Using a Multi Point Internal Calibrant dGH Probe.**

In this example, a whole-genome fluorescent *in-situ* hybridization--based assay comprised of dGH probes, similar to those disclosed in Table 1, designed for use in metaphase directional genomic hybridization (dGH), was used to assess on- and off--target integrations, the relative copy number of inserts present at each integration site, and any genomic structural rearrangements present in genetically-modified induced pluripotent stem cells (iPSCs). Metaphase spreads from cells that had been genetically-modified with a recombinant nucleic acid insert in their genomes were prepared as in Example 1. For the whole genome directional Genomic Hybridization (dGH) reaction, dGH insert probes designed to bind to the genomic sequence of the recombinant nucleic acid insert (also referred to herein as the insert cassette) (e.g., designed to bind to the dGH insert probe target DNA sequence), a dGH bystander (e.g., marker or babysitter) probe designed to bind the intended target genome editing locus, and a calibrant ladder dGH probe with a single point probe (e.g., a single point internal calibrant dGH probe) were hybridized to the metaphase spreads and imaged using a 100x objective.

In this example, a calibrant ladder dGH probe set was used, as an alternative to the single point calibration of Example 3. The calibrant ladder dGH probe set included probes designed with defined and distinct sizing and spacing. For instance, on human chromosome 3 such that each probe can be separated from the ladder and produced and analyzed individually, thereby allowing for multiple ladder formats to be used as needed. Table 14 is a summary of one such calibrant ladder design.

Table 14. Calibrant Ladder Design.

| Probe | Actual size | # oligos | bp coordinates |
|---|---|---|---|
| 2kb | 2084 | 15 | 30682606-30684690 |
| 5kb | 5073 | 38 | 60554840-60559913 |
| 10kb | 10298 | 76 | 120165097-120175395 |
| 30kb | 32045 | 228 | 147356004-147388049 |

Referring to Table 14, as an example, the 2 kb-sized probe consisted of 15 single-stranded DNA oligonucleotides, or oligos, that covered chromosome 3 from 30682606 bp to 30684690 bp. Each probe of the calibrant ladder was designed to be roughly equivalent in oligonucleotide density to the dGH insert probe (which was 10 kb, a pool of 77 oligos), scaled up or down according to target size in order to limit the amount of variability in coverage between the dGH insert probe and the calibrant ladder dGH probes. The calibrant ladder can be labeled with the same fluorophore as the dGH insert probe or in a different fluorophore in order to measure signal intensity and size, if an adjustment was made for the relative difference in fluorescence between the two fluorophores.

Calibrant Ladder Data Comparing Ladders Labelled in Two Separate Fluorophores (ATTO643N versus ATTO550)

Due to microscope and fluorophore differences, the area and intensity of the ladder labelled with different fluorophores was compared. Referring to Table 15, the area and intensity (RFU) values from each distinct ladder signal using calibrant ladders labeled in two different fluorophores (ATTO643N and ATTO550) was measured and used to calculate sum intensity (*I*) for each spot, using Formula 7.

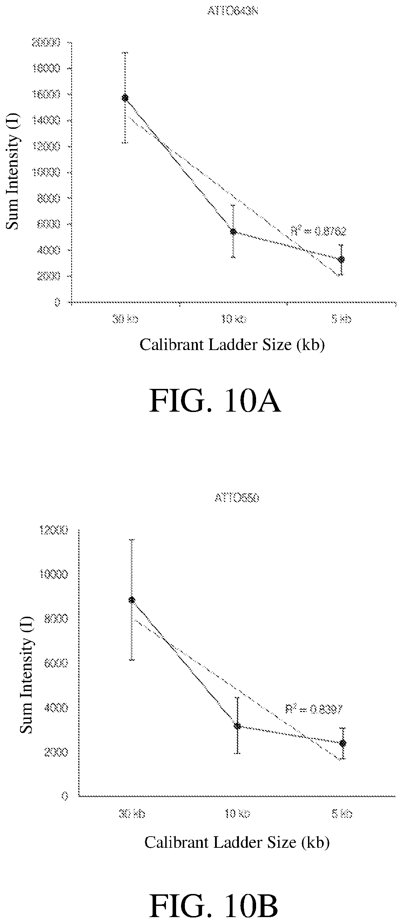

Table 15 shows the sum intensity values for each ladder spot (i.e., 30, 10 and 5 kb) labelled with the fluorophores ATTO643N and ATTO550. These values are plotted out as a line graph in FIG. 10A and FIG. 10B, respectively, which shows the sum intensity (*I*) values for 30, 10, and 5 kb ladder spots plotted as a linear graph with $R^2$ values displayed.

Table 15. Ladder Intensity Values

| | ATTO643N | | | ATTO550 | | |
|---|---|---|---|---|---|---|
| | 30 kb | 10 kb | 5 kb | 30 kb | 10 kb | 5 kb |
| Total avg | 15760 | 5452 | 3273 | 8853 | 3192 | 2408 |
| STDev | 3492 | 2000 | 1161 | 2696 | 1257 | 677 |
| % STDev | 22 | 37 | 36 | 30 | 39 | 28 |

As can be seen in FIG. 10A-10B, the trend lines are similar between the two fluorophores, ATTO643N and ATTO550. However, across all three ladder spots, ATTO643N sum intensity values are roughly 1.4 to 1.8-fold higher compared with ATTO550 spots, due to both fluorophore and microscope filter differences.

Example Estimation of Copy Number Per Integration Event

To evaluate cell samples for the effects of cassette integration on genotoxicity, the copy number for each integration event present in the edited cell lines was estimated as described in Example 3. To reduce the effects of minor observation errors, the data were binned. The bins represent copy number based on sum intensity values measured for each insert signal, and were used to estimate insert cassette copy number for each integration event measured, as seen in Table 16. Bins were created to conservatively take in the error bars present for each signal sum intensity measurement.

Table 16 shows the copy number sum-intensity data bins.

Table 16. Copy Number Sum-Intensity Bins

| Bin | ICN |
| --- | --- |
| <0.5 | <1 |
| 0.6-1.5 | 1 |
| 1.6-2.5 | 2 |
| 2.6-3.5 | 3 |

Table 17 shows an example per spread analysis using Formula 11, above.

Table 17. Spread Analysis.

| | Ladder data | | | Insert: per spread analysis | | | |
| --- | --- | --- | --- | --- | --- | --- | --- |
| | 30 kb avg SxA | 10 kb avg SxA | 5 kb avg SxA | Ncnc | $I_{insert}$(full) | $K_{insert}$(m) | Per spread bin |
| Cell 7 | 15412.5 | 7511.5 | | 0.907000511 | 6812.934338 | 3.681233189 | <1 |
| Cell 13 | 14632.5 | 5479 | 4671 | 0.907000511 | 4969.4558 | 6990705099 | 1 |
| Cell 16 | 12685 | 4578 | | 0.907000511 | 4152.248339 | 9.293760114 | 1 |
| Cell 19, insert 1 | 19496 | 5663 | 3074 | 0.907000511 | 5136.343894 | 11.75933723 | 1 |
| Cell 19, insert 2 | 19496 | 5663 | 3074 | 0.907000511 | 5136.343894 | 10.52889003 | 1 |
| Cell 21, insert 1 | 16125 | 2400 | 2194 | 0.907000511 | 2176.801226 | 9.481802817 | 1 |
| Cell 21, insert 2 | 16125 | 2400 | 2194 | 0.907000511 | 2176.801226 | 13.58874648 | 1 |
| Cell 21, insert 3 | 16125 | 2400 | 2194 | 0.907000511 | 2176.801226 | 12.21976526 | 1 |
| Cell 22, insert 1 | 17648.5 | 7396.5 | 5068 | 0.907000511 | 6708.62928 | 12.99073125 | 1 |
| Cell 22, insert 2 | 17648.5 | 7396.5 | 5068 | 0.907000511 | 6708.62928 | 5.634534034 | <1 |
| Cell 29, insert 1 | 14319.5 | 4814 | 3430.5 | 0.907000511 | 4366.30046 | 7.507499839 | 1 |
| Cell 29, insert 2 | 14319.5 | 4814 | 3430.5 | 0.907000511 | 4366.30046 | 7.903716274 | 1 |
| Cell 29, insert 3 | 14319.5 | 4814 | 3430.5 | 0.907000511 | 4366.30046 | 7.860201174 | 1 |
| Cell 29, insert 4 | 14319.5 | 4814 | 3430.5 | 0.907000511 | 4366.30046 | 4.214093869 | <1 |
| Cell 29, insert 5 | 14319.5 | 4814 | 3430.5 | 0.907000511 | 4366.30046 | 4.088129107 | <1 |
| Cell 29, insert 6 | 14319.5 | 4814 | 3430.5 | 0.907000511 | 4366.30046 | 4.369832121 | <1 |
| Cell 29, insert 7 | 14319.5 | 4814 | 3430.5 | 0.907000511 | 4366.30046 | 9.497743085 | 1 |
| Cell 29, insert 8 | 14319.5 | 4814 | 3430.5 | 0.907000511 | 4366.30046 | 6.802097169 | 1 |
| Cell 37 | 16095.5 | 4890 | 1822 | 0.907000511 | 4435.232499 | 4.425923558 | <1 |

(continued)

| | Ladder data | | | Insert: per spread analysis | | | |
|---|---|---|---|---|---|---|---|
| | 30 kb avg SxA | 10 kb avg SxA | 5 kb avg SxA | Ncnc | $I_{insert}$(full) | $K_{insert}$(m) | Per spread bin |
| Cell 42, insert 1 | 17021 | 7439 | 5713 | 0.907000511 | 6747.176801 | 5.62457471 | 1 |
| Cell 42, insert 2 | 17021 | 7439 | 5713 | 0.907000511 | 6747.176801 | 11.61967476 | 1 |
| Cell 48, insert 1 | 16344 | 8491 | | 0.907000511 | 7701.341339 | 3.630536392 | <1 |
| Cell 48, insert 2 | 16344 | 8491 | | 0.907000511 | 7701.341339 | 8.980253823 | 1 |

Standard curve analysis was performed to determine copy number (CN) per insertion event. For each spread that contained visible signals for all three calibrant probe spots (5, 10, and 30 kb), a linear standard curve was generated using the sum intensity values for each spot. $R^2$ values for all analyzed spreads varied between 0.9689-0.9998. The slope and intercept of the line generated from the calibrant standard curve were then used to calculate the estimated CN per integration event.

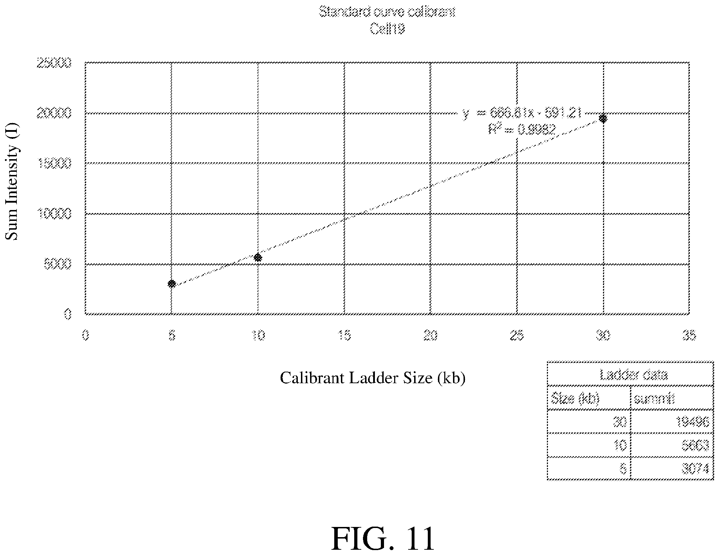

For example, Cell 19 ladder data and linear standard curve is shown in FIG. 11. Using the equation x = [(sumint$_{insert}$ + 591.21)/666.81], the estimated CN per integration event was calculated for each insert present in Cell 19. Each spread was analyzed using this method where the equation was unique to each calibrant standard curve.

Comparison Of Copy Per Insertion Event Methods

$K_{insert}$(m) values, calculated either by standard curve analysis or Formula 10, were compared and are shown in Table 18, which shows a comparison of $K_{insert}$(m) calculation methods. Although there were some differences in actual $K_{insert}$(m) values, the binning strategy data trend aligned between the two methods.

Table 18. Rate Comparison of Copy per Insertion Values (Kinsert(m) Values).

| | Standard curve analysis | | Formula 10 | |
|---|---|---|---|---|
| | Kinsert(m) | Bin 1 | Kinsert(m) | Bin 2 |
| Cell 19, insert 1 | 9.9 | 1 | 11.8 | 1 |
| Cell 19, insert 2 | 9 | 1 | 10.5 | 1 |
| Cell 21, insert 1 | 6.8 | 1 | 9.5 | 1 |
| Cell 21, insert 2 | 8.4 | 1 | 13.6 | 1 |
| Cell 21, insert 3 | 7.9 | 1 | 12.2 | 1 |
| Cell 29, insert 1 | 5.5 | 1 | 7.5 | 1 |
| Cell 29, insert 2 | 5.9 | 1 | 7.9 | 1 |
| Cell 29, insert 3 | 5.8 | 1 | 7.9 | 1 |
| Cell 29, insert 4 | 2.3 | <1 | 4.2 | <1 |
| Cell 29, insert 5 | 2.2 | <1 | 4.1 | <1 |
| Cell 29, insert 6 | 2.4 | <1 | 4.7 | <1 |
| Cell 29, insert 7 | 7.4 | 1 | 9.5 | 1 |
| Cell 29, insert 8 | 4.8 | <1 | 6.8 | 1 |
| Cell 37 | 5.1 | 1 | 4.4 | <1 |
| Cell 42, insert 1 | 1.4 | <1 | 5.6 | 1 |
| Cell 42, insert 2 | 10.2 | 1 | 11.6 | 1 |

Analysis of 50 Cells

Samples (50 cells per sample) were analyzed as described above, and insert events across the 50 cells per sample are summarized in Table 19. Samples 1-3 were metaphase spreads from genetically-modified cells, Sample 4 was metaphase spreads from cells that were treated with plasmid alone, and Sample 5 was metaphase spreads from untreated cells. The number of possible on-target insertions per cell was two (i.e., autosomic target, diploid genome).

Table 19. Cell Sample Insert Events.

| Sample | Total insertions | On-target insertions | Inverted on-target insertions | Off-target insertions | Average number of insertions per cell | % of cells with insertions | Average copy number of on-target insertions | Average copy number of off-target insertions |
|---|---|---|---|---|---|---|---|---|
| 1 | 1089 | 100 | 3 | 989 | 21.8 | 100.00 | 1.1 | 0.65 |
| 2 | 503 | 72 | 32 | 431 | 10.1 | 72.00 | 2.2 | 8.2 |
| 3 | 104 | 78 | 1 | 26 | 2.08 | 78.00 | 0.99 | 1.01 |
| 4 | 0 | 0 | 0 | 0 | 0 | 0.00 | 0 | 0 |
| 5 | 0 | 0 | 0 | 0 | 0 | 0.00 | 0 | 0 |

Structural Rearrangement Event Summary of the Bystander (e.g., Babysitter) Probe

Table 20 shows a summary of rearrangement events to target genomic locus (bystander probe signal).

Table 20. Bystander (e.g., Babysitter) Probe Rearrangement Event Summary

| Sample | Deletions | Gain-Insert | Gain-Aneuploidy | Translocations | Inversions/SC''s |
|---|---|---|---|---|---|
| 1 | 5 | 1 | 1 | 0 | 38 |
| 2 | 12 | 6 | 3 | 2 | 41 |
| 3 | 1 | 0 | 1 | 0 | 15 |
| 4 | 2 | 1 | 0 | 0 | 18 |
| 5 | 1 | 0 | 1 | 0 | 12 |

## Example 5. Measuring off-target effects of CRISPR edits in HEK293T Cells

In this experiment, two concurrent CRISPR edits were performed to the P53 gene on chromosome 17 in HEK293FT cells and purposefully designed dGH assay was used to assess genomic structural rearrangements to the P53 gene. HEK2953FT cells do not have a normal human duplex genome, but instead have four copies of chromosome 17. Three of the copies of chromosome 17 have a normally situated P53 gene, on the fourth chromosome 17, the P53 gene has been deleted.

For this experiment, a standard two dGH probe fluorescent *in-situ* dGH hybridization-based assay design was used. This assay design is comprised of a dGH probe, designed for centromere enumeration and a dGH probe covering the gene target. By defining a normal signal pattern, this assay design provides a method for enumerating normally structured P53 genes and also locating those genes on the C17 chromosome in the correct orientation. Changes to the signal pattern indicated changes to the Copy number, structure or location of the P53 gene.

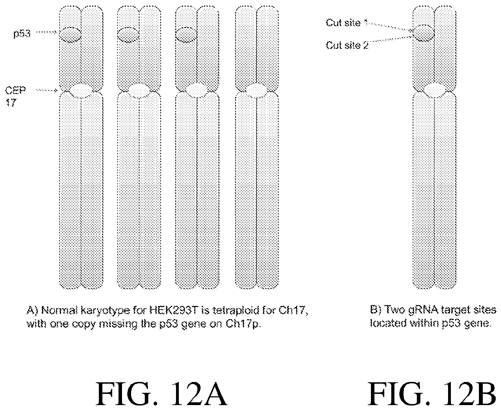

The dGH insert probe was designed against the genomic sequence of the P53 gene and the centromere enumeration probe (CEP) was designed to a sub-centromeric region abutting the centromere on C17. These probes were concurrently hybridized to HEK293T metaphase spreads derived from three different test population of cells (i.e., study samples) using the dGH reaction method as described in Example 1. For this example, study samples were: 1) unmodified HEK293T cells 2) genetically modified HEK293T cells with two recombinant nucleic acid inserts (i.e., two concurrent edits to the P53 gene) and 3) a control sample of HEK293T cells exposed to inactive CRISPR reagents, as shown in FIG. 12. The resulting dGH probe bound (hybridized) metaphase chromosomes were imaged at 100X and the fluorescence signal patterns of individual metaphase spreads analyzed for normal and aberrant patterns.

Editing of the HEK293FT cells was performed by transfection with plasmid-expressing Cas9-RFP and guide RNA in U6-gRNA:PGK-puro-2A-tagBFP plasmid backbone, for two different sites in the p53 gene. The gene-edited cells were sorted via flow cytometry and then expanded to provide sufficient sample for analysis.

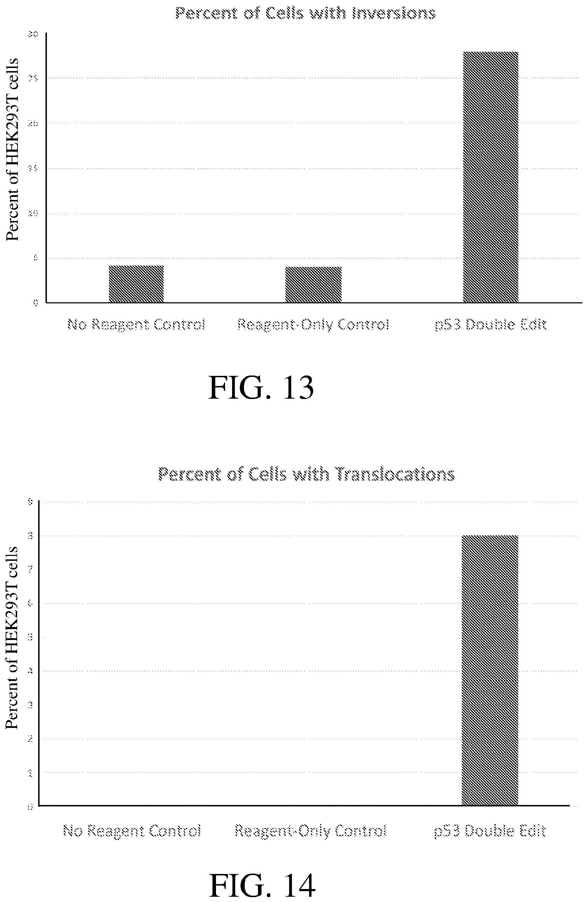

Graphs of inversions and translocations from the repair events (e.g., mis-repair of mis-aligned free DNA ends), a pair of which are present whenever there is a DNA double strand breaks such as caused by CRISPR editing, are shown in FIGs. 13 and 14. Two concurrent double strand breaks are required to form a structural rearrangement and any cell edited by CRISPR or other nuclease that cuts the DNA can have multiple double strand breaks concurrently arising at the target site(s), off-target sites at random locations in the genome.

In this experiment, there was the potential for any cell in the edited sample to have 0, 1, 2, 3, 4, 5 or 6 concurrent, on-target, double stranded DNA breaks derived from either 0, 1, or 2 CRISPR cuts at each of the three P53 genes present on homologous copies of chromosome 17. As the fourth homologous copy of chromosome 17 has no P53 gene, it presents no target sites and thus cannot contribute to this total of double strand breaks.

Off-target editing and random double strand break points can however add to the total chromosome variants and so it is expected that in extreme cases complex translocations involving more than six double strand breaks can be present concurrently in a single cell.

The concurrency of double-strand breaks has implications for both genotoxicity and stability, particularly when multiple high-prevalence, directed double strand breaks are directed at a single chromosome, as in this experiment.

The resulting chromosome variants (e.g., deletions, translocations, inversions) are summarized for the system before editing (control), in cells that had been treated with CRISPR reagents but omitting gRNA (reagents-only control), and after editing. Structural rearrangement rates to the p53 region, including translocations, inversions, and deletions of the entire region, were measured in the CRISPR-Cas9 edited cells at elevated rates above both the control and the reagent-only control. In the edited sample, 28% of the cells were observed to have inversions involving one of the three P53 loci and 8% of the cells were observed to have a translocation involving the P53 locus. Also, in the edited sample P53 was observed to be deleted from 100% of the fourth copy of chromosome 17.

This represents a 24% elevation in the inversion rate and an 8% elevation of the translocation rate over both the control and unedited HEK293FT cells. The deletion rated of P53 from the fourth copy of chromosome 17 was observed to be 100% in all samples.

This extremely high structural variant burden is directly caused by the large number of concurrent double strand breaks and the resultingly large pool of free DNA ends with repair events that can be mis-aligned and mis-repaired.

The excess number and variability of inversions over translocations measured in the test population of cells was a consequence of the spatial relationship of the edit sites co-located in the P53 gene. The free DNA ends available for mis-repair are naturally closely located and easily accessible, while a free DNA end arising from another cut to a different chromosome, may or may not be accessible for mis-repair. One consequence of this spatial relationship in this system is that an alternative editing strategy of sequential single cuts to the P53 gene would eliminate inversions arising between the edit sites while at the same time reducing the maximum number of concurrent double strand breaks on the homologous copies of chromosome 17 from 6 to 3, and thereby reducing the rate of translocations.

Two edits to a single gene on each of three homologous chromosomes yielded the same maximum number of double strand breaks as one edit to three separate genes on each of two homologous chromosomes, six in each case. And, so while the HEK293FT cells have a non-natural number of chromosomes and do not model a normal human genome, they can provide a reasonable model for certain editing situations, such as a triple knockout where three genes are modified using CRISPR editing. Regardless of the ploidy of the genome, a large numbers of concurrent double strand breaks poses a number of genotoxicity risks.

First, inversions and translocations, as structural rearrangements to the genome can have metabolic consequences to the cell itself, causing cell death or a change in cellular growth rate which could lead to an advantage and a sub-clonal population of cells with unwanted structural changes. In this experiment, the use of HEK293FT cells likely underrepresented the consequences of such a high burden of structural rearrangements as the non-normal genome gives these cells an advantage over a normal duplex genome. For instance a translocation involving two copies of the P53 gene that resulted in a double knock-out for HEK293FT cell still left a single functional copy of the P53 gene.

A second risk of high numbers of concurrent double strand breaks is genomic instability or chromothripsis. This risk increases with the addition of off-target editing or random double strand breaks and in particular if multiple double strand breaks occur on the same chromosome.

A third risk of a high numbers of concurrent double strand breaks is the specific genotoxicity risk of any specific structural variant arising from the mis-repair. When defined by the specific break points of the edit sites or known off-target sites, and regardless of if they take the form of inversions, translocations, or copy number variant, these potentially genotoxic variants can be predicted and evaluated through traditional means for risk of say, oncogenesis, and weights can be assigned to a risk model according to methods herein. However, many translocations observed in this experiment had a breakpoint located within the P53 gene at one of the integration sites of the recombinant nucleic acid inserts (i.e., edit sites) and a second breakpoint located at some location that can only be defined by the chromosome and arm. Since these arise randomly, the test population of cells, while likely to have the same rate of translocations with a single undefined breakpoint, will not have the same set of translocations with a single undefined breakpoint. The consequence of which is that even perfect measurements of the translocation identity in a test population of cells is not sufficient to define the risk arising from the actual translocations in say, a dose of drug product administered to a patient.

Mitigating the risk of undefinable structural variants requires minimizing the concurrent double strand breaks in any cell by minimizing the number of edits, by employing techniques that do not cut the double strands such as base editing, by performing multiplex editing sequentially rather than concurrently, and wherever possible minimizing any additional cutting at off-target sites.

The disclosed embodiments, examples, and experiments are not intended to limit the scope of the disclosure or to represent that the experiments herein are all or the only experiments performed. Efforts have been made to ensure accuracy with respect to numbers used (e.g., amounts, temperature, etc.) but some experimental errors and deviations should be accounted for. It should be understood that variations in the methods as described may be made without changing the fundamental aspects that the experiments are meant to illustrate.

Those skilled in the art can devise many modifications and other embodiments within the scope and spirit of the present disclosure. Indeed, variations in the materials, methods, drawings, experiments, examples, and embodiments described may be made by skilled artisans without changing the fundamental aspects of the present disclosure. Any of the disclosed embodiments can be used in combination with any other disclosed embodiment.

In some instances, some concepts have been described with reference to specific embodiments.

## Claims

1. A method of determining a genotoxicity risk for a test population of cells, the method comprising the steps of:

   a) performing a directional genomic hybridization (dGH) reaction by contacting one or more pairs of single-stranded sister chromatids on at least four cells from the test population of cells, with a plurality of sets of dGH probes, wherein the dGH probes of each set of dGH probes binds to a different target DNA sequence on a same single-stranded sister chromatid, wherein each set of dGH probes binds to a corresponding set of target DNA sequences on a different single-stranded sister chromatid, and wherein each single-stranded sister chromatid can be assigned to a chromosome number based on a color of the set of dGH probes that binds thereto and other visual features of the single-stranded sister chromatid;
   b) assessing a presence or absence of two or more types of chromosome variants and/or repair events in each of the at least four cells of the test population of cells individually based on results of the dGH reaction;
   c) measuring the number and/or variability of each of the two or more chromosomal variant and/or repair event types in each of the at least four cells of the test population of cells; and
   d) comparing the number and/or variability of each of the two or more chromosomal variant and/or repair event types in the test population of cells to a number and/or variability for a corresponding type of chromosomal variant and/or repair event of a control cell or a population of control cells, wherein a weight is assigned to the number and/or variability for each type of chromosomal variant and/or repair event to provide a series of weighted factors, wherein the weight assigned to obtain each weighted factor of the series of weighted factors depends on a characteristic of and/or a type of cells of the test population of cells to determine a weighted chromosomal stability score, wherein the weighted chromosomal stability score is used to determine the genotoxicity risk of the test population of cells, and wherein an increased weighted chromosomal stability score in the test population of cells compared to the control cell or the population of control cells is indicative of an increased genotoxicity risk for the test population of cells.

2. The method of claim 1, wherein the test population of cells comprises genetically-modified cells having a recombinant nucleic acid insert, and wherein the dGH reaction, or a parallel dGH reaction is performed using a dGH insert probe that binds to the recombinant nucleic acid insert.

3. The method of claim 2, wherein the recombinant nucleic acid insert comprises a chimeric antigen receptor sequence, a recombinant T cell receptor sequence, a transgenic sequence, a gene-edited sequence, a deleted gene sequence, an inserted gene sequence, a DNA sequence for binding guide RNA, a transcription activator-like effector binding sequence, or a zinc finger binding sequence.

4. The method of claim 2, wherein the method further comprises determining a number of recombinant nucleic acid inserts in each metaphase spread.

5. The method of claim 2, wherein the method further comprises determining a number of recombinant nucleic acid inserts at each integration site of the recombinant nucleic acid insert.

6. The method of claim 5, wherein the method further comprises determining a number of full single recombinant nucleic acid inserts, a number of fractional recombinant nucleic acid inserts, and a number of concatemer recombinant nucleic acid inserts.

7. The method according to any one of claims 1 to 6, wherein the population of test cells is a population of genetically-modified cells.

8. The method of claim 7, wherein the test population of cells is a population of genetically-modified T cells and/or genetically-modified NK cells.

9. The method according to any one of claims 1 to 6, wherein the two or more types of chromosome variant or repair events are two, three, four, five, six, seven, eight, or all of the chromosome variant or repair events selected from the group consisting of a change in genome ploidy, a change in total chromosome copy number, a gain of an individual chromosome, a loss of an individual chromosome, a deletion of a chromosome, an at least 1kb deletion of a portion of a chromosome, an at least 1 kb insertion of a portion of a chromosome, insertion of an entire chromosome, a balanced translocation, an acentric unbalanced translocation, a dicentric unbalanced translocation, a complex translocation involving three or more break points, a symmetrical translocation, an asymmetrical translocation, an inversion, an insertion, a marker chromosome, chromothrypsis, a chromatid-type break, a sister chromatid recombination, a complex chromosome event, a micronucleus, a chromosome fragment that is at least 1bkb, an extrachromosomal DNA (ecDNA) that is at least 1 kb, a multi-radial chromosome, an isochromosome, an acentric ring, a centric ring, a chromosome condensation defect, a centromere abnormality, and a sister chromatid exchange (SCE).

10. The method according to any one of claims 1 to 6, wherein a weighted chromosomal stability score is determined by adding two or more weighted factors of the series of weighted factors, by multiplying two or more weighted factors of the series of weighted factors, by using one of the weighted factors as an exponent of another weighted factor, or by a combination of the adding, the multiplying, and/or the using the exponent.

11. The method of claim 10, wherein the method further comprises using a mutation status of a target gene for the population of cells as a weighted factor in determining the weighted chromosomal stability score.

12. The method of claim 11, wherein a weighted factor value of 0 or 1 is assigned to the mutation status depending on a presence or absence of the mutation.

13. The method according to any one of claims 1 to 6, wherein the population of cells is selected from blood cells, cancer cells, primary mammalian cells, mammalian stem cells, induced pluripotent stem cells iPSCs, immortalized cells, T cells, NK cells, and neuronal cells.

14. The method according to any one of claims 1 to 6, wherein the comparing is comparing the variability of each of the two or more chromosomal variants and/or repair events.

15. The method according to any one of claims 1 to 6, wherein the two or more types of chromosome variant or repair events are two, three or all of the chromosome variant or repair events selected from the group consisting of somatic mosaicism, a deletion, a copy number variation, and a structural rearrangement, wherein the structural rearrangement involves PS1, PS2, MAPT, APP, NPC1, SNCA, chromosome 21 (21q21.3), NEGRI1, PTBP2, CADPS, KMT2, E KCNN2, MACROD2, MMP12, NTM, ANTXRL, CHST9, DNM3, NDST3, SDK1, STRC, SKY, SCN1A, SCN2A, SETD2, ARID1B, AKT1, AKT3, MTOR, PIK3CA, TSC1, TSC2, mTOR, PI3K-Akt, p53, or PTEN, or the structural rearrangement involves a cluster of genes on chromosome 17 comprising two, three, or all of the KANSLI, WNT3, MAPT, and CRHRI genes.

**Patentansprüche**

1. Verfahren zum Bestimmen eines Genotoxizitätsrisikos für eine Testpopulation von Zellen, wobei das Verfahren die folgenden Schritte umfasst:

   a)Durchführen einer gerichteten genomischen Hybridisierungsreaktion (directional Genomic Hybridization, dGH) durch Inkontaktbringen eines oder mehrerer Paare von einzelsträngigen Schwesterchromatiden an mindestens vier Zellen aus der Testpopulation von Zellen, mit einer Mehrzahl von Sätzen von dGH-Sonden, wobei die dGH-Sonden jedes Satzes von dGH-Sonden an eine unterschiedliche Ziel-DNA-Sequenz auf demselben einzelsträngigen Schwesterchromatid binden, wobei jeder Satz von dGH-Sonden an einen entsprechenden Satz von Ziel-DNA-Sequenzen auf einem anderen einzelsträngigen Schwesterchromatid bindet, und wobei jedes einzelsträngige Schwesterchromatid basierend auf einer Farbe des Satzes von dGH-Sonden, die daran bindet, und anderen visuellen Merkmalen des einzelsträngigen Schwesterchromatids einer Chromosomenzahl zugeordnet werden kann;

b)Beurteilen einer Anwesenheit oder Abwesenheit von zwei oder mehreren Arten von Chromosomvarianten und/oder Reparaturereignissen in jeder der mindestens vier Zellen der Testpopulation von Zellen, jeweils basierend auf Ergebnissen der dGH-Reaktion;

c)Messen der Anzahl und/oder Variabilität von jeder der zwei oder mehr Arten von chromosomalen Varianten und/oder Reparaturereignissen in jeder der mindestens vier Zellen der Testpopulation von Zellen; und

d)Vergleichen der Anzahl und/oder Variabilität von jeder der zwei oder mehr Arten von chromosomalen Varianten und/oder Reparaturereignissen in der Testpopulation von Zellen mit einer Anzahl und/oder Variabilität einer entsprechenden Art von chromosomalen Varianten und/oder Reparaturereignissen einer Kontrollzelle oder einer Population von Kontrollzellen, wobei der Anzahl und/oder Variabilität jeder Art von chromosomalen Varianten und/oder Reparaturereignissen eine Gewichtung zugewiesen wird, um eine Reihe von gewichteten Faktoren bereitzustellen, wobei die zugewiesene Gewichtung zum Erhalten des jeweiligen gewichteten Faktors aus der Reihe von gewichteten Faktoren von einer Eigenschaft und/oder einer Art von Zellen aus der Testpopulation von Zellen abhängt, um eine gewichtete chromosomale Stabilitätsbewertung zu bestimmen, wobei die gewichtete chromosomale Stabilitätsbewertung verwendet wird, um das Genotoxizitätsrisiko der Testpopulation von Zellen zu bestimmen, und wobei eine erhöhte gewichtete chromosomale Stabilitätsbewertung in der Testpopulation von Zellen im Vergleich zu der Kontrollzelle oder der Population von Kontrollzellen ein erhöhtes Genotoxizitätsrisiko für die Testpopulation von Zellen angibt.

2. Verfahren nach Anspruch 1, wobei die Testpopulation von Zellen genetisch modifizierte Zellen umfasst, die eine rekombinante Nukleinsäureinsertion aufweisen, und wobei die dGH-Reaktion oder eine parallele dGH-Reaktion unter Verwendung einer dGH-Insertionssonde durchgeführt wird, die an die rekombinante Nukleinsäureinsertion bindet.

3. Verfahren nach Anspruch 2, wobei die rekombinante Nukleinsäureinsertion eine chimäre Antigenrezeptorsequenz, eine rekombinante T-Zell-Rezeptorsequenz, eine transgene Sequenz, eine genveränderte Sequenz, eine deletierte Gensequenz, eine insertierte Gensequenz, eine DNA-Sequenz zur Bindung von guide-RNA, eine transkriptionsaktivatorähnliche Effektorbindungssequenz oder eine Zinkfingerbindungssequenz umfasst.

4. Verfahren nach Anspruch 2, wobei das Verfahren ferner ein Bestimmen einer Anzahl von rekombinanten Nukleinsäureinsertionen in jeder Metaphasenspreitung umfasst.

5. Verfahren nach Anspruch 2, wobei das Verfahren ferner ein Bestimmen einer Anzahl von rekombinanten Nukleinsäureinsertionen an jeder Integrationsstelle der rekombinanten Nukleinsäureinsertion umfasst.

6. Verfahren nach Anspruch 5, wobei das Verfahren ferner ein Bestimmen einer Anzahl von vollständigen einzelnen rekombinanten Nukleinsäureinsertionen, einer Anzahl von fraktionierten rekombinanten Nukleinsäureinsertionen und einer Anzahl von konkatemeren rekombinanten Nukleinsäureinsertionen umfasst.

7. Verfahren nach einem der Ansprüche 1 bis 6, wobei die Population von Testzellen eine Population von genetisch modifizierten Zellen ist.

8. Verfahren nach Anspruch 7, wobei die Testpopulation von Zellen eine Population von genetisch modifizierten T-Zellen und/oder genetisch modifizierten NK-Zellen ist.

9. Verfahren nach einem der Ansprüche 1 bis 6, wobei die zwei oder mehr Arten von chromosomalen Varianten oder Reparaturereignissen zwei, drei, vier, fünf, sechs, sieben, acht oder alle chromosomalen Varianten oder Reparaturereignisse sind, die aus der Gruppe ausgewählt sind, die aus einer Veränderung der Genom-Ploidie, einer Änderung in der Gesamtchromosomkopienanzahl, einem Zuwachs eines individuellen Chromosoms, einem Verlust eines individuellen Chromosoms, einer Deletion eines Chromosoms, einer Deletion von mindestens 1 kb eines Abschnitts eines Chromosoms, einer Insertion von mindestens 1 kb eines Abschnitts eines Chromosoms, einer Insertion eines gesamten Chromosoms, einer ausgeglichenen Translokation, einer azentrischen unausgeglichenen Translokation, einer dizentrischen unausgeglichenen Translokation, einer komplexen Translokation, die drei oder mehr Unterbrechungsstellen beinhaltet, einer symmetrischen Translokation, einer asymmetrischen Translokation, einer Inversion, einer Insertion, einem Markerchromosom, einer Chromothripsis, einer chromatidartigen Unterbrechung, einer Schwesterchromatid-Rekombination, einem komplexen Chromosomenereignis, einem Mikrokern, einem Chromosomfragment, das mindestens 1 kb beträgt, einer extrachromosomalen DNA (ecDNA), die mindestens 1 kb beträgt, einem multiradialen Chromosom, einem Isochromosom, einem azentrischen Ring, einem zentrischen Ring, einem Chromosomenkondensationsfehler, einer Zentromer-Abnormalität, und einem Schwesterchromatidaustausch (SCE) besteht.

10. Verfahren nach einem der Ansprüche 1 bis 6, wobei eine gewichtete chromosomale Stabilitätsbewertung durch ein Addieren von zwei oder mehr gewichteten Faktoren aus der Reihe von gewichteten Faktoren, durch ein Multiplizieren von zwei oder mehr gewichteten Faktoren aus der Reihe von gewichteten Faktoren, durch ein Verwenden eines der gewichteten Faktoren als Exponent eines anderen gewichteten Faktors oder durch eine Kombination aus dem Addieren, dem Multiplizieren und/oder dem Verwenden als Exponenten bestimmt wird.

11. Verfahren nach Anspruch 10, wobei das Verfahren ferner ein Verwenden eines Mutationsstatus eines Zielgens für die Population von Zellen als gewichteten Faktor bei dem Bestimmen der gewichteten chromosomalen Stabilitätsbewertung umfasst.

12. Verfahren nach Anspruch 11, wobei dem Mutationsstatus in Abhängigkeit von einer Anwesenheit oder Abwesenheit der Mutation ein Wert als gewichteter Faktor von 0 oder 1 zugewiesen wird.

13. Verfahren nach einem der Ansprüche 1 bis 6, wobei die Population von Zellen ausgewählt ist aus Blutzellen, Krebszellen, primären Säugetierzellen, Säugetierstammzellen, induzierten pluripotenten Stammzellen (iPSCs), immortalisierten Zellen, T-Zellen, NK-Zellen und neuronalen Zellen.

14. Verfahren nach einem der Ansprüche 1 bis 6, wobei das Vergleichen ein Vergleichen der Variabilität von jeder/jedem der zwei oder mehr chromosomalen Varianten und/oder Reparaturereignisse ist.

15. Verfahren nach einem der Ansprüche 1 bis 6, wobei die zwei oder mehr Arten von chromosomalen Varianten oder Reparaturereignissen zwei, drei oder alle der chromosomalen Varianten oder Reparaturereignisse sind, die aus der Gruppe ausgewählt sind, die aus somatischem Mosaizismus, einer Deletion, eine Kopienanzahlvariation und einer strukturellen Umordnung besteht, wobei die strukturelle Umordnung PS1, PS2, MAPT, APP, NPC1, SNCA, Chromosom 21 (21q21.3), NEGRI1, PTBP2, CADPS, KMT2, E KCNN2, MACROD2, MMP12, NTM, ANTXRL, CHST9, DNM3, NDST3, SDK1, STRC, SKY, SCN1A, SCN2A, SETD2, ARID1B, AKT1, AKT3, MTOR, PIK3CA, TSC1, TSC2, mTOR, PI3K-Akt, p53 oder PTEN betrifft oder die strukturelle Umordnung einen Cluster von Genen auf Chromosom 17 betrifft, der zwei, drei oder alle aus den Genen KANSLI, WNT3, MAPT und CRHRI umfasst.

**Revendications**

1. Procédé de détermination d'un risque de génotoxicité pour une population de cellules à l'essai, le procédé comprenant les étapes consistant à :

   a) réaliser une réaction d'hybridation génomique directionnelle (dGH) en mettant en contact d'une ou de plusieurs paires de chromatides sœurs simple brin sur au moins quatre cellules de la population de cellules à l'essai, avec une pluralité d'ensembles de sondes dGH, dans lequel les sondes dGH de chaque ensemble de sondes dGH se lient à une séquence d'ADN cible différente sur une même chromatide sœur simple brin, dans lequel chaque ensemble de sondes dGH se lie à un ensemble correspondant de séquences d'ADN cibles sur une chromatide sœur simple brin différente, et dans lequel chaque chromatide sœur simple brin peut être attribuée à un numéro de chromosome sur la base d'une couleur de l'ensemble de sondes dGH qui se lie à celle-ci et d'autres caractéristiques visuelles de la chromatide sœur simple brin ;
   b) évaluer une présence ou une absence d'au moins deux types de d'événements de variant chromosomique et/ou de réparation dans chacune des au moins quatre cellules de la population de cellules à l'essai individuellement sur la base de résultats de la réaction dGH ;
   c) mesurer le nombre et/ou la variabilité de chacun des au moins deux types d'événements de variant chromosomique et/ou de réparation dans chacune des au moins quatre cellules de la population de cellules à l'essai ; et

d) comparer le nombre et/ou la variabilité de chacun des au moins deux types d'événements de variant chromosomique et/ou de réparation dans la population de cellules à l'essai à un nombre et/ou une variabilité pour un type correspondant d'événement de variant chromosomique et/ou de réparation d'une cellule témoin ou d'une population de cellules témoins, dans lequel un poids est attribué au nombre et/ou à la variabilité pour chaque type d'événement de variant chromosomique et/ou de réparation pour fournir une série de facteurs pondérés, dans lequel le poids attribué pour obtenir chaque facteur pondéré de la série de facteurs pondérés dépend d'une caractéristique et/ou d'un type de cellules de la population de cellules à l'essai pour déterminer un score de stabilité chromosomique pondéré, dans lequel le score de stabilité chromosomique pondéré est utilisé pour déterminer le risque de génotoxicité de la population de cellules à l'essai, et dans lequel un score de stabilité chromosomique pondéré accru dans la population de cellules à l'essai comparativement à la cellule témoin ou à la population de cellules témoins indique un risque de génotoxicité accru pour la population de cellules à l'essai.

2. Procédé selon la revendication 1, dans lequel la population de cellules à l'essai comprend des cellules génétiquement modifiées ayant un insert d'acide nucléique recombinant, et dans lequel la réaction dGH, ou une réaction dGH parallèle est réalisée à l'aide d'une sonde d'insert dGH qui se lie à l'insert d'acide nucléique recombinant.

3. Procédé selon la revendication 2, dans lequel l'insert d'acide nucléique recombinant comprend une séquence de récepteur d'antigène chimérique, une séquence de récepteur de cellule T recombinante, une séquence transgénique, une séquence modifiée par un gène, une séquence de gène délété, une séquence de gène inséré, une séquence d'ADN pour lier un ARN de guidage, une séquence de liaison d'effecteur de type activateur de transcription, ou une séquence de liaison en doigt de zinc.

4. Procédé selon la revendication 2, dans lequel le procédé comprend en outre la détermination d'un nombre d'inserts d'acide nucléique recombinant dans chaque diffusion en métaphase.

5. Procédé selon la revendication 2, dans lequel le procédé comprend en outre la détermination d'un nombre d'inserts d'acide nucléique recombinant au niveau de chaque site d'intégration de l'insert d'acide nucléique recombinant.

6. Procédé selon la revendication 5, dans lequel le procédé comprend en outre la détermination d'un nombre d'inserts d'acide nucléique recombinant uniques complets, d'un nombre d'inserts d'acide nucléique recombinant fractionnaires, et d'un nombre d'inserts d'acide nucléique recombinant concatémères.

7. Procédé selon l'une quelconque des revendications 1 à 6, dans lequel la population de cellules à l'essai est une population de cellules génétiquement modifiées.

8. Procédé selon la revendication 7, dans lequel la population de cellules à l'essai est une population de cellules T génétiquement modifiées et/ou de cellules NK génétiquement modifiées.

9. Procédé selon l'une quelconque des revendications 1 à 6, dans lequel les au moins deux types d'événements de variant chromosomique ou de réparation sont deux, trois, quatre, cinq, six, sept, huit, ou l'ensemble des événements de variant chromosomique ou de réparation sélectionnés dans le groupe constitué par un changement de la ploïdie génomique, un changement du nombre total de copies chromosomiques, un gain d'un chromosome individuel, une perte d'un chromosome individuel, une délétion d'un chromosome, une délétion d'au moins 1 kb d'une partie d'un chromosome, une insertion d'au moins 1 kb d'une partie d'un chromosome, l'insertion d'un chromosome entier, une translocation équilibrée, une translocation déséquilibrée acentrique, une translocation déséquilibrée dicentrique, une translocation complexe impliquant au moins trois points de rupture, une translocation symétrique, une translocation asymétrique, une inversion, une insertion, un chromosome marqueur, la chromothripsie, une rupture de type chromatidique, une recombinaison de chromatides sœurs, un événement chromosomique complexe, un micronoyau, un fragment chromosomique qui est d'au moins 1 bkb, un ADN extrachromosomique (ADNec) qui est d'au moins 1 kb, un chromosome multiradial, un isochromosome, un anneau acentrique, un anneau centrique, un défaut de condensation chromosomique, une anomalie de centromère, et un échange de chromatides sœurs (SCE).

10. Procédé selon l'une quelconque des revendications 1 à 6, dans lequel un score de stabilité chromosomique pondéré est déterminé par addition d'au moins deux facteurs pondérés de la série de facteurs pondérés, multiplication d'au moins deux facteurs pondérés de la série de facteurs pondérés, utilisation de l'un des facteurs pondérés comme exposant d'un autre facteur pondéré, ou par une combinaison de l'addition, de la multiplication, et/ou de l'utilisation de l'exposant.

**11.** Procédé selon la revendication 10, dans lequel le procédé comprend en outre l'utilisation d'un statut de mutation d'un gène cible pour la population de cellules en tant que facteur pondéré dans la détermination du score de stabilité chromosomique pondéré.

**12.** Procédé selon la revendication 11, dans lequel une valeur de facteur pondérée de 0 ou 1 est attribuée au statut de mutation en fonction d'une présence ou d'une absence de la mutation.

**13.** Procédé selon l'une quelconque des revendications 1 à 6, dans lequel la population de cellules est choisie parmi des cellules sanguines, des cellules cancéreuses, des cellules primaires de mammifère, des cellules souches de mammifère, des cellules souches pluripotentes induites iPSC, des cellules immortalisées, des cellules T, des cellules NK, et des cellules neuronales.

**14.** Procédé selon l'une quelconque des revendications 1 à 6, dans lequel la comparaison consiste à comparer la variabilité de chacun des au moins deux événements de variant chromosomiques et/ou de réparation.

**15.** Procédé selon l'une quelconque des revendications 1 à 6, dans lequel les au moins deux types d'événements de variant chromosomique ou de réparation sont deux, trois ou l'ensemble des événements de variant chromosomique ou de réparation sélectionnés dans le groupe constitué par le mosaïcisme somatique, une délétion, une variation du nombre de copies, et un réarrangement structurel, dans lequel le réarrangement structurel implique PS1, PS2, MAPT, APP, NPC1, SNCA, le chromosome 21 (21q21.3), NEGRI1, PTBP2, CADPS, KMT2, E-KCNN2, MACROD2, MMP12, NTM, ANTXRL, CHST9, DNM3, NDST3, SDK1 STRC, SKY, SCN1A, SCN2A, SETD2 ARID1B, AKT1, AKT3, MTOR, PIK3CA, TSC1, TSC2, mTOR, PI3K-Akt, p53, ou PTEN, ou le réarrangement structurel implique un groupe de gènes sur le chromosome 17 comprenant deux, trois, ou l'ensemble des gènes KANSLI, WNT3, MAPT, et CRHRI.

FIG. 1

FIG. 2B

FIG. 2A

FIG. 3A

FIG. 3B

FIG. 4

FIG. 5A

FIG. 5B

FIG. 5C

FIG. 5D

FIG. 5E

FIG. 5F

FIG. 6A

FIG. 6B

FIG. 7

FIG. 8

FIG. 9

FIG. 10A

FIG. 10B

FIG. 11

A) Normal karyotype for HEK293T is tetraploid for Ch17, with one copy missing the p53 gene on Ch17p.

B) Two gRNA target sites located within p53 gene.

# FIG. 12A

# FIG. 12B

FIG. 13

FIG. 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100304994

**Non-patent literature cited in the description**

- **LUXTON JARED J. et al.** Telomere Length Dynamics and Chromosomal Instability for Predicting Individual Radiosensitivity and Risk via Machine Learning. *JOURNAL OF PERSONALIZED MEDICINE*, 08 March 2021, vol. 11 (3), 188
- **RAY F. ANDREW et al.** Directional genomic hybridization for chromosomal inversion discovery and detection. *CHROMOSOME RESEARCH*, 10 April 2013, vol. 21 (2), 165-174
- **RAY et al.** *Chrom*, 2013, vol. 21, 165-174
- **S. M. BAILEY et al.** Strand-Specific Fluorescence in situ Hybridization: The CO-FISH Family. *Cytogenet. Genome Res.*, 2004, vol. 107, 11-14
- **ASANO et al.** *Current Protocols in Cell Biology*, 2018, vol. 80, e56
- **TUCK-MULLER.** *Cell Genetics*, 2000
- **PORUBSKY D et al.** *Cell*, 26 May 2022, vol. 185 (11), 1986-2005